# EUROPEAN PATENT APPLICATION

(11) **EP 2 299 274 A1**
(43) Date of publication of application: **23.03.2011**
(21) Application number: 10012418.9
(22) Date of filing: 07.03.2003
(51) Int. Cl.: G01N 33/68, G01N 33/574

(54) **SCD fingerprints**

(30) Priority: 07.03.2002 GB 0205394; 03.04.2002 GB 0207746; 03.12.2002 GB 0228195
(62) Divisional of application: 03715080.2
(71) Applicant: Medical Research Council, 20 Park Crescent London W1B 1AL (GB); Cambridge Enterprise Ltd., Cambridge Cambridgeshire CB2 1TN (GB)
(72) Inventor: Woolfson, Adrian, Newnham Cambridge CB3 9HZ (GB); Hales, C. N., Cambridge CB2 2QR (GB); Milstein, Cesar, deceased (GB)
(74) Representative: Richards, William John

(57) **Abstract**

The present invention relates to the use of cluster of differentiation (CD) molecules in detecting the presence and progression of one or more disease states in an individual. In particular it relates to the use of profiles of shed CD (sCD) molecules in detecting and assessing the progression of one or more disease states in an individual. Further uses of sCD profiles according to the present invention are also described.

## Description

The present invention relates to the use of cluster of differentiation (CD) molecules in detecting the presence and/or assessing the progression and/or assessing the response to therapeutic intervention of one or more disease states in an individual. In particular it relates to the use of profiles/fingerprint/s of shed CD (sCD) molecules in body fluids in detecting and/or assessing the progression of one or more disease states in an individual. Further uses of sCD profiles according to the present invention are also described.

### Background to the invention

Rapid and accurate diagnosis is essential in medicine as in many cases early diagnosis and successful treatment correlates with a better outcome and reduced hospitalisation. Currently, the clinical diagnosis and staging of many diseases of global significance involve different invasive procedures such as histopathological analysis of biopsy samples which are usually obtained when the disease process is at a relatively advanced stage. In many cases, a classic histopathological approach may not be sufficient to produce accurate diagnosis and any delay in confirming the diagnosis would have financial and morbidity repercussions for the healthcare institution and most importantly for the individual. Disease states and disease staging are also determined by different imaging techniques such as X-rays, nuclear magnetic resonance (NMR), CT analysis and others, however, these are expensive and impractical when dealing with large numbers of individuals, or when it is necessary to monitor disease progression closely, or in health institutes or clinical situations where such equipment is unavailable. Furthermore such investigations are impractical for individuals because it would result in such individuals obtaining high radiation doses. For this reason such tests cannot be carried out serially and are thus of little use in monitoring drug responses and monitoring disease progression.

A variety of diseases or the predisposition to such a disease can be characterised by changes in the overall patterns and/or expression levels of various genes and their proteins. For example, some cancers are associated with changes in the expression of oncogenes or tumour suppressor genes. Furthermore, disease conditions or disorders associated with disregulated cell cycle and development can be attributed to changes in transcriptional regulation of specific genes.

Although there are several genetic assays available to assess gene mutations, the identification of specific genetic changes may not always be a direct indicator of a disease or disorder and thus cannot be relied upon as an accurate prognostic indicator.

Certain genetic changes are exhibited by alterations in cell surface antigens. Again, however, prior attempts to develop a diagnostic assay for complex disease conditions or disorders based on the identification of single antigen or very small numbers of antigens have not been uniformly successful.

In addition, or alternatively, biochemical analysis of a patient may be used to diagnose a disease state. For example, the presence of Bence Jones proteins in urine is an indicator that an individual has multiple myeloma. However, classical biochemical methods are limited, for example an elevated cholesterol in serum indicates hypercholesterolaemia but does not definitively indicate atherosclerosis. A further disadvantage of biochemical methods of diagnosis is that they generally permit the measurement of only one or two indicator/s of disease in any one test. Consequently, they provide an incomplete picture of the disease state of an individual. Moreover, if several tests are performed in an attempt to provide a more complete picture, this inevitably increases the number of variables which complicates interpretation. Furthermore, for many diseases there are no reliable biochemical markers, especially for diseases of global importance such as breast cancer, colorectal cancer and lung cancer. In the case of solid tumours such as colorectal cancer, a number of carcinoembryonic antigen (CEA) markers have been identified, however they have poor sensitivity and very low specificity. The situation is similar with disease conditions requiring surgical intervention. There is still, for example, no marker for acute appendicitis and consequently, a great many patients undergo unnecessary invasive surgery. It has been estimated that more than 40,000 unnecessary appendicitis operations occur each year due to misdiagnosis with associated costs of $700 million. In a recent larger retrospective study, Flume and colleagues show that misdiagnosis occurs in 15% of instances.

Therefore, there is a pressing need in the art to provide a simple and complete picture of the disease state or condition of an individual. Such a 'picture' would be of use in predicting and/or detecting the presence of a disease or condition, in assessing the therapeutic strategies and the potential of various agents and in monitoring the progression and successful treatment of disease states or conditions

Lymphocytes and other leukocytes express a large number of different antigens associated with their outer plasma membranes that can be used to identify distinct functional cell subsets. Many of these antigens were "classically" known to be receptors for growth factors, cell-cell interactions, viruses eg CD4, CD 112 and CD 155 are the HIV, poliovirus receptor 2 and poliovirus receptor respectively), and immunoglobulins; molecules for cell adhesion or complement stimulation; enzymes and ion channels. A single systematic nomenclature has been adopted to classify monoclonal antibodies to human leukocyte cell surface antigens termed cluster of differentiation (CD) antigens, also referred to as CD molecules/antigens (Kishimoto et al., 1996 Proceedings of the Sixth International Workshop and Conference held in Kobe, Japan. 10-14 Garland Publishing Inc, NY, USA). This work originated as the direct result of the work of one of the inventors (Dr. César Milstein) of the present application who invented monoclonal antibody technology with his colleague Georges Kohler (Kohler and Milstein. Continuous cultures of fused cells secreting antibody of defined specificity (1975), Nature Aug 7, 256 (5517), 495-7) and who identified and raised the first monoclonal antibodies to both non-human and human CD antigens (McMichael et al. A human thymocyte antigen defined by a hydrid myeloma monoclonal antibody).

The data required in order to define a CD has changed over the years, not surprisingly in view of the advances in modern technology. Initially, clustering depended absolutely on the statistical revelation of similarities in reaction pattern of two or more antibodies, analysed on multiple tissues. It is now accepted that CD molecules may also be classified by molecular characteristics. Thus it has become customary to use the CD marker (for example CD21) to indicate the molecule recognised by each group of monoclonal antibodies. The current list of CD markers is constantly updated as new antigens are identified and eventually, the CD list will encompass all human lymphocyte cell surface antigens and their homologues in other mammalian and non-mammalian species (Mason et al., 2001, Immunology, 103, 401-406). It should be noted that although CD antigens were initially defined in the basis that they are expressed on the cell surface of leukocytes, a great many of them are also expressed on numerous other cell types including brain, liver, kidney, red blood cells, bone marrow, dendritic antigen presenting cells, epithelial cells, stem cells, thymocytes, osteoclasts, NK cells, B cells, macrophages, to name but a few.

Historically, CD cell surface antigens have been used as markers in diagnosis. Indeed leukemias are diagnosed on the basis of cell morphology, expression of specific CD antigens, lymphoid (LY) and myeloid (MY) antigens, enzyme activities and cytogenetic abnormalities such as chromosome translocations. The expression of up to three CD antigens on leukemia cells is determined using labelled antibodies to particular CD antigens with analysis by flow cytometry.

Significantly, however, it has been observed that often (if not always in normal or disease states) the surface bound CD immunological specificity molecules (intact CD molecules or fragments thereof) are found soluble in the serum and in other body fluids. Subsequent research has shown that indeed CD molecules can be secreted from cells as a result of "active" processes such as alternative splicing (Woolfson and Milstein, PNAS, 91 (14) 6683-6687 (1994)) or "passive" processes such as cell surface shedding. Thus, CD molecules can be found in three forms, membrane associated CD molecules, shed CD molecules (sCD) produced by alternative splicing or other mechanisms and intracellular CD molecules. Each of these can be complete molecules or fragments thereof.

It is generally accepted however that the change in levels of any one sCD is not specific to a given disease state and cannot therefore usefully be used in the diagnosis of disease states.

Recent studies (those of WO 00/39580) have described a system for the diagnosis of haematological maligancies, whereby immunoglobulins are immobilised on a solid support and are used to detect cell-surface antigen levels, in particular cell-surface CD antigen levels in samples of cells. Using this approach, a pattern of expression of cell-surface bound CD antigens is generated which the inventors have shown to be indicative of the presence of various defined leukemias in a patient. However, there are several disadvantages with this technique. Firstly and importantly, it is a cell-based technique. Such techniques have many disadvantages associated with them, for example that of background noise and the difficulty of measuring antigen levels accurately. Such methods only allow semiquantitative determination of the relative densities of sub-populations of cells of distinct immunophemotypes, indeed absolute quantification using this method may not be possible. Another problem with this prio art method is that at equilibrium, the number of cells captured by the immobilised antibody dot depends not only on the affinities of the interactions, the concentration of the antibody dot, the level of expression of the CD antigen on the cell surface and in addition to this the stereochemical availability and accessibility of the monoclonal antibody immobilised on the nitrocellulose membrane of the CD antibody array. Furthermore computerised quantification of the cell density as indicated by the pixel intensity corresponding to each dot of arrayed antibody, depends not only on the number of cells in the test sample, but in addition to the cell size and morphology. In addition to all of these factors, the absolute requirement for purification of cells from whole blood and the possible need to fractionate blood cells still further makes such an approach both labour intensive and time consuming.

Therefore, there still exists a need in the art for a simple method for diagnosis of different diseases and conditions by the measurement of CD antigens wherein such method produces a complete, sensitive, specific and accurate picture of disease.

### Summary of the invention

The present inventors have surprisingly found that particular disease states can be characterised by specific patterns of levels of shed/soluble/secreted (sCD) (as herein defined) CD molecules derived from the body fluids of an individual. That is, the profile or 'sCD print' of the levels of sCD antigens correlate with particular diseases or disorders or physiological states such as those induced by administration of a drug or toxin. This finding is especially surprising since the levels of sCDs found in the body fluids of an individual are generally very low, and the sCD released by cells would only be expected to change in some, and not all cell types of an individual when affected by one or more diseases, the change of levels of shed CD levels, as herein defined detectable in the body fluids of diseased individuals as compared with non-diseased individuals would be expected to be minimal.

Thus, in a first aspect, the present invention provides a shed CD (sCD) fingerprint (sCD print) of one or more disease states.

In the context of the present invention, the term 'CD' refers to a different cell surface leukocyte molecule recognised by a given monoclonal or group of monoclonal antibodies which specifically 'cluster' to the antigen/molecule in question. Many, if not all of these molecules produce forms which are released from the cell surface by alternative splicing, proteolytic cleavage, dissociation or other mechanisms. Thus in the context of the present invention, the term 'shed CD molecule (sCD)' is synonymous with the term secreted/soluble CD (sCD) and refers to a released form of a cell surface leukocyte molecule in which at least a portion of that molecule is recognised by a given monoclonal or group of monoclonal antibodies as herein described. It should be noted however, that the antibody used to recognise the CD molecule may not be monoclonal. It may be engineered, an artificial construct consisting of an expressed fragment derived from an antibody molecule with intact recognition, or it may be a non-protein molecular recognition agent, or a protein recognition agent which is not an antibody, or is an antibody hydrid, for example made by introducing antibody binding sites into a different scaffolding. Advantageously, as herein defined a shed form of sCD is generated by various mechanisms including but not limited to any of those selected from the group consisting of the following: alternative splicing, proteolytic cleavage and dissociation.

In the context of the present invention it is important to note that the CD nomenclature is a simple method for representing a whole range of molecules. For example: CD14 is the lipopolysaccharide receptor, LP5-R; CD21 is the EBV receptor; CD 25 is the IL-2Ralpha receptor; CD 31 is PECAM-1; CD 44 is H-CAM; CD 50 is ICAM-3; CD 54 ICAM-1; CD 62E is LECAM-2; CD 62L is LECAM-1; CD 86 is B 70; CD 95 is FAS apoptosis antigen; CD 102 is ICAM-2; CD 106 is VCAM-1; CD 116 is GM-CSFR alpha; CD 117 is c-kit stem cell factor receptor; CD 124 is IL-4R alpha; CD 126 is IL-6Ralpha; CD 130 is gp 130; CD 138 is syndican-1; CD 141 is thrombomodulin; CD 91 is low density lipoprotein receptor-related antigen; CD 132 is common cytokine receptor gamma), CD 89 is IgA Fc receptor, CD 74 is class II specific chaperone, CD 95 is apoptosis antigen; CD220 is the insulin receptor and CD 184 is the chemokine receptor 4. (CXCR4) CD8 is Lin 2; CD 27 is low affinity IgE-R; CD 30 is Ki-1.

The present inventors realised that sCDs act as representives/ambassadors for the families of molecules from which they are shed. Thus sCD 184 stands as an ambassador for all shed (as defined herein) cell surface chemokine receptors and for example sCD54 acts as an ambassador for all intercellular adhesion molecules. Furthermore they realised that cell behaviour can be interregated on the basis of the patterns of sCD molecules shed by cells.

In this regard it should be noted, as mentioned above that sCDs are ambassadors for a vast range of molecules including but not limited to the following: integrins, adhesion molecules, Fc receptors, apoptosis antigens, blood group antigens, viral receptors, coagulation factors, selectins, chemokine receptors, macrophage receptors, insulin receptors, prion proteins, glycophorins, rhesus antigens, T cell receptor zeta chain and pregnancy specific antigens.

As herein defined, the term 'shed CD fingerprint (sCD)' describes the pattern or profile of levels of more than one shed CDs in one or more individuals. A sCD fingerprint as herein defined may be representative of one or more non-diseased individual/s or one or more diseased individual/s. Preferably, a shed CD fingerprint describes the level of five or more shed CD molecules, more preferably it is 6, 7, 8, 9, 10 or more sCD molecules, more preferably still a shed CD fingerprint describes the levels of 15 or more sCD molecules. More preferably a shed CD fingerprint describes the levels of 20 or more sCD molecules. Most preferably, it comprises the levels of sCDs for the complete set of sCDs for a given individual.

sCD levels from normal and/or diseased individuals may be collated in order to generate one or more reference sCD fingerprints. A 'reference' sCD fingerprint (sCD print) is a fingerprint which is advantageously generated from sCD measurements from more than one individual and is representative of the sCD levels of either a 'normal' or diseased individual. Advantageously, these reference fingerprints are collected together to form a database so that abnormal fingerprints generated from patient samples can be distinguished from normal reference fingerprints in the database. In addition, by comparing one or more patient sample fingerprints with one or more reference fingerprint/s corresponding to one or more diseases, then the disease state of an individual may be established.

A shed CD (sCD) fingerprint (sCDprint) may be generated from one individual. Preferably however, each fingerprint is generated from more than one individual. Advantageously, it is generated from more than five, ten, fifteen or twenty, 50, 100, 500, 1000, 5000, 10,000, 50,000 or 100,000 individuals. One skilled in the art will appreciate that the greater the number of individuals used to generate the reference fingerprint, then the more representative of any given disease state or of a normal individual the reference sCD profile/s will be. Fingerprints may be simplified by using the average values of the data obtained for each sCD for a number of individuals. For example, the modal value is used for data obtained from a very small number of samples. Such information may then be placed within a database as herein described. One skilled will appreciate that often more than one disease state may be present in an individual at a given time. This may complicate the CD fingerprint obtained, such that the fingerprint is an aggregate fingerprint of several disease states. The effect of multiple disease states (composites) in an individual may be minimised if the reference fingerprint for any given disease state is generated from several or many individuals. Importantly, composites may generate their own patterns and be used as reference in their own right.

Measuring the levels of sCD molecules is carried out using methods known to one skilled in the art and described herein.

sCD levels are measured in samples of body fluids. Suitable body fluids for measuring sCDs include any one or more selected from the group consisting of the following: tissue fluid, serum, blood, cerebrospinal fluid, synovial fluid, urine, plural fluid, saliva, lymphatic fluid, aspirate, bone marrow aspirate and mucus. One skilled in the art will appreciate that this list is not intended to be exhaustive.

In a further aspect the present invention provides a method of generating a shed CD (sCD) fingerprint of one or more disease state/s comprising the step of measuring in parallel the levels of more than one shed CD in one or more samples from one or more individuals and collating the data.

According to the above aspect of the invention, the term 'measuring in paralell' (sCD levels) refers to the process where sCD levels are measured in one or more samples taken from an individual at substantially the same time. Those skilled will appreciate that in the case where sCD levels are measured from more than one body fluid sample, then it may not be practical to take more than one sample of body fluid from the same individual at precisely the same time. Thus according to the above aspect of the invention, when more than one body fluid sample is to be taken from an individual for the generation of a sCDprint, then such samples should be taken from the same individual as close together in time as possible. Advantageously, the term 'close together' (in time) means within 5 hrs of one another, more advantageously within 4 hrs, 3hrs, 2 hrs, 1 hr, 30 mins, 20 mins, 10 mins, 5 mins, 1 min of one another. Those skilled will appreciate that so long as there is no change in levels of measured sCDs between the first sample and the last sample being taken, then such a time interval can be considered 'close together' as described herein. Thus so long as there is no change in sCD levels between the first sample and the last sample being taken, then such samples can be considered to be taken 'in paralell' and likewise such sCD levels can be considered to be measured 'in paralell' as referred to herein.

According to the above aspect of the invention, the samples for testing are used to generate a given sCD profile/fingerprint/pattern/barcode (sCDprint) and may be from one body fluid type or more than one body fluid type. Advantageously the one or more samples for testing and used to generate a sCD profile are taken from more than one body fluid for any given disease state. Advantageously, a number of sCD levels are measured from the same body fluid sample. More advantageously, all of the sCD levels comprising a fingerprint are measured in paralell from one body fluid sample.

As used herein, the term 'collating' the data means to put the data into a form so that one or more pattern/s of the levels of sCDs within that disease state is apparent. Advantageously, the data will be entered into a database as described herein. More advantageously, the database is an integrated database as described herein, comprising clinical data linked to specific sCD fingerprint patterns/profiles.

As the present inventors surprisingly found that a sCD fingerprint/profile (sCDprint) of a diseased individual is different from that of a non-diseased individual, it was realised that by comparing the sCD fingerprint/profile (sCDprint) of a sample from a diseased patient with that of one or more reference sCD fingerprints representing one or more defined disease states then the presence and nature of a disease in that individual could be ascertained.

Thus, in a further aspect, the present invention provides a method for predicting the presence of one or more disease states in an individual comprising the step of comparing one or more sCD fingerprint/s (sCDprint/s) generated from that individual with one or more reference sCD fingerprint/s.

In the context of the present invention, the term 'predicting the presence of one or more disease states' refers to the process of detecting the presence of one or more disease states before the onset of the clinical signs of the disease are apparent in the individual. The clinical signs of disease are characteristic of each disease state or group of disease states, as long as the disease is present in that individual.

As referred to herein, the comparing step may refer to comparing an individuals sCD fingerprint/profile (sCDprint) with one or more reference sCD fmgerprint/s of one or more disease state/s and/or with a reference sCD fingerprint of a non-diseased 'normal' individual.

In a further aspect still, the present invention provides a method for detecting the presence of one or more disease states in an individual comprising the step of comparing one or more sCD fingerprint/s generated from that individual with one or more reference sCD fingerprints.

The term 'detecting the presence of one or more disease states' refers to the detection of the presence of one or more disease states in an individual once the clinical signs of one or more disease states are apparent in that individual. In addition, the term refers to the process of detecting the presence of one or more disease states in an individual other than the disease state whose clinical signs are apparent in that individual.

According to the above two aspects of the invention, the reference sCD fingerprint/s may be from non-diseased (normal) individuals and/or from diseased individuals. Alternatively, or in addition the reference sCD fingerprints/profiles may be derived from normal subjects who have undergone some form of intervention. Such interventions include but are not limited to treatment with chemotherapeutic or other agents, exposure to radiation and exposure to pathogens. Those skilled in the art will be aware of other interventions as used herein. According to the above two aspects of the invention, preferably, the sCD fingerprint/s/profiles (sCDprint) are from diseased individuals.

In a further aspect still, the present invention provides a method for detecting the extent of one or more disease states in an individual comprising the step of comparing one or more sCD fingerprint/s generated from that individual with one or more reference sCD fingerprint/s.

As referred to above, the term 'detecting the extent of one or more disease states' includes within its scope detecting the severity of one or more disease states within an individual. For example it may allow low grade and high grade forms of the disease to be distinguished. It allows localised and metastasised forms of a particular disease to be distinguished. In such cases one or more sCD fingerprints of an individual are compared with one or more reference disease sCD fingerprints representative of disease states at one or more degrees of severity. For example, in the case of neoplastic disease the presence or absence of metastasis may be detected using the method of the present invention.

In a further aspect, the present invention provides a method for assessing the progression of a disease state in an individual comprising the step of comparing the sCD fingerprint of an individual at two or more periods during the occurrence of the disease.

In the context of the present invention, the term 'assessing the progression of a disease state' means assessing whether the disease has increased in severity, decreased in severity or remains the same severity compared with a different period during the life-span of the disease. In addition the term 'assessing the progression of a disease state' includes within its scope monitoring the progression of a disease state.

The term 'period' in the context of the present invention, generally refers to a time period.

As defined herein, the term a 'disease state' refers to any impairment of the normal physiological functions affecting an organism or any disease condition, disorder or the presence of a particular microbial, viral, parasitic or other pathogenic agent known to one skilled in the art. Suitable disease states for analysis as described herein include but are not limited to: infectious, neoplastic, autoimmune, immunological, metabolic, degenerative, psychological, psychiatric, iatrogenic, inflammatory, drug or toxin related, vascular, traumatic and endocrine diseases. Advantageously, 'a disease state' as herein defined refers to any one or more disease selected from the group which includes but is not limited to: infections such as bacterial, fungal, protozoan, parasitic, prion and viral infections, non-neoplastic disorders; stroke; heart condition; atherosclerosis; pain; diabetes, obesity; anorexia; bulimia; asthma; Parkinson's disease; thrombosis; acute heart failure; hypotension; hypertension; urinary retention; metabolic bone diseases such as osteoporosis and osteo petrosis; angina pectoris; hepatitis; myocardial infarction; ulcers; asthma; allergies; rheumatoid arthritis; inflammatory bowel disease; irritable bowel syndrome benign prostatic hypertrophy; pancreatitis; chronic renal failure and psychotic and neurological disorders, including anxiety, schizophrenia, manic depression, delirium, dementia, severe mental retardation and dyskinesias, such as Huntington's disease or Gilles de la Tourett's syndrome and others. Most preferably it refers to appendicitis; Bence Jones Proteinuria; Chronic Myoloid Leukemia; Colorectal cancer; chronic renal failure; Crohn's Disease; Diabetic Nephropathy; Cardiac pathology; Infection; Liver damage; Lymphoma; macrocytic anaemia; Prostate Cancer; Oligoclonal Banding and Pulmonary Embolism/Deep Vein Thrombosis (eg DVT/PE). One skilled in the art will appreciate that this list is not intended to be exhaustive. Indeed this method should be suitable for most if not all diseases.

Examples of shed cluster of differentiation molecules suitable for measurement to generate a sCD fingerprint for use in the methods of the present invention include but are not limited to CD14, CD25, CD31, CD44, CD50, CD54, CD62E, CD62L, CD86, CD95, CD106, CD116, CD124, CD138, CD141, CD40L, CD8, CD23, CD30, CD40. Those skilled in the art will be aware of other suitable sCD molecules for analyses according to the methods of the present invention. They will also be aware of other members of the family that each sCD stands as an ambasador for, such as chemokine receptors, interleukin receptors and inter-cellular adhesion molecules.

Measuring CD levels may be carried out using methods known to those skilled in the art and described herein. Shed CD (sCD) levels may suitably be measured in samples of tissue fluids which include, but are not limited to: serum, plasma, lymph fluid, pleural fluid, synovial fluid, follicular fluid, seminal fluid, amniotic fluid, milk, whole blood, urine, cerebrospinal fluid (CSF), ascites, saliva, sputum, tears, perspiration, and mucus. Advantageously, sCD levels are measured from samples of serum using reagents suitable for detecting shed CDs that include but are not limited to antibodies raised against those CDs. Preferably monoclonal antibodies or engineered antibodies, including phage antibodies raised against shed CDs or their membrane bound forms are used for their detection. However non-protein agents may also in principle be ised to detect sCD molecules. Similarly the detecting molecule may contain antibody bonding site fragments incorporated into the scaffold of another molecules or an engineered scaffold. Commercially available kits for measuring CD levels include those from Diaclone 1, Bd A Fleming BP 1985 F-25020 Besancon Cedex-France and Medsystems Diagnostics GmbH, Rennweg 95b, A-1030 Vienna Austria.

Suitable techniques for measuring levels of sCDs include but are not limited to immunoassay including ELISA using commercially available kits such as those described above, flow cytometry particularly multiplexed particle flow cytometry as herein described. Those skilled in the art will be aware of other suitable techniques for measuring CD levels in samples from an individual including antibody 'chip' array type technologies or chip technologies utilizing non-classical antibody binding site grafted molecules. Suitable techniques for measuring levels of sCDs are described in more detail in the detailed description of the invention.

Shed CD levels are also measured in a number of individuals with one or more disease states as herein defined, such as appendicitis and the like. Generally one or more shed CDs levels will be elevated in a given disease state as compared with the range found in normal individuals. However, some sCD levels decrease in some disease states compared with the range found in 'normal' individuals and such decreases may also form part of a sCD fingerprint of the present invention. Thus, by measuring the ranges of levels of various shed CDs found in a number of individuals with one or more defined disease state/s a 'fingerprint' of shed CD levels for any defined one or more diseases is generated. Likewise by measuring the ranges of levels of various shed CDs found in a number of individuals who have undergone one or more interventions (such as chemotherapeutic treatment, exposure to pathogens, exposure to radiation, individuals who have undergone a given vaccination program etc) then a 'fingerprint' (sCDprint) of shed CD levels for a given intervention may be generated. Those skilled in the art will appreciate that a sCD fingerprint (sCDprint) representative of an intervention may be generated form diseased or non-diseased individuals.

Preferably, the sCD fingerprint of an individual is generated from any two or more sCDs selected from the group consisting of the following: CD14, CD25, CD31, CD44, CD50, CD54, CD62E, CD62L, CD86, CD95, CD106, CD116, CD124, CD138, CD141, CD40L, CD8, CD23, CD30, CD40. One skilled in the art will appreciate that this list is not intended to be exhaustive and may include CD homologues of human and other mammalian or non-mammalian species. One skilled in the art will appreciate though that in general animal reference sCD fingerprints cannot be used to analyse human diseases and *vice-versa.* For instance sCD patterns/profiles/fingerprints could be defined in the rat or mouse using rat or mouse equivalent CD monoclonal antibodies as herein described. This would be an invaluable adjunct for studying these animal model systems, especially in the area of therapeutics, gene knockouts and other such proteomic and genomic studies.

The invention can also be used for testing human and other mammalian and non-mammalian species using sCD fingerprints from the appropriate animal.

One skilled in the art will appreciate that the methods of the present aspect of the invention can be used to test potential therapeutic agents suitable for the prophylaxis and/or treatment of diseases. An agent of therapeutic potential will affect the sCD profile or 'fingerprint' of the disease: If several fingerprints are taken at various stages of a disease and compared with those obtained from samples in which an individual has been treated with a potential therapeutic agent, then the effect on one or more sCD fingerprints can readily be assessed.

In addition, the method of the present invention may also be used to monitor patient compliance with taking a particular drug (agent), and/or undergoing a particular treatment regime.

Thus, in a further aspect still, the present invention provides a method for assessing the effect of one or more agent/s on one or more disease states in an individual comprising the step of comparing a sCD fingerprint of an individual at two or more different time periods.

According to the above aspect of the invention, preferably the agent is a potentially therapeutic agent.

In the context of the present invention the term to 'assess the effect' means to detect any changes in the severity or other characteristics of any one or more diseases in an individual. Such changes will be reflected in a change in sCD profile/fingerprint of an individual.

Preferably the agent is a potentially therapeutic agent. The term 'potentially therapeutic agent' means any agent that may cause a beneficial effect on an individual suffering from one or more diseases. Such beneficial effects may be for example reducing the clinical signs of the one or more diseases. It is an important feature of the present invention though, that a change in level of any one sCD in isolation is not always indicative of a change in severity of a disease. It will appreciated though that in some cases, a change in the level of one sCD in isolation will be indicative of a change in severity of a disease.

Generally, individual sCD levels will be elevated in a disease state as compared with a 'normal' non-diseased individual. Occasionally however, the level of an individual sCD will decrease in a disease state as compared with a normal non-diseased individual. However, according to the present invention, it is the changes of the profile of a number of sCDs (that is a fingerprint) during a disease which provides an accurate measure of the effect of one or more agents on a disease state in an individual.

One skilled in the art will appreciate that on occasion a selection of the complete repertoire of sCDs available for testing may be measured. The selection chosen may vary according to the disease state being tested.

According to this aspect of the invention, sCDs suitable for generating a sCD fingerprint are as described herein.

Therapeutic agents may be tested for their effect on any one or more disease states selected from the group consisting of the following: infections, autoimmune disease, neoplastic, vascular endocrinological, metabolic, inflammatory degenerative, psychiatric psychological, traumatic, drug/toxin-related, bacterial, fungal, protozoan and viral infections, non-neoplastic disorders; pain; diabetes, obesity; anorexia; bulimia; asthma; pregnancy; endocrine; vascular; metabolic; gastro-intestinal; iatrogenic; psychiatric; psychoclogical; exercise-induced; diet-related; ME; degenerative; Parkinson's disease; thrombosis; atherosclerosis; acute heart failure; hypotension; hypertension; erectile dysfunction; urinary retention; metabolic bone diseases such as osteoporosis; angina pectoris; hepatitis; myocardial infarction; ulcers; allergies; rheumatoid arthritis; inflammatory bowel disease; irritable bowel syndrome benign prostatic hypertrophy; psychosis; psychiatric disorders; including anxiety; schizophrenia; manic depression; delirium; dementia; severe mental retardation and dyskinesias, such as Huntington's disease or Gilles de la Tourett's syndrome; and preferably tumours which can be benign or malignant cancers; breast cancer; myeloma; melanoma; bladder cancer; leukaemia; plasmocytoma and others, but most preferably appendicitis; Bence Jones Proteinuria; Chronic Myeloid Leukaemia; Colorectal cancer; chronic renal failure; Crohn's Disease; Diabetic Nephropathy; Cardiac pathology; Infection; Liver damage; Lymphoma; macrocytic anaemia; Prostate Cancer; Oligoclonal Banding and PE/DVT

Suitable agents for assessment according to the method of the present invention may be naturally occurring or synthetic. Naturally occurring agents include proteins, peptides or nucleic acids. They may be agents known to be of therapeutic value or they may be of unknown therapeutic value.

In a further aspect, the present invention provides a method for sub-categorising a sCD fingerprint profile comprising the steps of identifying within one or more disease states one or more sub-group/s of sCDs wherein each sub-group of sCDs exhibits common characteristics distinguishing it from any other sub-group within that disease category.

As used herein the term a 'sCD sub-category' describes a sub-group of sCDs which show a defined fingerprint/profile (sub-fingerprint) of sCD levels within a larger fingerprint of one or more disease states wherein each sub-group of sCDs exhibits common characteristics distinguishing it from any other sub-group within those one or more disease states.

In a further aspect still, the present invention provides a sCD reference database comprising pathological and/or normal sCD fingerprint patterns.

As herein described the term 'a reference database' refers to a collection of sCD fingerpruits from normal 'non-diseased' and/or diseased individuals. Advantageously, the database is computer generated and/or stored. Advantageously the data from more than 5 individuals is present in the database. More advantageously the data from more than 10, 100, or 1000 individuals comprises the database. More advantageously still the data from more than 10,000 or more than 50,000 individuals comprises the database. Most advantageously the data from more than 100,000 individuals comprises the database. Advantageously the database, in addition to sCD data will also comprise clinical information relating to various patients and/or disease conditions. Alternatively or in addition, a database according to the present invention comprises genomic information such as mRNA expression profiles, and/or sCD body fluid profiling data, and/or CD cell surface pattern data, and/or clinical data. Most advantageously, the database will be in the form of an integrated clinical database comprising accurate patient details including co-morbidity, age, sex, smoking status etc.

Recent studies which have investigated the impaired expression of NKG2D and T-cell activation by tumour-derived soluble MHC ligands (Nature, vol 419, 17 October 2002). Studies have shown that tumours release large amounts of the MHC class I homologue MIC into the serum. Activation of the NKG2D receptor on natural T cells is known to stimulate their ability to destroy tumours, but the high levels of tumour derived MIC seem to downregulate the NKG2D receptor and block the antitumour effect. (Nature, vol 419, 17th October 2002, p679, pg 734). These soluble forms of MHC are produced either by enzymatic cleavage or by alternative splicing (Nature, vol 419, 17th October 2002, p679, pg 734).

It is apparent from the present disclosure that sCDs may be produced by alternative splicing (Woolfson and Milstein, PNAS Vol 91, pp 6683-6687), enzymatic cleavage or other mechanisms and that such shed forms are associated with disease (Sugiyama et al,. Non-invasive detection of bladder cancer by identification of abnormal CD44 proteins in exfoliated cancer cells in urine, Journal of Clinical pathology, 48, 3, 142-147; Yoshida, K et al, Abnormal retention of intron 9 in CD44 transcripts in human gastrointestinal tumours. Cancer research 55, 4273-4277). The present inventors consider that sCD molecules may also bind to a ligand/receptor and thereby block down stream effects. Thus, the present inventors have realised that the blockage of the production of sCD molecules via the inhibition of any of the methods of sCD generation described herein, may be a therapeutically. useful method for the prophylaxis or treatment of one or more diseases or disorders including but not limited to any of those in the group consisting of infections, inflammation, vascular, iatra genic, endocrine, drug-related disorders, toxin related disorders, and cancer in particular metastasis and leukemia.

Thus in a further aspect still, the present invention provides a method for treating one or more diseases comprising the step of inhibiting the production of one or more sCDs within an individual.

In a further aspect still, the present invention provides the use of an inhibitor of the production of one or more sCDs in the preparation of a medicament for the treatment of disease.

According to the above aspect of the invention, the term 'an inhibitor of the production of one or more sCDs' refers to one or more agents which inhibit the production of a shed form of sCD as herein defined. Advantageously, the inhibitor is a specific inhibitor of those one or more sCDs. Suitable inhibitors include alternative splicing inhibitors and/or enzymatic cleavage inhibitors. Advantageously, the inhibitor is an alternative splicing inhibitor. Such alternative splicing inhibitors include for example inhibitors of exonic splicing enhancers (Fairbrother et al, Science, vol 297, 9th August 2002).

According to the above aspects of the invention, the production of any one or more sCDs present in the body fluid of an individual may be inhibited. Advantageously, the one or more sCDs are any one of those selected from the group consisting of the following: CD14, CD25, CD31, CD44, CD50, CD54, CD62E, CD62L, CD86, CD95, CD106, CD116, CD124, CD138, CD141, CD40L, CD8, CD23, CD30, CD40. More advantageously the sCD is CD1. Advantageously, the sCD is CD1 and the inhibitory agent is an alternative splicing inhibitor and/or a gene specific CD 1 inhibitor.

It should be noted that the present inventors consider that the invention described herein can be used to post hoc re-analyse clinical trial data, assigning patients to different sub-groups for analysis on the basis of their sCDprints and in doing so potentially revealing previously unseen statistical effects and at the same time identifying responders or non-responders to a therapeutic intervention, and those who respond adversely to the intervention in the case in which a therapeutic agent has not been taken further due to adverse responses in small numbers of individuals.

### Definitions

The term **'CD'** refers to a different cell surface leukocyte molecule recognised by a given monoclonal or group of monoclonal antibodies which specifically 'cluster' to the antigen/molecule in question. Many, if not all of these molecules produce forms which are released from the cell surface by alternative splicing, proteolytic cleavage, dissociation or other mechanisms.

Thus in the context of the present invention, the term **'shed CD molecule (sCD)'** refers to a released form of a cell surface leukocyte molecule in which at least a portion of that molecule is recognised by a given monoclonal or group of monoclonal antibodies as herein described. Advantageously, as herein defined a shed form of sCD is generated by various mechanisms including but not limited to any of those selected from the group consisting of the following: alternative splicing, proteolytic cleavage and dissociation.

As herein defined, the term **'shed CD fingerprint/profile (sCD)'** or **'sCDprint'** describes the pattern or profile of levels of more than one shed CD measured in one or more body fluids from one or more individuals. A sCD fingerprint as herein defined may be representative of one or more non-diseased individual or a one or more diseased individual/s. Preferably, a shed CD fingerprint describes the level of five or more shed CD molecules, more preferably it is 6, 7, 8, 9, 10 or more sCD molecules, more preferably still a shed CD fingerprint describes the levels of 15 or more sCD molecules. Most preferably a shed CD fingerprint describes the levels of 20 or more sCD molecules.

As used herein the term a **'sCD sub-category'** describes a sub-group of sCDs which show a defined fingerprint/profile **(sub-fingerprint)** of sCD levels within a larger fingerprint of one or more disease states wherein each sub-group of sCDs exhibits common characteristics distinguishing it from any other sub-group within those one or more disease states.

As defined herein, the term a **'disease state'** refers to any impairment of the normal physiological functions affecting an organism or any disease condition, disorder or the presence of a particular microbial, viral, parasitic or other pathogenic agent known to one skilled in the art. Suitable disease states for analysis as described herein include but are not limited to: infectious, neoplastic, autoimmune, immunological, metabolic, degenerative, psychological, psychiatric, iatrogenic, inflammatory, drug or toxin related, vascular, traumatic and endocrine diseases. Advantageously, 'a disease state' as herein defined refers to any one or more disease selected from the group which includes but is not limited to: infections such as bacterial, fungal, protozoan, parasitic, prion, and viral infections, non-neoplastic disorders; stroke; heart condition; atherosclerosis; pain; diabetes, obesity; anorexia; bulimia; asthma; Parkinson's disease; thrombosis; acute heart failure; hypotension; hypertension; urinary retention; metabolic bone diseases such as osteoporosis and osteo petrosis; angina pectoris; hepatitis; myocardial infarction; ulcers; asthma; allergies; rheumatoid arthritis; inflammatory bowel disease; irritable bowel syndrome benign prostatic hypertrophy; pancreatitis; chronic renal failure and psychotic and neurological disorders, including anxiety, schizophrenia, manic depression, delirium, dementia, severe mental retardation and dyskinesias, such as Huntington's disease or Gilles de la Tourett's syndrome and others. Most preferably it refers to appendicitis; Bence Jones Proteinuria; Chronic Myoloid Leukemia; Colorectal cancer; chronic renal failure; Crohn's Disease; Diabetic Nephropathy; Cardiac pathology; Infection; Liver damage; Lymphoma; macrocytic anaemia; Prostate Cancer; Oligoclonal Banding (myaesthenis gravis) and Pulmonary Embolism/Deep Vein Thrombosis (eg DVT/PE). One skilled in the art will appreciate that this list is not intended to be exhaustive.

Examples of shed cluster of differentiation molecules suitable for measurement to generate a sCD fingerprint for use in the methods of the present invention include but are not limited to CD14, CD25, CD31, CD44, CD50, CD54, CD62E, CD62L, CD86, CD95, CD106, CD116, CD124, CD138, CD141, CD40L, CD8, CD23, CD30, CD40. Those skilled in the art will be aware of other suitable sCD molecules for analyses according to the methods of the present invention.

As defined herein the term **'an antibody'** includes within its scope for example IgG, IgM, IgA, IgD or IgE) or fragment (such as a FAb, F(Ab')₂, Fv, disulphide linked Fv, scFv, diabody) whether derived from any species naturally producing an antibody, or created by engineered DNA technology (for example fluorobodies, green fluorescently labelled antibodies); whether isolated from serum, B-cells, hybridomas, transfectomas, yeast or bacteria). It also includes within its scope, non-protein binding agents which comprise the binding specificity of an antibody molecule, or a binding capacity in general for a determinant or sub-determinant of a component of the protein structure/glycoprotein structure of a CD molecule.

### Brief description of the figures

- **Figure 1.**: **Disease Groups. Multiples of upper limit of normal (ULN). All sCD's included**
The limits indicated by each point are:
- No shading ≤ 1 x ULN
- Lightly shaded 1 - 2 x ULN
- Darkly shaded >2 x ULN
- A white slash in the box indicates no data available.
- **Figure 2.**: All sCD's that appear not to discriminate from the normals (sCD's 21; 102; 117, 126; 130; 26; 44v5; 44v6; 62P).
- **Figure 3.**: Disease Groups. Mode of Response for Remaining 20 sCD's. To simplify the data further the modal response for each disease group was plotted.
- **Figure 4.**: Disease Groups. Mode of Response for remaining sCD's. Data has been ranked in order of increased expression.
- **Figure 5.**: Disease Groups. Mode of Response for remaining sCD's.
- **Figure 6.**: Disease Groups. Mode of Response for all sCD's. As for Figure 3 (except all sCD's are included). The limits indicated by each point are:
- No shading ≤ 1 x ULN
- Lightly shaded 1- 2 x ULN
- Darkly shaded > 2 x ULN
A white slash in the box indicates no data available.
- **Figure 7.**: Disease Groups. Mode of Response for all sCD's.
- **Figure 8.**: Disease Groups. Mode of Response for all sCD's.

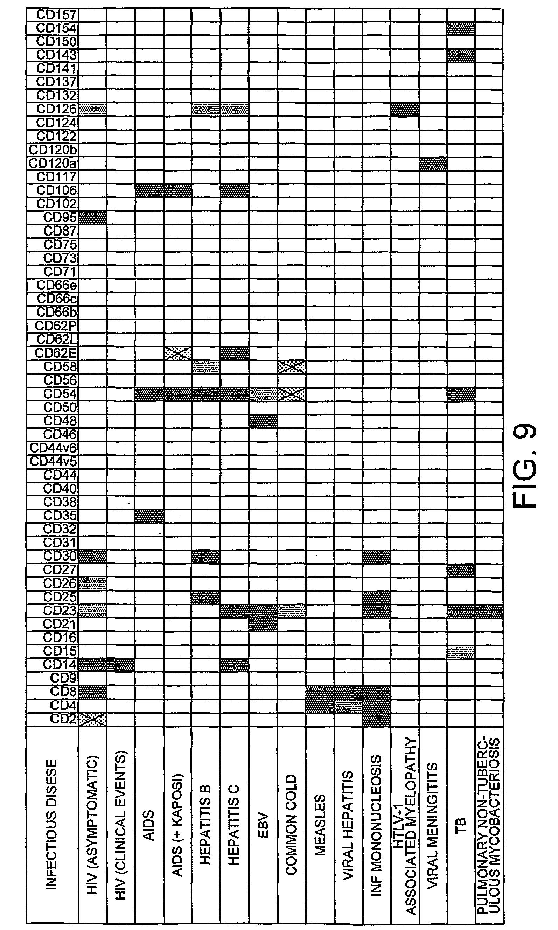

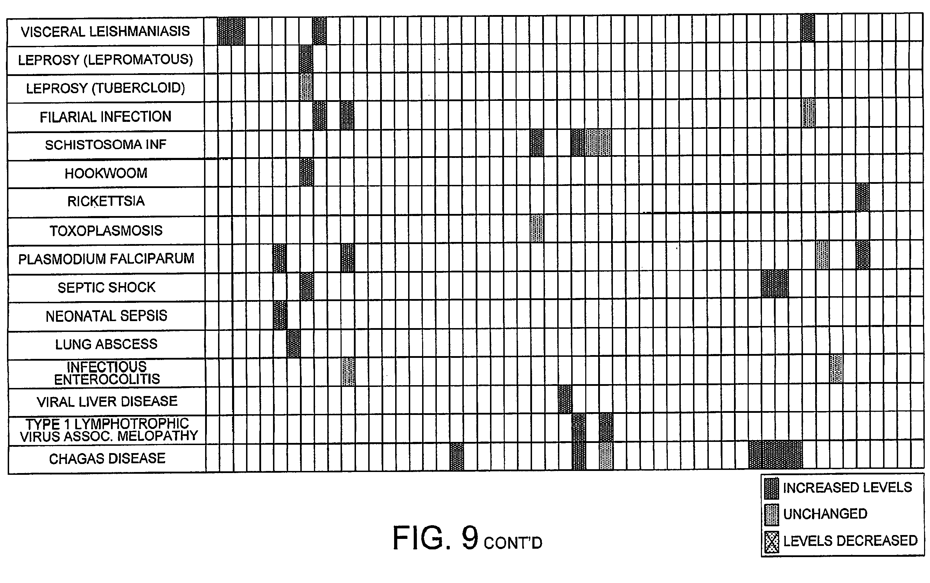

- **Figure 9.**: Shows the patterns of levels of sCDs during various infectious disease states as compared with a group of 'normal' non-diseased individuals.

- **Key:**: Lightly shaded box-sCD levels unchanged Darkly shaded box-sCD levels increased Shaded box with diagonal line-sCD levels decreased.

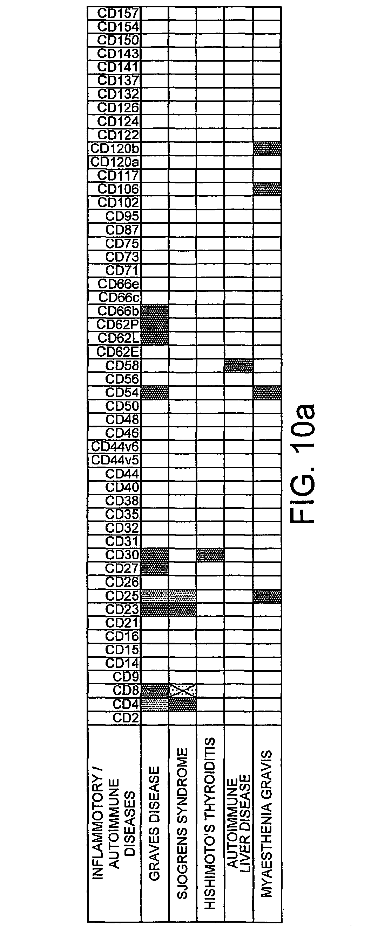

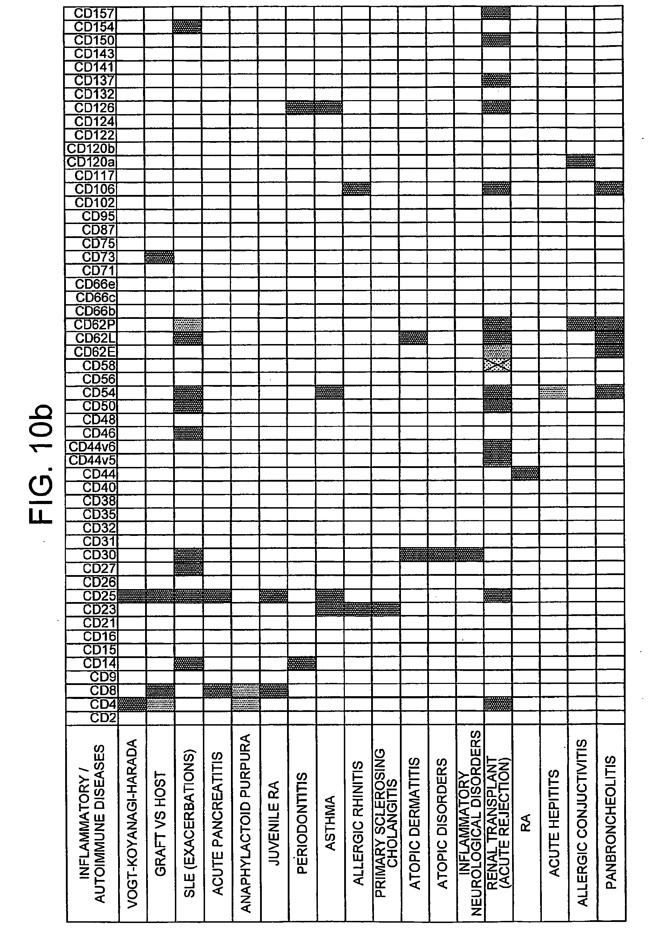

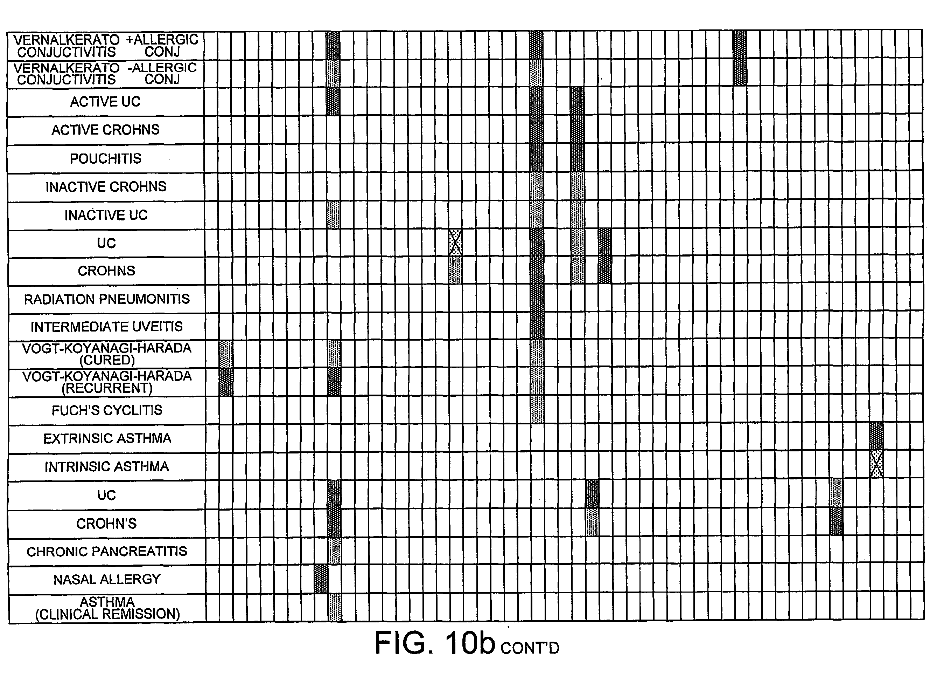

- **Figure 10.**: Shows the patterns of levels of sCDs during various inflammatory/autoimmune diseases as compared with a group of 'normal' non-diseased individuals.

- **Key:**: Lightly shaded box-sCD levels unchanged Darkly shaded box-sCD levels increased Shaded box with diagonal line-sCD levels decleased.

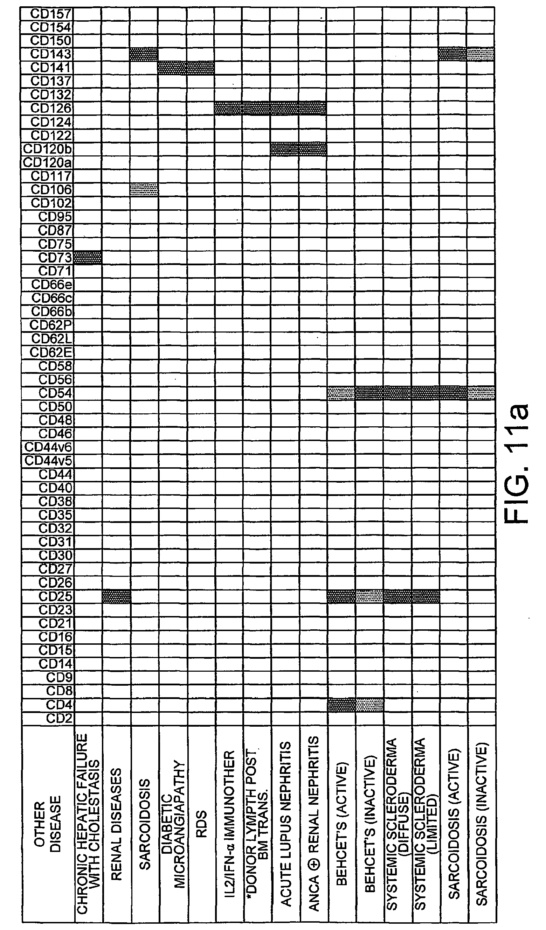

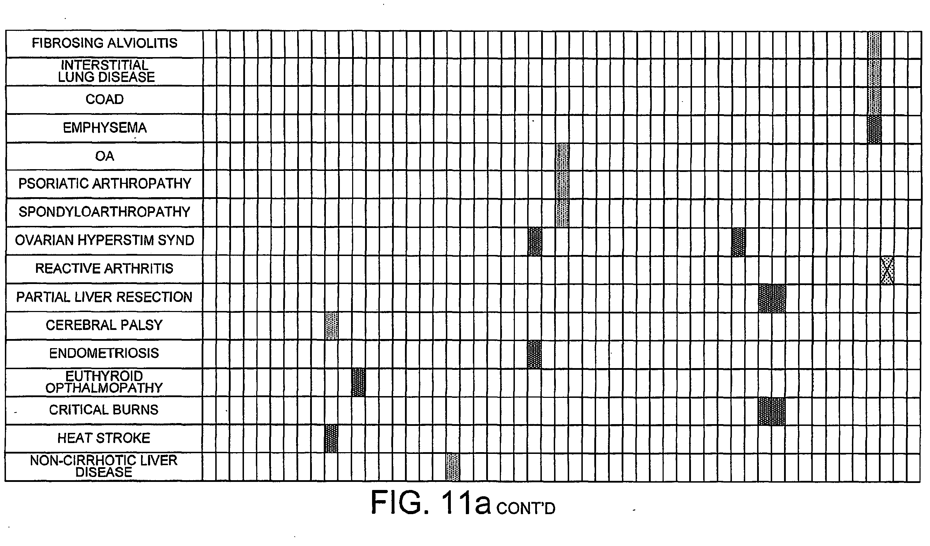

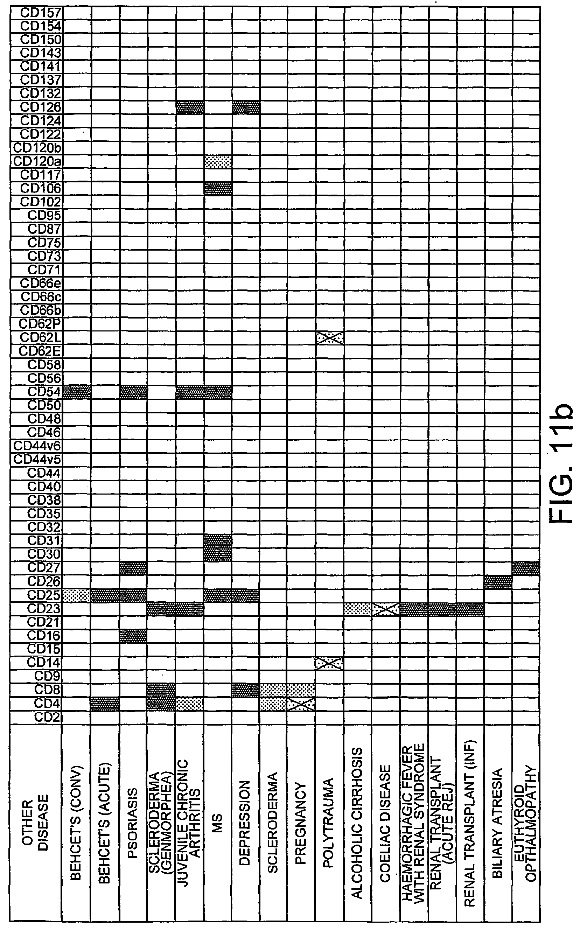

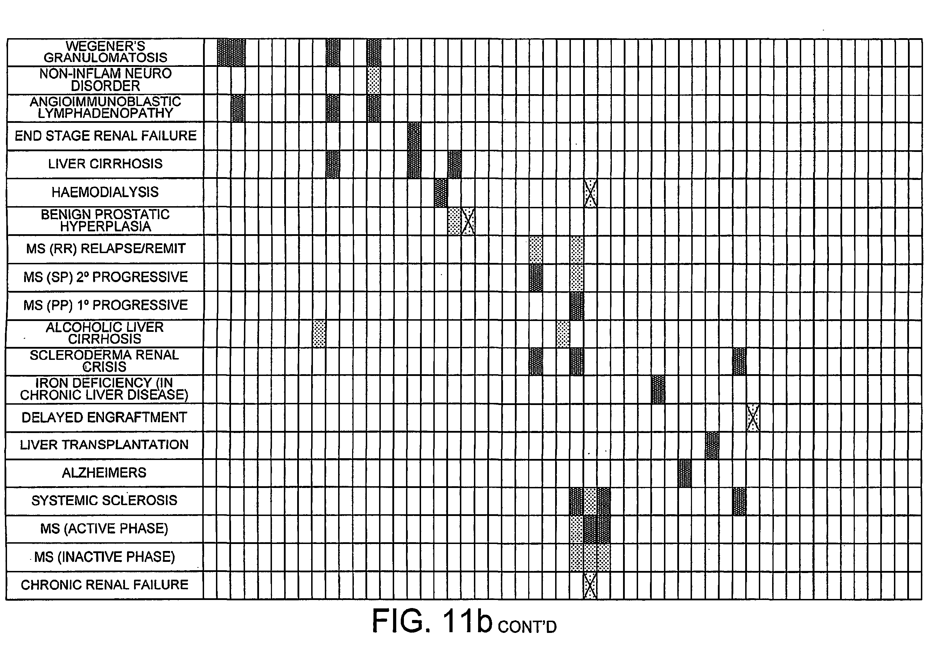

- **Figure 11.**: Shows the patterns of levels of sCDs during various 'other diseases' as compared with a group of 'normal' non-diseased individuals.

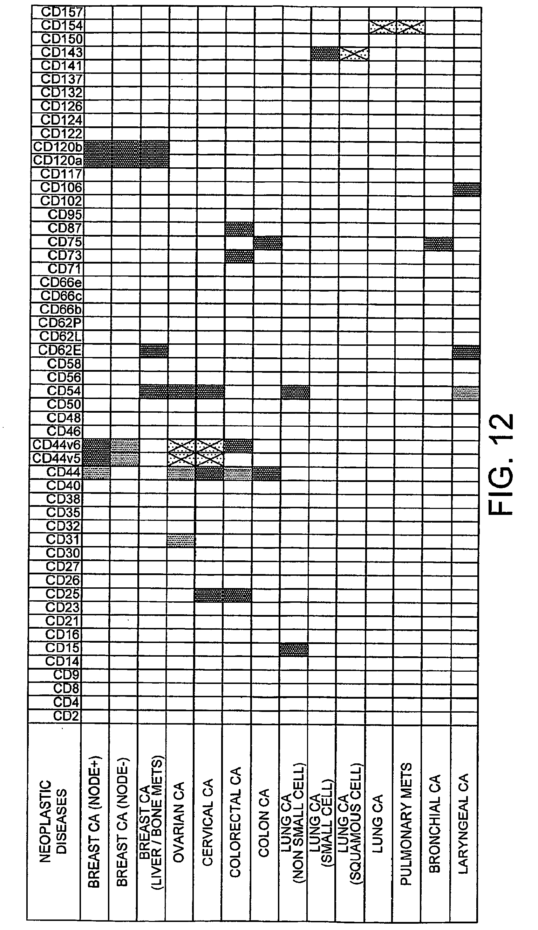

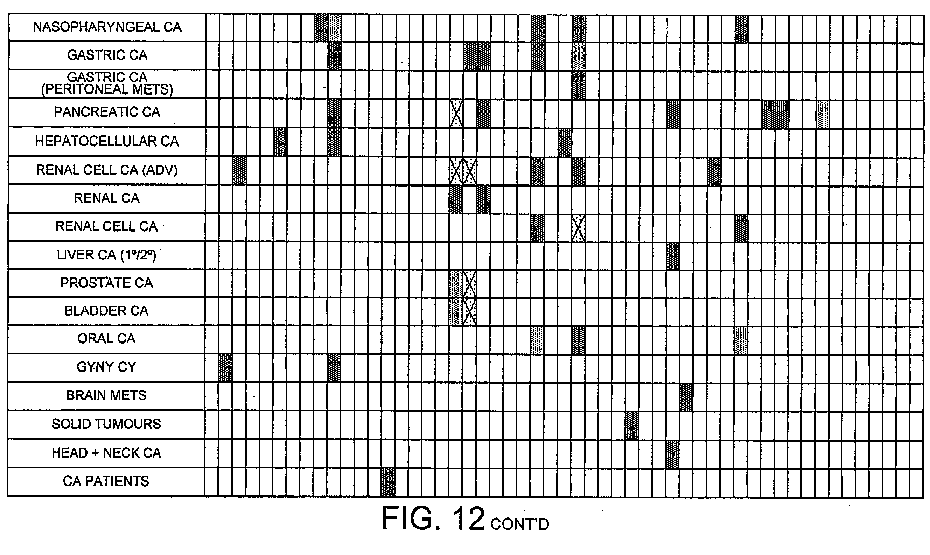

- **Key:**: Lightly shaded box-sCD levels unchanged Darkly shaded box-sCD levels increased Shaded box with diagonal line-sCD levels decreased
- **Figure 12.**: Shows the patterns of levels of sCDs during various neoplastic disease states as compared with a group of 'normal' non-diseased individuals.

- **Key:**: Lightly shaded box-sCD levels unchanged Darkly shaded box-sCD levels increased Shaded box with diagonal line-sCD levels decreased

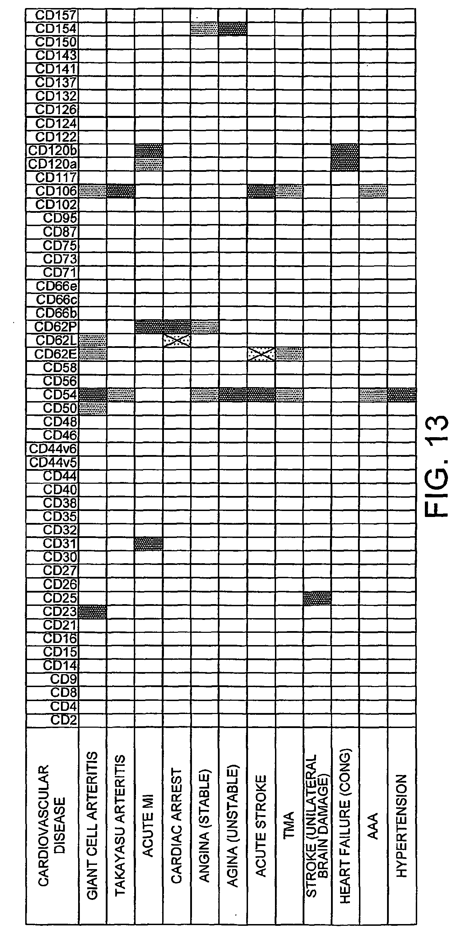

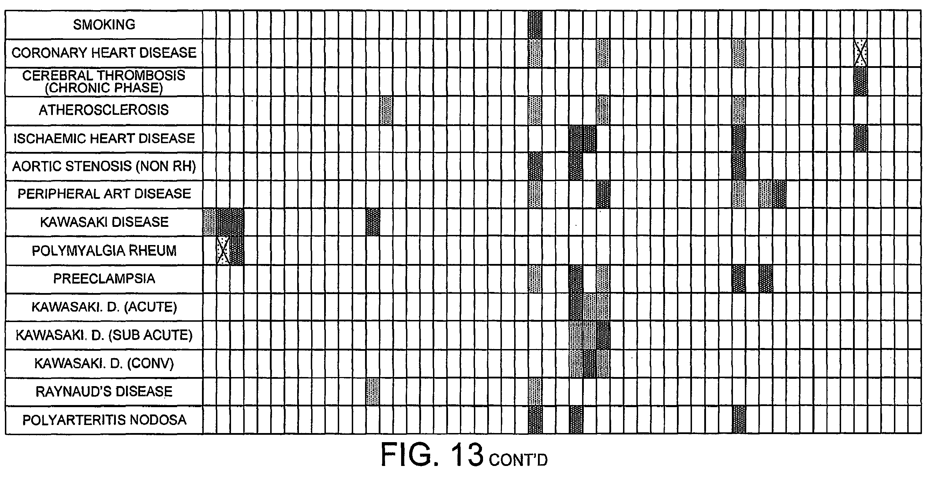

- **Figure 13.**: Shows the patterns of levels of sCDs during various cardiovascular diseases as compared with a group of 'normal' non-diseased individuals.

- **Key:**: Lightly shaded box-sCD levels unchanged Darkly shaded box-sCD levels increased Shaded box with diagonal line-sCD levels decreased

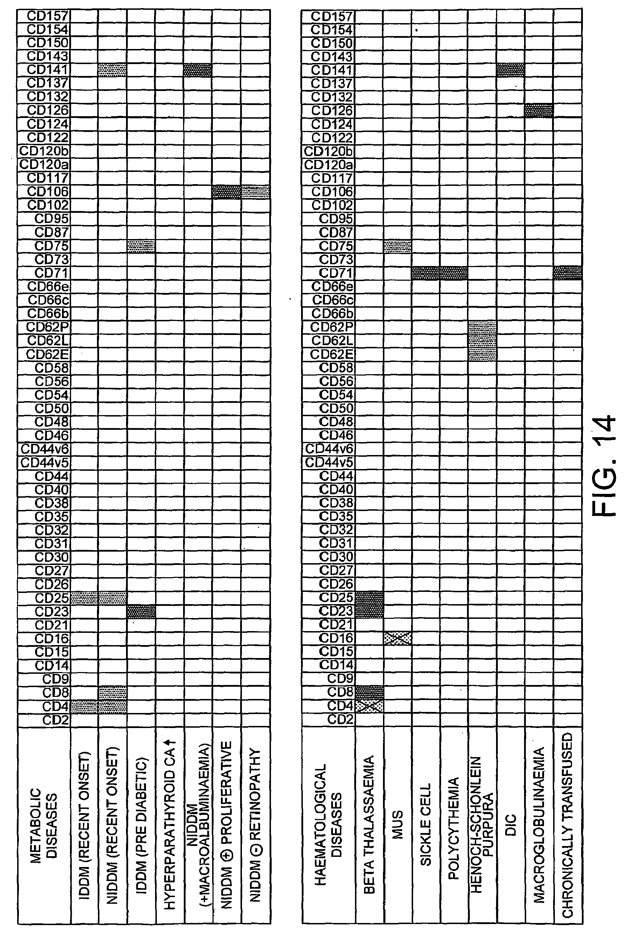

- **Figure 14.**: Shows the patterns of levels of sCDs during various metabolic and haematological diseases as compared with a group of 'normal' non- diseased individuals.

- **Key:**: Lightly shaded box-sCD levels unchanged Darkly shaded box-sCD levels increased Shaded box with diagonal line-sCD levels decreased

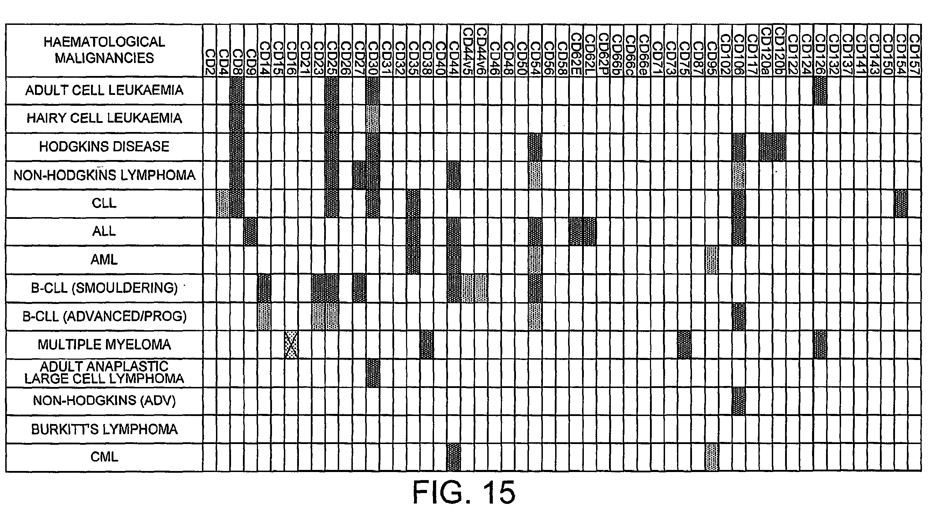

- **Figure 15.**: Shows the patterns of levels of sCDs during various haematological malignancies as compared with a group of 'normal' non-diseased individuals.

- **Key:**: Lightly shaded box-sCD levels unchanged Darkly shaded box-sCD levels increased Shaded box with diagonal line-sCD levels decreased

### Detailed description of the Invention

### General techniques

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods. In addition Harlow & Lane., A Laboratory Manual Cold Spring Harbor, N.Y, is referred to for standard Immunological Techniques.

### sCD molecules according to the invention

In a first aspect, the present invention provides a shed CD (sCD) fingerprint (sCD print) of one or more disease states.

In the context of the present invention, the term 'CD' refers to a different cell surface leukocyte molecule recognised by a given monoclonal or group of monoclonal antibodies which specifically 'cluster' to the antigen/molecule in question. Many, if not all of these molecules produce forms which are released from the cell surface by alternative splicing, proteolytic cleavage, dissociation or other mechanisms.

Thus in the context of the present invention, the term 'shed CD molecule (sCD)' refers to a released form of a cell surface leukocyte molecule in which at least a portion of that molecule recognised by a given monoclonal or group of monoclonal antibodies as herein described.

### Location of sCD molecules.

Although first identified on leukocytes. CD antigens have been located on other blood cells and non-blood cells. CD molecules have been found on many different blood cell types including the following blood cell types: erythroid, dendritic cells, B cells, pre-B cells, T cells (cytotoxic, suppressor and helper subtypes), monocytes, myeloid cells, endothelial cells, platelets, NK cells ( natural killer cells), red blood cells, thymocytes. Those skilled in the art will appreciate that this list is not intended to be exhaustive.

In addition, CD antigens have been found on the following non-immune cells myocytes, peripheral nerve, liver, platelet precursors, lung cells, cerebellum, cortex, glia, neuroepithelium, placenta, prostate, spinal cord, brain, muscle, kidney, salivary glands, muscle, melanoms, leukemias, lymphoma, hematopoietic cells, lymphoid progenitor cells, breast, astrocytes, thyroid, lung, pancreas, trachea, schwann cells, trophoblast, erthroblast, microglia.

In the context of the present invention it is important to note that the CD nomenclature is a simple method for representing a whole range of molecules. For example: CD14 is the lipopolysaccharide receptor, LP5-R; CD21 is the EBV receptor; CD 25 is the IL-2Ralpha receptor; CD 31 is PECAM-1; CD 44 is H-CAM; CD 50 is ICAM-3; CD 54 ICAM-1; CD 62E is LECAM-2; CD 62L is LECAM-1; CD 86 is B 70; CD 95 is FAS apoptosis antigen; CD 102 is ICAM-2; CD 106 is VCAM-1; CD 116 is GM-CSFR alpha; CD 117 is c-kit stem cell factor receptor; CD 124 is IL-4R alpha; CD 126 is IL-6Ralpha; CD 130 is gp 130; CD 138 is syndican-1; CD 141 is thrombomodulin; CD8 is Lin 2; CD 27 is low affinity IgE-R; Cd 30 is Ki-1;

The present inventors realised that sCDs act as representives/ambassadors for the molecules from which they are shed. Furthermore they realised that cell behaviour can be interrigated on the basis of the patterns of sCD molecules shed by cells.

### Generation of a fingerprint of one or more disease states

In a first aspect, the present invention provides a shed CD (sCD) fingerprint of one or more disease states.

Clinical signs and symptoms and various biochemical indicators of disease are used to identify individuals with one or more defined disease states. sCD levels are then measured for a number of sCDs present in one or more body fluid samples from each individual, preferably in a number of individuals using methods known to those skilled in the art and described herein, in order to generate a reference disease state or reference composite disease state fingerprint for those one or more given disease states.

### (A) Diagnosis of disease states

### i. Diagnostic Indicators Used.

### Appendicitis

- Request for Amylase at admission A&E/MAU Subsequent Histopathological Diagnosis

### Bence Jones Proteinuria

- Multiple myeloma in which the malignant plasma cells excrete only light chains of one type (either 2 or 3); lytic bone lesions occur in about 60% of the cases, and light chains (Bence Jones protein) can be detected in the urine
- Positive finding

### Chronic Myeloid Leukaemia

- Histopathological Diagnosis

### Colorectal Carcinoma

- Histopathological Diagnosis

### Chronic Renal Failure

- Prolonged elevation of serum creatinine.

### Crohn's Disease

- Histopathological diagnosis

### Diabetic Nephropathy

- Identified by abnormal urine Albumin/Creatinine ratio from subjects attending diabetic clinic.

### Cardiac Pathology

- MI (as diagnosed by increased CK, symptoms and ECG changes).

### Infection

CRP (C reactive protein) > 250 g/l (e.g. *Staphylococcus aureus* infections).

### Liver Damage

- Clinical Details Alcoholic Liver Disease/Poisoning. Abnormal liver function tests.

### Lymphoma

- Histopathological Diagnosis

### Macrocytic Anaemia

- Diagnosed by haematological parameters. Hb <10 g/dL; MCV > 100 fL

### Oligoclonal Banding

- small discrete bands in the gamma globulin region of the spinal fluid electrophoresis, indicating local central nervous system production of IgG; bands are frequently seen in patients with multiple sclerosis but can also be found in other diseases of the central nervous system including syphilis, sarcoidosis, and chronic infection or inflammation.

### VO (pulmonary angiogram)

### Pulmonary Embolism/Deep Vein Thrombosis

- ultrasound VQ or CT pulmonary angiogram scan (ventilation perfusion mismatch)

### Prostate Carcinoma

- Histopathological diagnosis and elevated PSA.

In general, a combination of the patient's history, medical examination, general health and indicators provided from biochemical, histochemical, radiochemical and other types of tests and disease and/or clinical signs of disease will be used in the diagnosis of disease. For the avoidance of doubt, the term 'clinical signs and symptoms of disease' means the same as 'clinical details' of disease.

### (B) Samples of body fluids from disease states

For each sCD the following information is generally obtained a) the dynamic range of the assay b) the range of concentrations expected in health c) the range of concentrations expected in disease. From this information an approximate dilution factor for each assay may be obtained, allowing maximum use of subject samples. One skilled will appreciate thought that in some circumstances body fluid samples may not be diluted for testing.

Suitable body fluids for measuring sCD levels as herein defined include whole blood, serum, urine, tissue fluid, cerebrospinal fluid, lymphatic fluid, synovial fluid, aspirate, bone marrow aspirate, mucus or other tissue or body fluid. One skilled in the art will appreciate that this list is not intended to be exhaustive. Preferably sCD levels are measured in serum which is prepared from whole blood using methods familiar to those skilled in the art At least 1.5ml of sample is required for testing of all the sCDs. Advantageously, less than 1.5 mls of sample is required for such testing. More advantageously, much smaller volumes of sample will be needed for such testing. In addition, the present inventors have shown that haemolysis and lipaemia can interfere with some immunoassays used for detecting sCDs and therefore samples are used which exhibit minimal haemolysis and lipaemia.

Body fluid samples may be diluted in order to measure the sCD levels and the dilution factor for each sCD should be the same for the generation of the fingerprints for all disease states tested. One skilled in the art will appreciate that the dilution factor may be adjusted in order to focus on either high or low concentrations of sCDs. Advantageously, the dilution factor will be adjusted to focus on high concentrations of sCDs.

### (C) Methods of measuring sCD levels.

Suitable methods for measuring levels of sCDs in body fluids include flow cytometry, in particular multiplexed particle flow cytometry, immunoassay and microarray technologies utilising antibody or ligand interactions. Advantageously, sCDs levels are measured using multiplexed particle flow cytometry and/or chip based monoclonal antibody technology, engineered antibody molecules or non-antibody or non-protein molecules that recognise sCD antigens. These methods will be familiar to those skilled in the art.

### (i) Immunoassays

Immunoassays such as immunoblotting (detecting membrane-bound and soluble proteins), and enzyme linked immunoassays (ELISA) provide a sensitive and specific means of detecting target substances.

Although the various types of immunoassays are performed differently, they have one thing in common-they all involve antibodies. Used in an appropriate immunoassay system, specificity leads to sensitivity. As herein defined the term 'antibodies' includes antibody fragments, engineered immunoglobulin folds or scaffolds which have a binding affinity for soluble CD molecules.

One skilled in the art will appreciate that the 'immunoassay technique' may be adapted to use other molecules which selectively bind sCDs. Those skilled in the art will be aware of such molecules.

In a direct immunoassay, the antibody used as the primary reagent is advantageously given a fluorescent, enzymatic, or radio-active detection means. In indirect immunoassays, the secondary antibody-usually polyclonal antisera produced by a goat or a rabbit against human immunoglobulins-carries the detection means. When a secondary antibody is used, the initial immune reaction between the primary antibody and the target antigen is amplified, producing a more readily detectable signal.

Western blots of electrophoretically separated proteins (immunoblots), on the other hand, are generally probed with antibodies labeled with an enzyme or a radioisotope such as 125I. Chromogenic or chemiluminescent substrates can also be used. For example, enzymes such as HRP and AP catalyze chromagenic reactions, in which a colourless substrate is converted into a coloured compound, and also chemiluminescent reactions where light is emitted.

Chromogenic substrate kits are commercially available and include but are not limited to for example alkaline phosphatase, horseradish peroxidase, and TMB peroxidase (TMB is tetramethylbenzidine, the substrate in this case). Boehringer Mannheim also has several enzyme substrates for immunoassays available. They include but are not limited to for example ABTS (2,2+-azino-di-3-ethylbenzthiazoline sulfonate) and TMB (tetramethylbenzidine), which are used with HRP; and 4-nitrophenyl phosphate and 5-bromo-4-chloro- 3-indolyl phosphate (BCIP) for immunoassays in which an alkaline phosphatase-conjugated antibody is used.

Chemiluminescent substrates are available from companies such as Pierce, which for example, produces the SuperSignal CL-HRP Substrate, an enhanced chemiluminescent substrate for horseradish peroxidase. This system detects specific proteins on immunoblots with a sensitivity that rivals radioactivity (reportedly to picogram levels). When the chemiluminescent substrate is applied to membrane-bound proteins on an immunoblot, an instantaneous but long-lasting flash of light is produced.

Commercially available immunoassay kits for measuring sCD levels include those from Diaclone 1, Bd A. Fleming BP 1985 F-25020 Besancon Cedex-France which provides kits for the measurement of a number of CD molecules including CD 14, CD21, CD25, CD31, CD44, CD50, CD54, CD62E, CD62L, CD86, CD95, CD102, CD106, CD116, CD117, CD124, CD126, CD130, CD138, CD141, CD40L. Medsystems diagnostics GmbH, Rennweg 95b, A-1030 Vienna Austria, also provides kits which measure sCD levels.

### (ii) Flow cytometry

Techniques for carrying out flow cytometry are familiar to those skilled in the art and are described in Flow Cytometry: A Practical Approach. Edited by MG Ormerod. IRL Press, Oxford. 1994. ISBN 0-19 963461-0. Practical Flow Cytometry. 3rd Edition. Howard M Shapiro. Alan R Liss, Inc. ISBN 0-471-30376-3. Flow Cytometry. First Principles. Alice Longobardi Givan. Wiley-Liss, New York, 1992. ISBN 0-471-56095-2. Handbook of Flow Cytometry Methods. Edited by J Paul Robinson. Wiley-Liss, New York, 1993. ISBN 0-471-59634-5.

### (iii) Multiplexed particle flow cytometry assay

Methods for simultaneously assaying different proteins in individual samples are commercially available. Some of those commercially available are detailed below:
The versatile laboratory multianalyte profiling (LabMAP™) system developed by Luminex Corp. of Austin, Texas, can be used for virtually any bioassay that is based on the specific binding of one molecule to another, for example a monoclonal antibody raised against a sCD and a CD molecule.

LabMAP assays for a sCD molecule can be based on the immunological detection, and/or may follow the gain or loss of fluorescence (e.g., when a mAb raised against a sCD binds to a sCD target). LabMAP assays employ three different fluorochromes: two to create color-coded microspheres, and the third for quantifying the reaction. Polystyrene microspheres are internally dyed with precise ratios of two spectrally distinct fluorochromes. This ratio confers a unique identifying "signature" or "spectral address" to each microsphere set.

Bioassays are conducted on the surfaces of the microspheres. Each capture probe (e.g., sCD-specific antibody or other affirmative detection reagent) is immobilized onto a color-coded set of microspheres using any of a variety of different surface chemistries. Luminex offers microspheres bearing Lumavidin™ (an avidin derivative), for immobilizing biotinylated molecules, or carboxyl groups, for covalently coupling protein. The binding of analyte to an immobilized probe is detected via a detection reagent labeled with the third fluorochrome. Luminex currently offers 100 different microsphere sets, each of which can be used for the simultaneous measurement of a different analyte. Thus, in theory, up to 100 different species can be simultaneously measured in a single tube or microplate well.

The Luminex microsphere product line is designed specifically to work with the instruments available from Luminex or their partners. The Luminex 100™ instrument uses microfluidics to align the microspheres in single file and employs two lasers, one for the detection of the fluorescent microsphere itself, and the other for the reporter reagent. The colour signals are captured by an optics system and translated into binding data via digital signal processing.

Instrumentation, reagents, and custom services for LabMAP technology users are also available from companies other than Luminex. For example, Bio-Rad Laboratories of Hercules, Calif., introduced the Bio-Plex™ Protein Array System. This system combines a fluorescent reader with the software, protocols, and supplies needed for performing LabMAP-based assays in a 96-well microplate format.The primary benefits of the Bio-Plex Protein Array System, other than up to a 100-fold increase in data, include significantly reduced sample requirements.

The Cytometric Bead Array (CBA) system from BD Biosciences of San Diego is flexible in that it accommodates multiple sizes and fluorescent intensities of particles. The system includes everything the researcher needs to implement this technology, including a cytometer setup kit with the requisite software, reagents and standards. The company's CBA assay kits employ their proprietary bead sets, which are internally dyed with varying intensities of a proprietary fluorophore. These sets are distinguished via one fluorescence parameter and two size discriminators. However, the system is also capable of handling assays based on the use of other types of spectrally distinct microsphere sets. The CBA analysis software is an "add-in" for Microsoft Excel®, and is compatible with contemporary data acquisition software such as CellQuest™. Researchers can employ a variety of preset configurations for generating standard dilution series and calibration curves, and data reports can be generated at each step in the process. (iv) "Antibody Chip" array technology.

The array format has revolutionised biomedical experimentation and diagnostics, enabling ordered high-throughput analysis. During the past decade, classic solid phase substrates, such as microtitre plates, membrane filters and microscopic slides, have been turned into high-density, chip-like structures.

Protein array technology allows high throughput screening for gene expression and molecular interactions. Protein arrays appear as new and versatile tools in functional genomics, enabling the translation of gene expression patterns of normal and diseased tissues into protein product. Protein function, such as enzyme activity, antibody specificity, and other ligand-receptor interactions and binding of nucleic acids or small molecules can be analysed on a whole genome level.

As the array technology develops, an ever increasing variety of formats become available (eg nanoplates, patterned arrays, three-dimensional pads, flat-surface spot arrays, microfluidic chips), and proteins can be arrayed onto different surfaces (e.g, membrane filters, polystyrene film, glass, silane, gold). Various techniques are being developed for producing arrays. The emerging future array systems will be used for high-throughput functional annotation of gene products.

Protein microarrays are particularly useful in molecular diagnostics The concept of the array library was central to this development which now extends from DNA to protein. Similar to the gene chip arrays measuring mRNA levels on a genome wide scale, the protein product of expressed cells can be used for the simultaneous assessment of protein levels on a proteome wide scale. Additionally, protein specific antibodies can be arrayed to produce "antibody chip arrays" (Cahill D., 2001, J. Imm. Meth. 250, 81-91). The availability of such antibody chip arrays can be used to simultaneously analyse numerous interactions within a single sample. The "antibody chip" can be used to demonstrate antibody-protein interactions by incubating the chip with target proteins which have been labelled with a traceable marker (ProteinChip, Ciphergen Biosystems, Fremont, CA, USA; BIAcore chips, Biacore, Upsala, Sweden) or by incubating the chip with protein molecules or fragments thereof and detecting association between antibody and protein molecule or fragment thereof using ELISA type assays (Caliper Technologies, Mountain View, CA, USA; Orchid Biocomputer Inc., Princeton, NJ, USA). It should be noted that sCDs according to the invention may be found complexed with a ligand and thus chip based techology described herein may be used to measure/demonstrate interactions of ligand bound sCD with other molecules.

Techniques for preparing antibody arrays are described below:
The antibodies may be covalently linked to a suitable membrane such as an Immobilon P membrane (PVDF; Millipore Corporation) Subsequent blocking with an excess of a protein solution such as a skim milk preparation is preferred. A blocking agent is designed to eliminate non-specific binding on the binding surface Other suitable blocking agents are Irish moss extract or other source of carrageenan or gelatin. The antibodies are also adsorbed to a nitrocellulose film on a glass microscope slide (Schleicher and Schuell, NH, USA) and the unbound nitrocellulose is then blocked with skim milk. Antibodies are also adsorbed to Nylon membranes. To increase the accessibility of bound anti- CD antibodies to antigens on cells, the solid support used for the array is initially coated with a recombinant, truncated form of Protein G from Streptococcus which retains its affinity for the Fc portion of IgG lacks albumin and Fab binding sites, and membrane-binding regions (Goward et al., 1990). Antibodies are applied to this coat of Protein G and bind via their domains leaving the Fab domains free to interact with cells. The Fab domains are also further from the solid support providing greater accessibility of CD antigens on cell membranes to antibodies.

The array of antibodies is also constructed on a membrane or a coverslip. In this case, the antibodies are covalently linked to the membrane as duplicate spots in a two dimensional matrix. The spots are arranged in a matrix such as but not limited to a 15x 15 matrix.

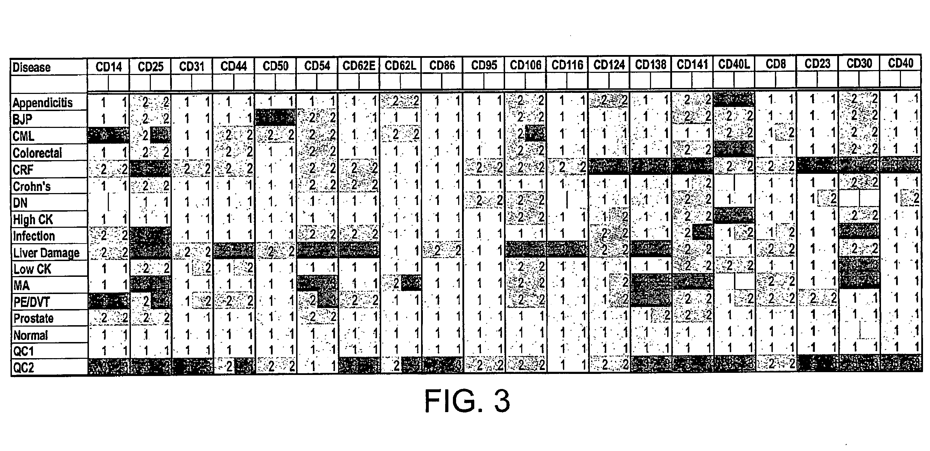

The antibodies are advantageously monoclonal and are specific for the cluster of differentiation (cluster designation) antigens (CD antigens). Details of CD antigens are available at http://www.ncbi.nlm.nih.gov/prow/cd/index molecule.htm. The spots are of microscopic size and are produced by the application of a drop (-10 nanolitres) of antibody solution (e.g. 10/.tg protein/ml) on designated portions of a membrane or glass surface such as a coverslip, first washed with a non-specific protein absorbent such as 30% w/v skim milk (Dutch Jug, Bonlac Foods Ltd, Melbourne, Australia) and then rinsed. Other protein solutions and other brands of skim milk may also be employed. The antibodies may be covalently coupled to the solid support such as through amino groups of lysine residues, the carboxylate groups of aspartate or glutamic acid residues or the sulthydryl groups of cysteine residues. The array of antibodies selectively binds cells from body fluids which express the respective antigens or may bind free antigens. A positive and/or negative control is included such as an antibody for surface molecules or soluble molecules known to be present in the sample. An example of one form of the assay device is shown in Fig. 3. The solid support is conveniently of similar size and shape to a microscope slide and may be constructed of glass or other polymeric material.

A wall around the microscope slide may be separately added or moulded with the slide and this facilitates retention of fluid material. The present invention extends to any other device capable of fulfilling the method of the present invention.

In the case of nitrocellulose based antibody arrays are preferably constructed using a Biodot Aspirate and Dispense System (Cartesian Technologies) where 5 nL dots are applied to a nitrocellulose film, on glass microscope slides (Schleicher and Schuell, Cat. No. 10484182). Purified monoclonal antibodies (Beckman Coulter, Becton Dickinson or Biosource International) are used at concentrations recommended for flow cytometric analysis and are applied in the same buffers as supplied by the manufacturers. The nitrocellulose is then blocked by incubation with 5 % w/v skim milk (Dutch Jug) for 1.5 h at 37'C. These blocking conditions are chosen to minimize background binding.

The stability of the arrays is further enhanced by adding protein stabilizing agents to the antibodies (e.g. polyethylene glycol or stabilizer products commercially available from Surmodics, MN, USA).

The following description provides a preferred method for preparing the antibody arrays: The panel of antibodies is generally used to construct antibody arrays with the Cartesian Technologies PixSysTM 3200 Aspirate and Dispense System. The antibodies are chosen for use in a particular diagnosis or detection protocol. Each antibody is generally applied in the volume of from about 1 to about 10 nanoliters in a dot format at approximately from 0.5 to 1.5 min intervals to create an appropriate array on a nitrocellulose film generally laid on a solid support such as but not limited to a microscope slide, plastic or gold support. After dotting, the supports are assessed on a light box and the corners of the arrays marked gently using, for example, a lead pencil. The antibody arrays are then immersed in a blocking agent such as but not limited to skim milk, Irish moss extract or other source of carrageenan or gelatin. From about 2 % to about 15 % w/v skim milk powder in PBS at 4'C overnight or at 37'C for from about 60-120 minutes is particularly useful. After application of the blocking agent, the solid supports are washed gently with purified water and allowed to dry at room temperature for a period of time from about 60-120 minutes.

The solid supports are then stored in an airtight bag at 4'C in the dark

An alternative method of detection is the use of acoustic detection based methods, such as those described by Akubio Technology. (DDT vol 7, No 5 (Suppl.) 2002). This form of detection technology is based on the sound made as molecular interactions are disrupted. It does not use any form of electromagnetic radiation. Very high accelerations, millions of times the force of gravity are used to disrupt such interactions. Such forces are generated by an acoustic wave device such as a quartz crystal resonator. By monitoring the change in resonant frequency of the crystal , which occurs on adsorption of mass to the surface, quartz crystal resonators can be used together with appropriate surface chemistry and fluidics to detect the adsorption of proteins, oligonucleotides, cells and other particles to surface-bound receptors. This allows the label-free determination of interaction affinities and kinetics in real time. (Janshoff A et al (2000) Ptiezoelectric mass-sensing devices as biosensors. An alternative to optical biosensors. Angew. Chem, 39, 4004-4032); Ward et al, (1990) In situ interfacial mass detection with piezoelectric transducers, Science 249, 1000-1007).

### (D) A sCD fingerprint for one or more disease states according to the present invention

Advantageously, levels of sCDs are measured in diseased individuals and absolute values may be divided by the upper limit of normal (ULN) obtained from healthy individuals. The data is collated and the resultant pattern of values obtained for each sCD for one or more given disease states, from one or more individuals forms the basis of a sCD fingerprint of one or more given disease states.

The statistical significance of the increases or decreases in sCD levels found in various disease states can be assessed using a number of methods.

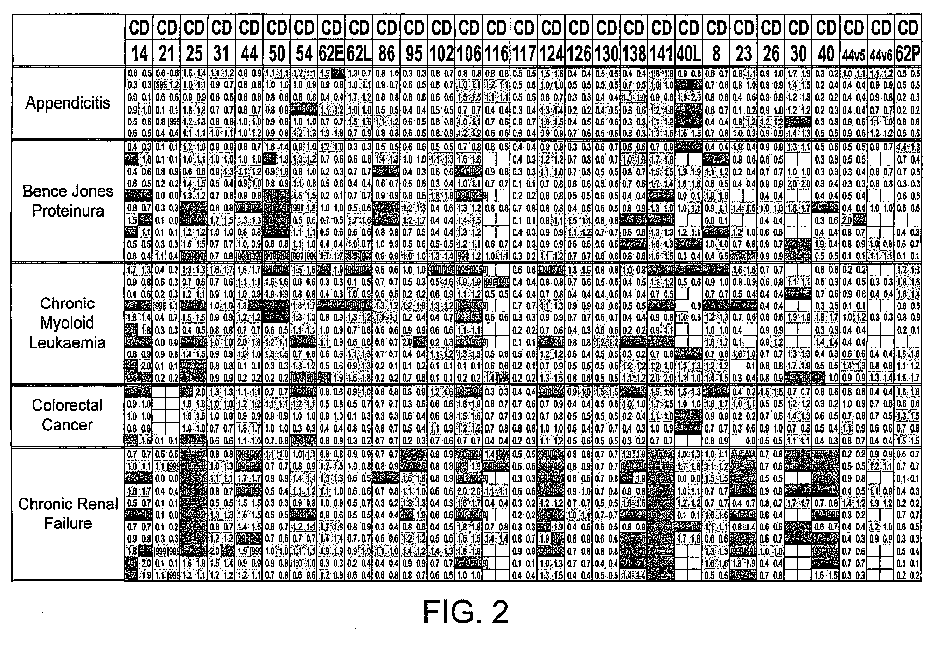

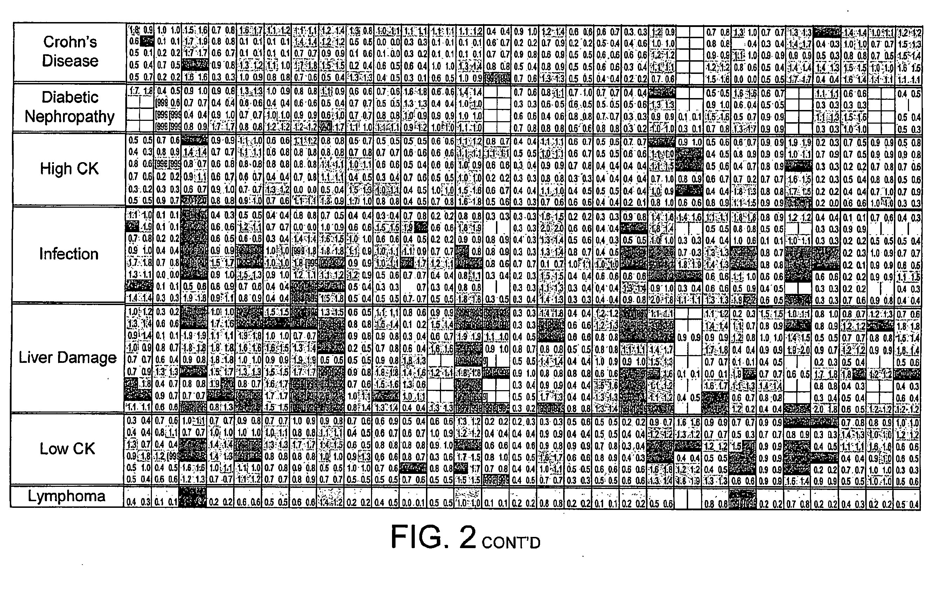

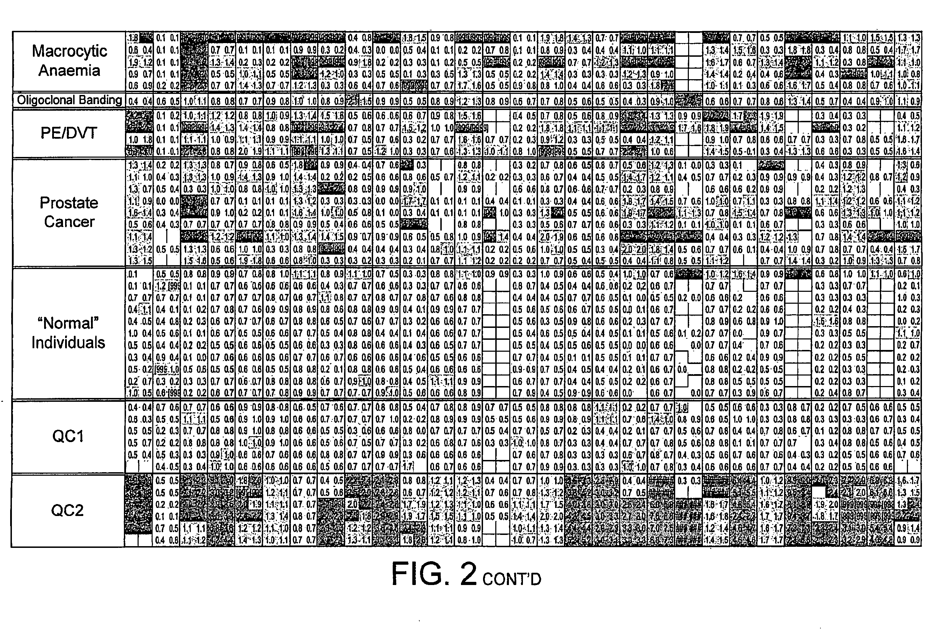

The sCD fingerprint can advantageously be simplified by removing those sCDs whose levels do not generally change significantly during one or more given disease states. Examples of such sCDs include but are not limited to CD21, CD102, CD117, CD 126, CD130, CD 26, CD44v5, CDv6, CD62P. For example see Figure 2.

To simplify the fingerprint further, instead of showing the data from each individual for any given sCD during a disease, a modal value for each sCD calculated from the group of individuals may be plotted. The rational of this is demonstrated in Figures 3, 5, 6, 7, 8. This provides an easily readable and simple 'fingerprint' of a disease.

One skilled in the art will appreciate that there are many methods suitable for the statistical analysis of the sCD level data measured as herein described. These include but are not limited to cluster analysis and other statistical methods for the detection of patterns.

### Statistical analysis

Methods for the statistical analysis of data are known to those skilled in the art. An example of a suitable protocol for the analysis of data is outlined below:
(i) The statistical analysis procedures described here relate to immunoprecipitation assays of soluble CD molecules in samples arrayed in 96-well microtitre trays.

Statistical analysis of the proposed antibody-chip arrays could essentially follow the same procedures, although modifications would inevitably need to be made to account for experimental design and data quality issues specific to that technology.

In the present analysis, each tray was designed to measure the concentration of a specific soluble CD molecule in a number of samples. Trays were prepared for each of 29 soluble CD molecules, for each of three sets of 37 patients. In all, the three sets of patients represented 17 disparate disease groups, including one group of healthy individuals. The 96 wells on each tray contained: sera from each of the 37 patients of one set (duplicated); a standard control preparation containing the target antigen at each of 7 different concentrations (duplicated); pooled sera from 'normal' control patients (replicated 3 times); and the same pooled sera spiked with the target antigen (replicated 3 times).

Each tray was washed with a solution containing fluorescently labelled monoclonal antibody specific to its designated target antigen, and exposed to light. For each well, a measurement of light absorption was recorded.

### (ii) Statistical analysis using artificial intelligence type neural networking

This method describes the ability of the computer system which analyses the data to learn to recognise patterns in data. Thus the more the system is used, the better able the system is to recognise patterns. Such a system is described in amongst other documents Sven Olsson et al (2002), Clin Physiol & Func Im (2002) 22, pp295-299; Sijo Perekattil et al (March 2003), Journal of Virology, vol 169, pg 917-920. This approach is reviewed in PJG Lisboa, (2002), Neural Networks 15, 11-39. All of these documents are herein incorporated by reference.

### Data preparation

The recorded light absorptions were mathematically transformed, as follows.
⑩ Absorptions were converted to antigen concentrations, via a calibration curve based on the absorptions recorded for the antigen standards in the same tray. Absorptions of dubious quality were flagged *missing,* and omitted from subsequent analysis.
⑩ Concentrations exceeding that of the most concentrated antigen standard of the tray were flagged as *high.* Concentrations corresponding to absorptions at the limit of instrumentation were similarly flagged.
⑩ Each flagged concentration was replaced by the concentration of its duplicate in the tray, provided the duplicate concentration was not also flagged.
⑩ The average of each concentration with its duplicate was computed. We refer to these as *mean concentrations.*
⑩ For each CD, the 10the and 90th centiles of the distribution of mean concentrations in normal individuals was computed. We refer to these as CD₁₀ and CD₉₀. Subtracting CD₁₀ from CD₉₀ gives a range, which we denote CD_{range}. *Concentration categories* were defined for each CD as follows:
⑩

| **Category** | **from** | **to** |
|---|---|---|
| -1 | 0 | CD₁₀ |
| 0 | CD₁₀ | CD₉₀ |
| 1 | CD₉₀ | CD₉₀ + CD_{range} |
| 2 | CD₉₀ + CD_{range} | CD₉₀ + 2 x CD_{range} |
| 3 | CD₉₀ + 2 x CD_{range} | CD₉₀ + 3 x CD_{range} |
| 4 | CD₉₀ + 3 x CD_{range} | - |

⑩ To control for differences in the scale of reactivity of different CDs, to stabilise variability between duplicates across the range of concentrations, and to relate concentrations to values obtained for normal individuals, each mean concentration was put into the appropriate concentration category of its CD. All concentrations flagged *high* were put into the highest category. We refer to these as *categorised concentrations.*
⑩

### Data analyses

### Cluster analysis

A cluster analysis was performed to identify clusters of CDs having similar patterns of reactivity across the patients, standards and controls. Likewise, a second cluster analysis was performed to identify clusters of patients with similar profiles of reactivity across the CDs. Cluster analyses were hierarchical and based on the categorised concentrations, described above, using a Euclidean distance metric and the average linkage criterion for cluster merging.

The results from the cluster analyses were displayed in the form of a level plot, a part of which is given in **figure 16.** In this level plot, the rows correspond to CDs and the columns correspond to patients, standards and controls. Rows and columns are ordered so that those in the same cluster are adjacent. Also, dendrograms were produced showing the hierarchy of clusters, separately for the clustering of CDs and for the clustering of patients, standards and controls. Each cell in the level plot is toned to indicate its categorised concentration, deep blue corresponding to Category -1 (below normal), and deep red corresponding to Category 4 (high). Missing concentrations are denoted by an 'X'.

### A sCD fingerprint database according to the invention

In a further aspect still, the present invention provides a sCD reference database comprising pathological and/or normal sCD fingerprint patterns.

As herein described the term 'a database' refers to a collection of sCD fingerprints from normal 'non-diseased' and/or diseased individuals. Advantageously, the database is computer generated and/or stored. Advantageously the data from more than 5 individuals is present in the database. More advantageously the data from more than 10, 100, or 1000 individuals comprises the database. More advantageously still the data from more than 10,000 or more than 50,000 individuals comprises the database. Most advantageously the data from more than 100,000 individuals comprises the database.

Advantageously the database, in addition to sCD data also comprises clinical information relating to various patients and/or disease conditions. Alternatively or in addition, a database according to the present invention comprises genomic information and/or sCD profiles relating to specific disease states and other pathological states and clinical data. Thus the inventors contemplate the use of a database in which sCD data and other data may be integrated and used to obtain a more complete analysis of one or more disease states.

### Uses of one or more 'fingerprints of disease' according to the present invention

In a further aspect, the present invention provides a method for predicting the presence of one or more disease states in an individual comprising the step of analysing the sCD fingerprint in that individual.

In a further aspect still, the present invention provides a method for detecting the presence of one or more disease states in an individual comprising the step of analysing the pattern/s of shed CD levels of more than one shed CD which is present in that individual.

In a further aspect still, the present invention provides a method for detecting the extent of one or more disease states in an individual comprising the step of comparing one or more sCD fingerprint/s generated from that individual with one or more reference sCD fingerprint/s.

In yet a further aspect, the present invention provides a method for assessing the progression of a disease state in an individual comprising the step of comparing the sCD fingerprint of an individual at two or more periods during the occurrence of the disease.

In a further aspect still, the present invention provides a method for assessing the effect of one or more agent/s on one or more disease states in an individual comprising the step of comparing a sCD fingerprint of an individual at two or more time periods.

In a further aspect, the data generated by the present invention is used to compile a reference database, comprising pathological and/or normal sCD fingerprints, against which the expression sCD pattern of any individual will be compared.

In a further aspect still, the present invention provides the use of a sCD fingerprint to assess the effect of one or more agents in an individual.

An embodiment of the present invention is its use as a tool for assessing the affect different diet and exercise regimes may have on human or other mammals.

Additionally, the present invention may be used to construct sub-categories of sCD fingerprint profiles, suitable for common therapeutic treatment.

### The therapeutic inhibition of the production of one or more sCDs according to the invention

Recent studies which have investigated the impaired expression of NKG2D and T-cell activation by tumour-derived soluble MHC ligands (Nature, vol 419, 17 October 2002). Studies have shown that tumours release large amounts of the MHC class I homologue MIC into the serum. Activation of the NKG2D receptor on noatural T cells is known to stimulate their ability to destroy tumours, but the high levels of tumour derived MIC seem to downregulate the NKG2D receptor and block the antitumour effect. (Nature, vol 419, 17th October 2002, p679, pg 734). These soluble forms of MHC are produced either enzymatic cleavage or by alternative splicing (Nature, vol 419, 17th October 2002, p679, pg 734).

It is apparent from the invention described herein that sCDs may be produced by alternative splicing (Woolfson and Milstein, PNAS Vol 91, pp 6683-6687), enzymatic cleavage or other mechanisms. The present inventors consider that sCD molecules may also bind to a ligand/receptor and thereby block down stream effects. Thus, the present inventors have realised that the blockage of the production of sCD molecules via the inhibition of any of the methods of sCD generation described herein, may be a therapeutically useful method for the prophylaxis or treatment of one or more diseases in particular tumourigenesis.

Thus in a further aspect still, the present invention provides a method for treating one or more diseases comprising the step of inhibiting the production of one or more sCDs within an individual.

In a further aspect still, the present invention provides the use of an inhibitor of the production of one or more sCDs in the preparation of a medicament for the treatment of disease.

According to the above aspect of the invention, the term 'an inhibitor of the production of one or more sCDs' refers to one or more agents which inhibit the production of a shed form of sCD as herein defined. In reducing the amount of shed molecule produced, the level of cell surface molecule should increase correspondingly. This should be advantageous to the cell. Advantageously, the inhibitor is a specific inhibitor of those one or more sCDs. Suitable inhibitors include alternative splicing inhibitors and/or enzymatic cleavage inhibitors. Advantageously, the inhibitor is an alternative splicing inhibitor. Such alternative splicing inhibitors are include for example inhibitors of exonic splicing enhancers (Fairbrother et al, Science, vol 297, 9th August 2002).

According to the above aspects of the invention, the production of any one or more sCDs present in the body fluid of an individual may be inhibited. Advantageously, the one or more sCDs are any one of those selected from the group consisting of the following: CD14, CD25, CD31, CD44, CD50, CD54, CD62E, CD62L, CD86, CD95, CD106, CD116, CD124, CD138, CD141, CD40L, CD8, CD23, CD30, CD40. More advantageously the sCD is CD1. Advantageously, the sCD is CD1 and the inhibitory agent is an alternative splicing inhibitor.

According to the above aspects of the invention, one or more inhibitory agents may be used for the prophylaxis or treatment of any one or more disease states selected from the group consisting of the following: infections, autoimmune disease, neoplastic, vascular endocrinological, metabolic, inflammatory degenerative, psychiatric psychological, traumatic, drug/toxin-related, bacterial, fungal, protozoan and viral infections, non-neoplastic disorders; pain; diabetes, obesity; anorexia; bulimia; asthma; pregnancy; endocrine; vascular; metabolic; gastro-intestinal; iatrogenic; psychiatric; psychoclogical; exercise-induced; diet-related; ME; degenerative; Parkinson's disease; thrombosis; atherosclerosis; acute heart failure; hypotension; hypertension; erectile dysfunction; urinary retention; metabolic bone diseases such as osteoporosis; angina pectoris; hepatitis; myocardial infarction; ulcers; allergies; rheumatoid arthritis; inflammatory bowel disease; irritable bowel syndrome benign prostatic hypertrophy; psychosis; psychiatric disorders; including anxiety; schizophrenia; manic depression; delirium; dementia; severe mental retardation and dyskinesias, such as Huntington's disease or Gilles de la Tourett's syndrome; and preferably tumours which can be benign or malignant cancers; breast cancer; myeloma; melanoma; bladder cancer; leukaemia; plasmocytoma and others, but most preferably appendicitis; Bence Jones Proteinuria; Chronic Myoeloid Leukaemia; Colorectal cancer; chronic renal failure; Crohn's Disease; Diabetic Nephropathy; Cardiac pathology; Infection; Liver damage; Lymphoma; macrocytic anaemia; Prostate Cancer; Oligoclonal Banding and PE/DVT. Advantageously, the disease is tumourigenesis.

The invention will now be described by the following examples which are in no way limiting of the invention.

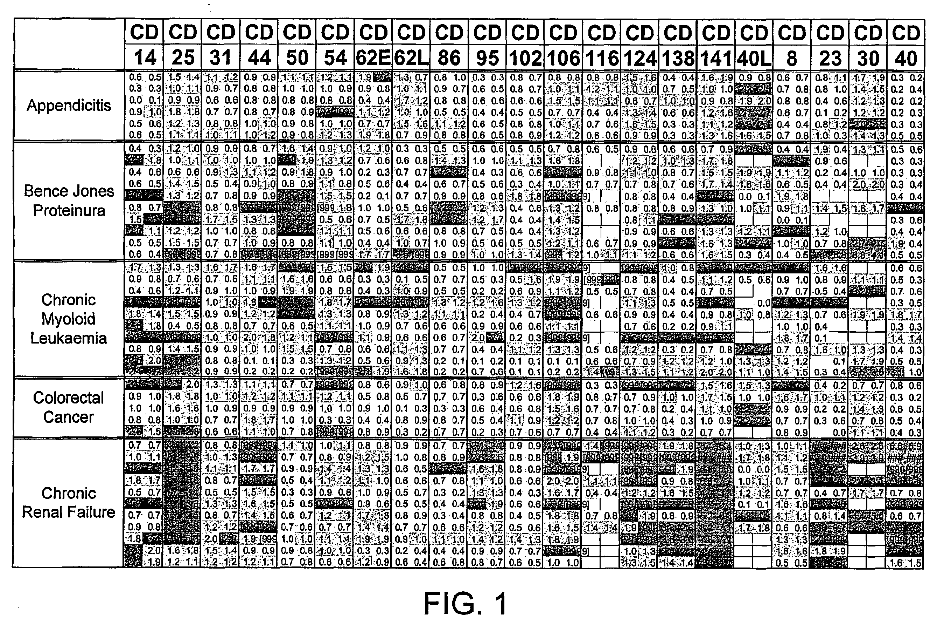

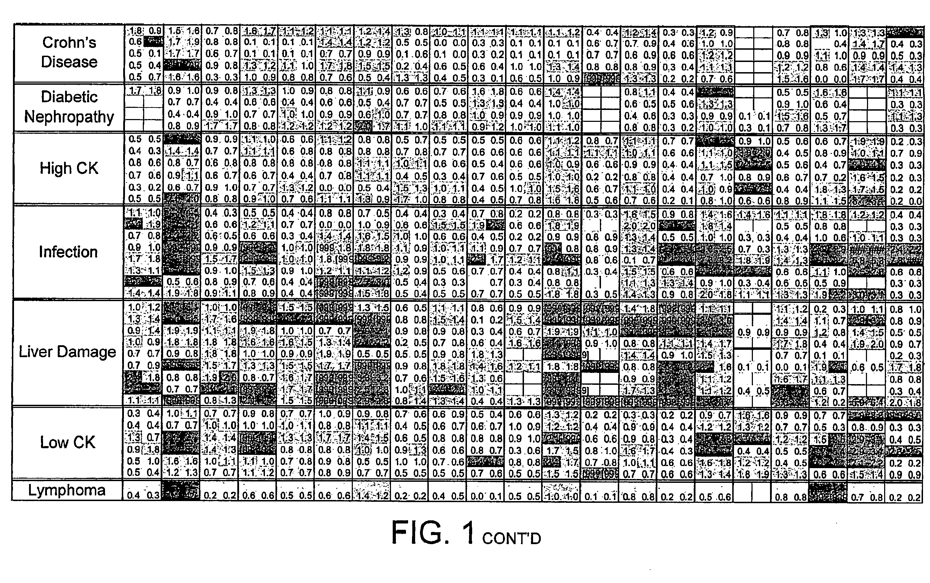

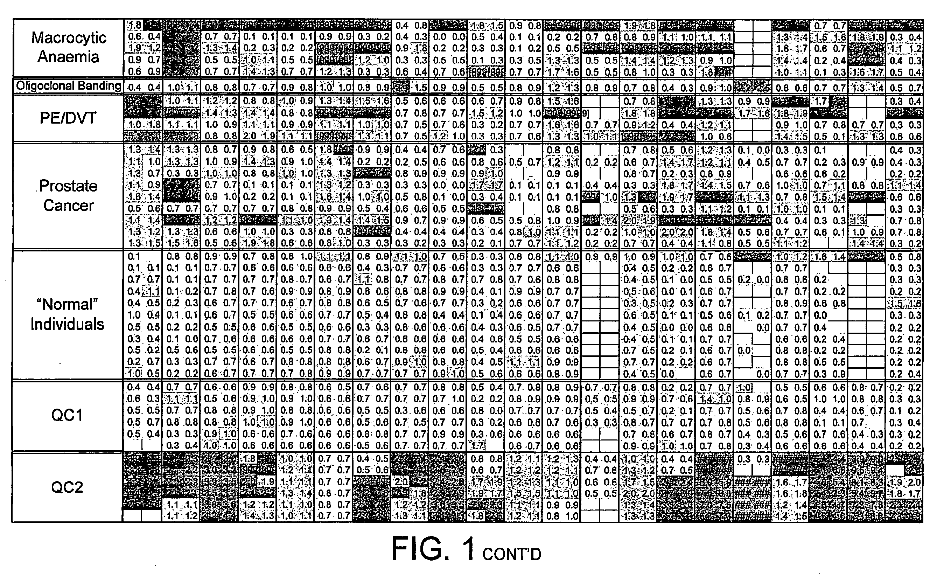

### Example 1. Figure 1. Disease Groups. Multiples of ULN All sCD's included

Two values obtained (CD40L and CD30) for the individual, classified as normal, with a suspected drug overdose were omitted from the calculation of the upper limit of normal. The dilution factor for each sCD was fixed throughout the study. The results obtained are those of the diluted sample and have not been multiplied by the dilution factor. The absolute value of each data point was divided by the upper limit of normal (ULN) as defined above. Where the absolute value was greater than the dynamic range of the assay the result [9999] was recorded.

The limits indicated by each point are:
- Green ≤ 1 x ULN
- Blue 1-2 x ULN
- Red >2 x ULN
- A white block indicates no data available.

### Example 2. Figure 2 Remove all sCD's that appear not to discriminate from the normals (sCD's 21; 102; 117; 126; 130; 26; 44v5; 44v6; 62P).

To simplify the diagram the above sCD plots were removed.

The data suggests (Figure 1.) that the concentration of some of these sCD may actually be lowered in disease. As we initially worked on the premise that there would be over-expression of these molecules in disease, samples have been diluted optimally to focus on high, rather than low concentrations.

### Example 3. Figure 3. Disease Groups. Mode of Response for Remaining 20 sCD's

To simplify the data further the modal response for each disease group has been plotted. As the lymphoma and "oligoclonal-banding positive" group contain only a single subject, they have been omitted. Where there is no clear mode, both responses have been shown.

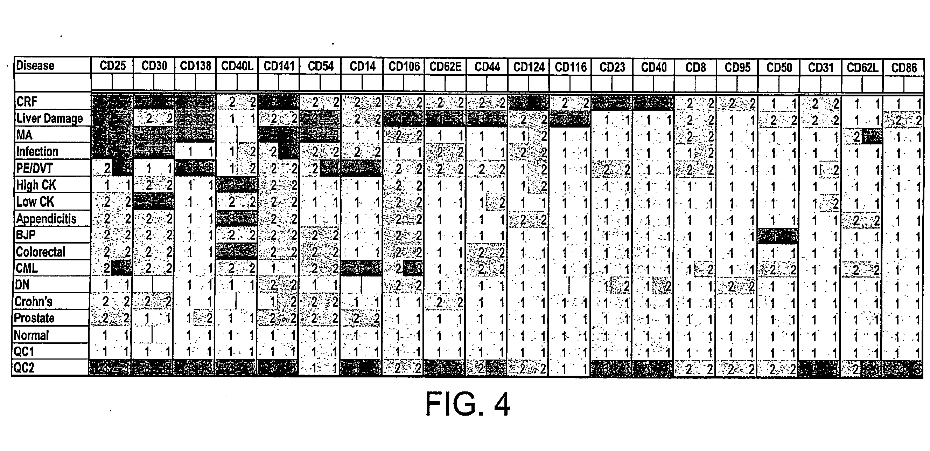

### Example 4. Figure 4. Disease Groups. Mode of Response for remaining sCD's

Data has been ranked in order of increased expression.

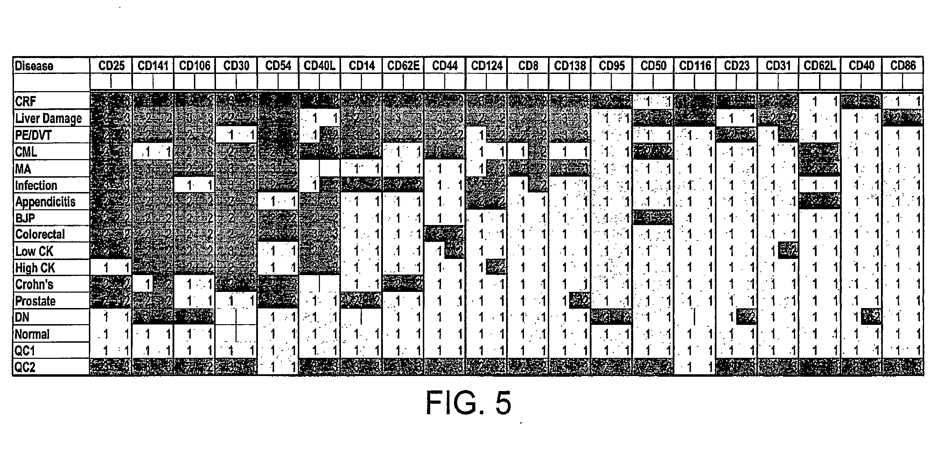

### Example 5. Figure 5. Disease Groups. Mode of Response for remaining sCD's

As for Figure 4. except responses have been classified as "normal" and "abnormal". Values >1 ULN have been classified as abnormal.

Both Figures 4 and 5 suggest that each disease state exhibit a unique pattern of elevated sCD expression.

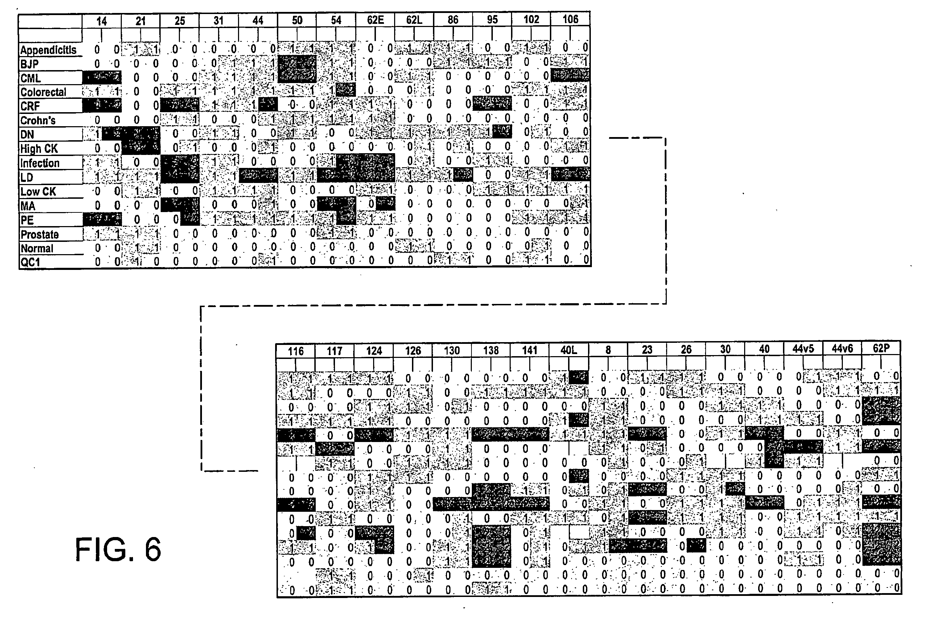

### Example 6. Figure 6. Disease Groups. Mode of Response for all sCD's

As for Figure 3 (except all sCD's are included).

The limits indicated by each point are:
- Green ≤ 1 x MoM
- Blue 1-2 x MoM
- Red > 2 x MoM
- A white block indicates no data available.

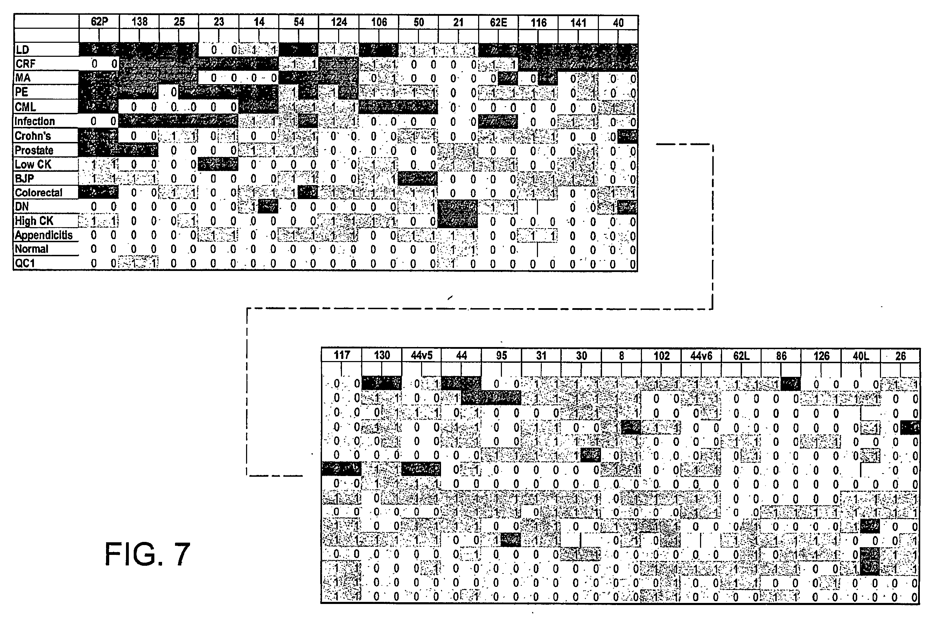

### Example 7. Figure 7. Disease Groups. Mode of Response for all sCD's

As for Figure 4.

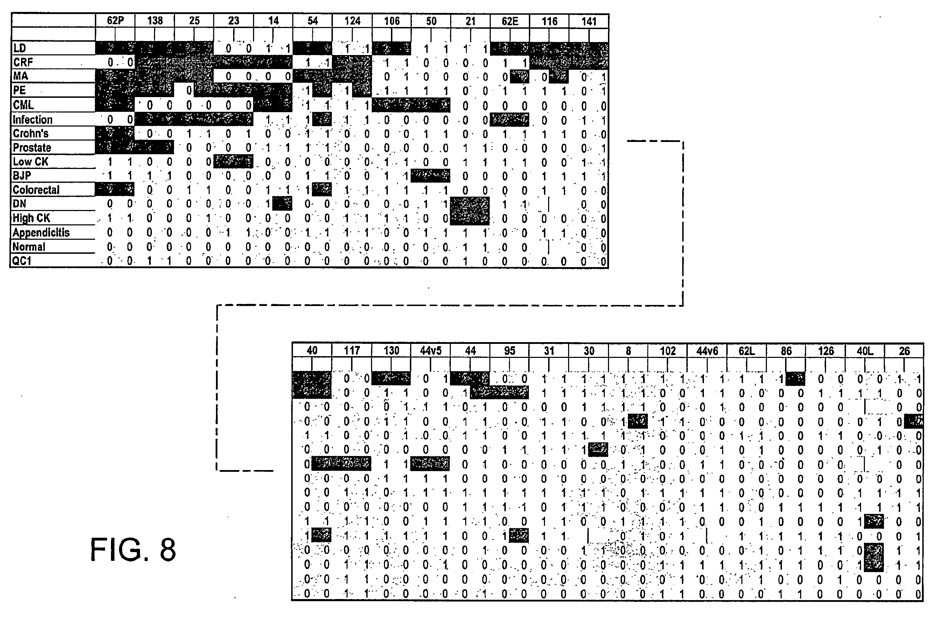

### Example 8. Figure 8. Disease Groups. Mode of Response for all sCD's

As for Figure 5. (except that values > 2 MoM have been classified as abnormal).

Comparison of figures 5 and 8 illustrate the importance of determining the cut-off threshold values in order to obtain a defined pattern.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry, molecular biology and biotechnology or related fields are intended to be within the scope of the following claims.

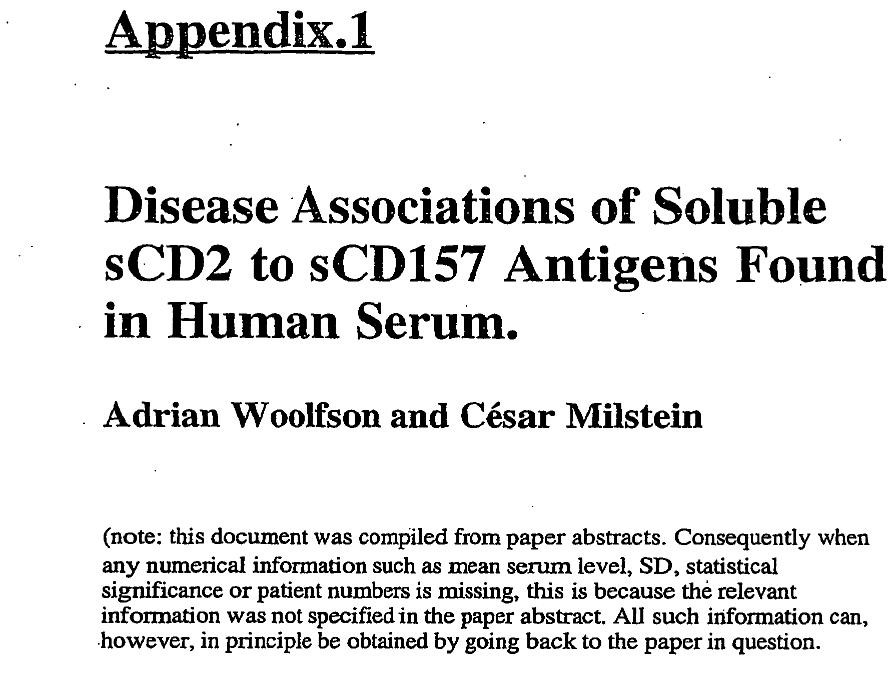

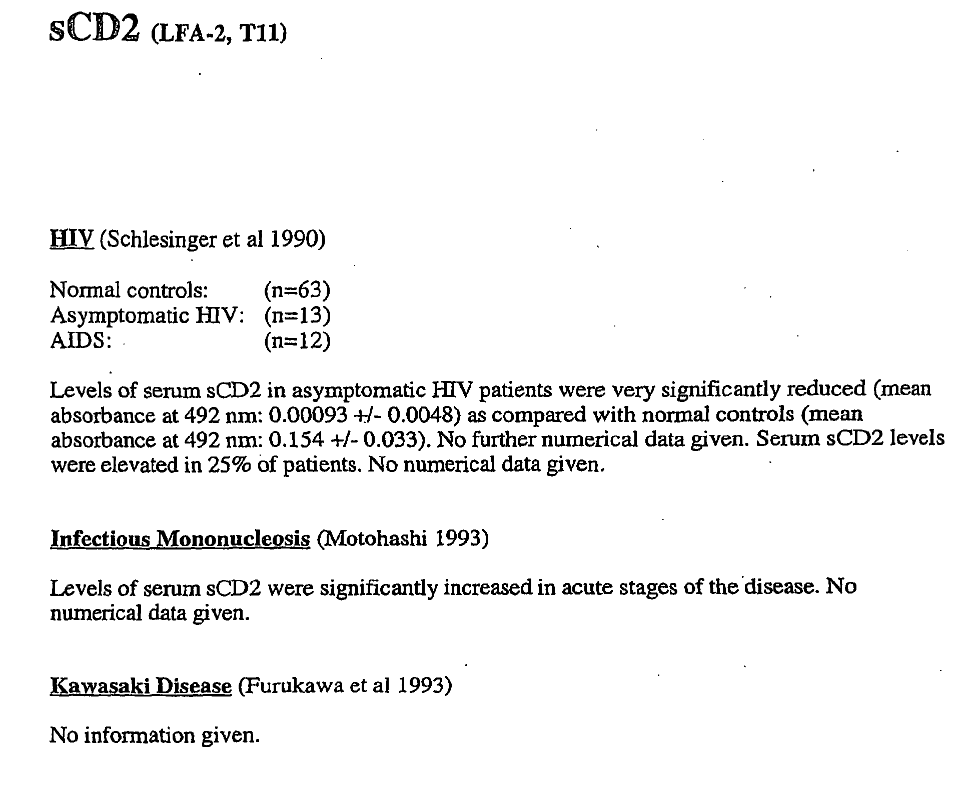

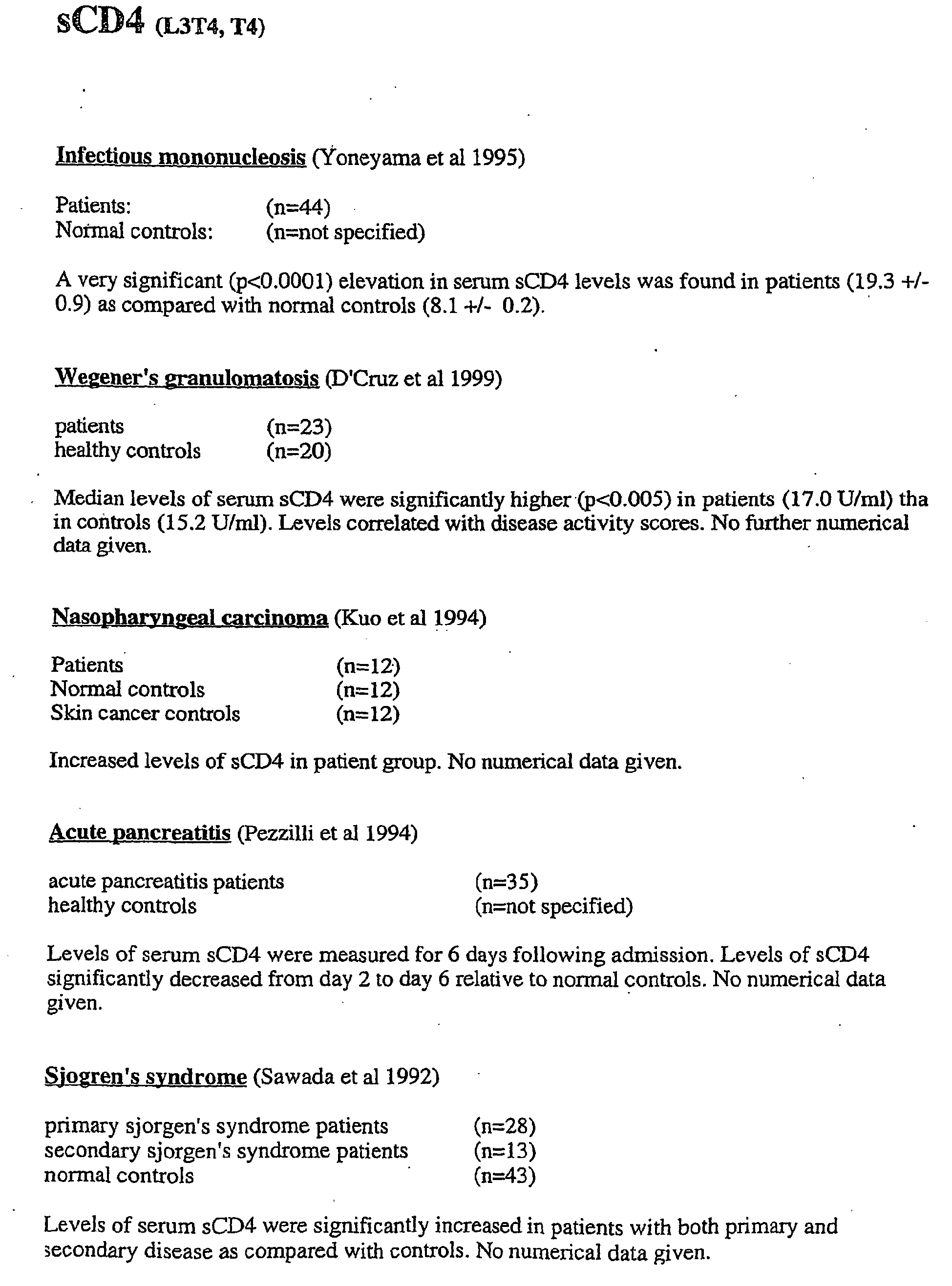

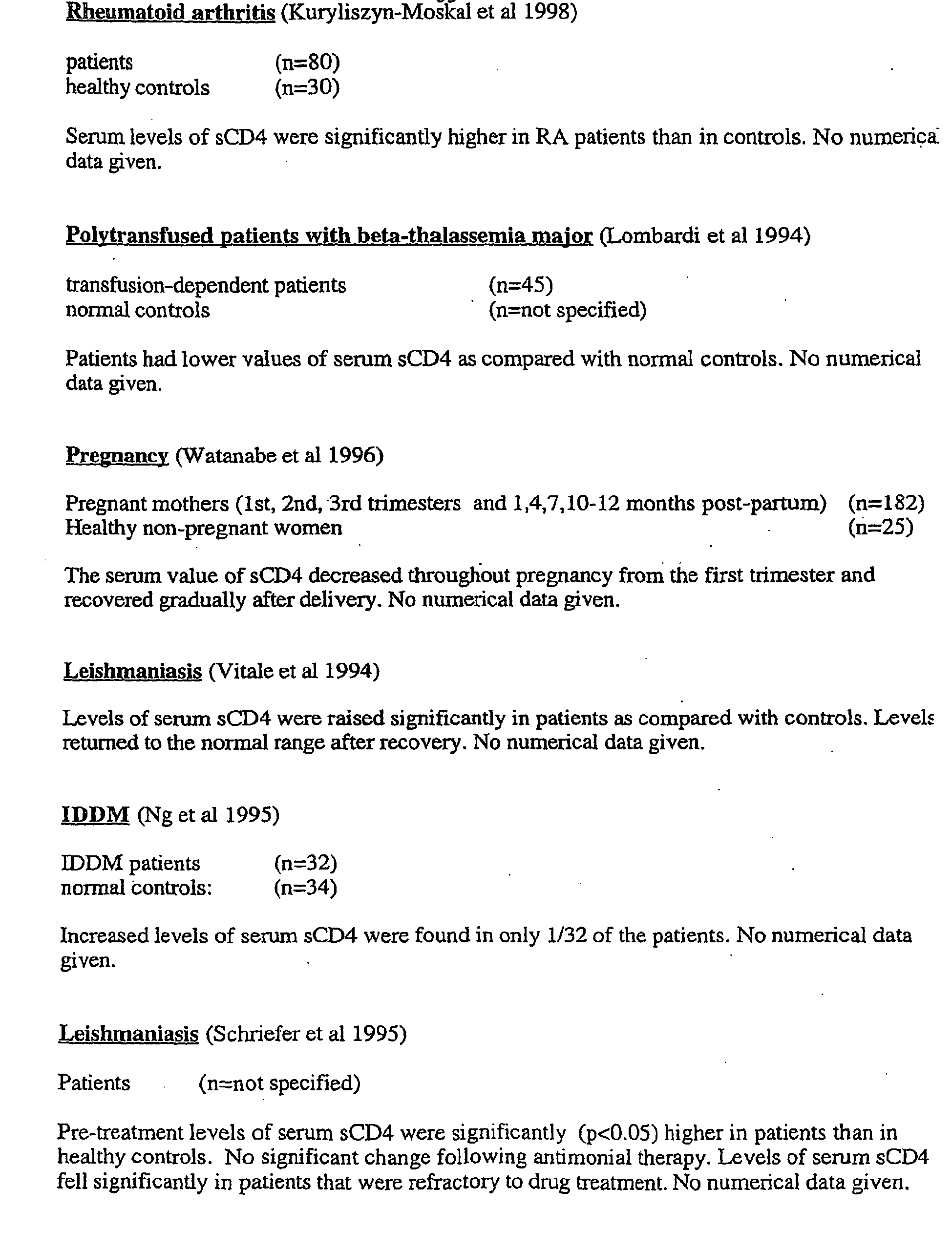

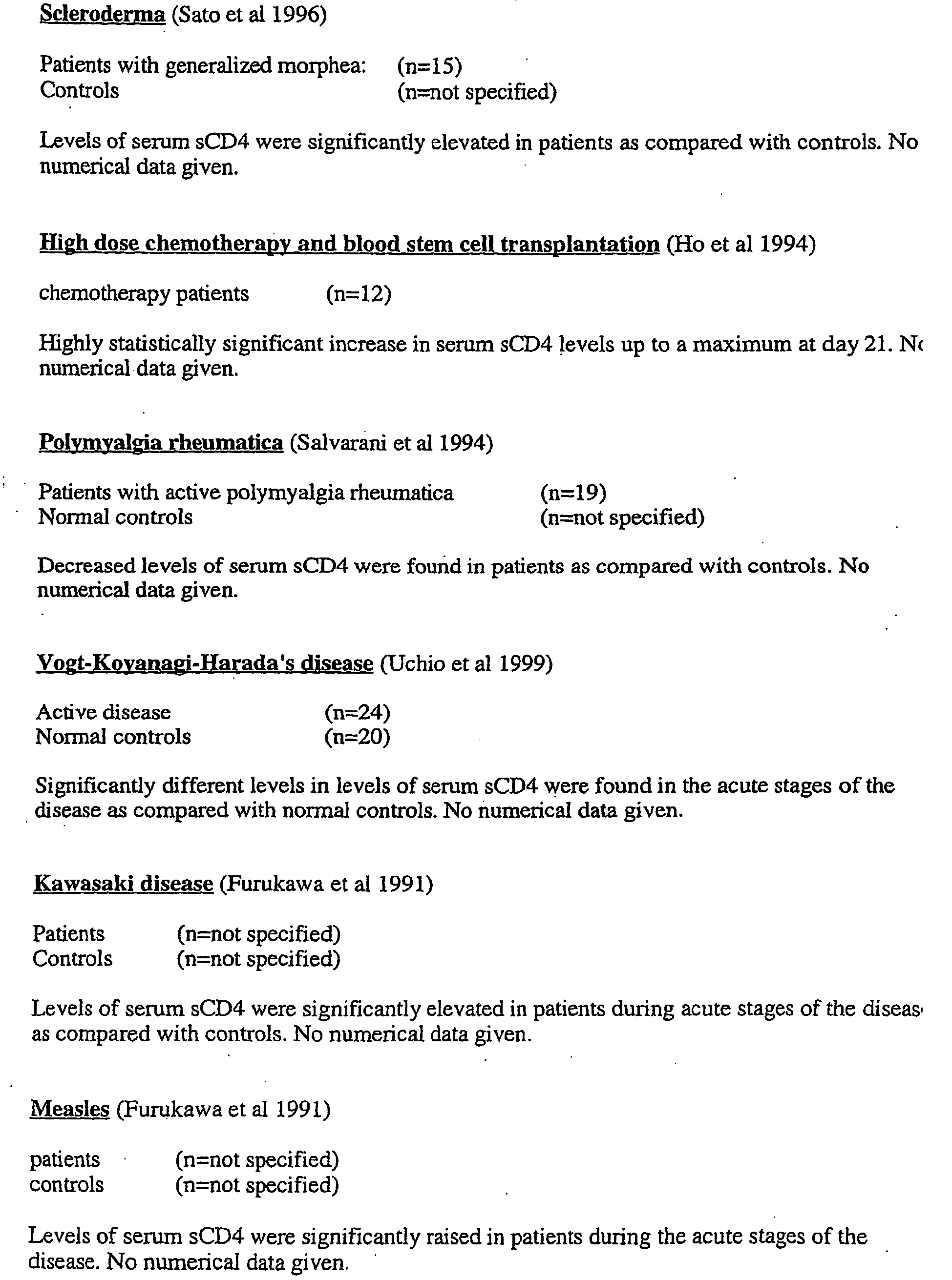

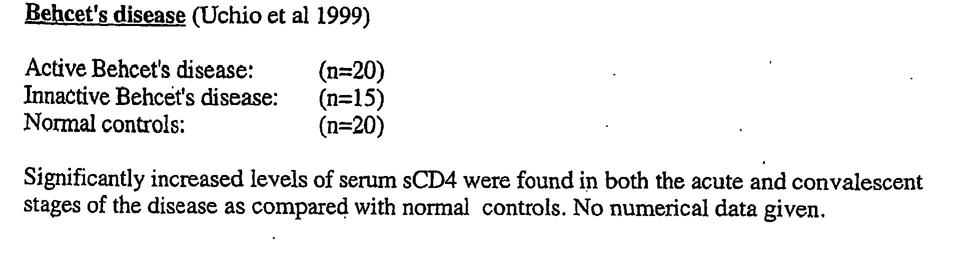

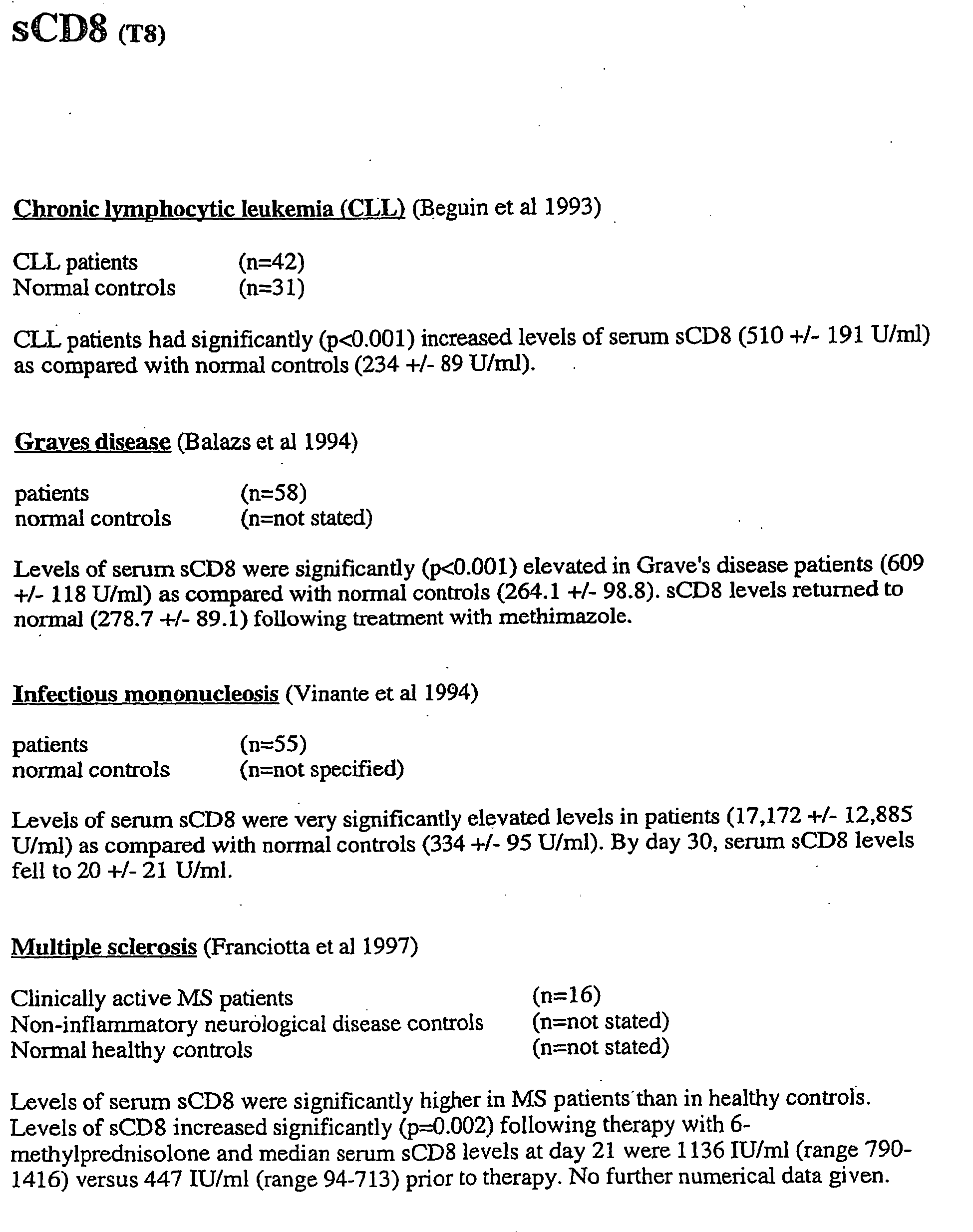

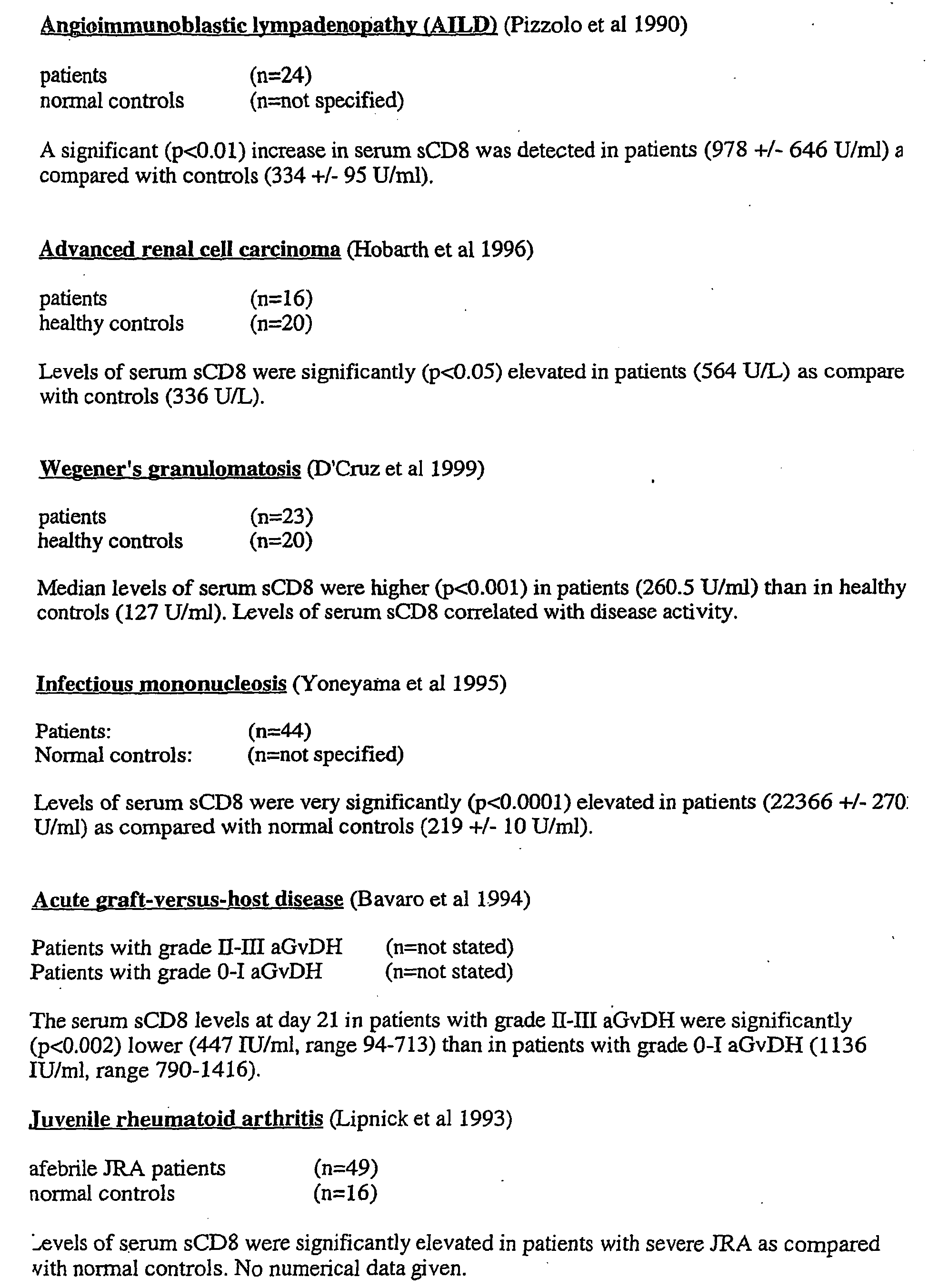

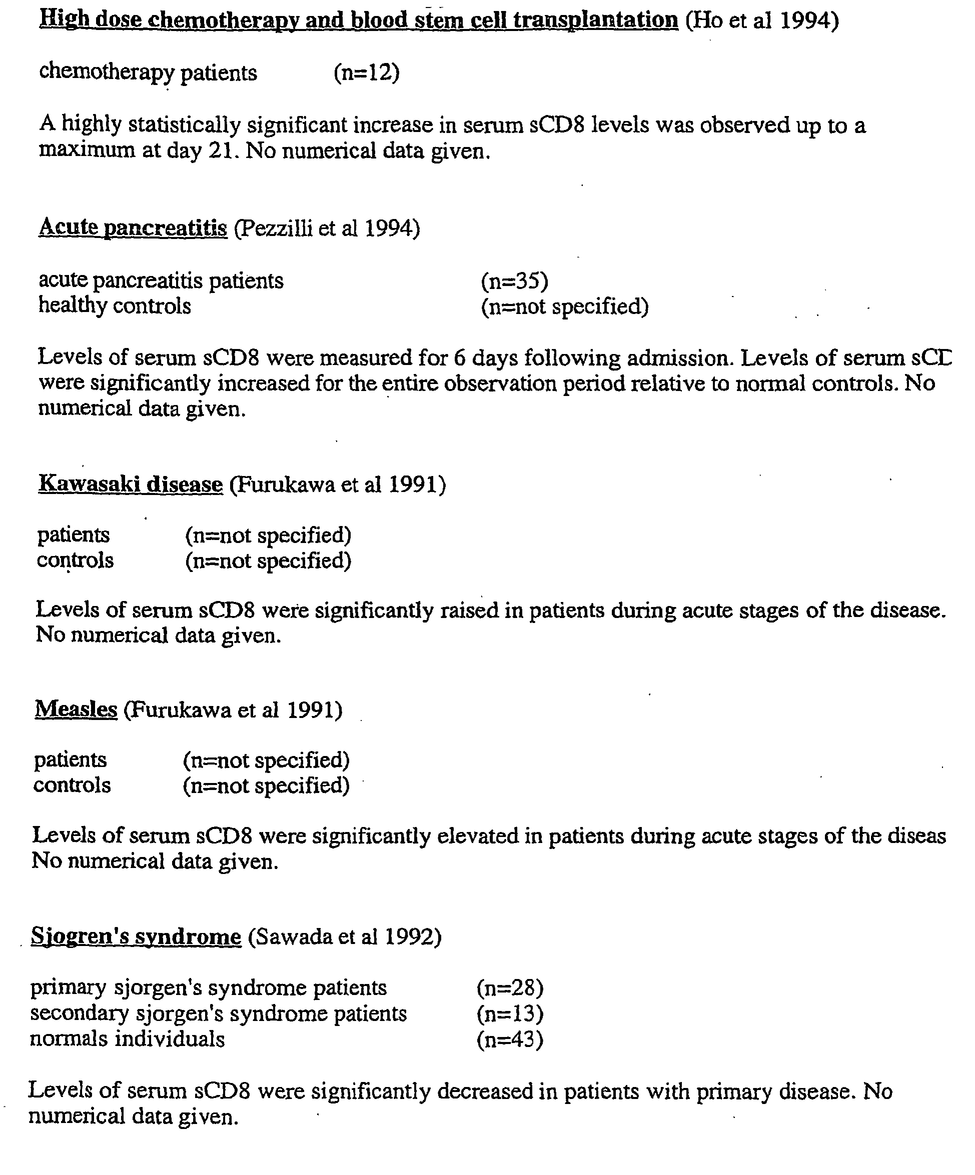

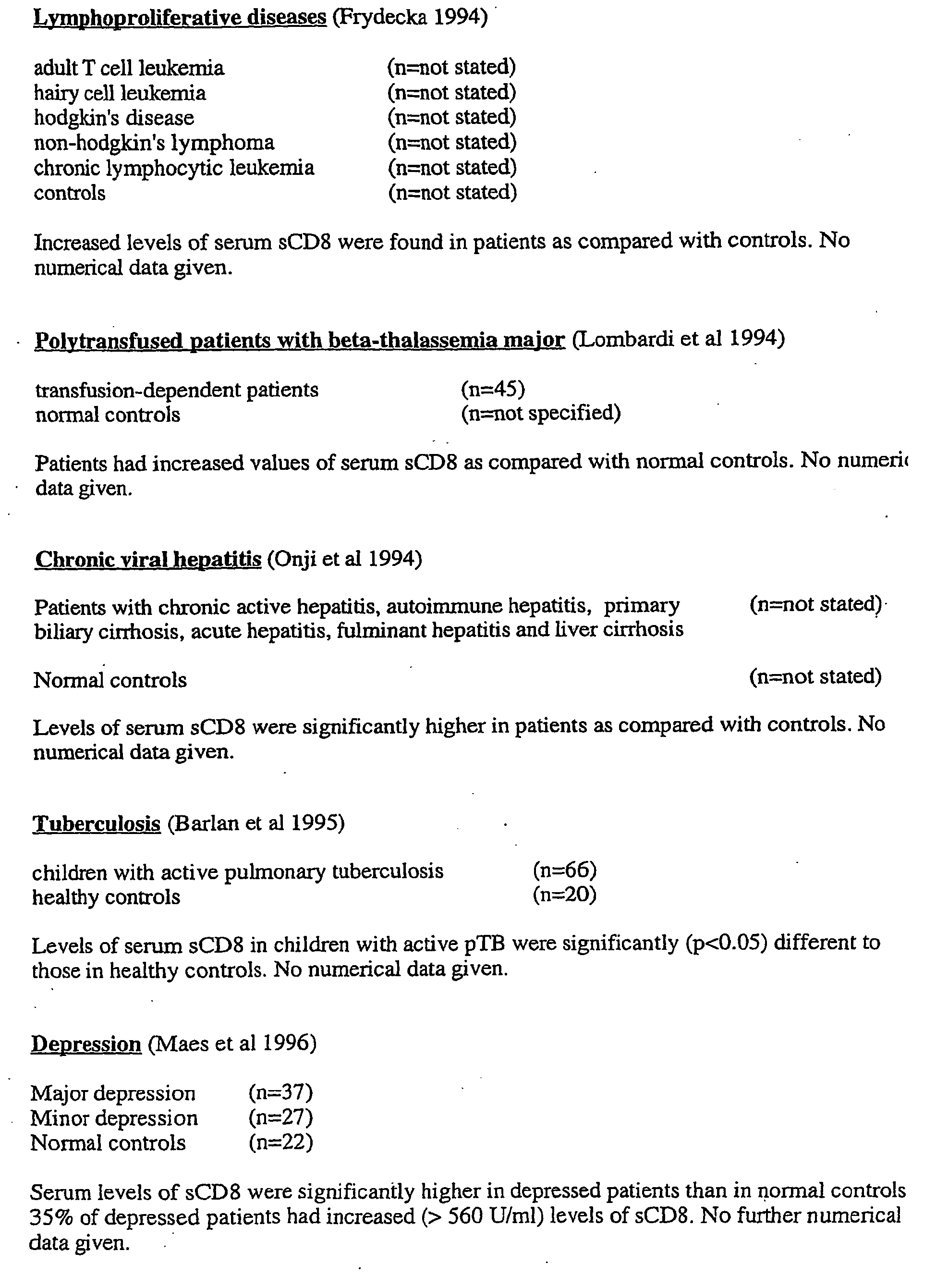

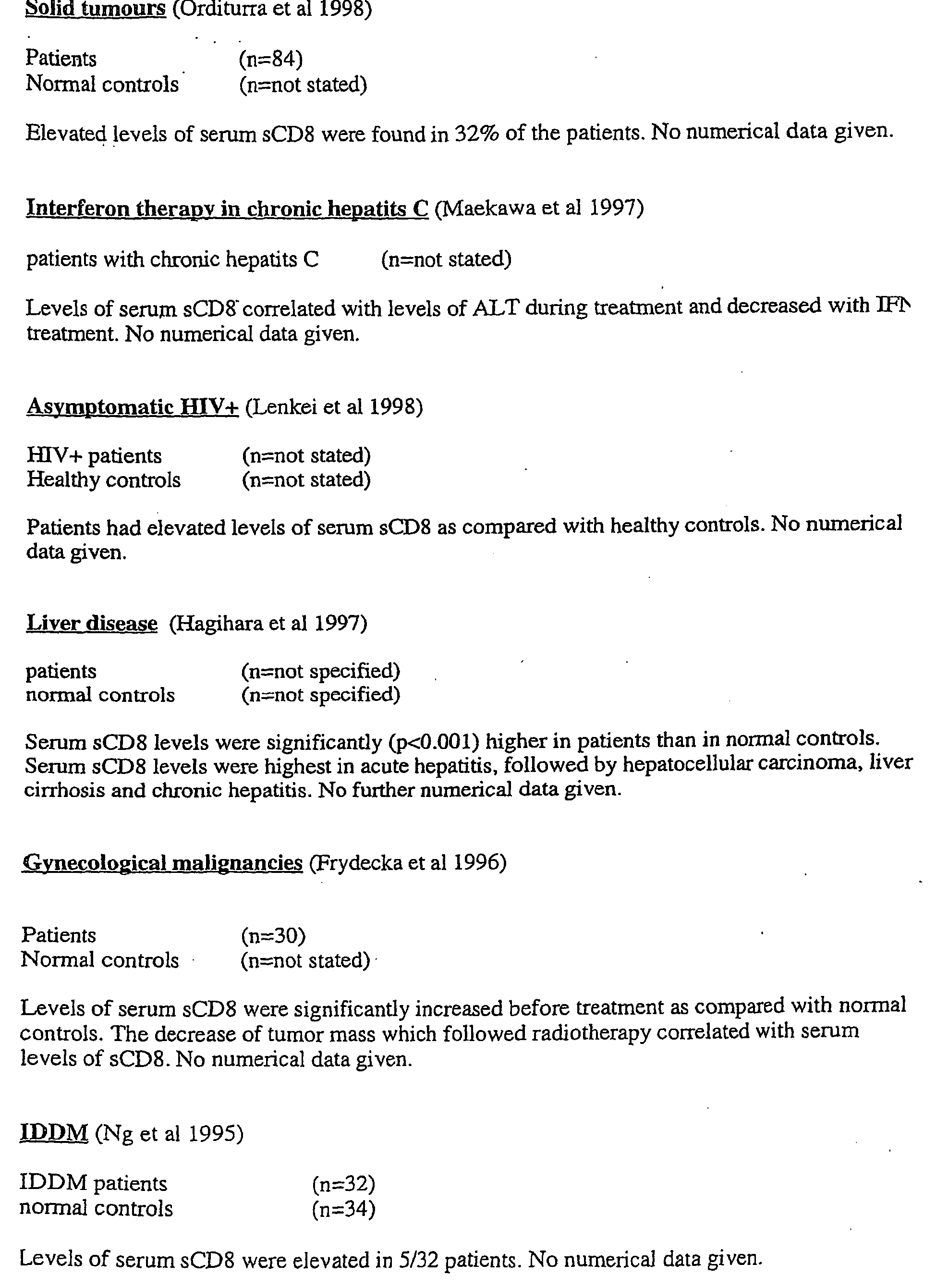

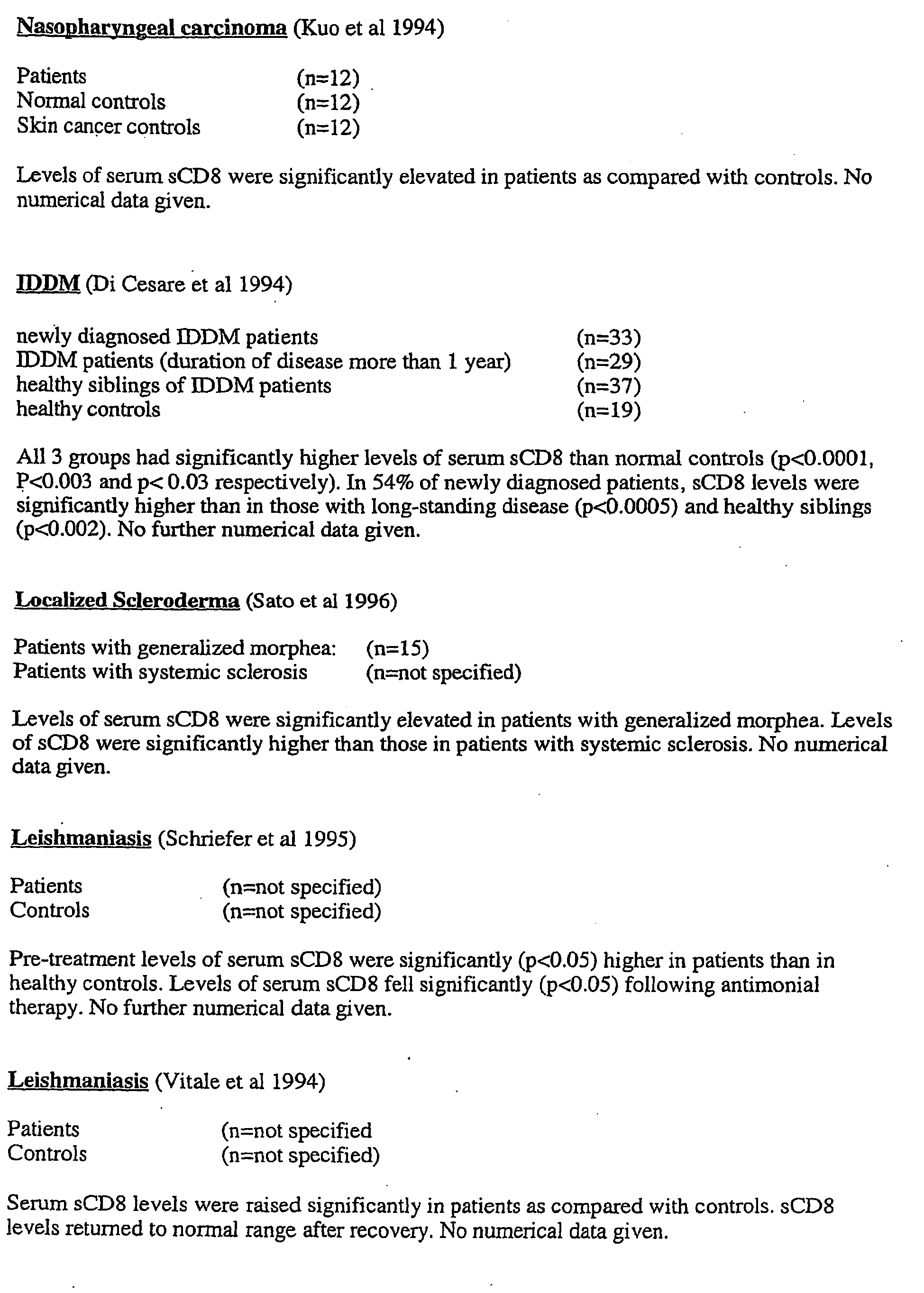

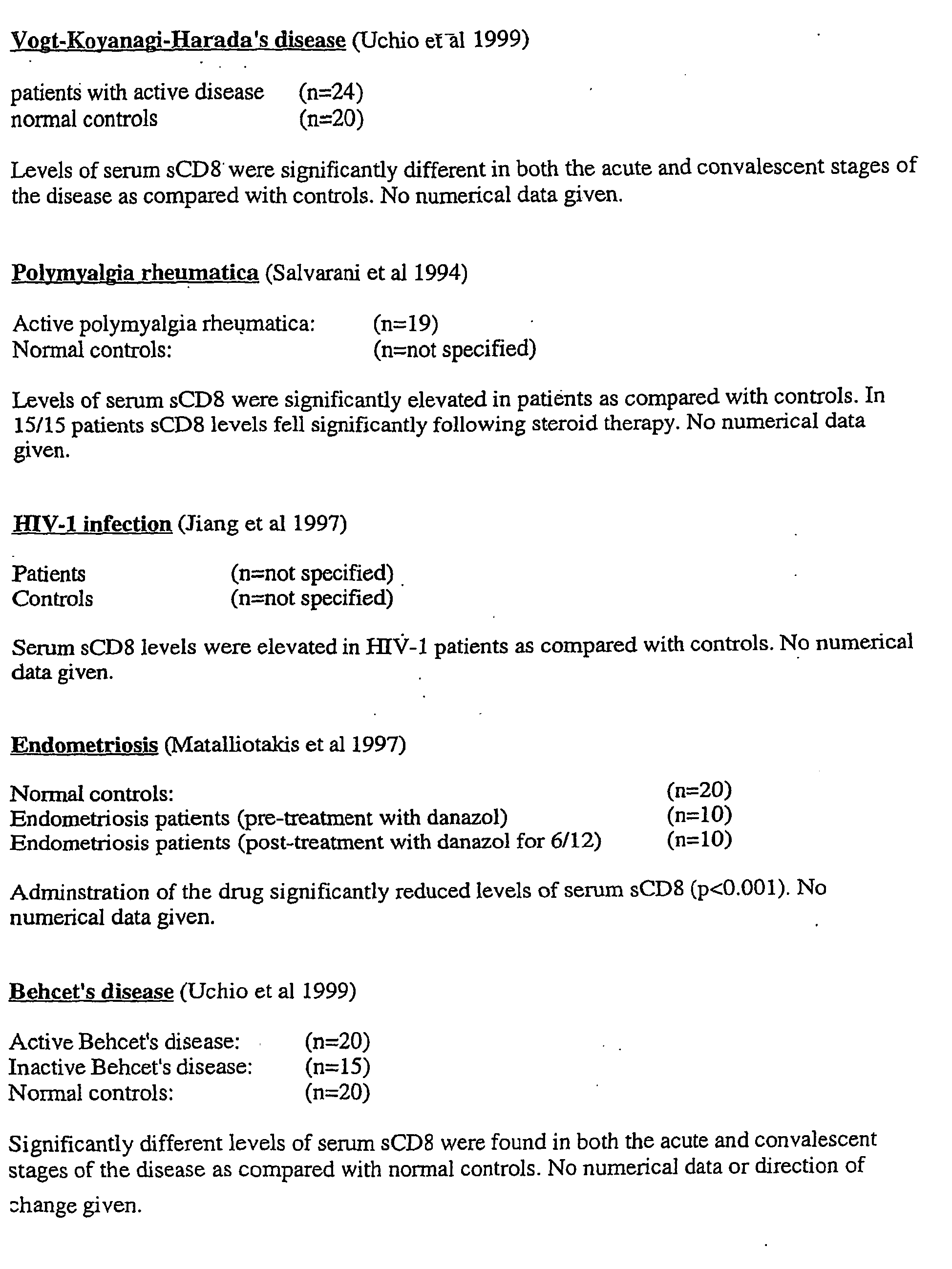

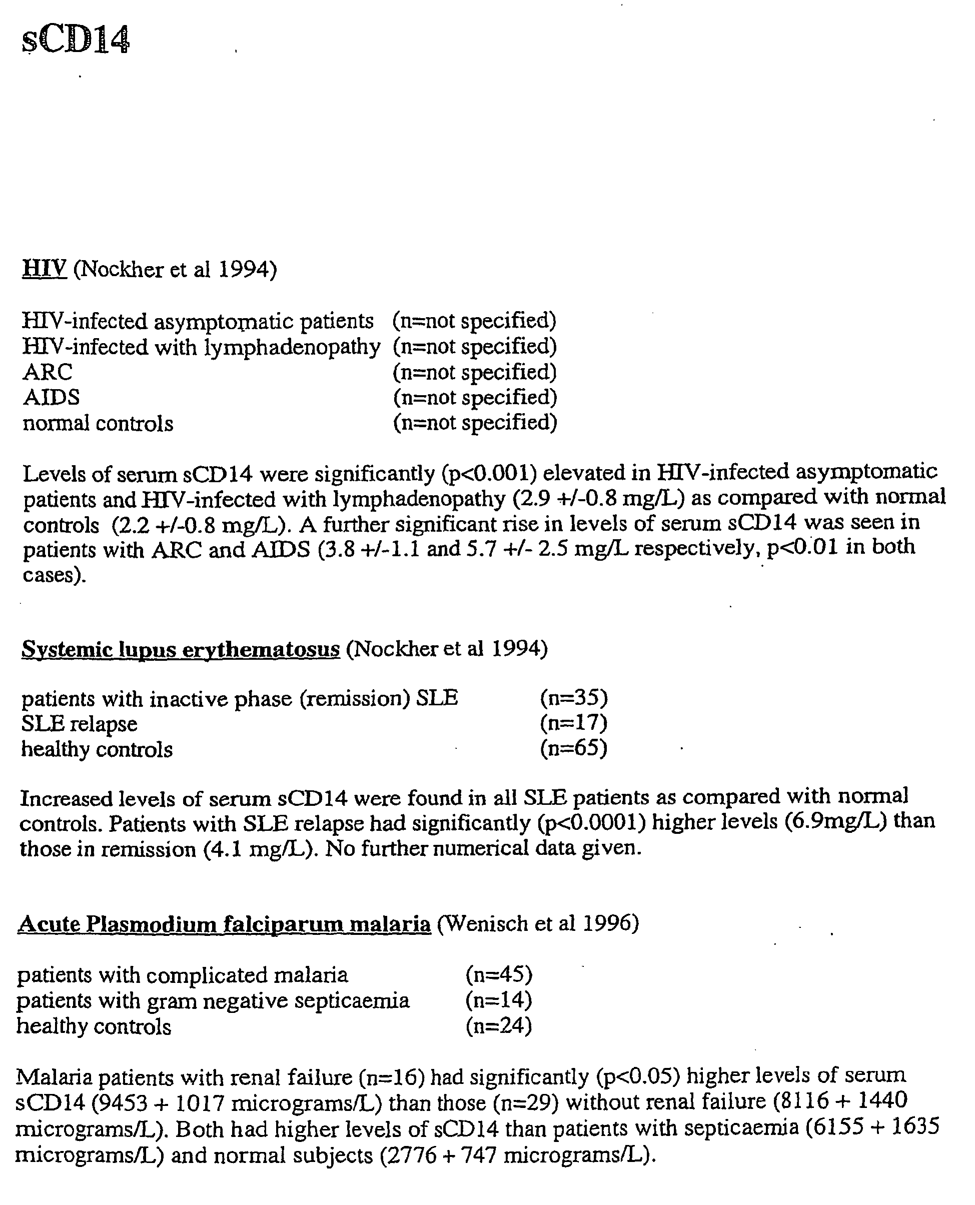

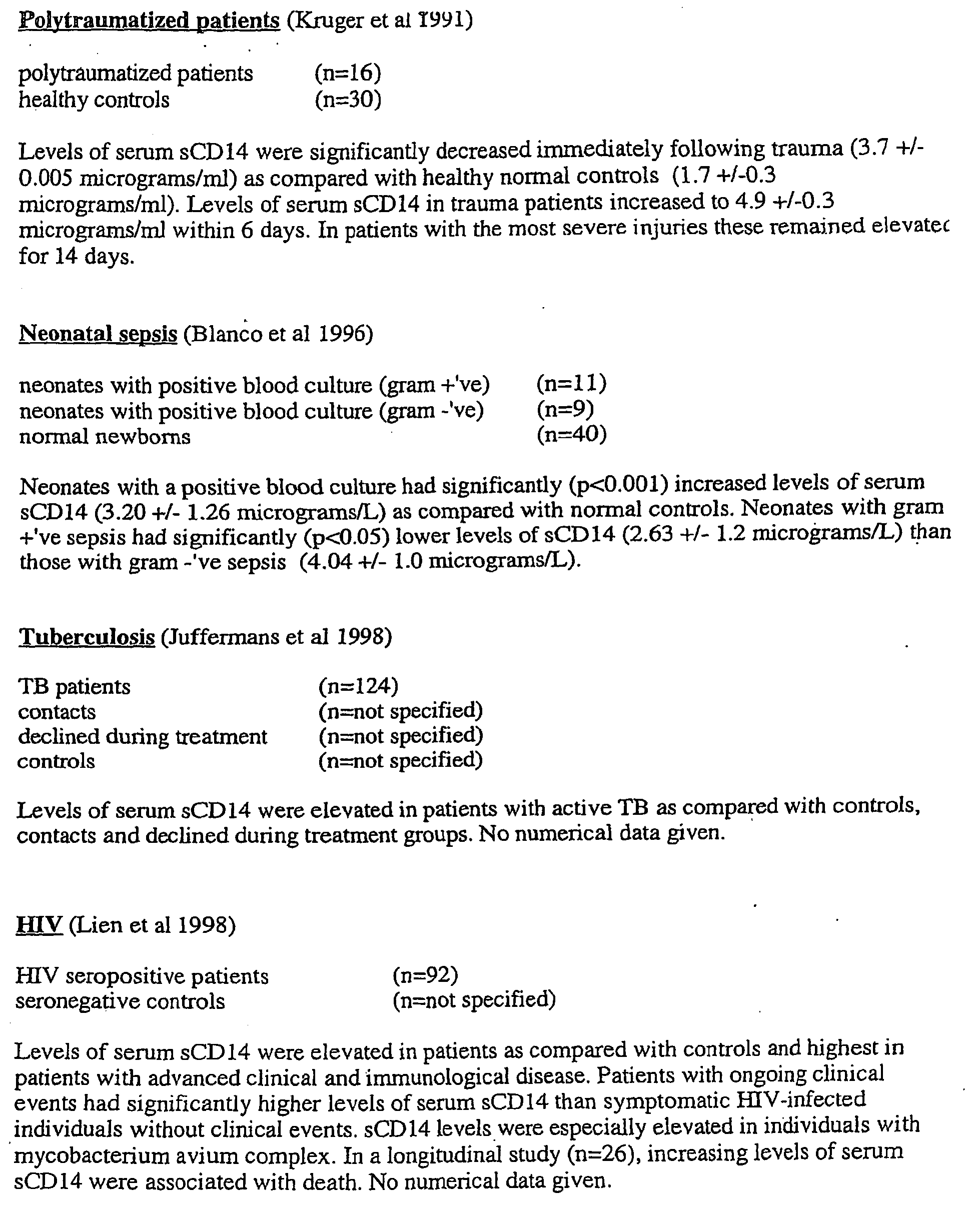

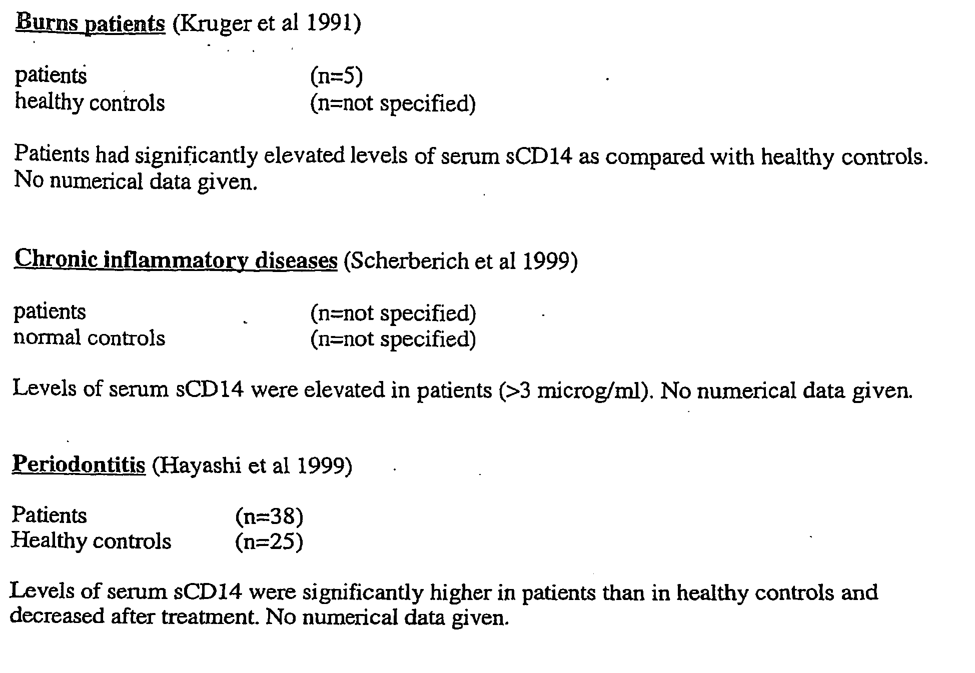

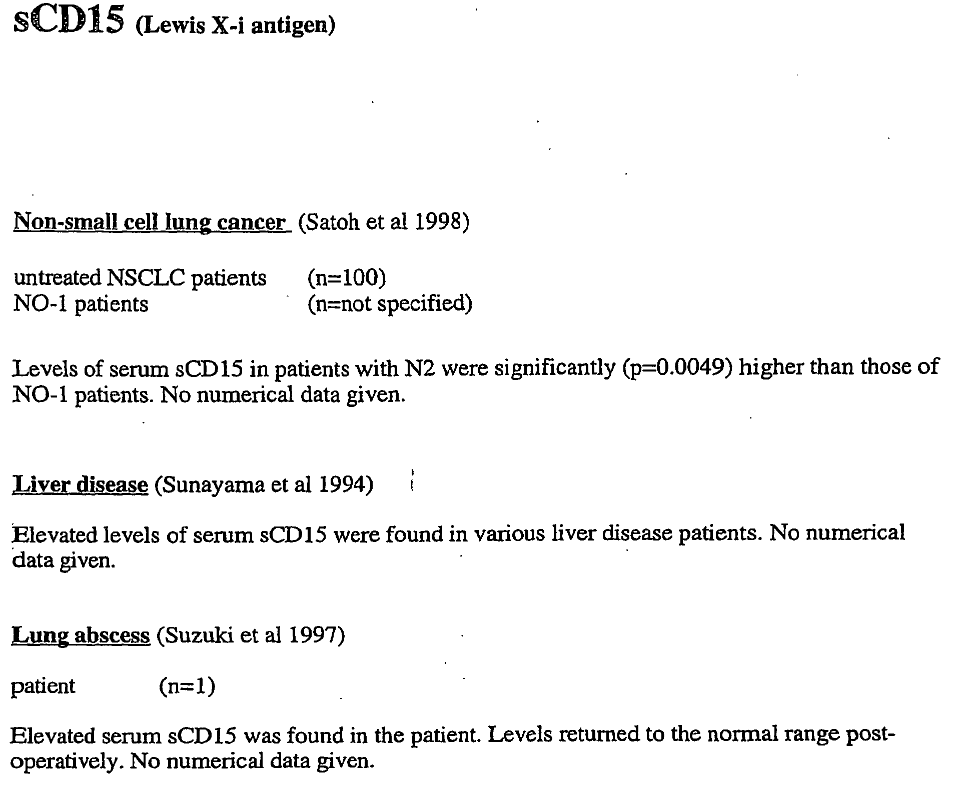

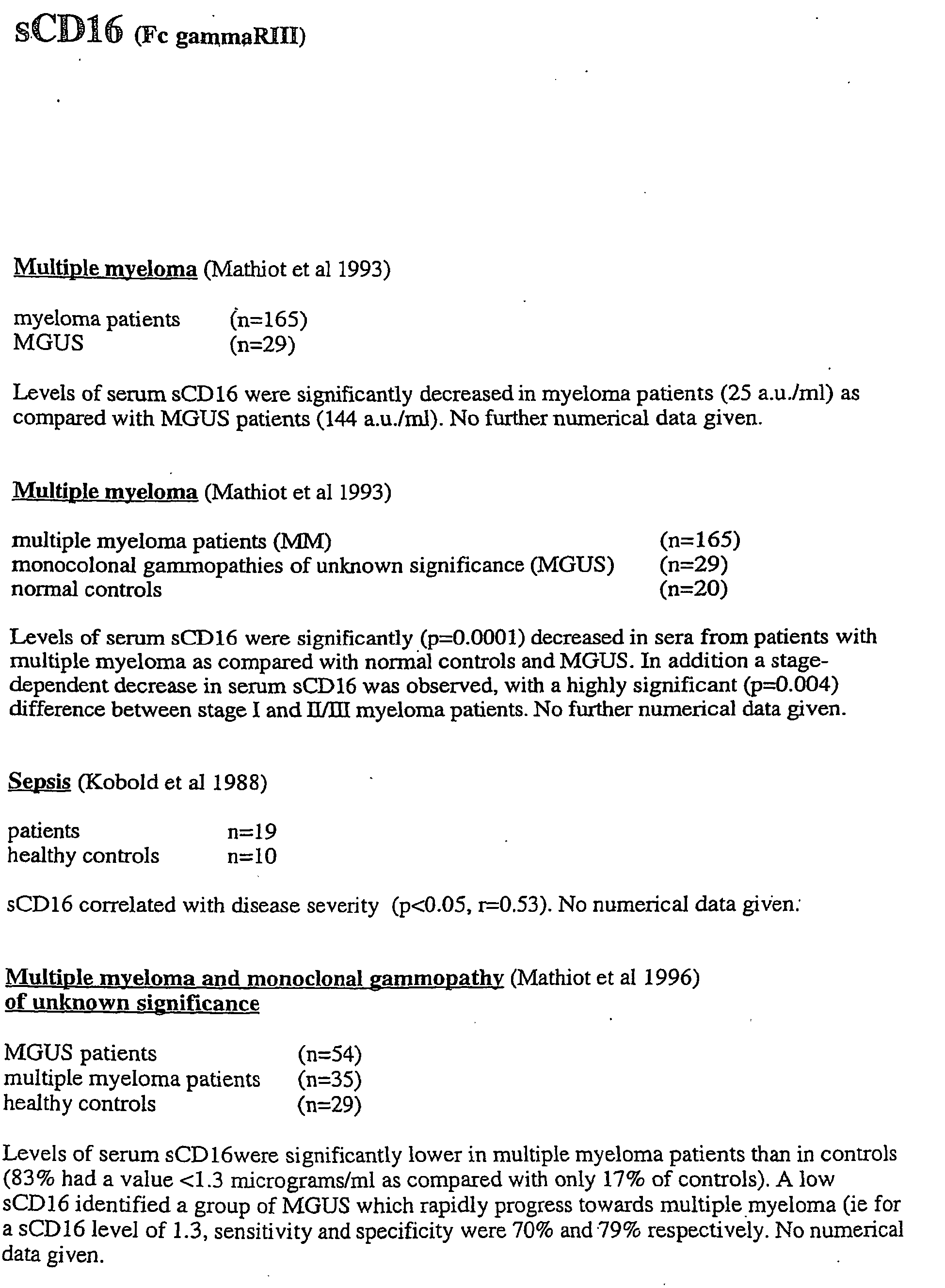

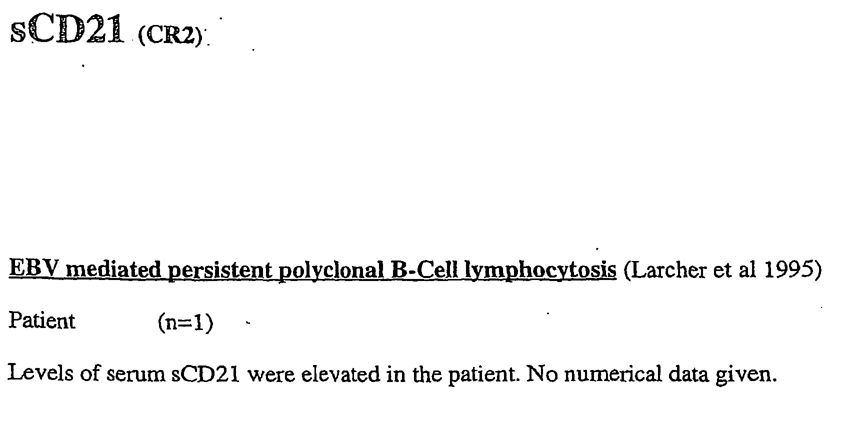

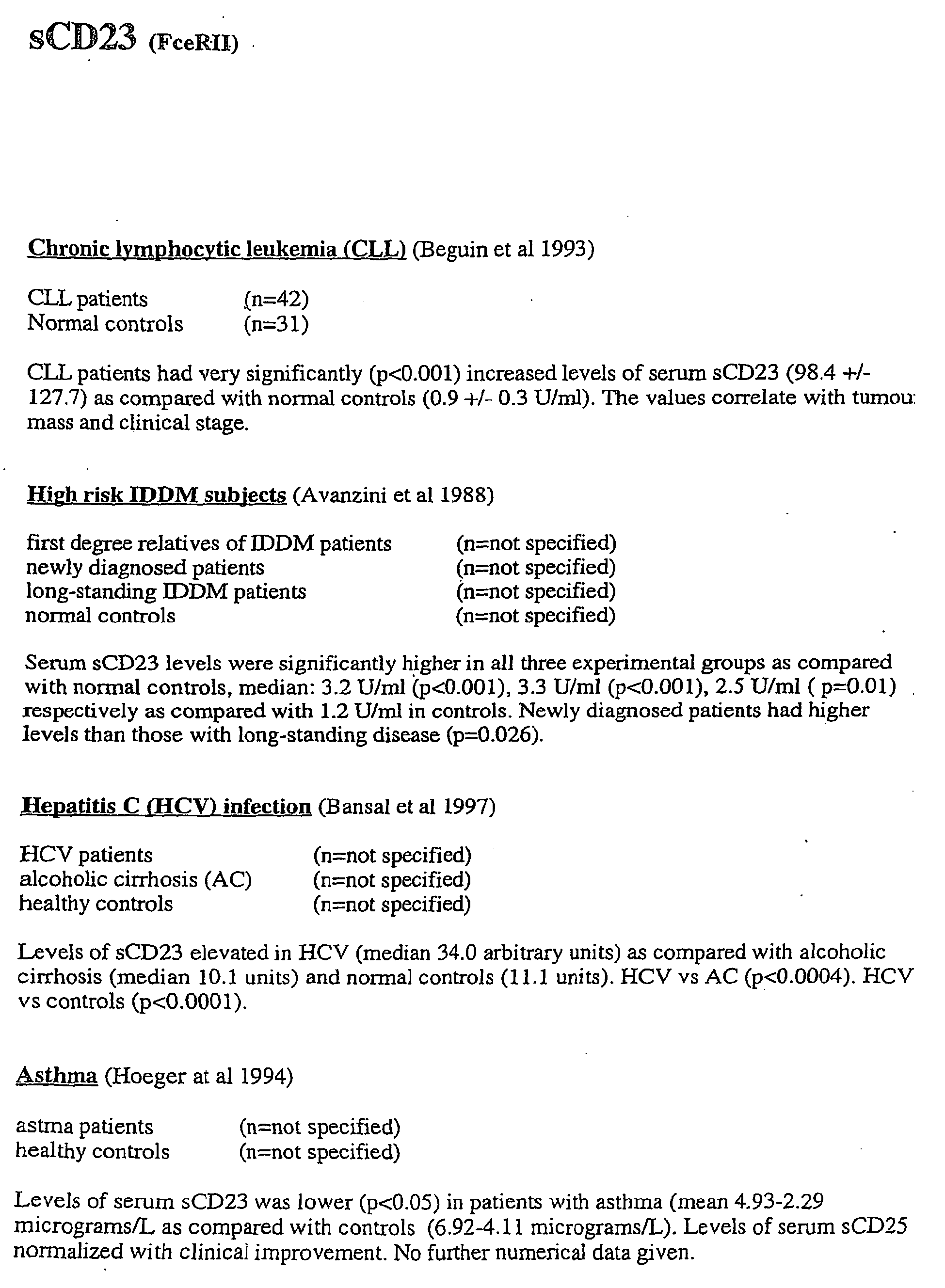

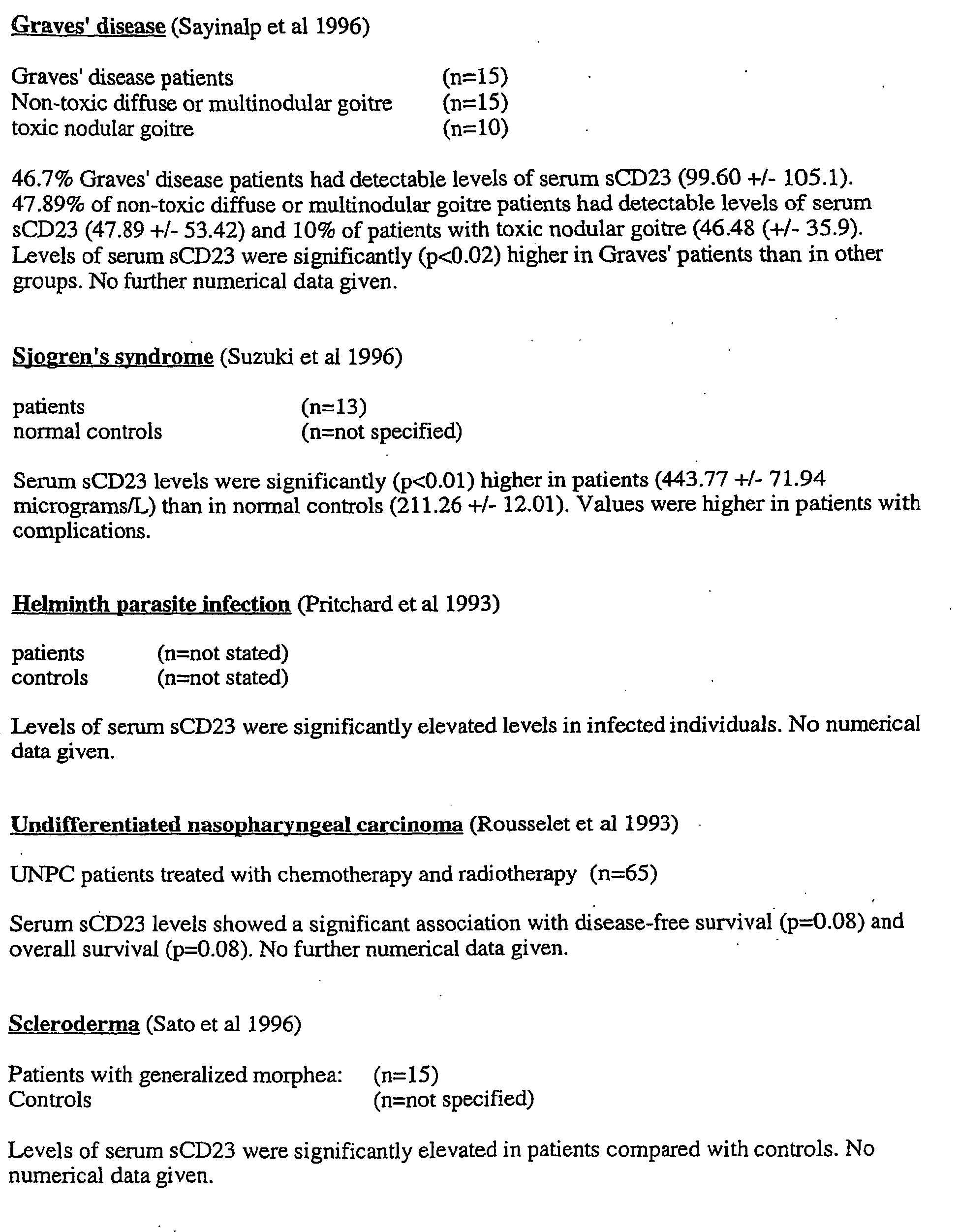

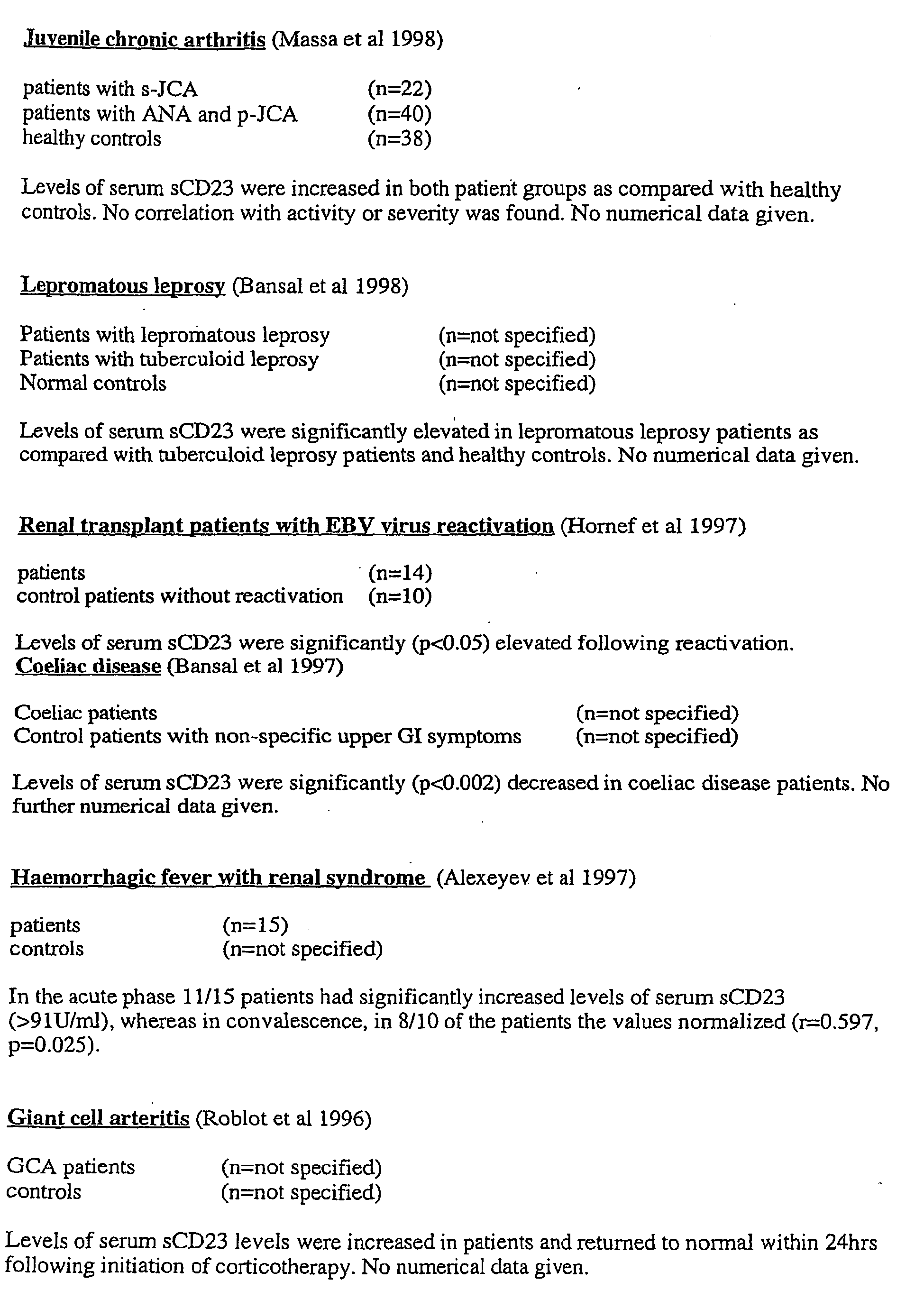

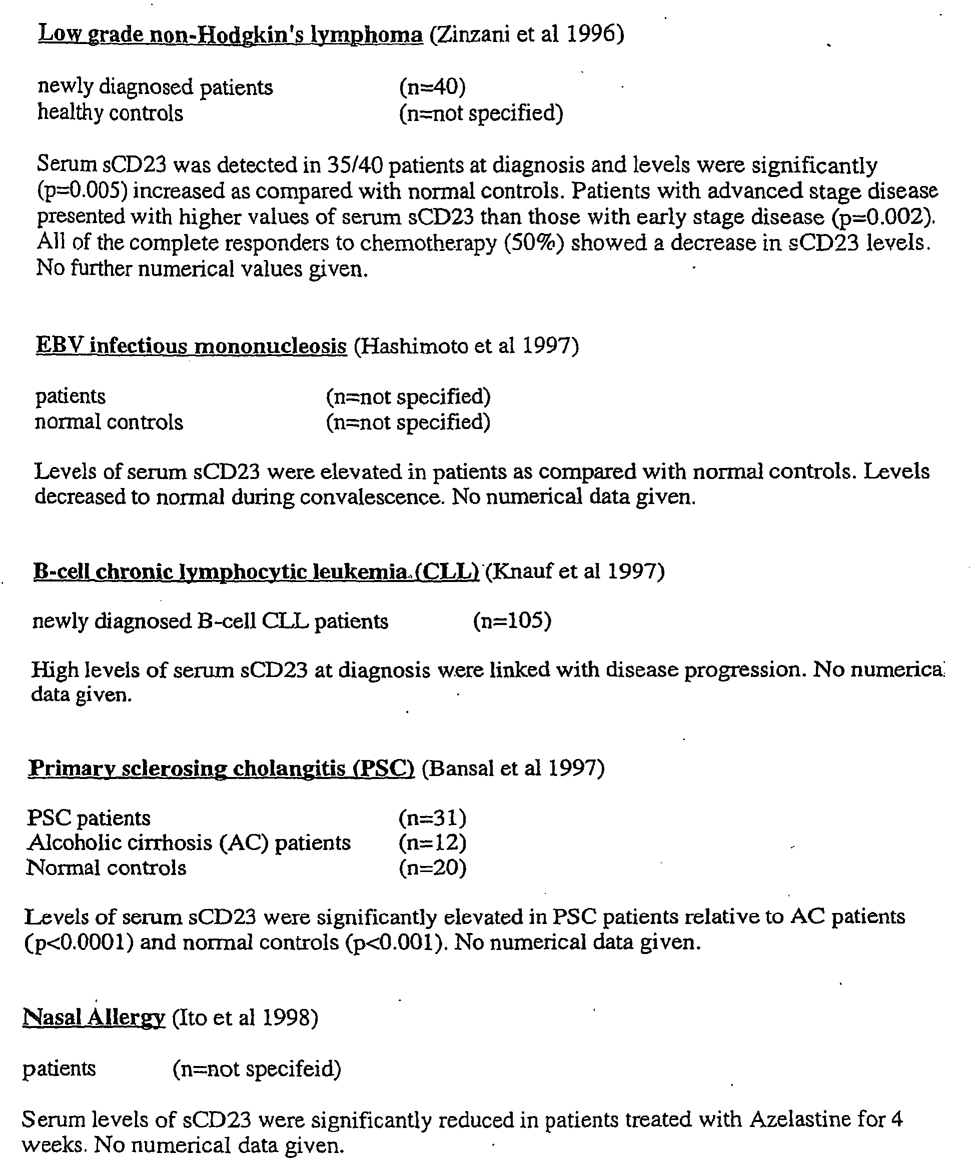

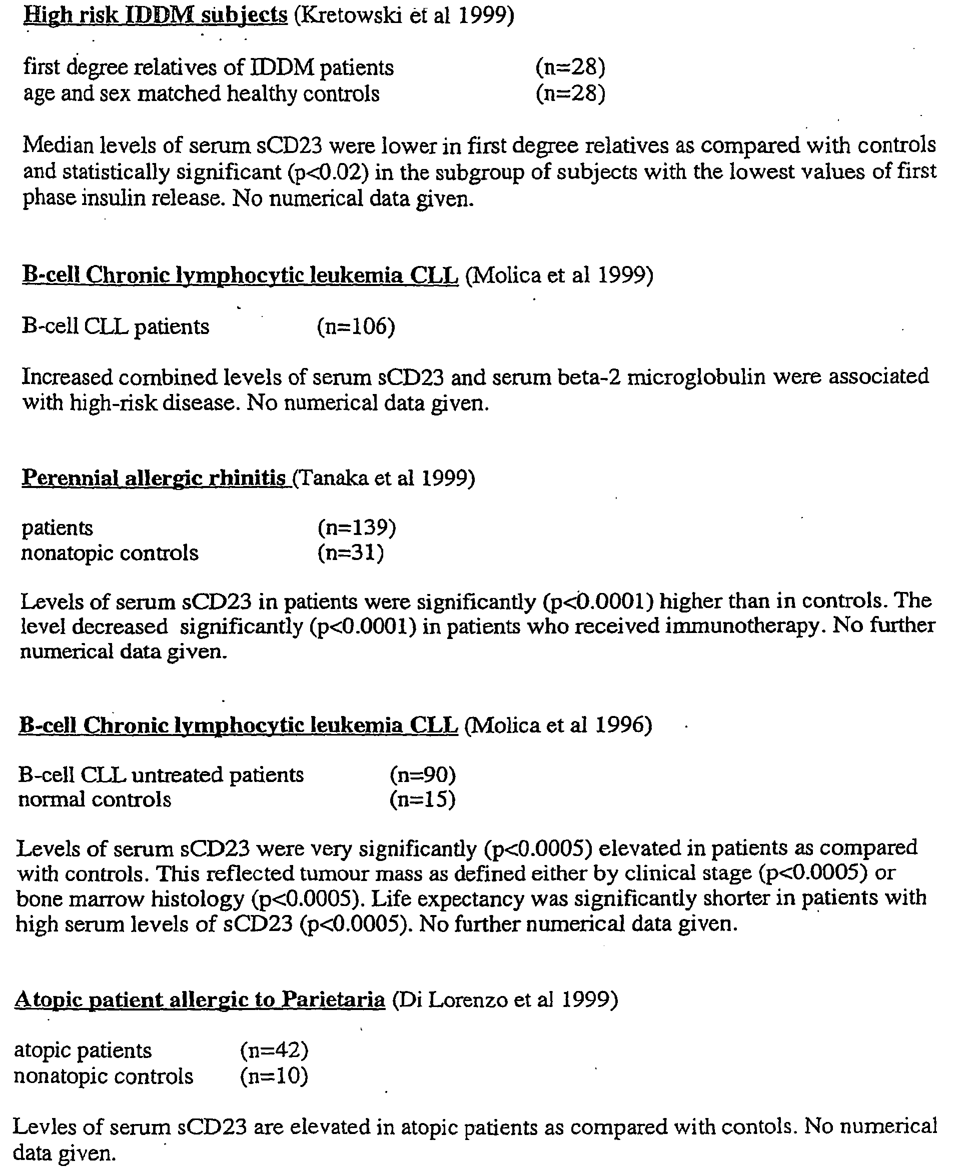

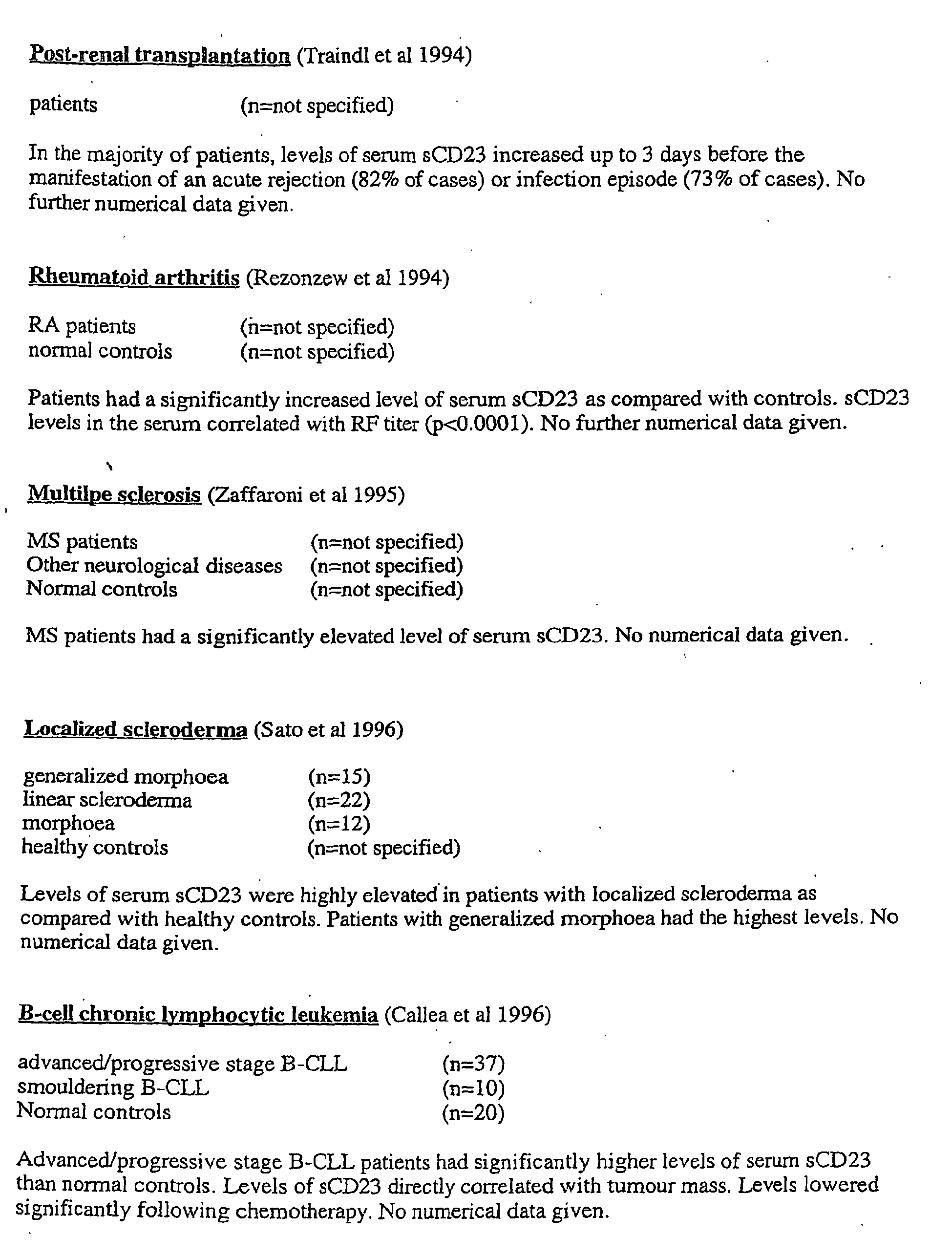

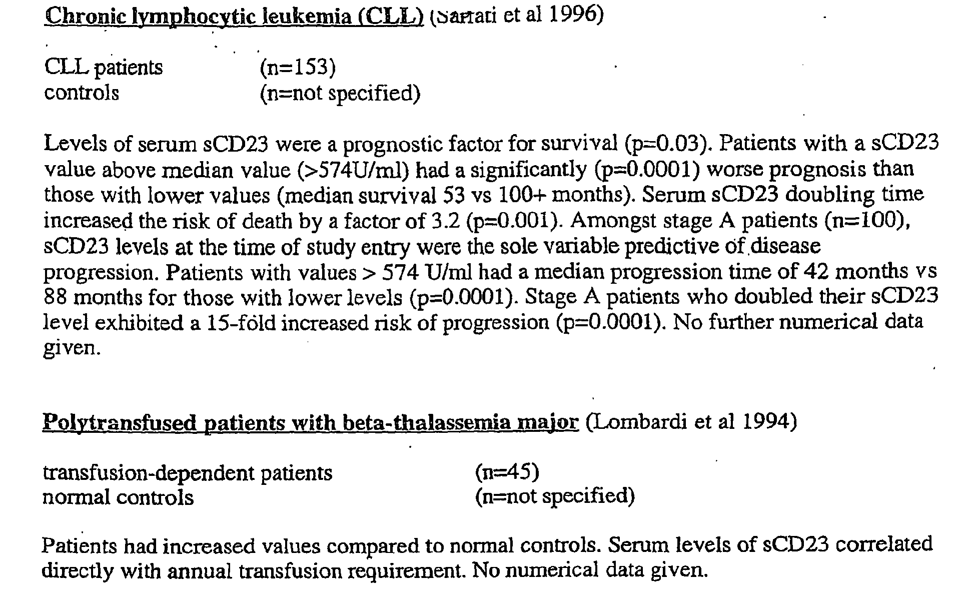

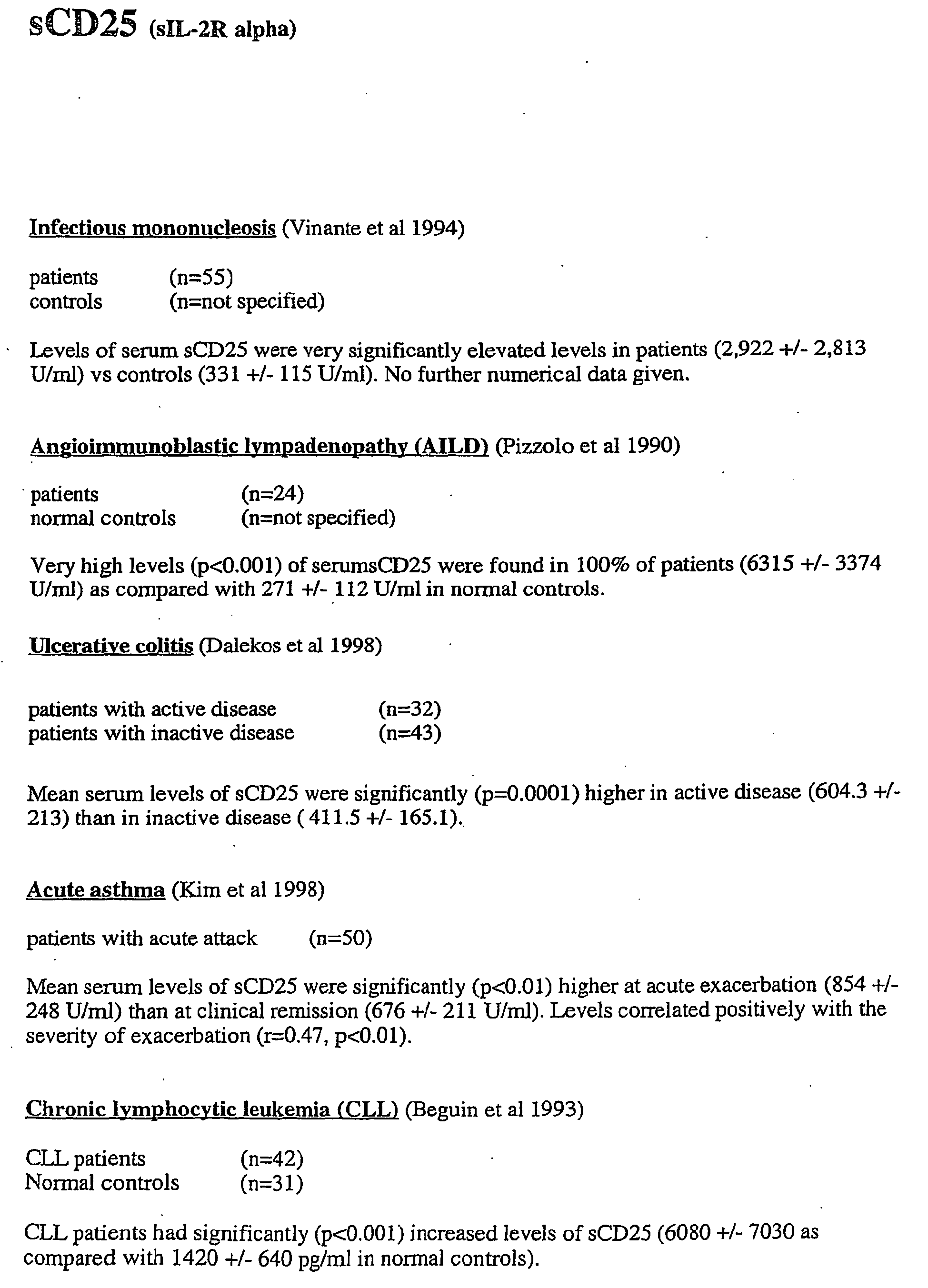

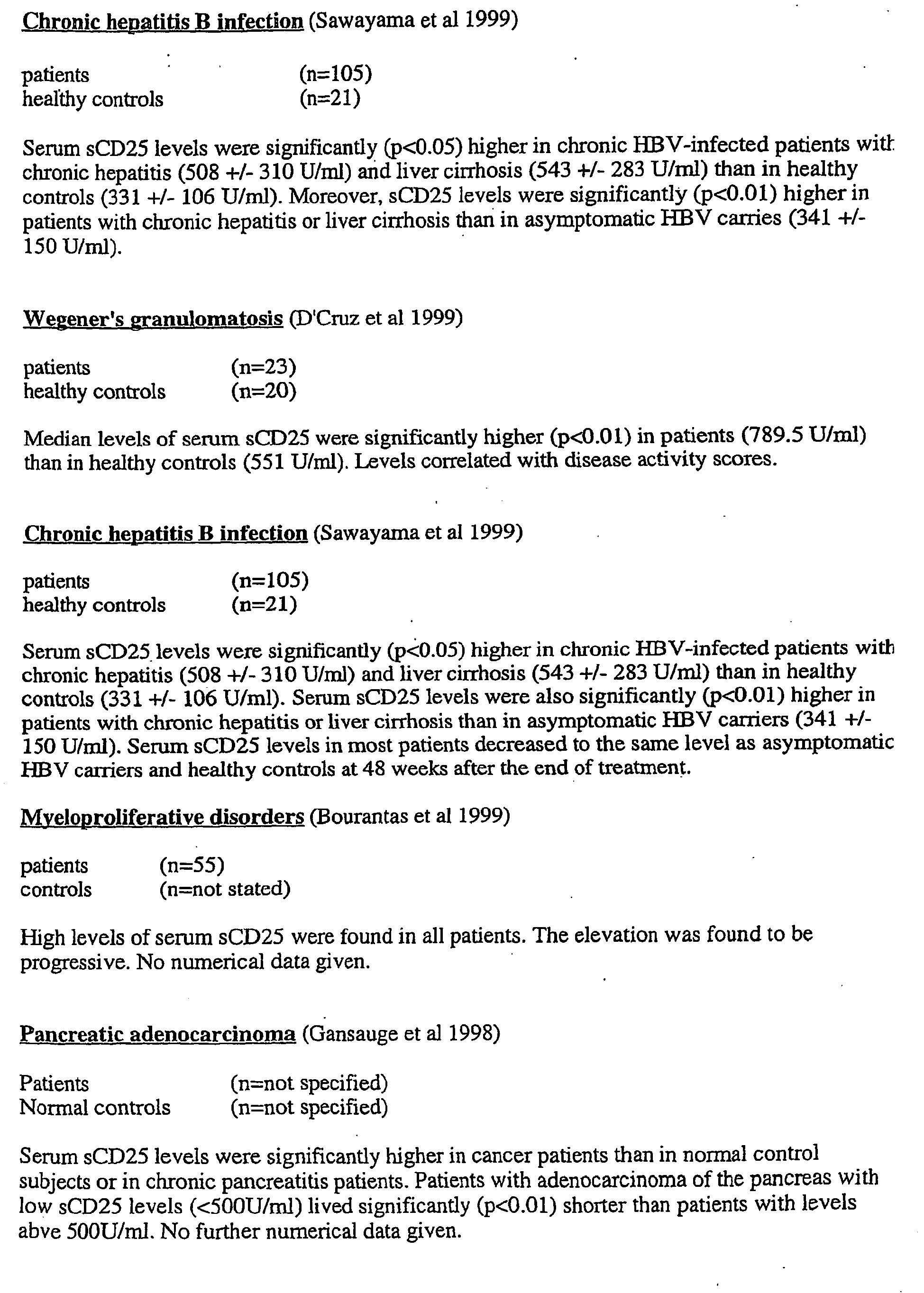

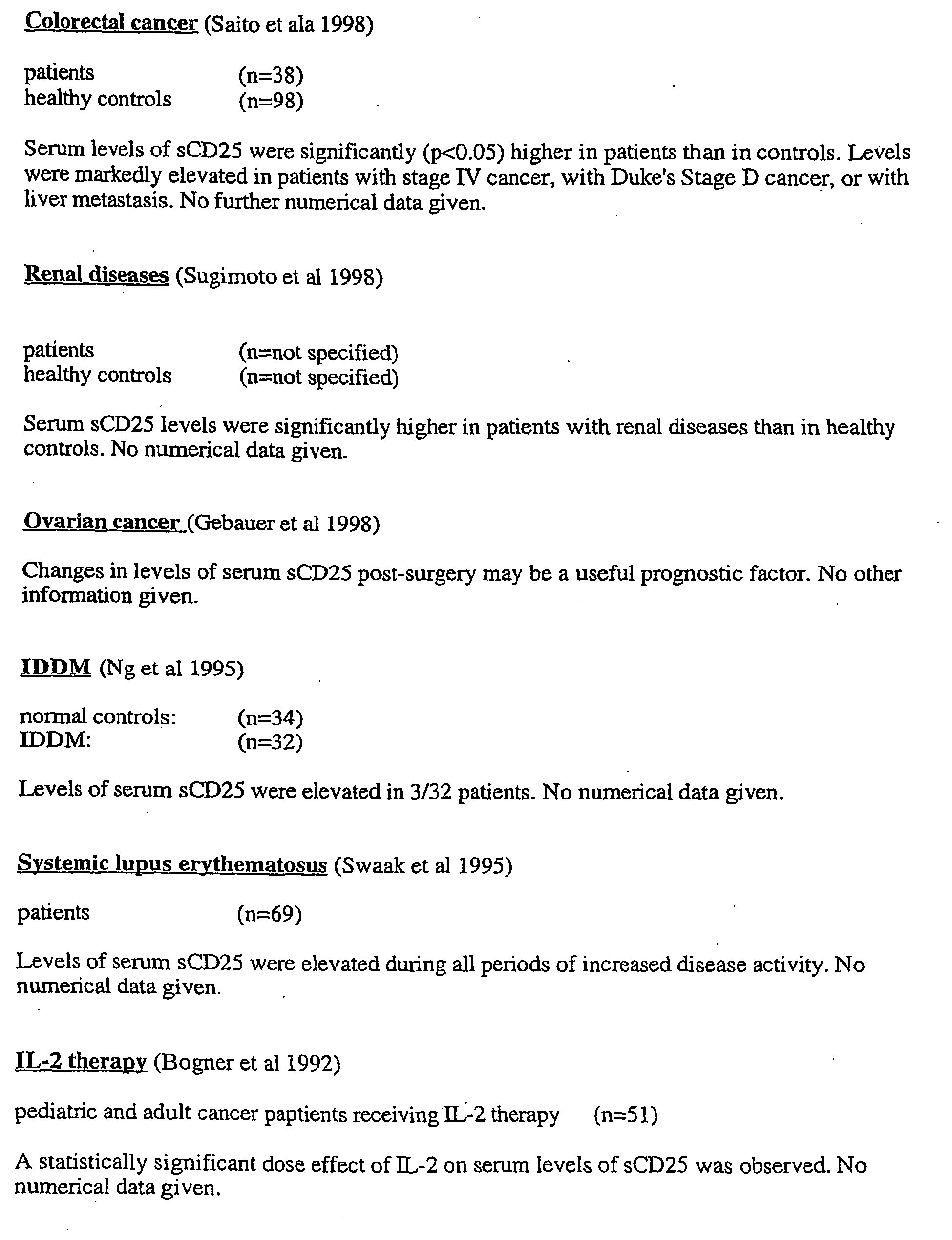

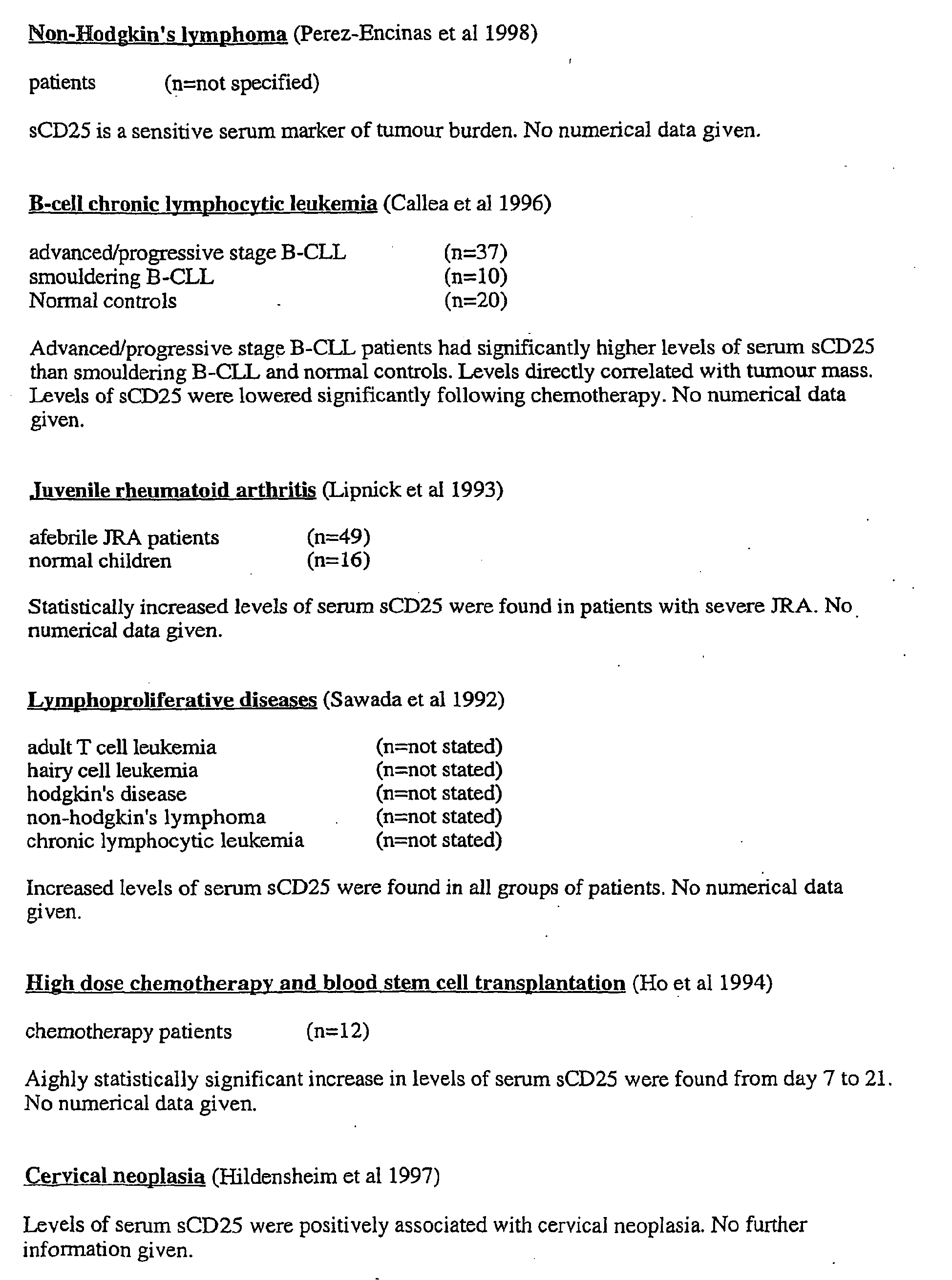

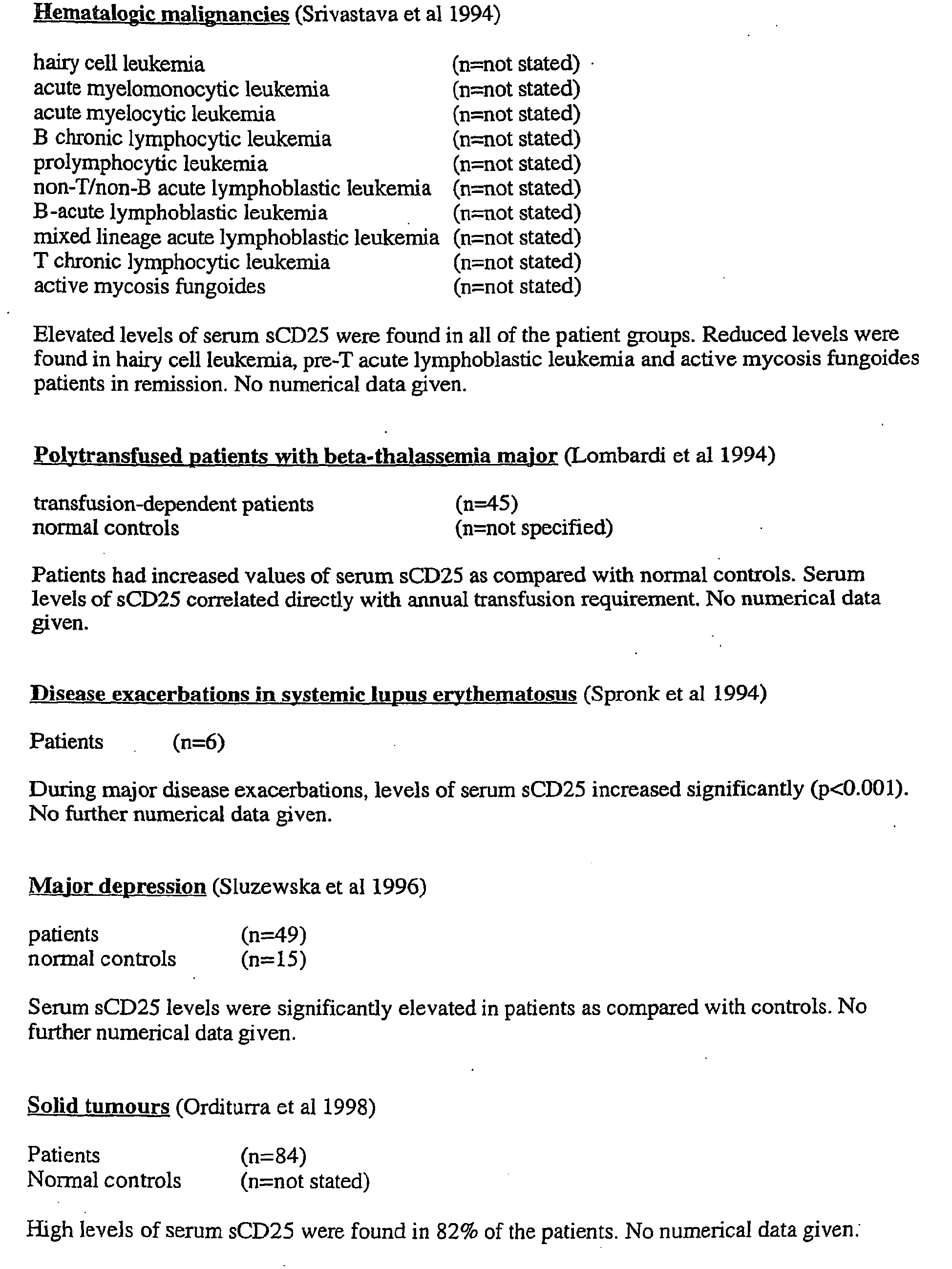

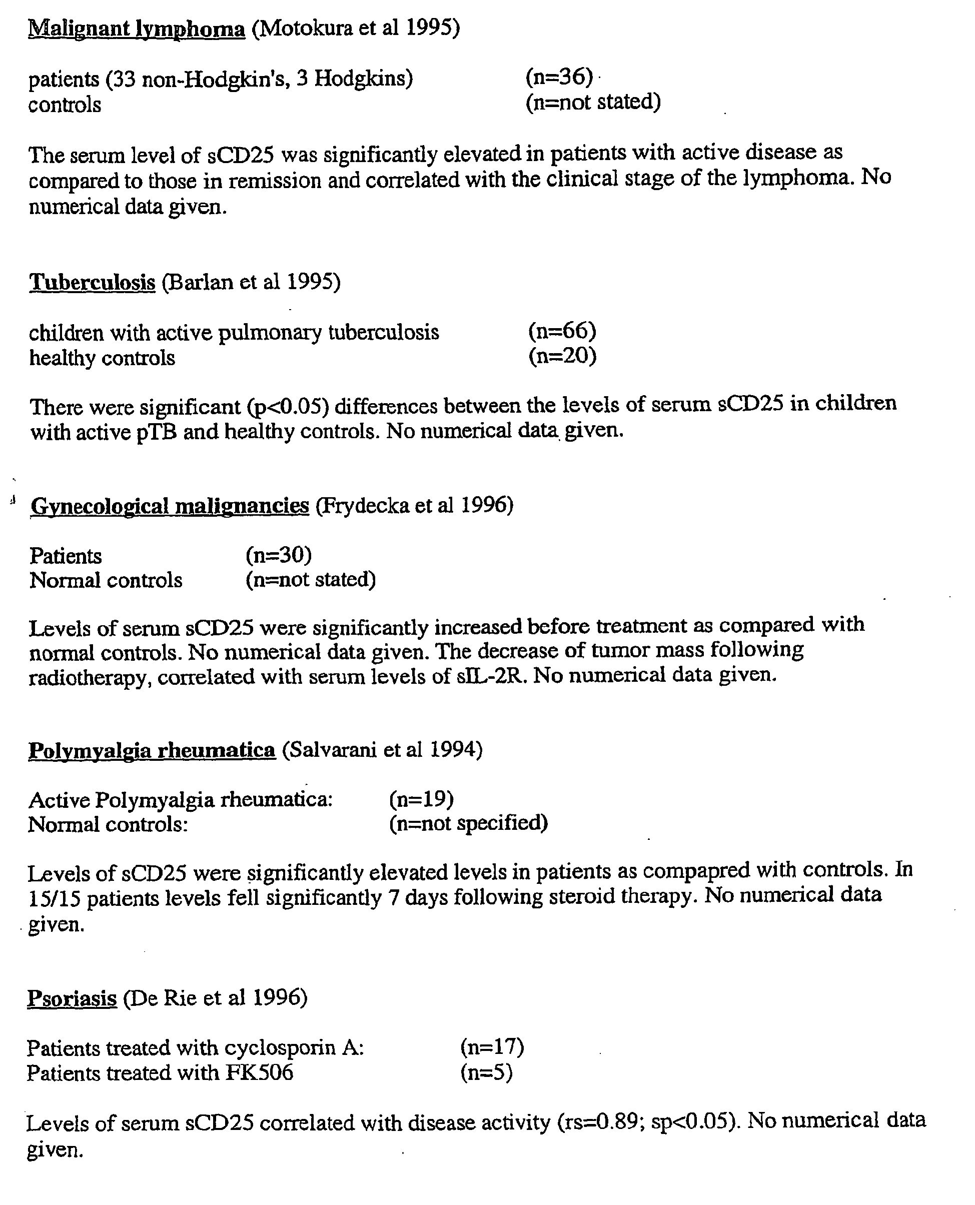

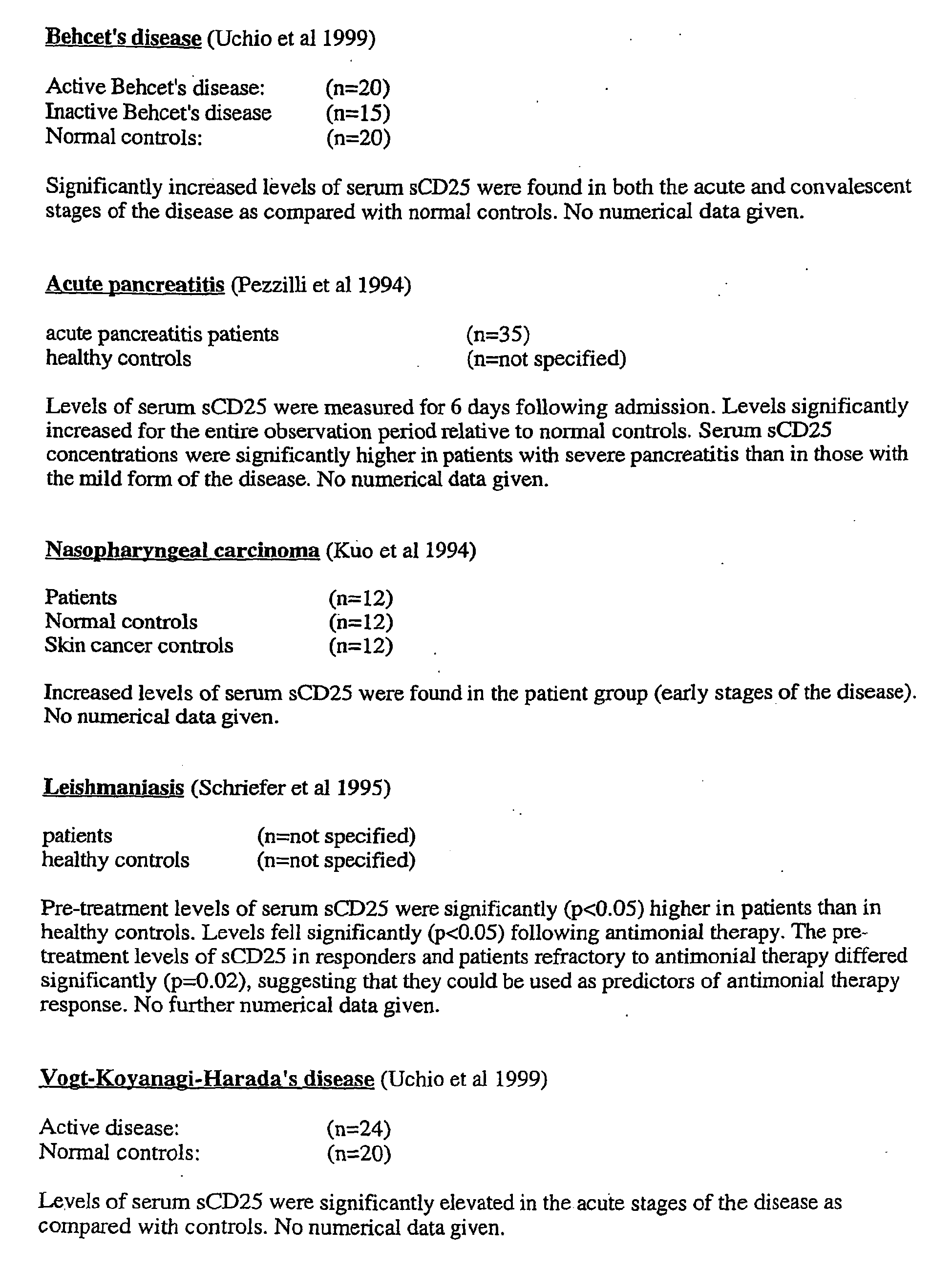

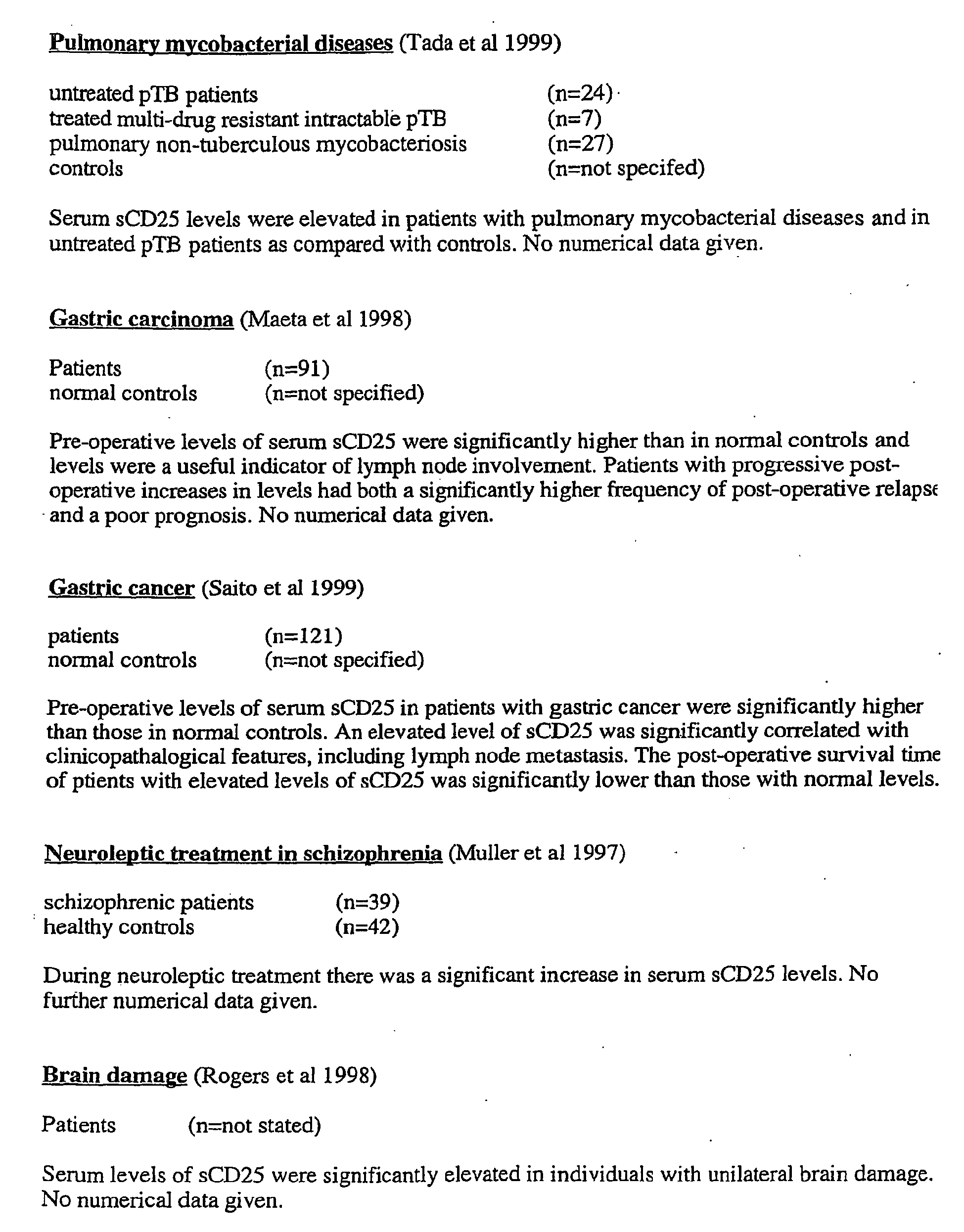

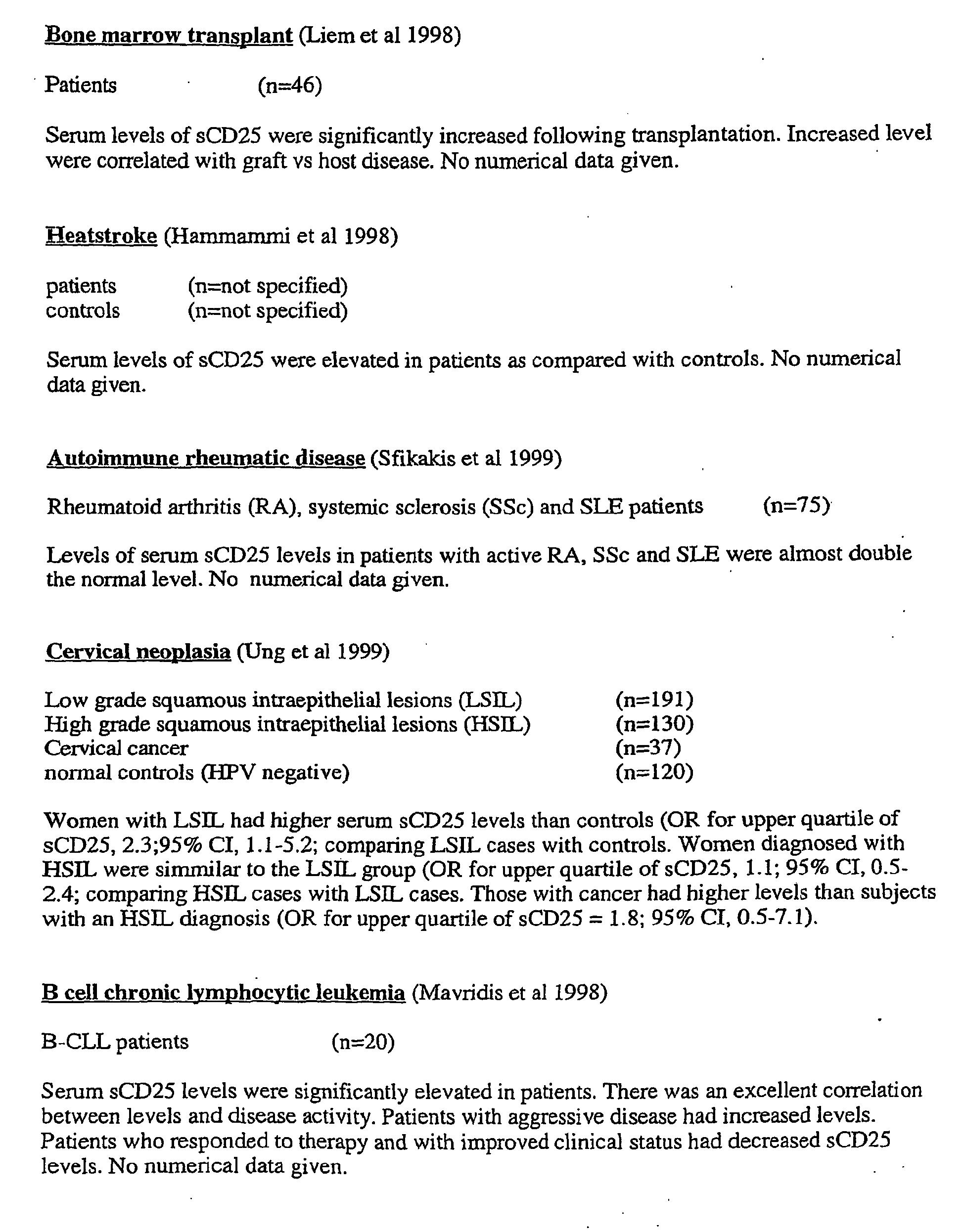

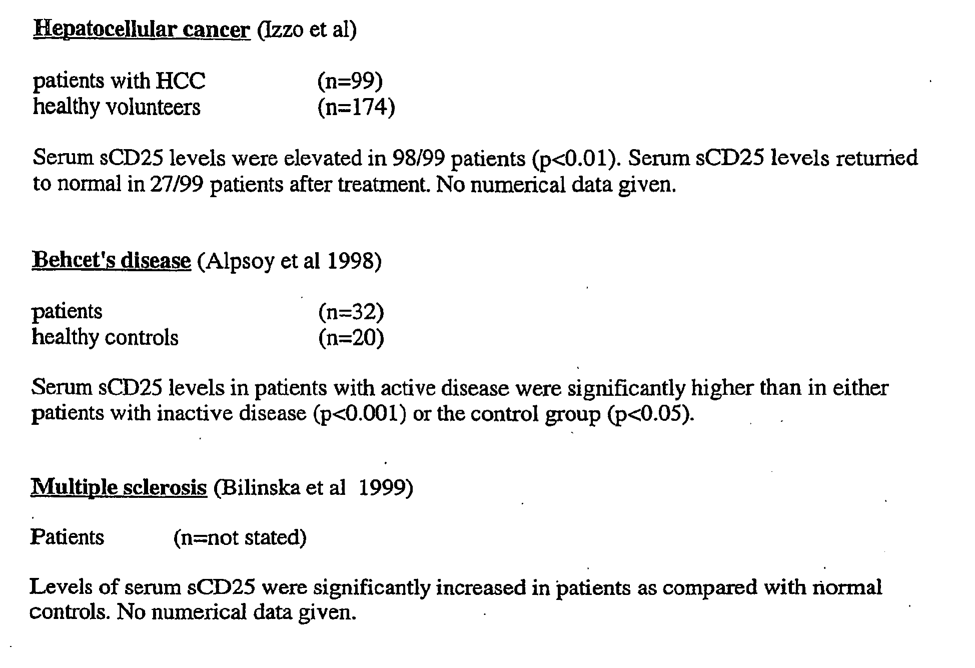

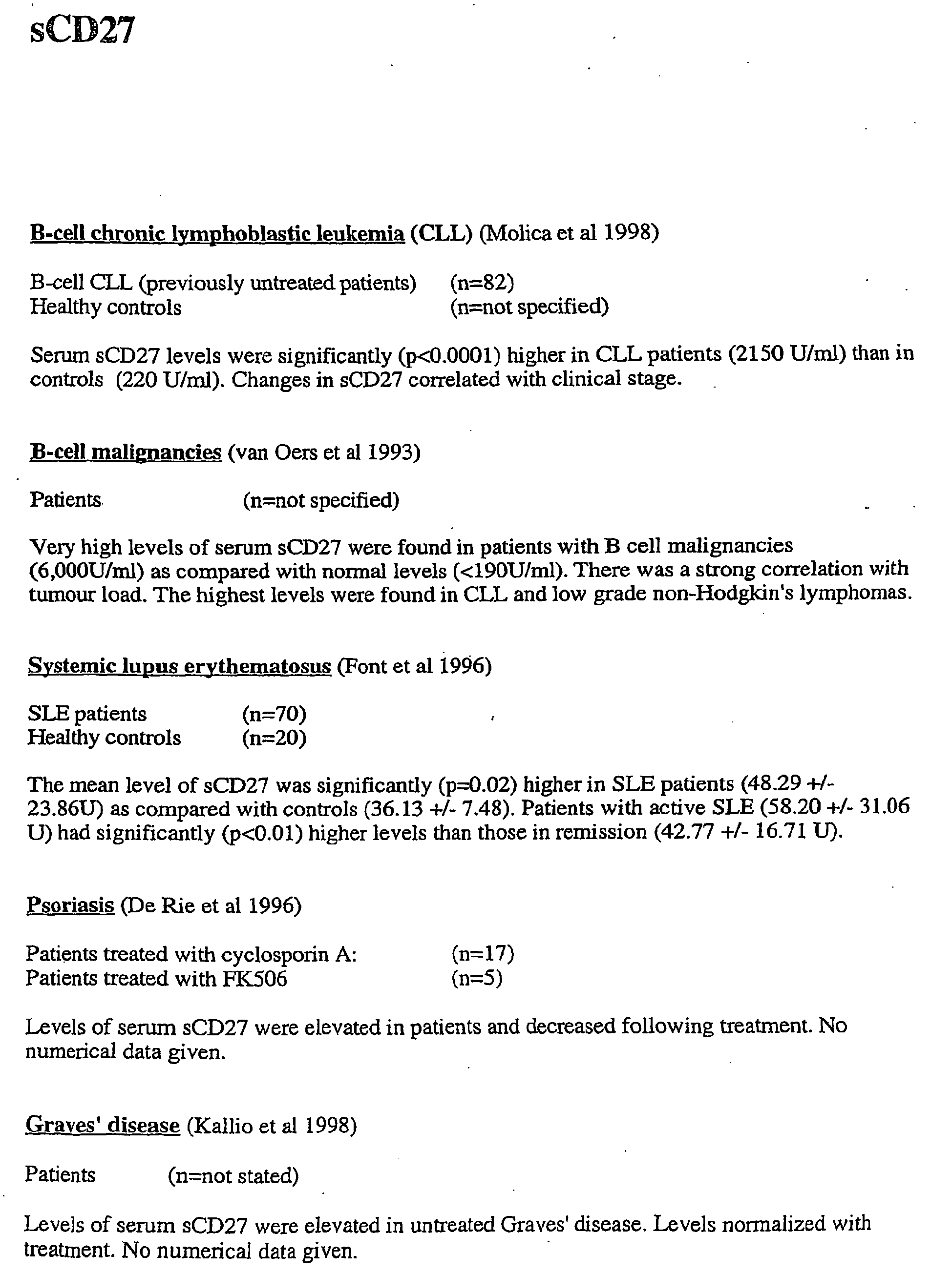

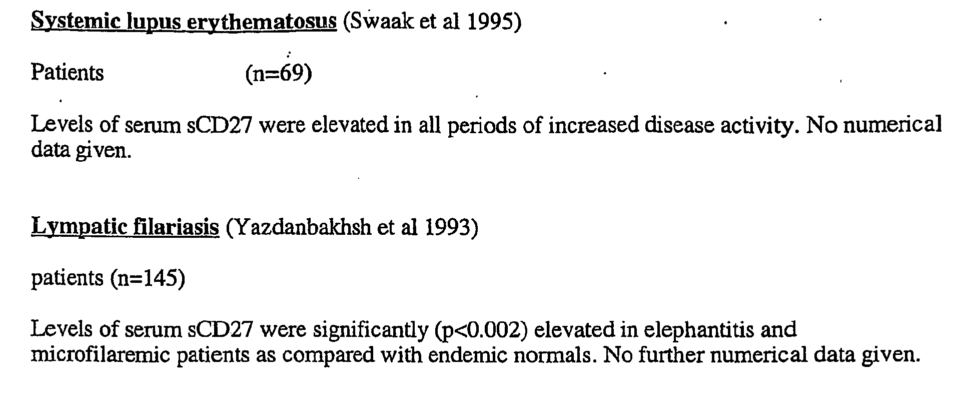

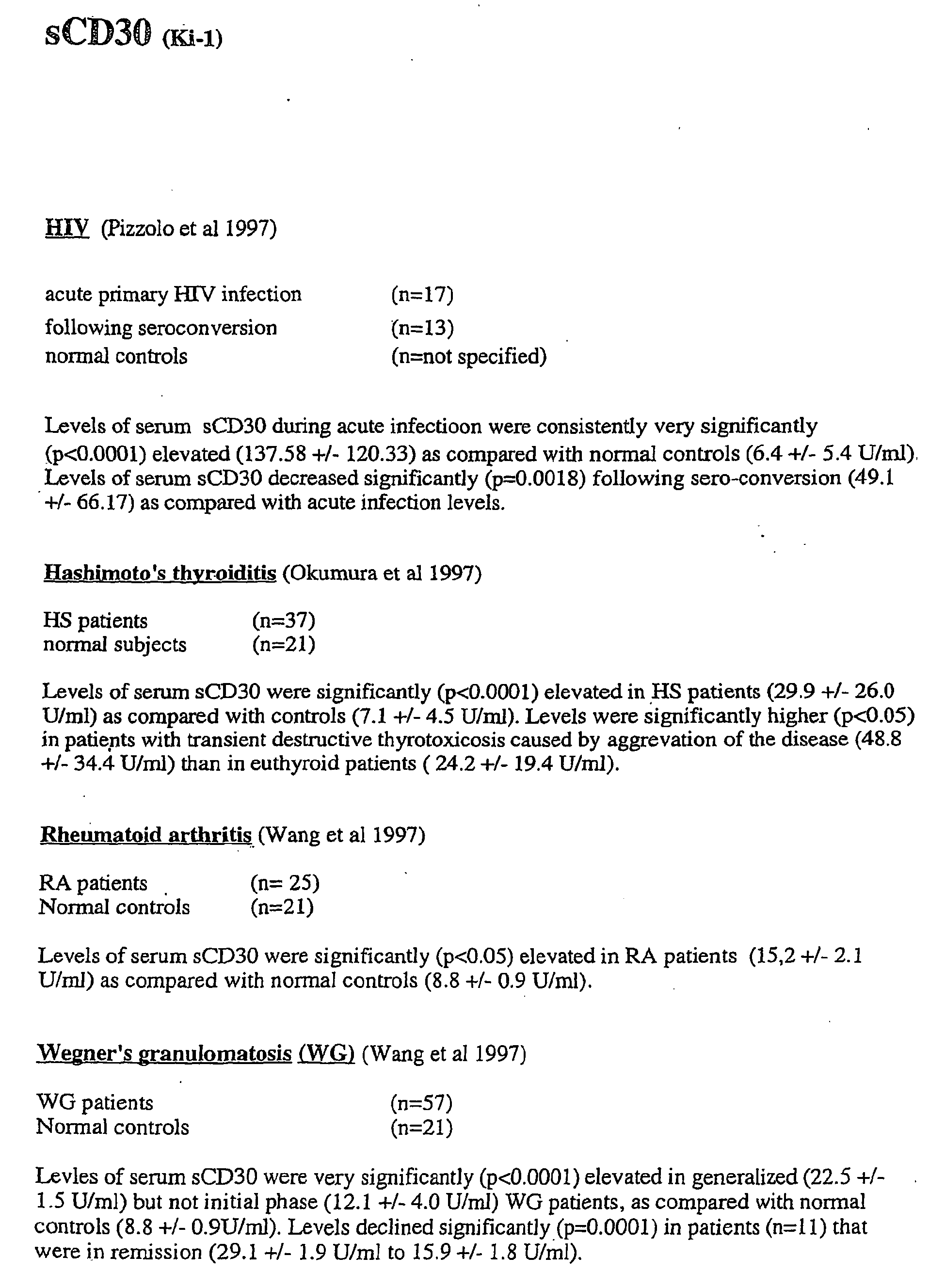

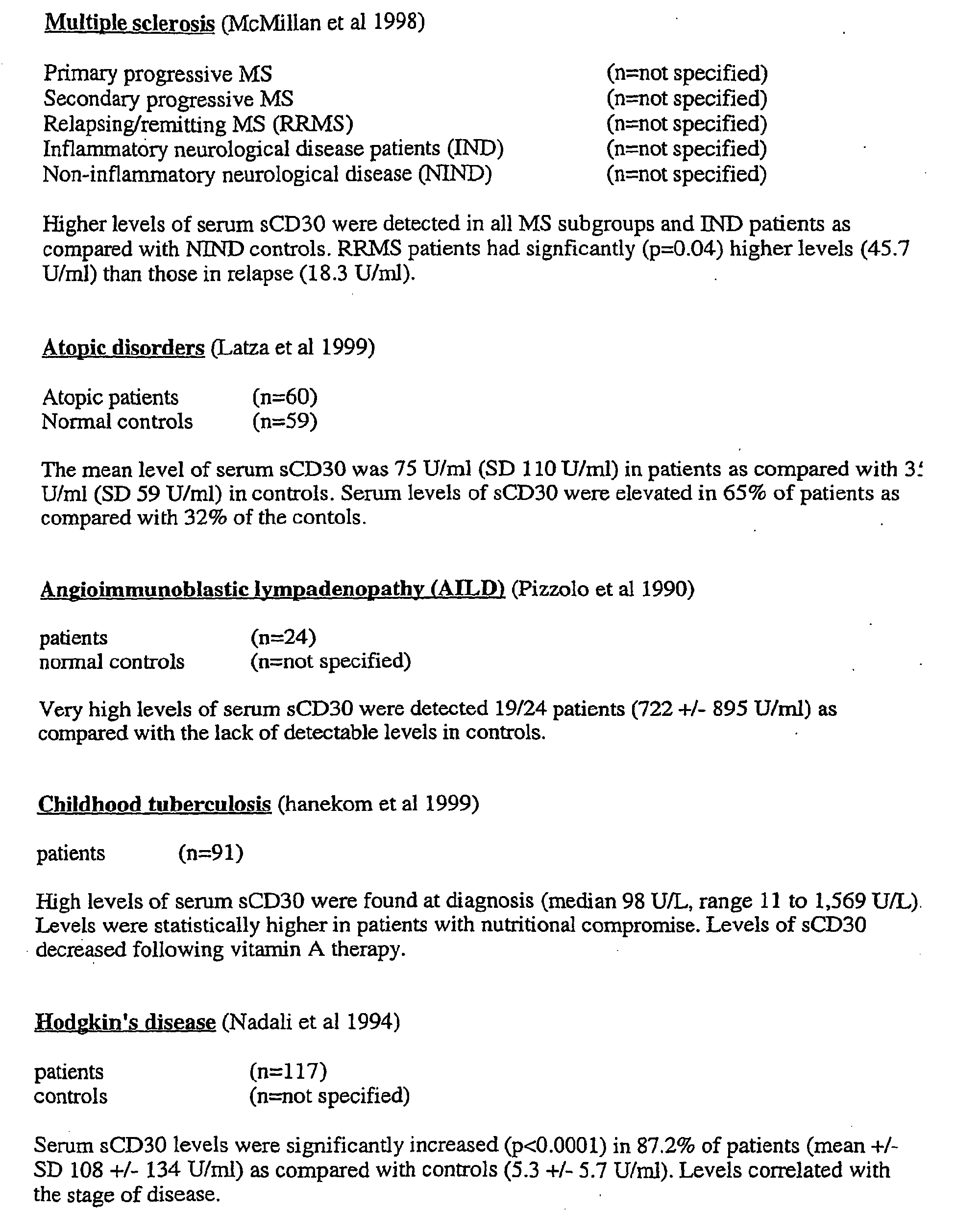

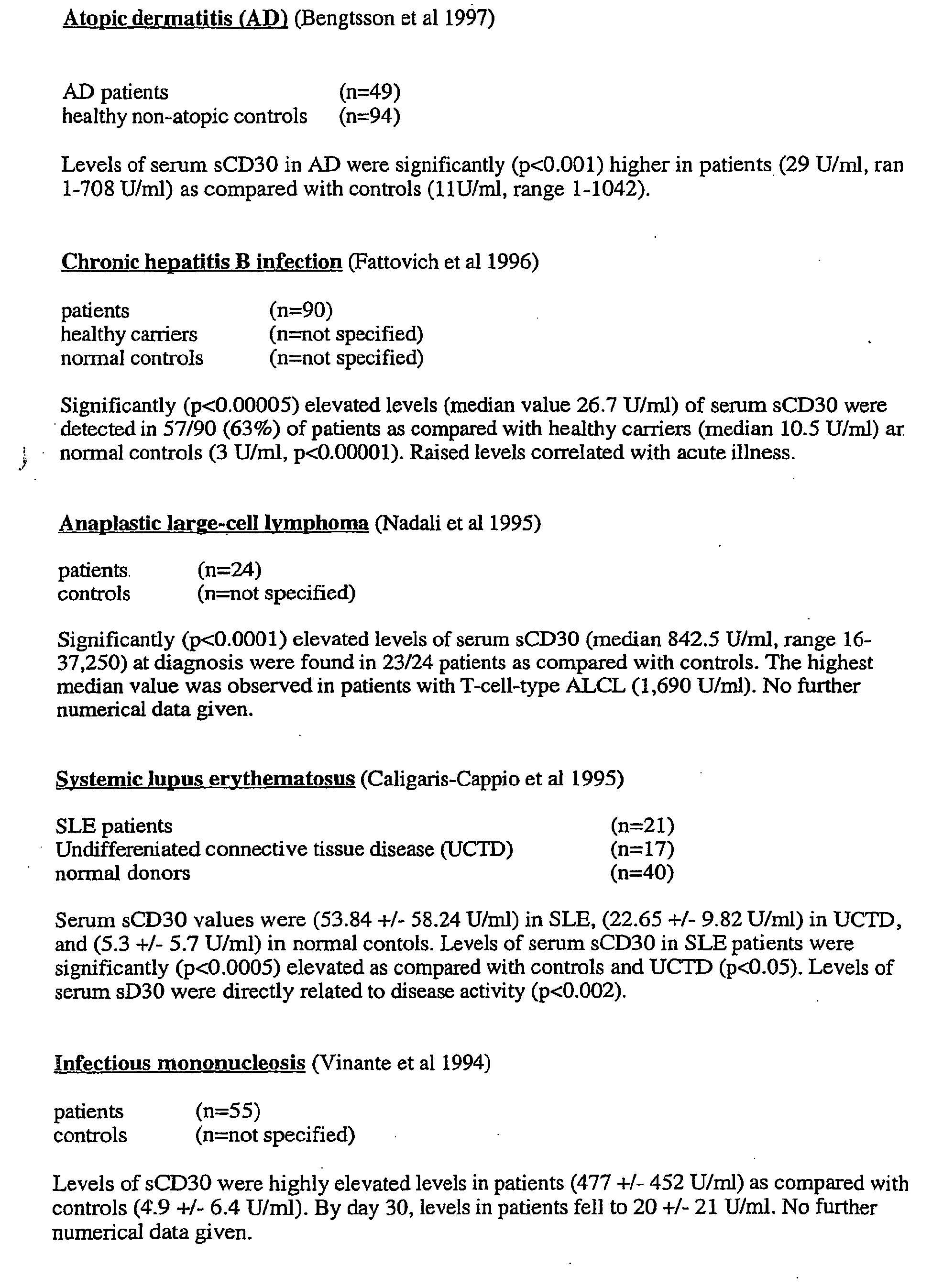

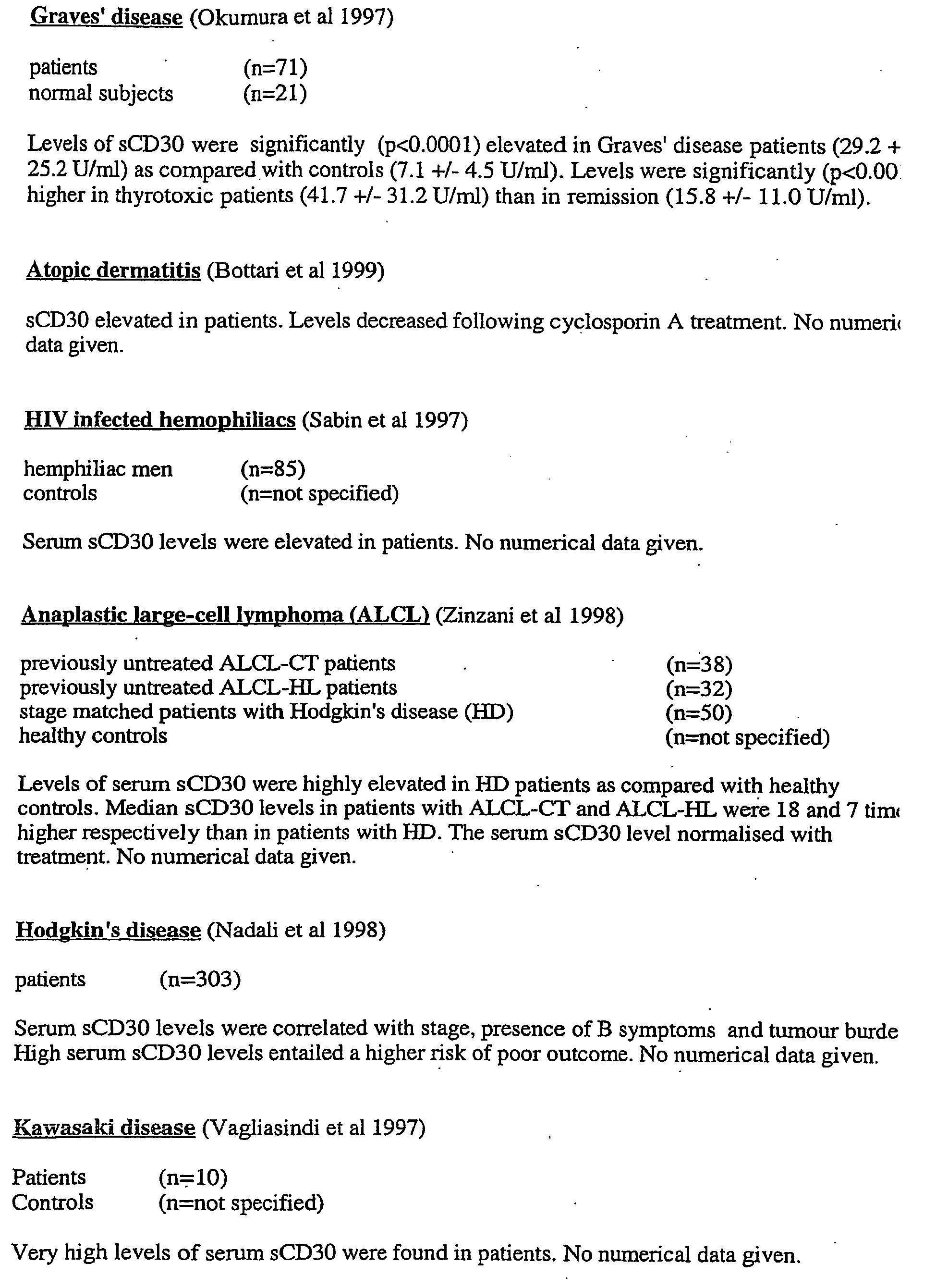

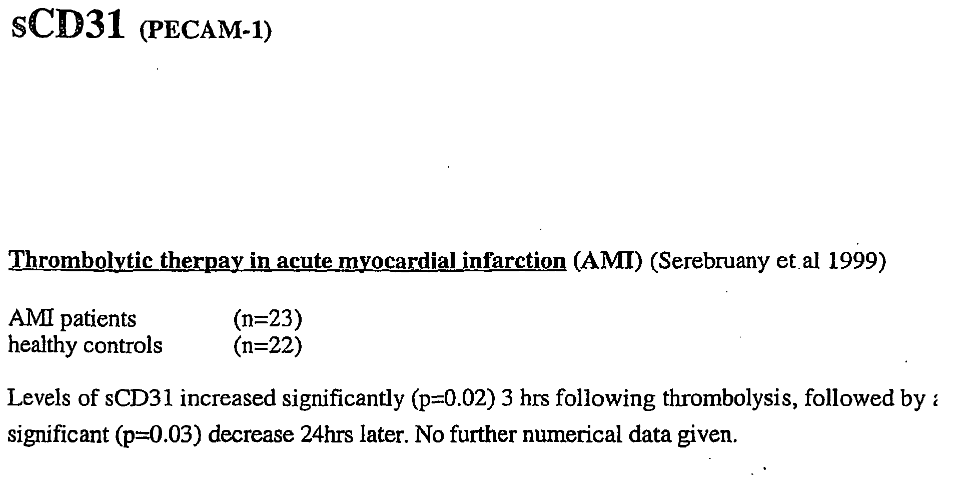

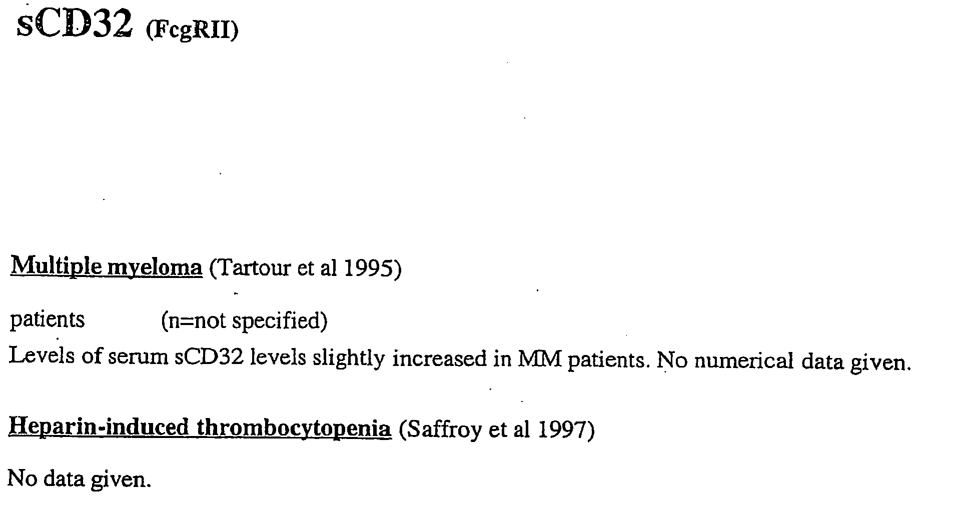

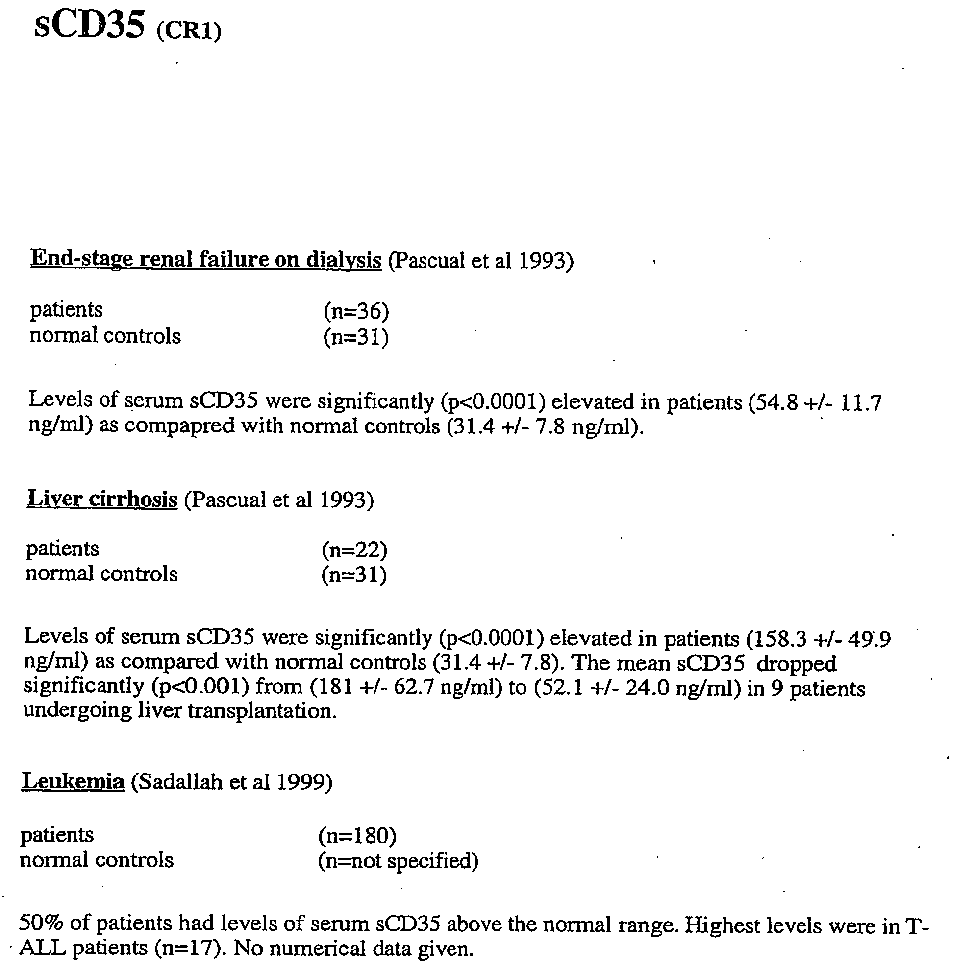

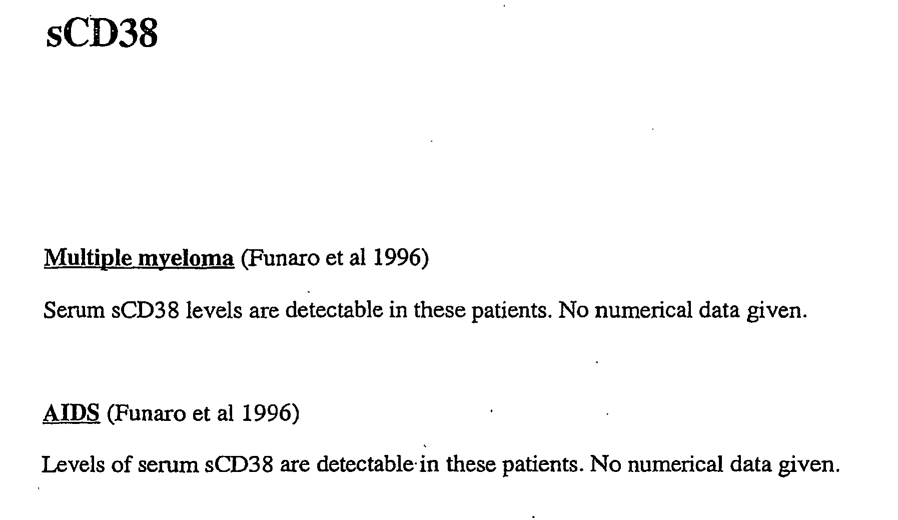

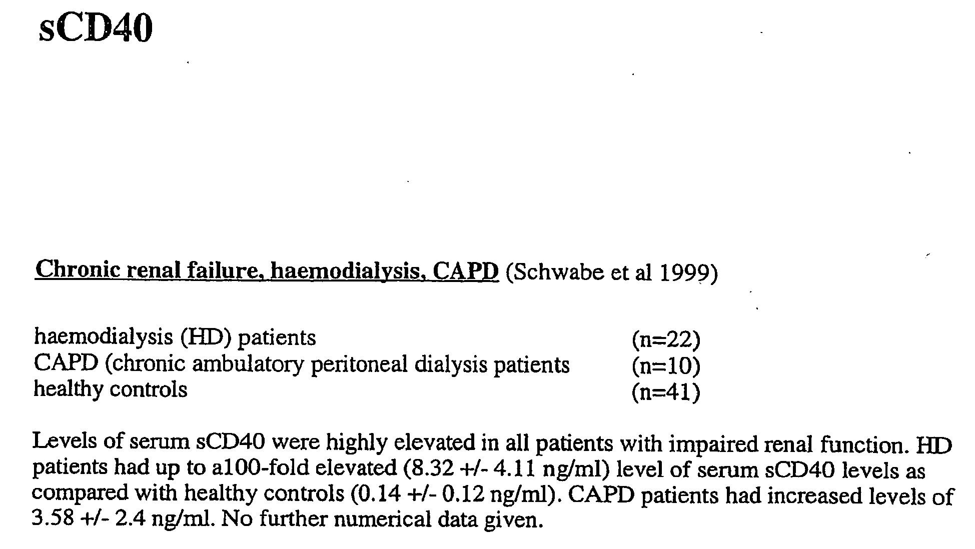

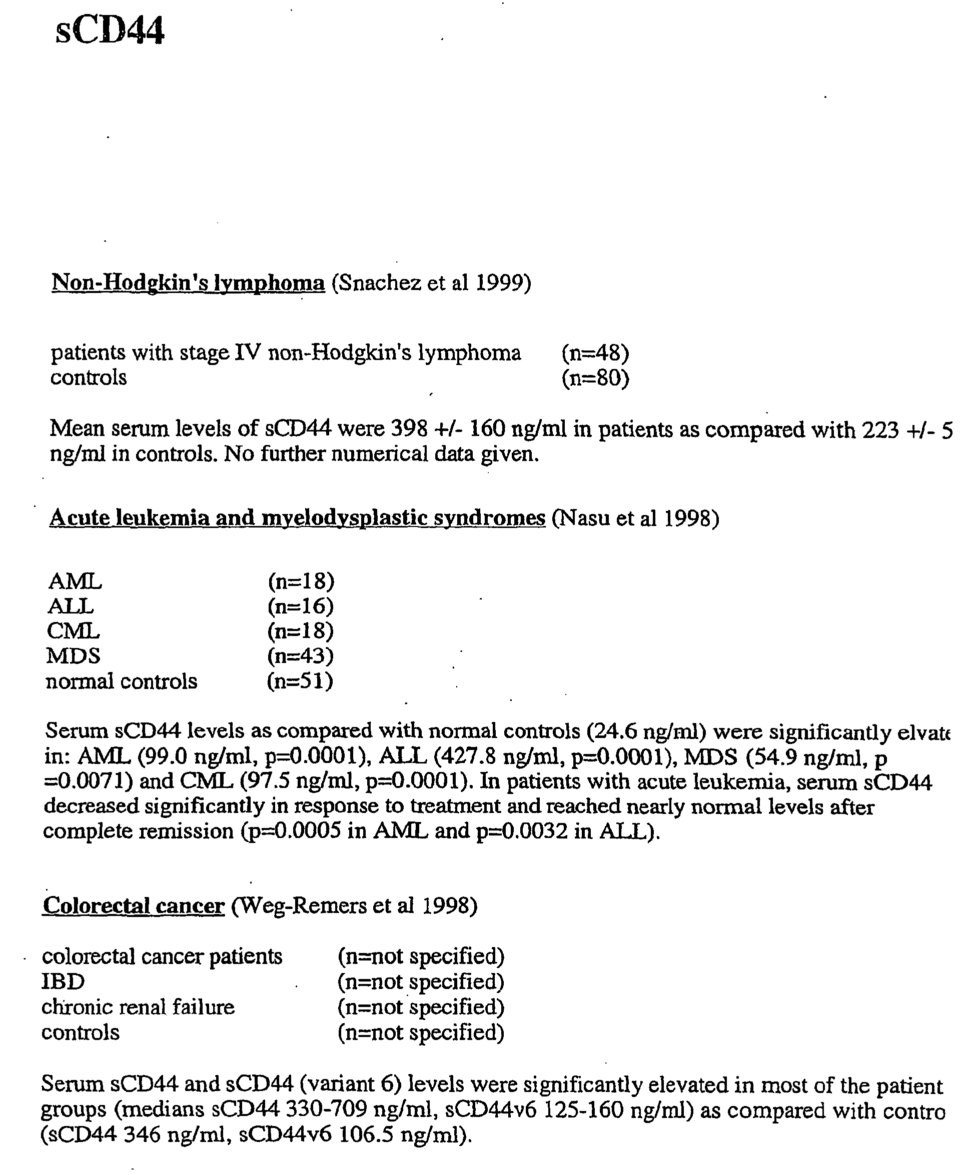

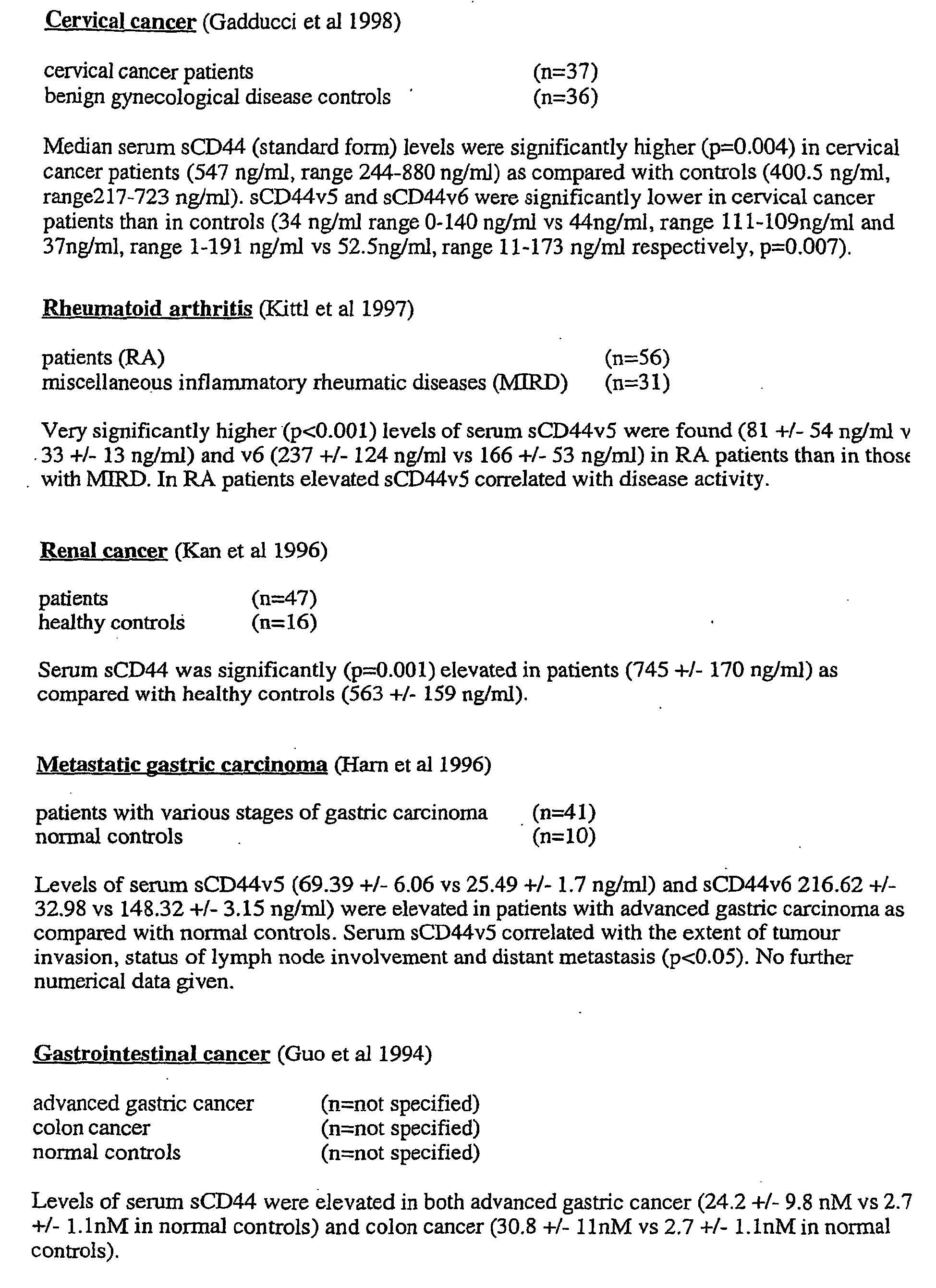

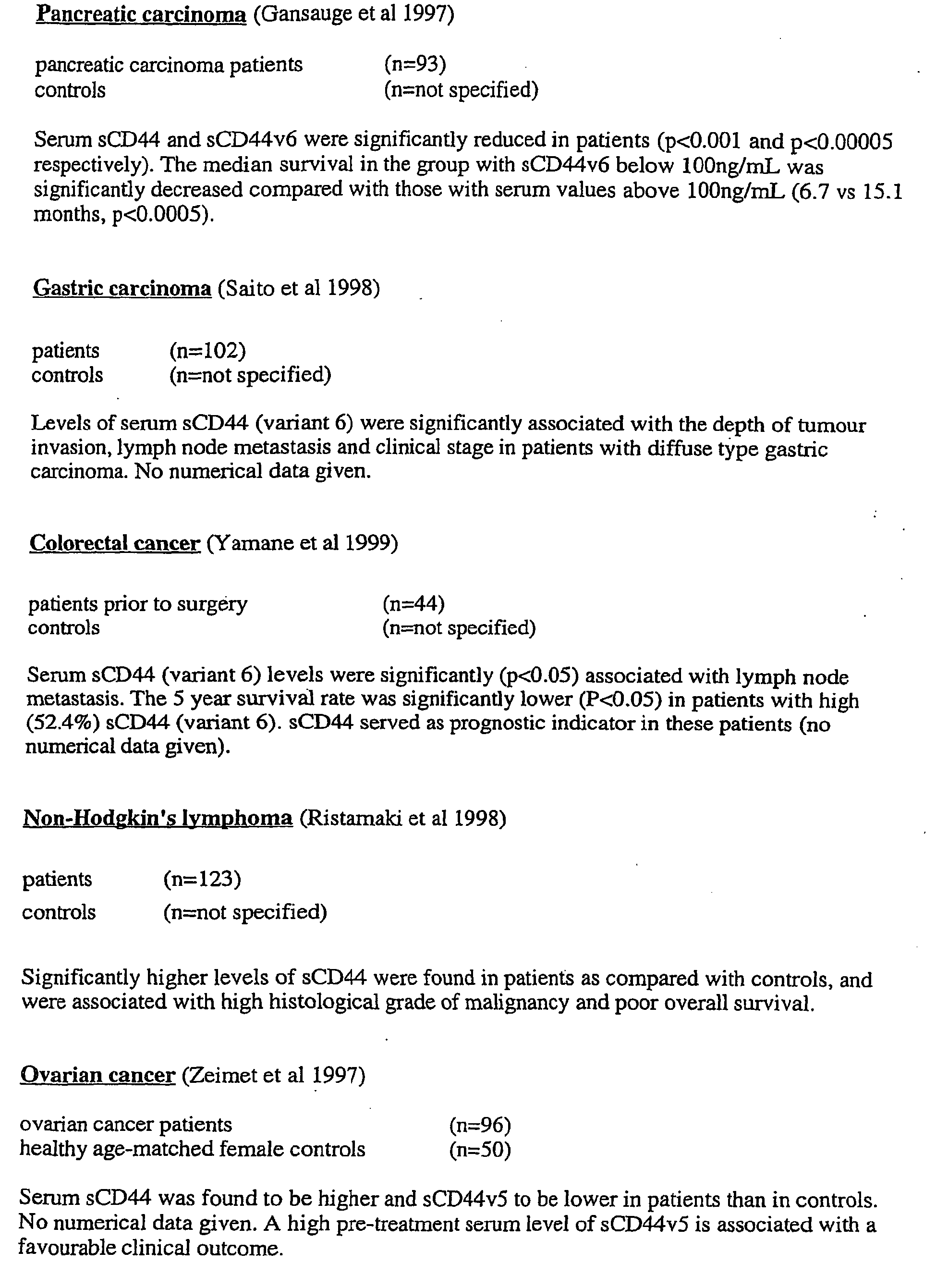

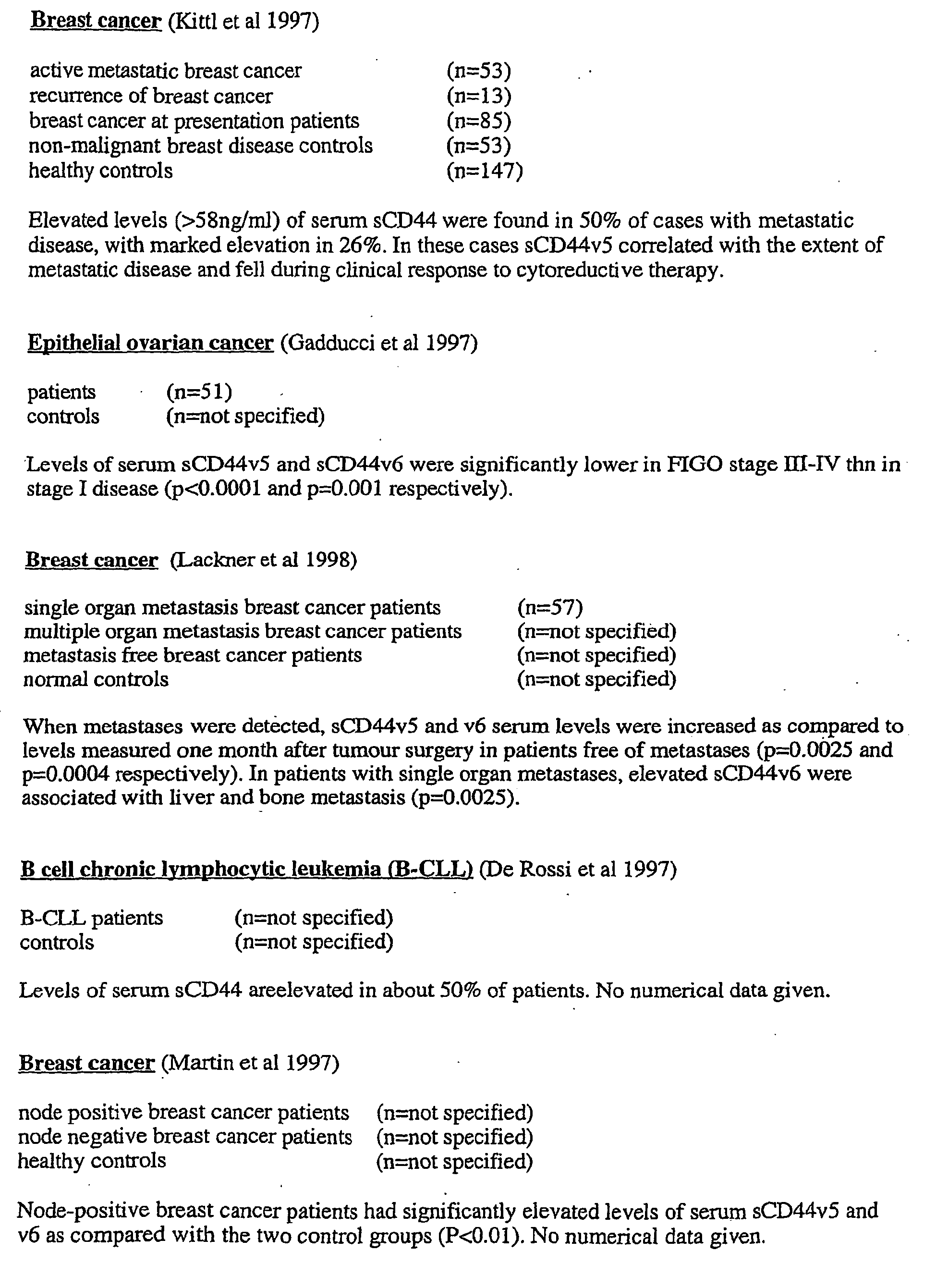

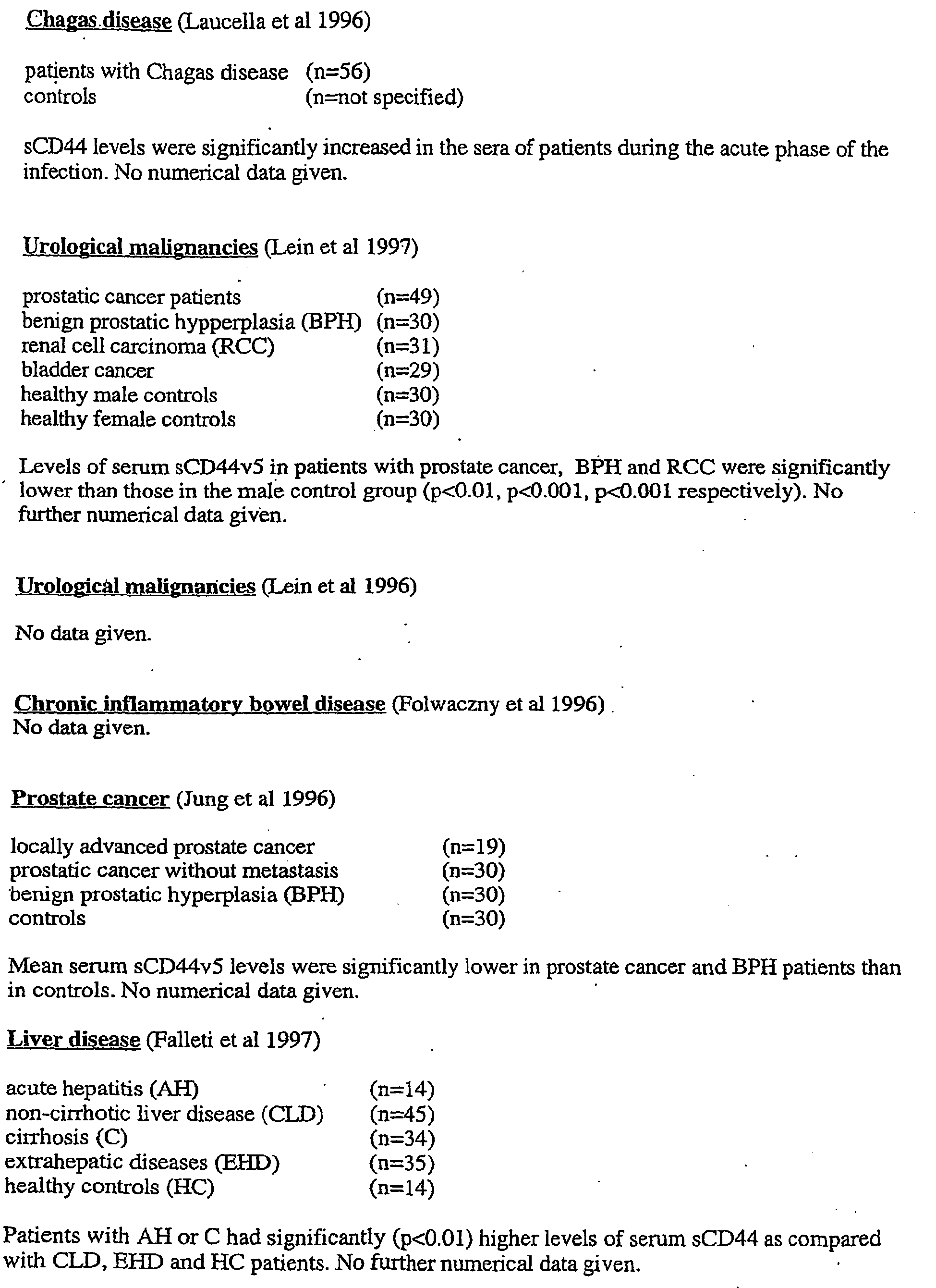

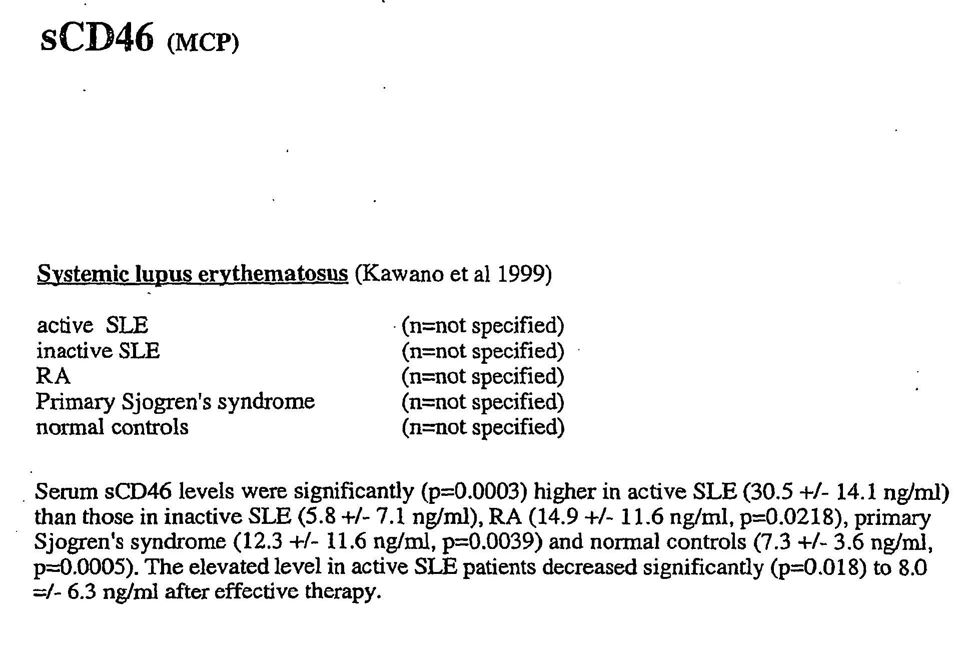

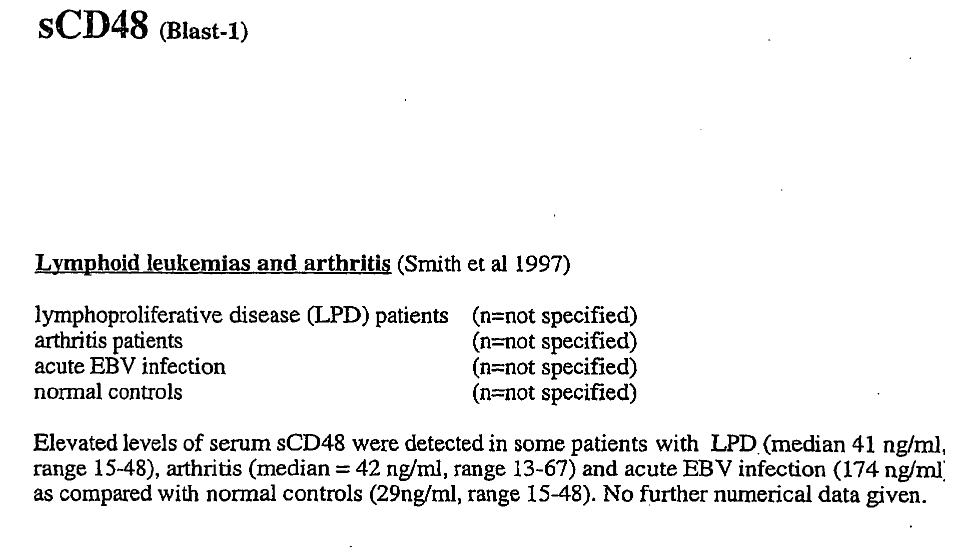

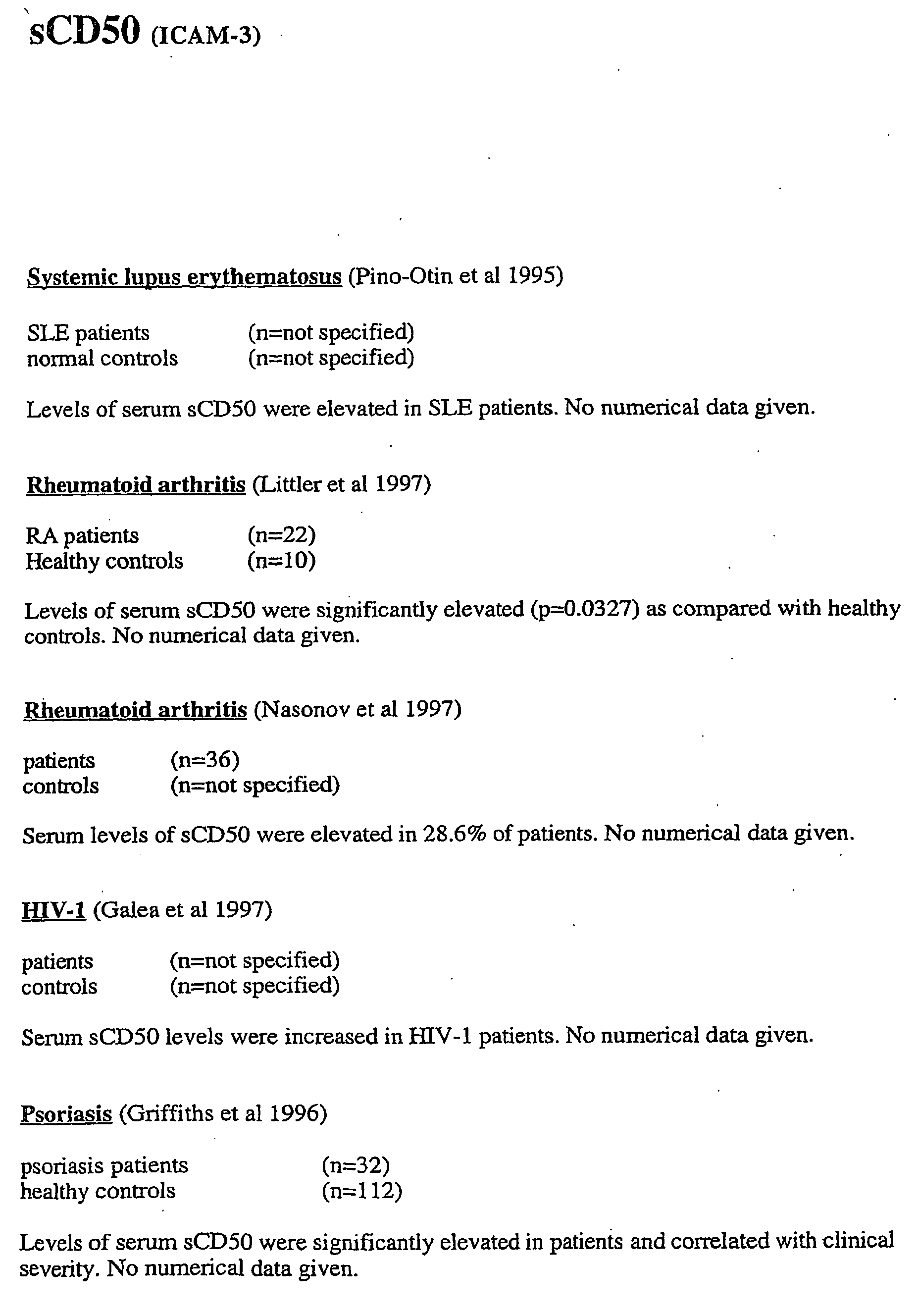

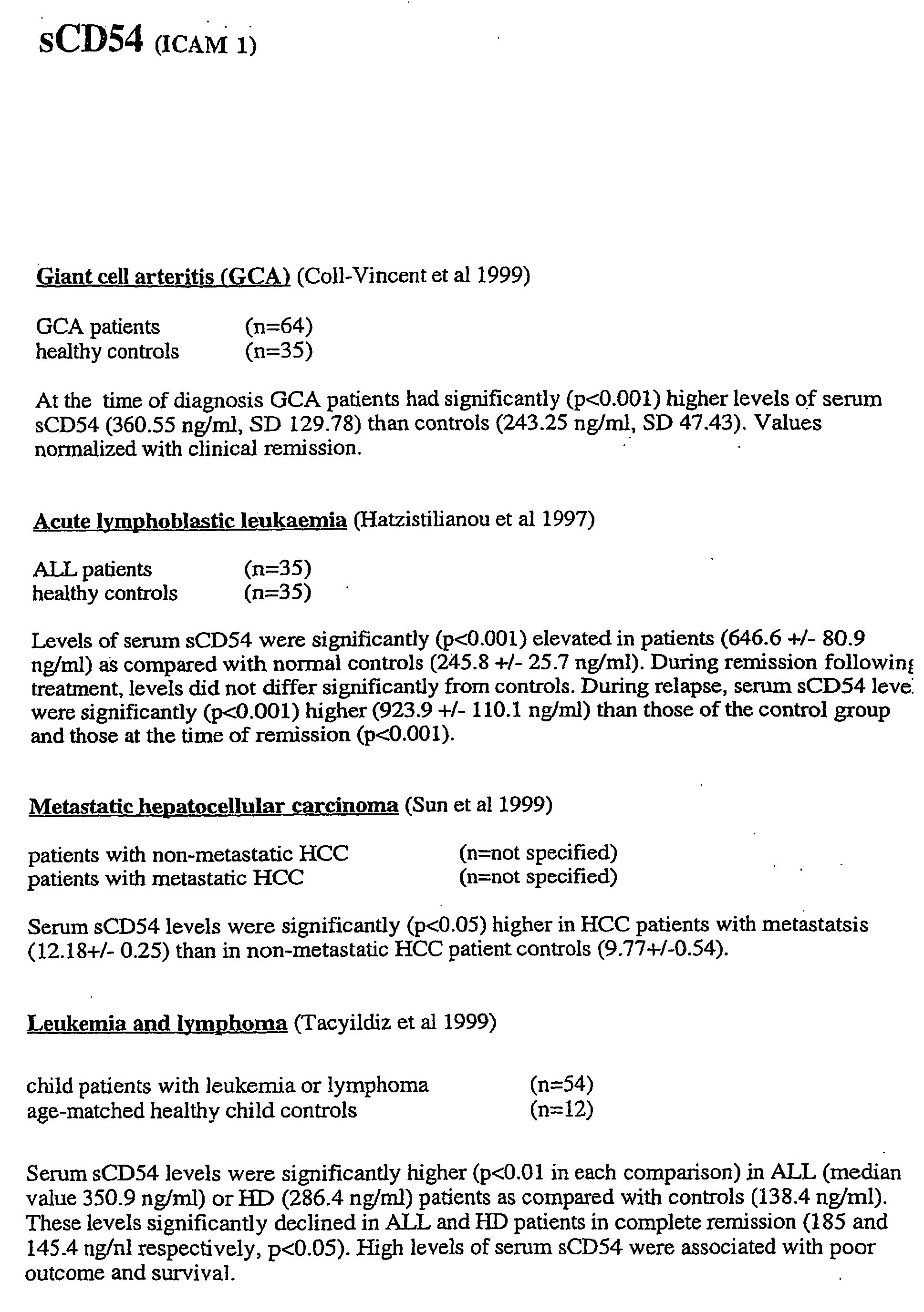

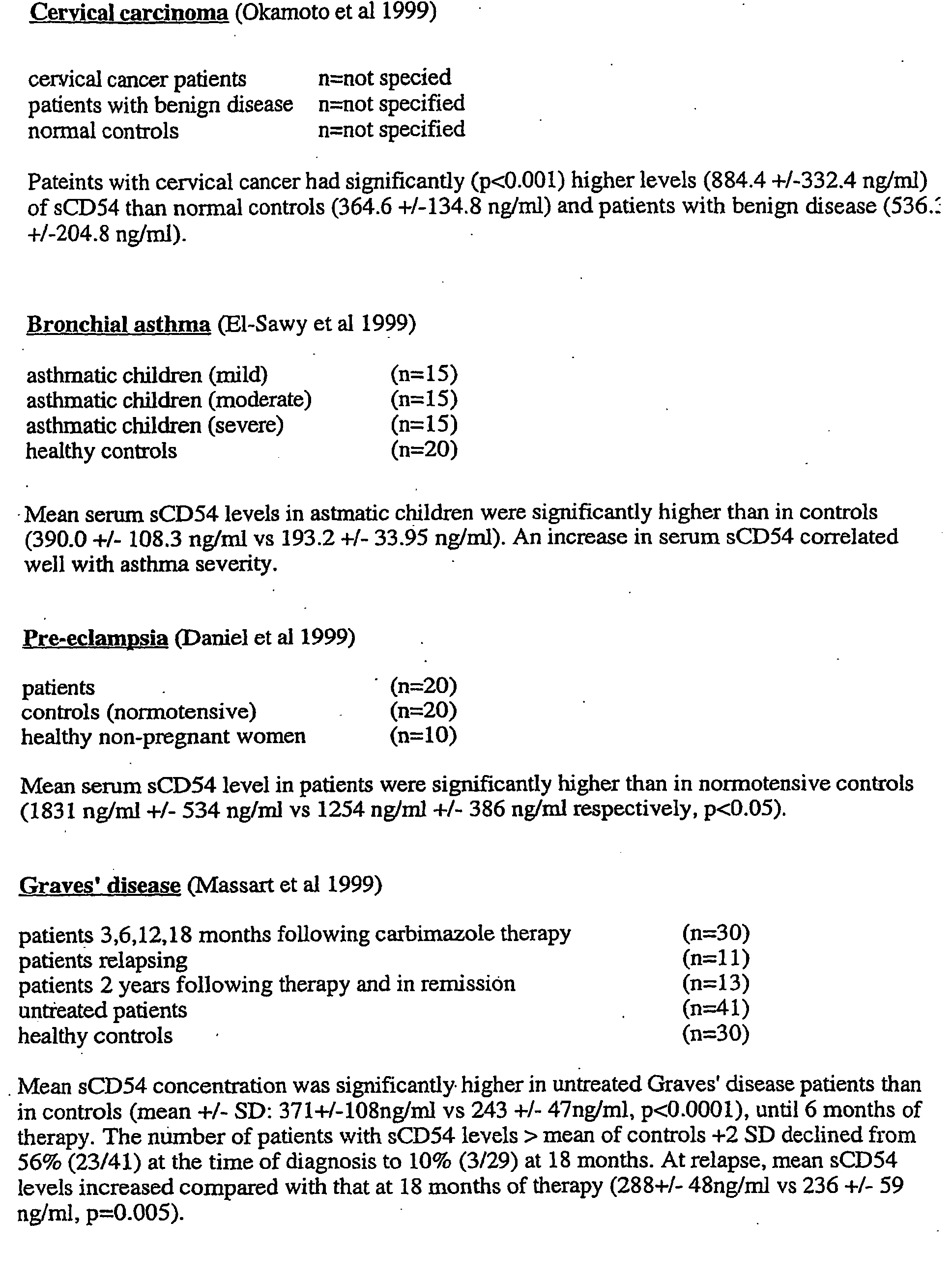

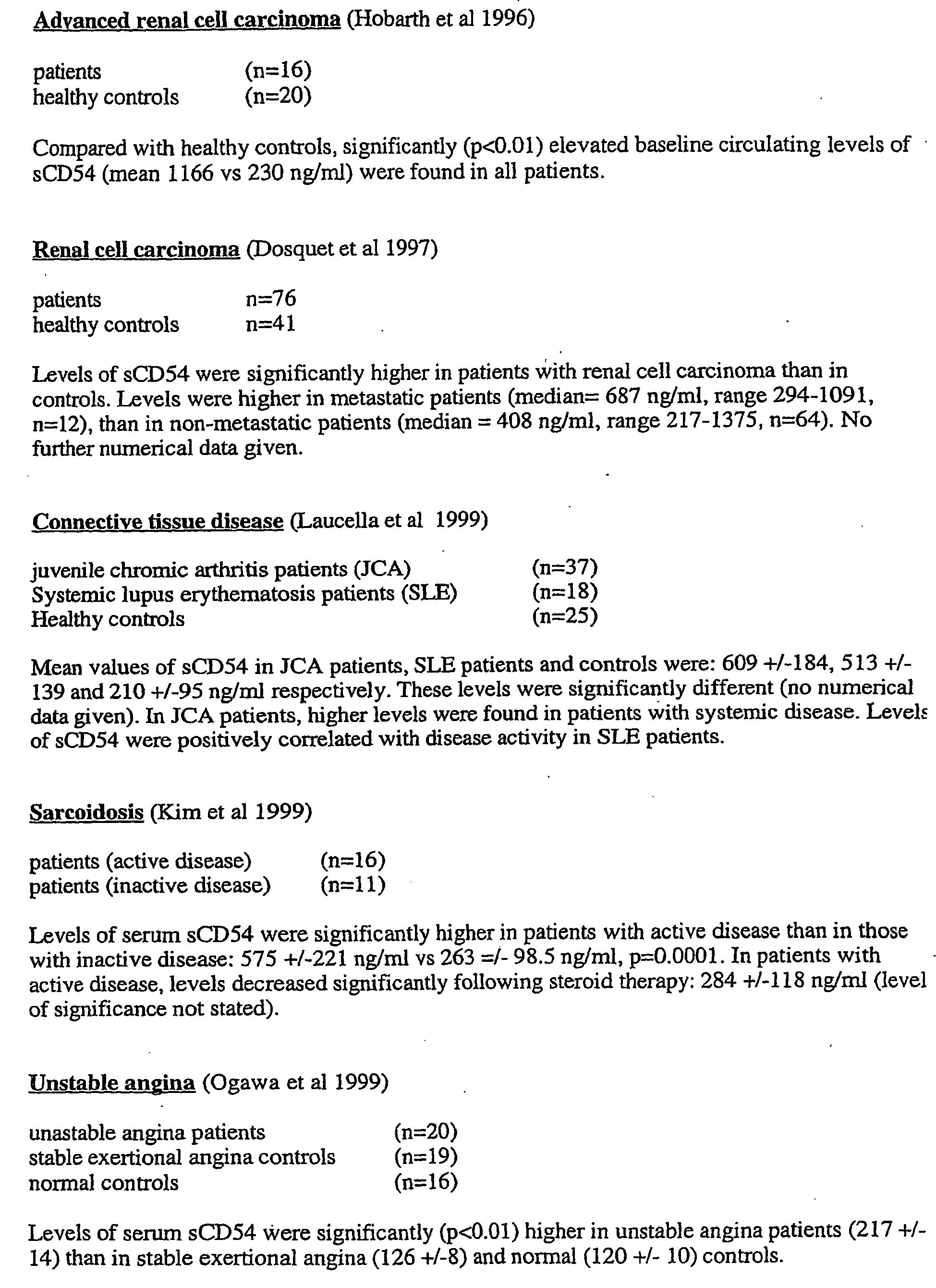

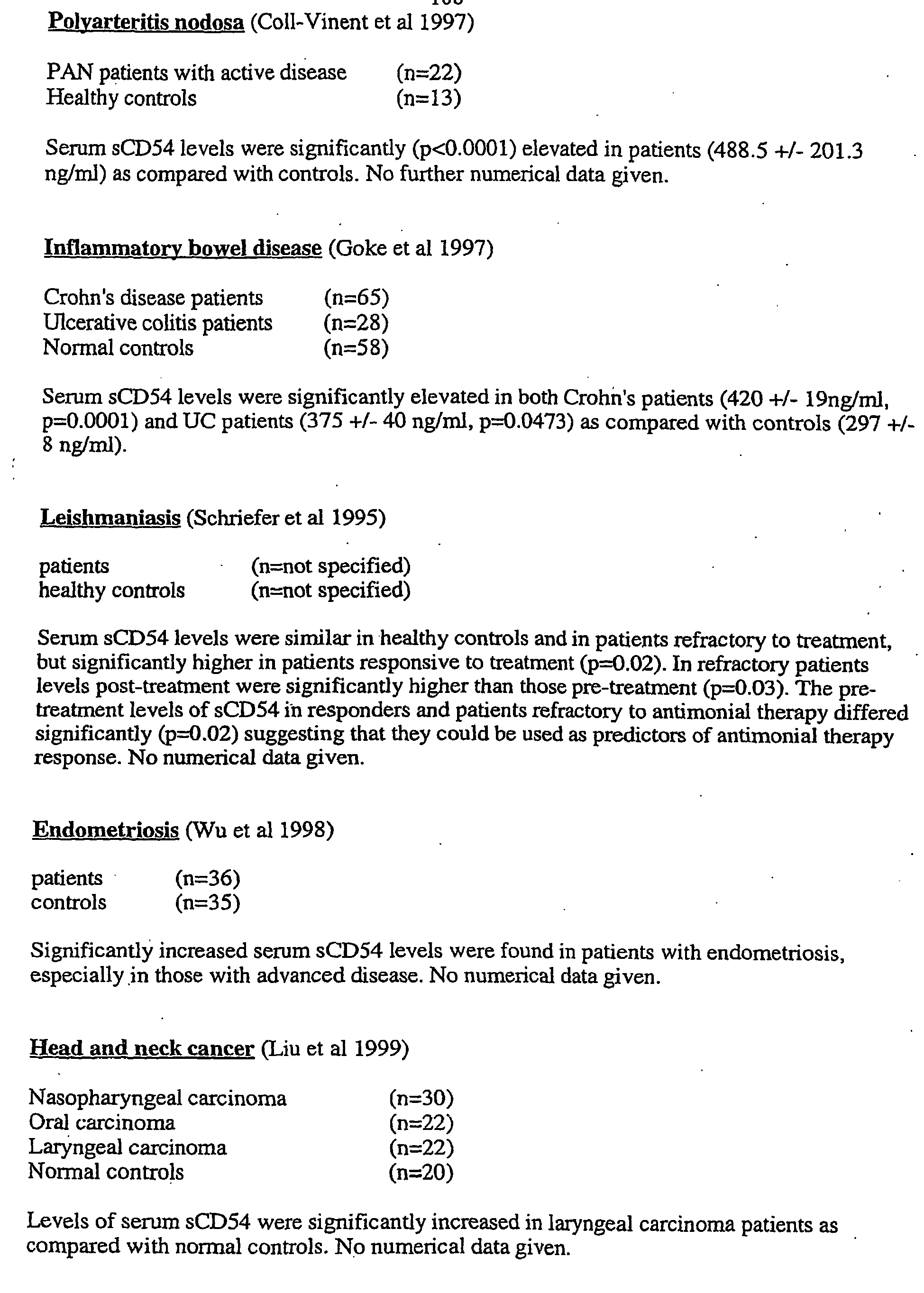

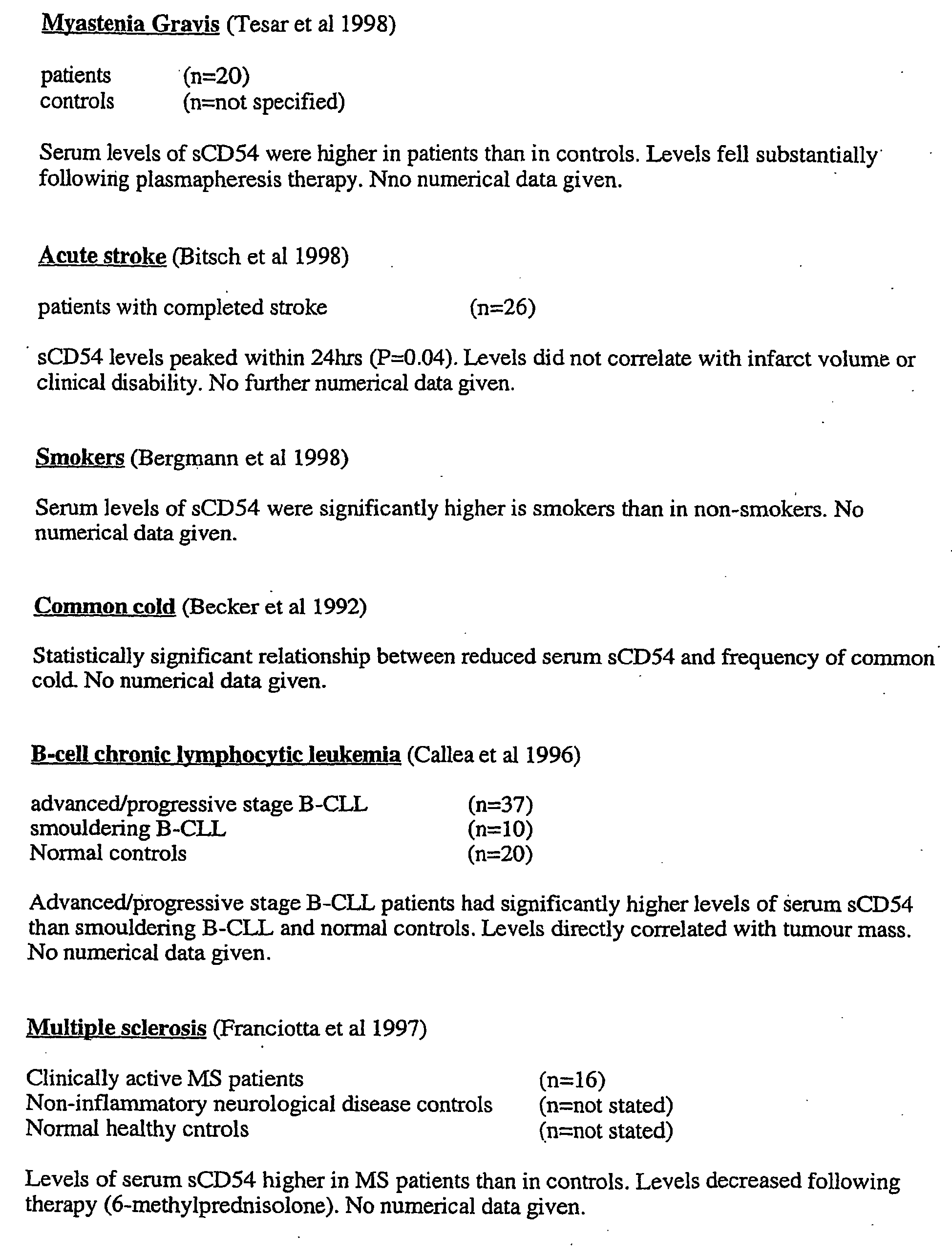

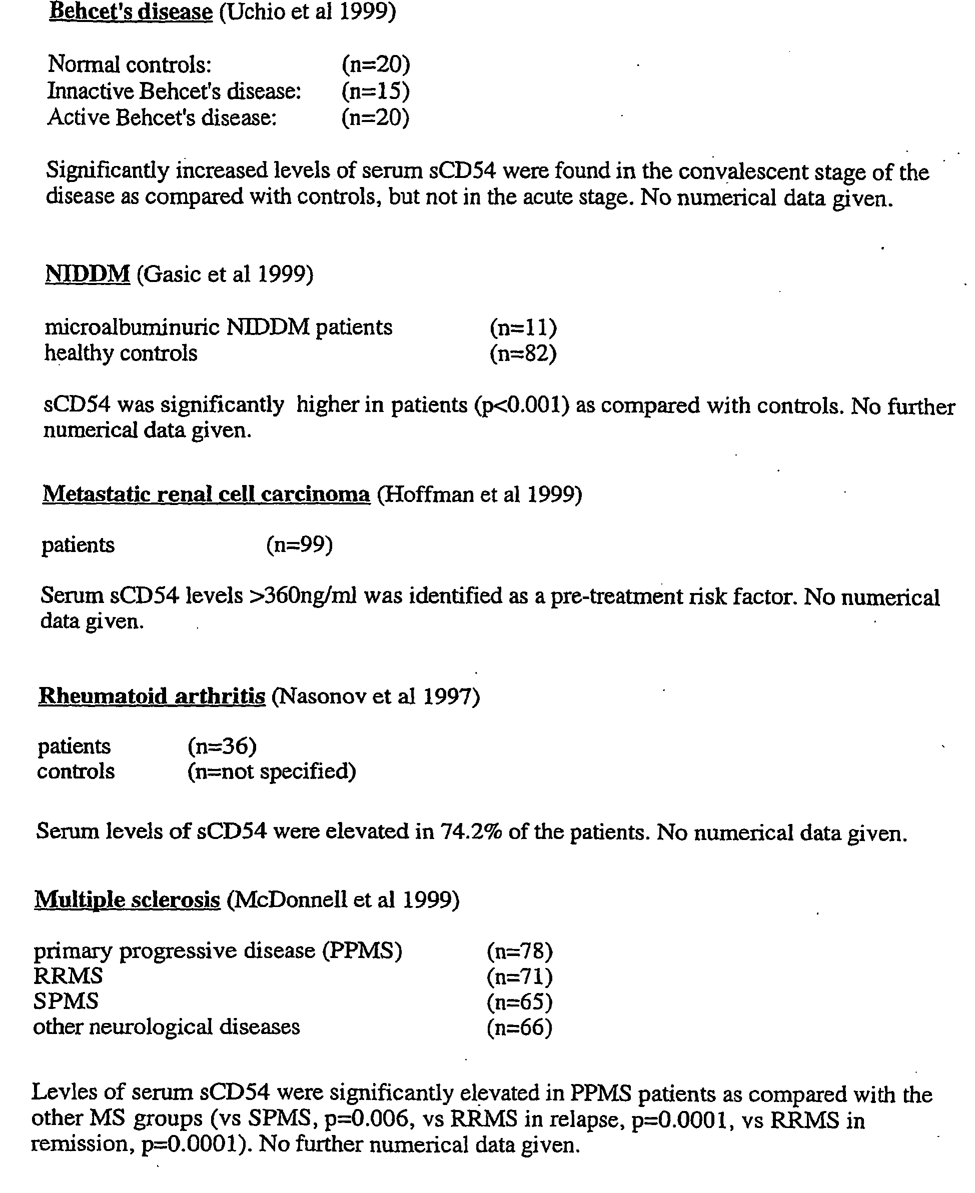

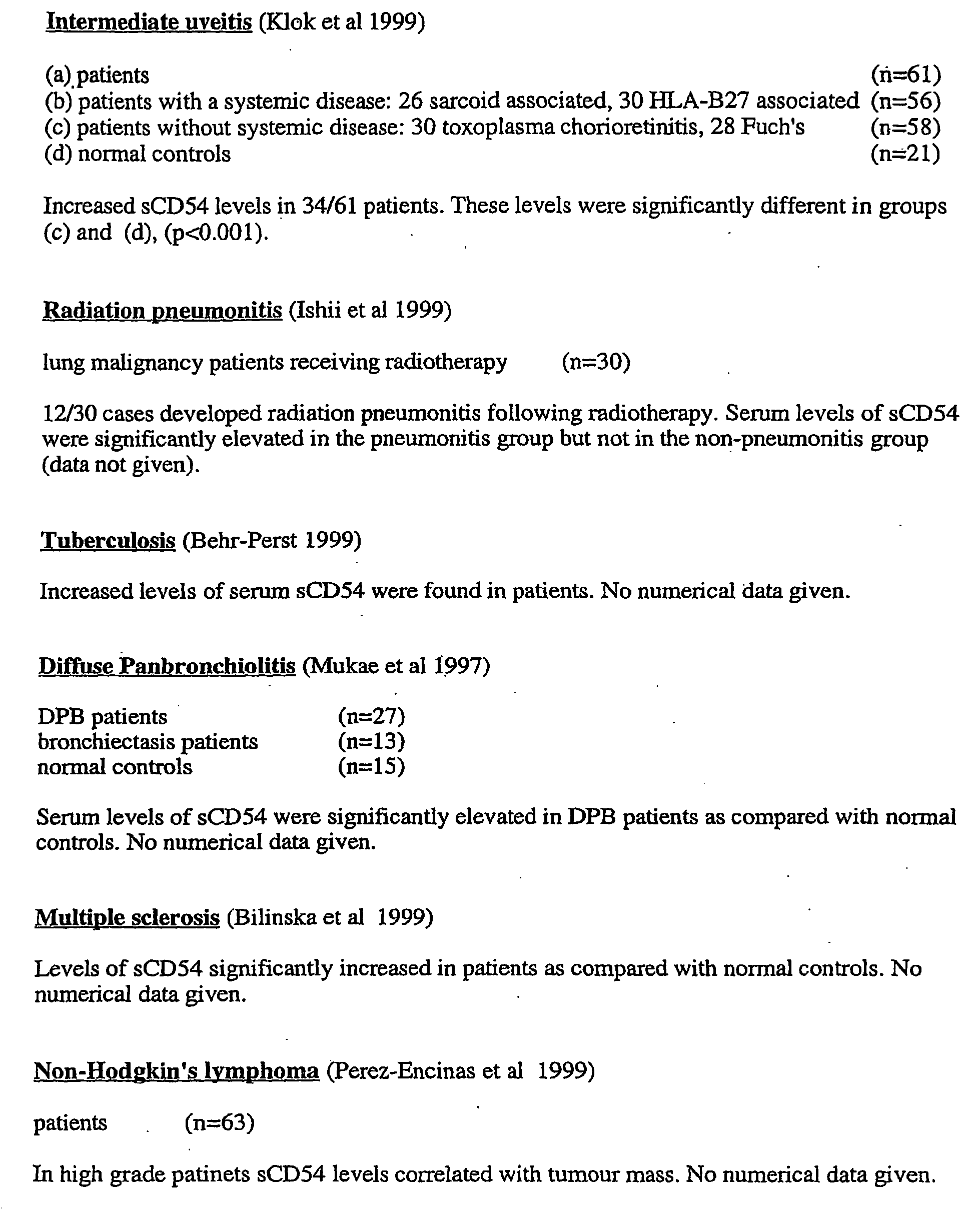

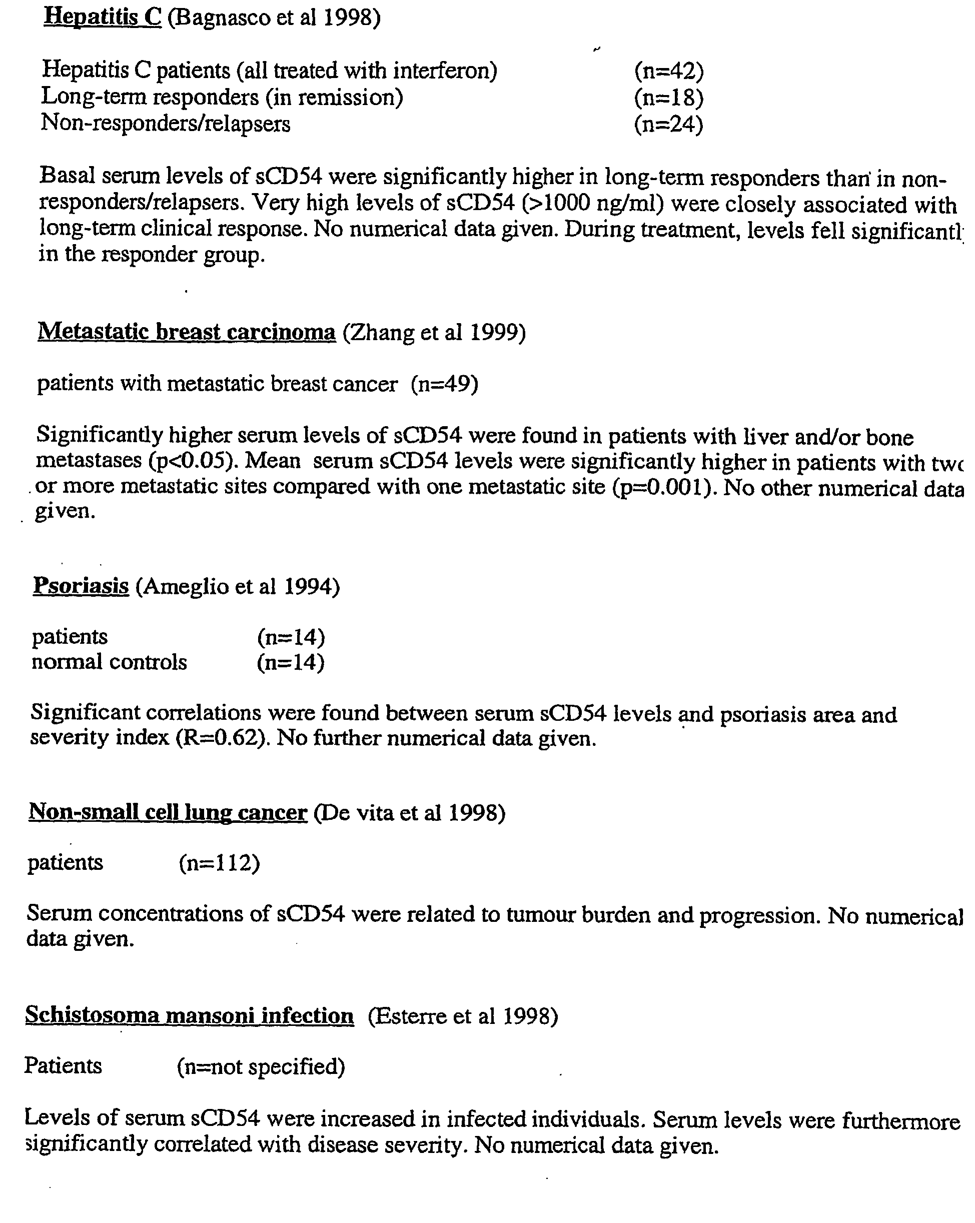

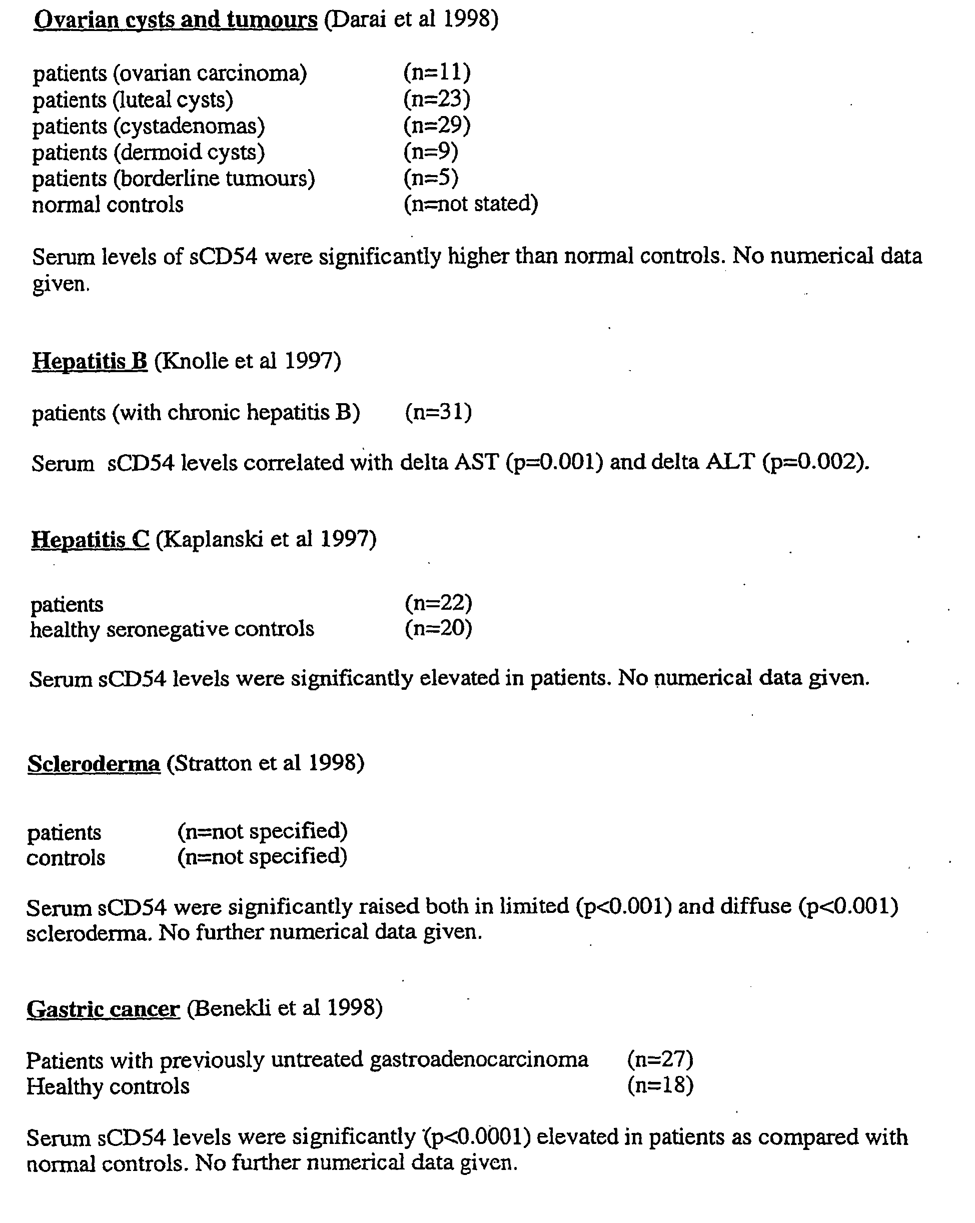

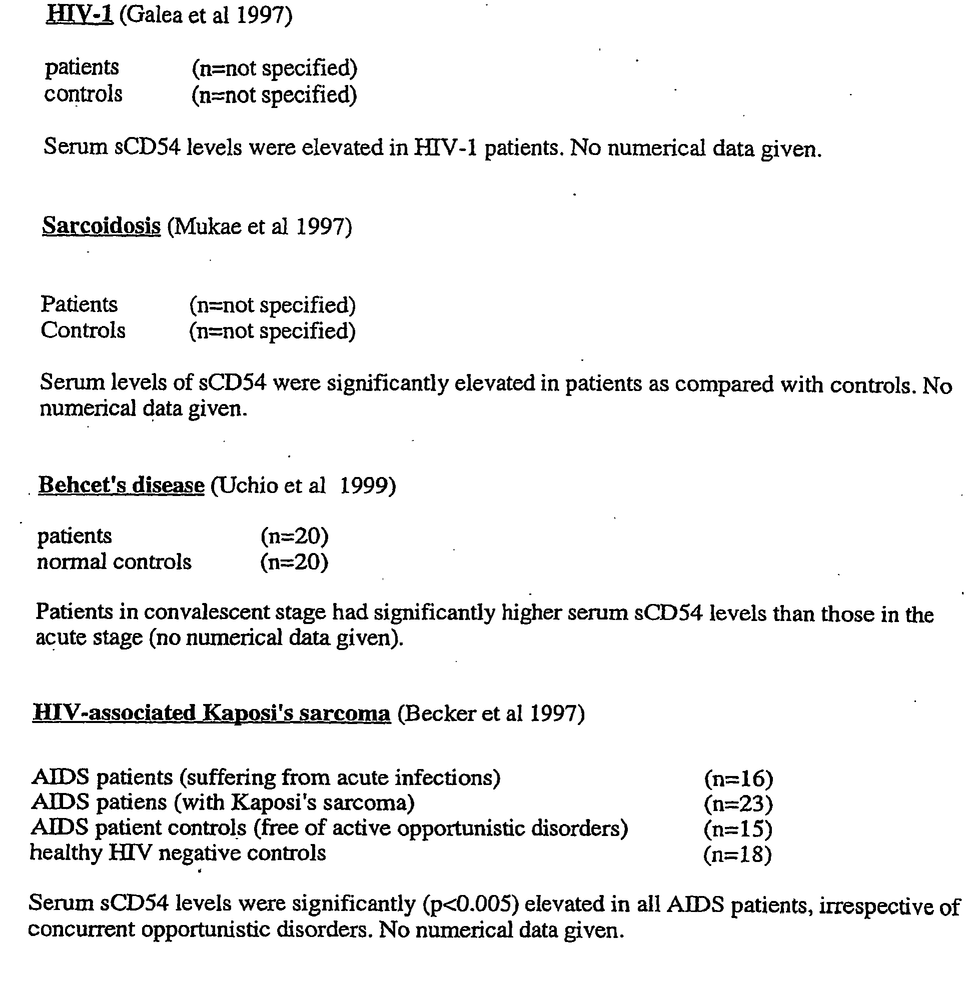

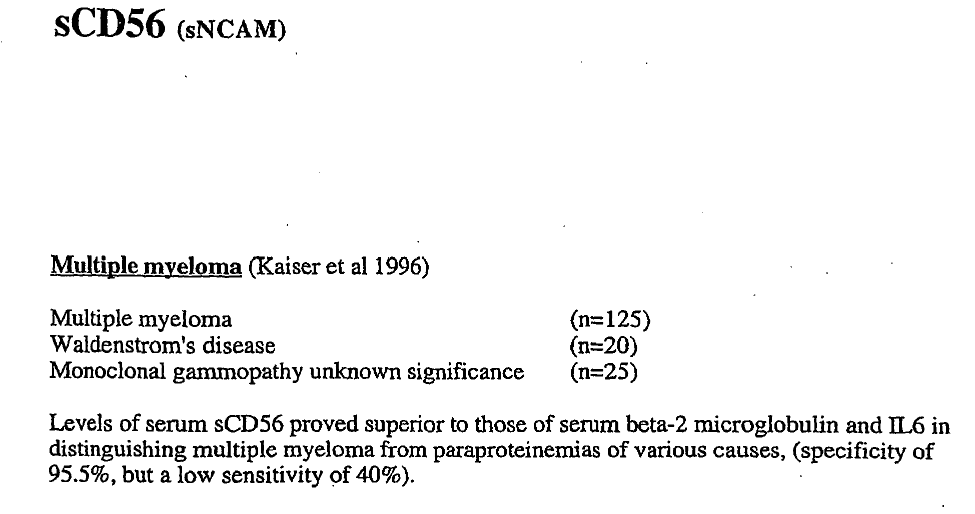

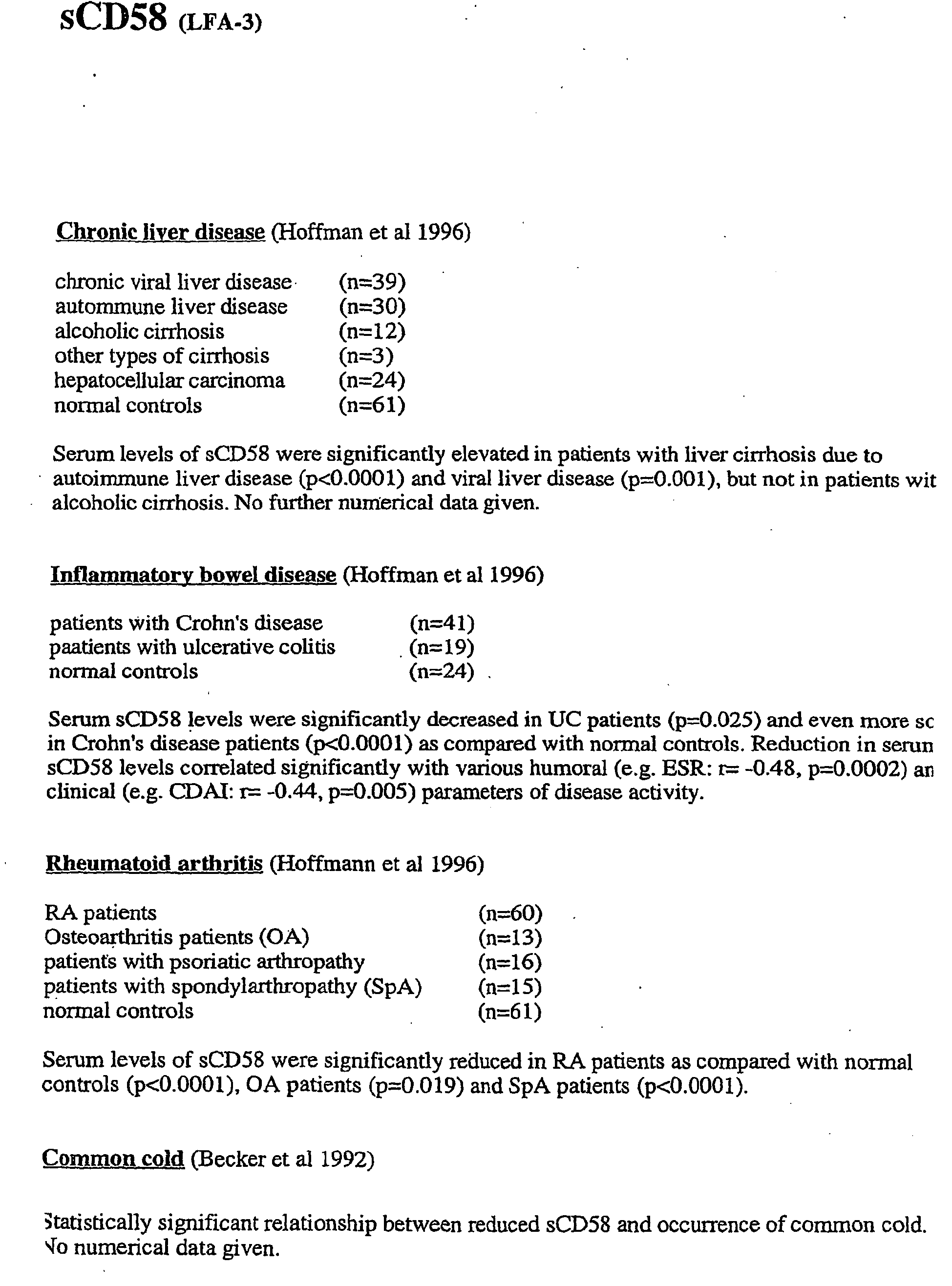

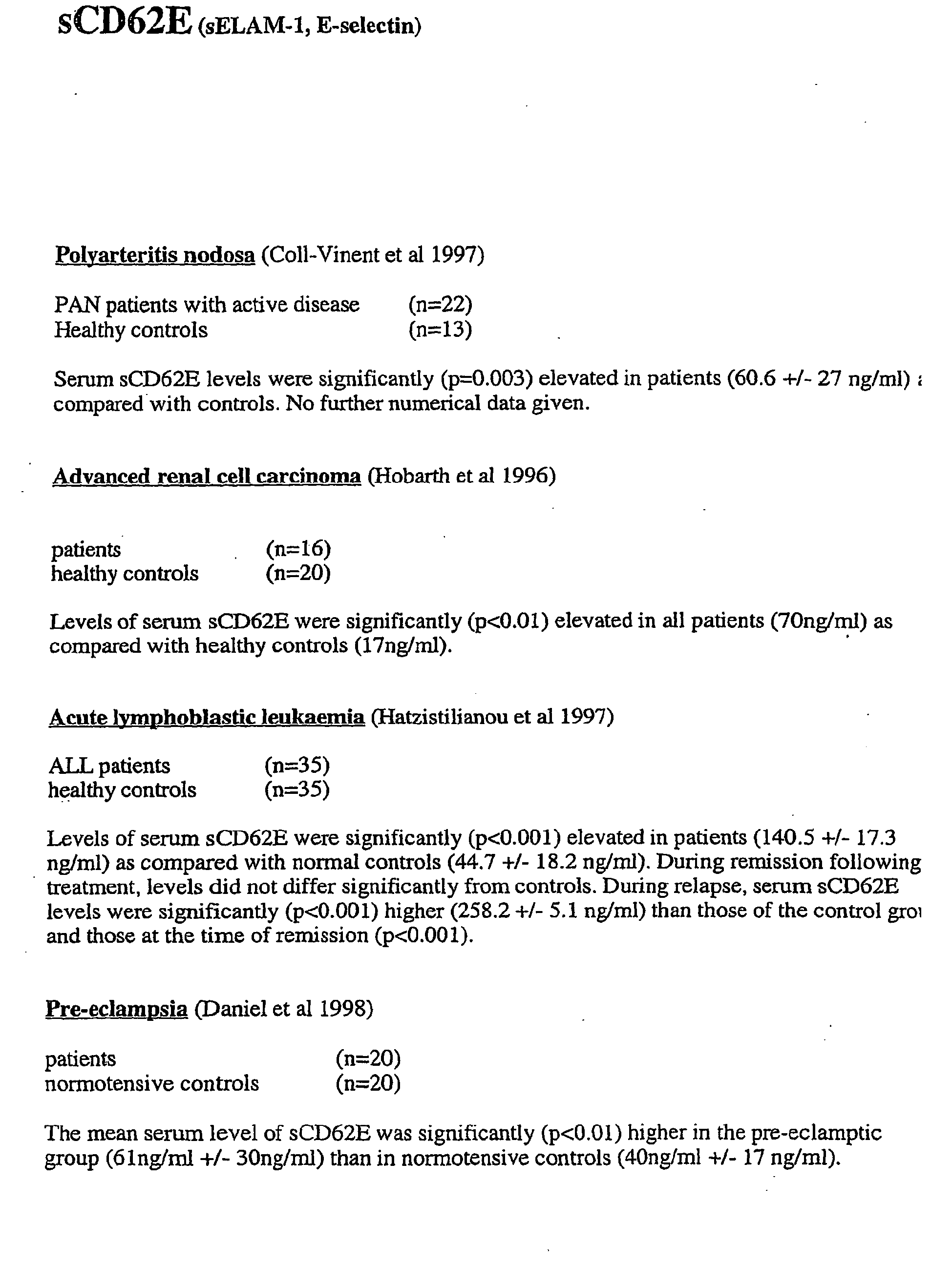

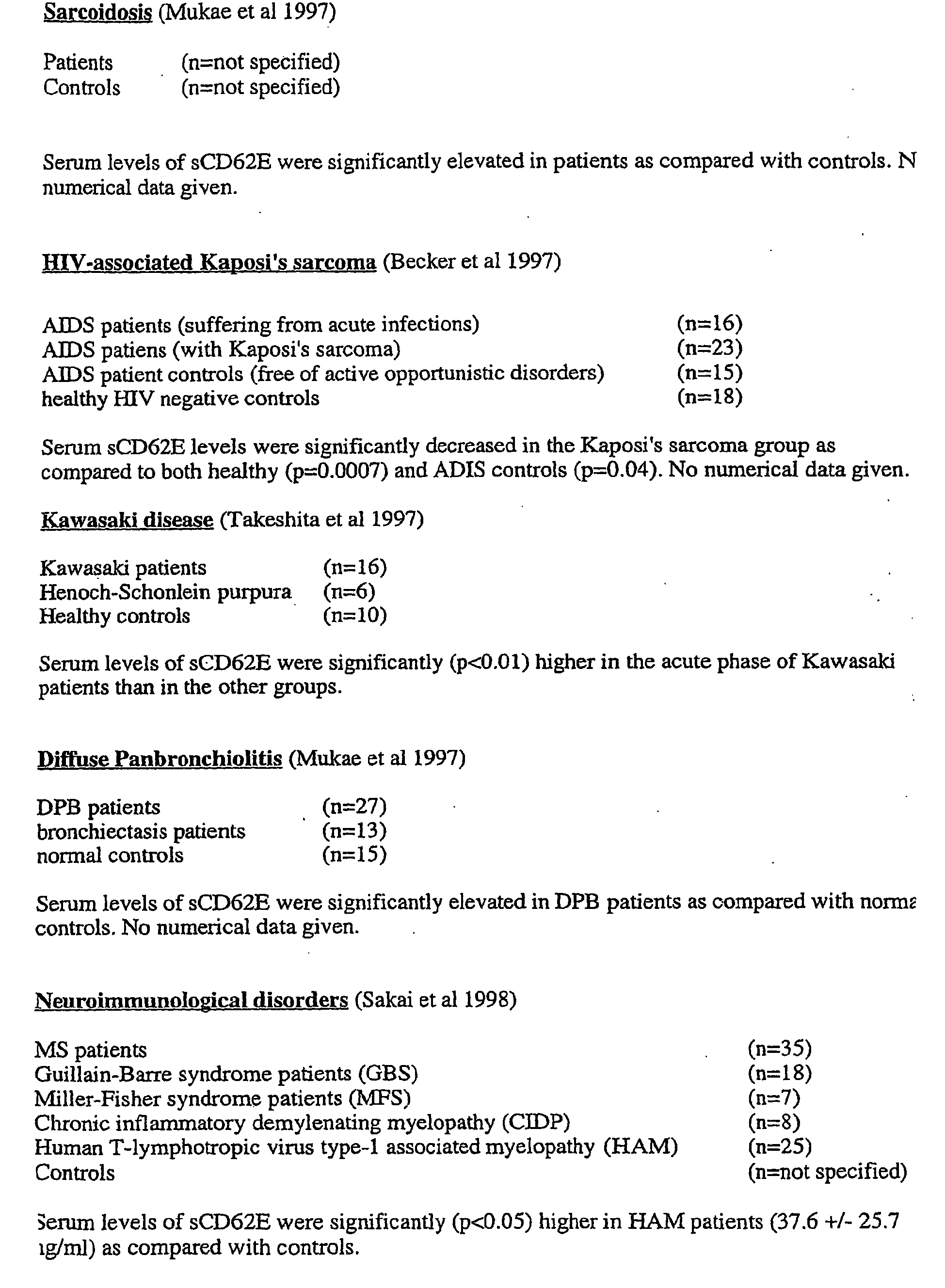

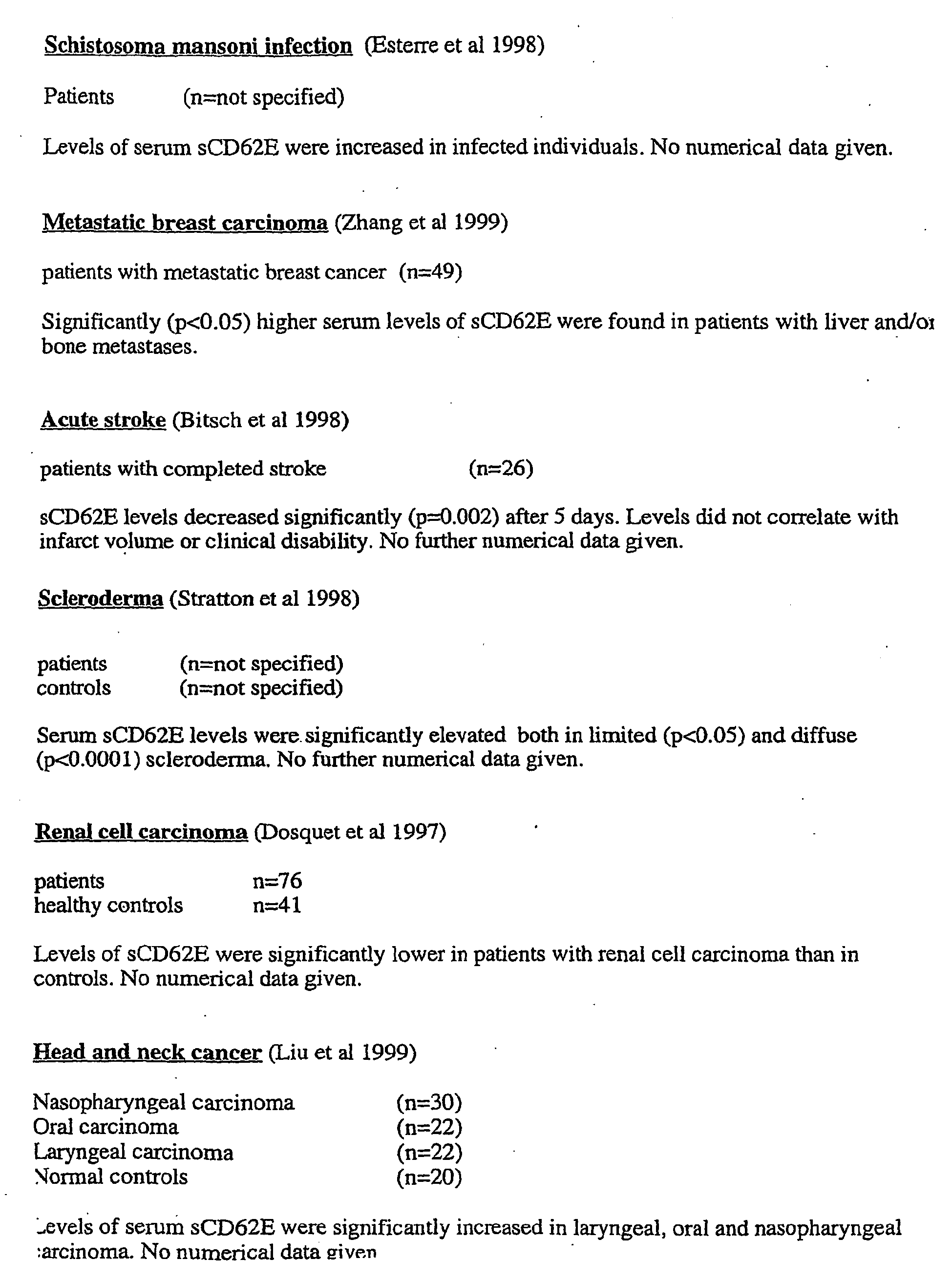

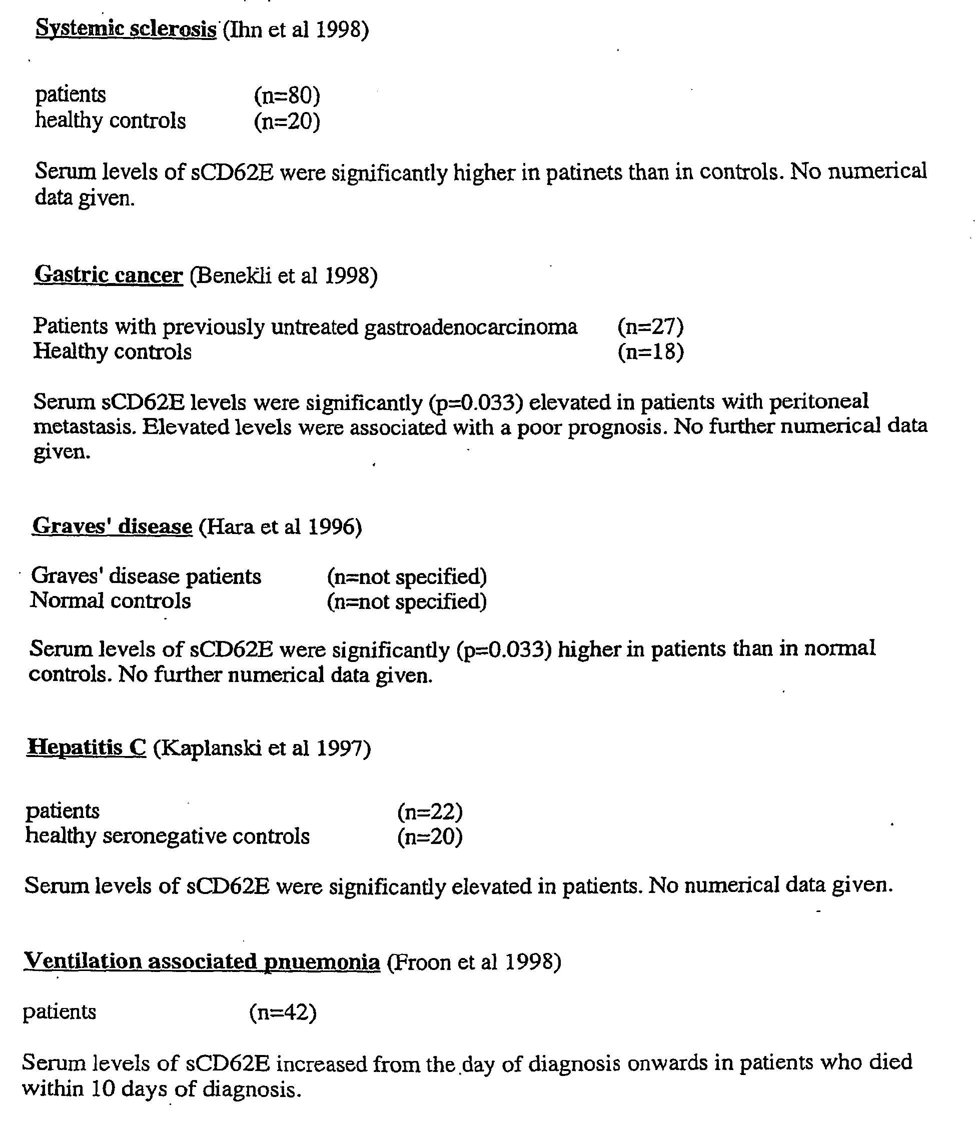

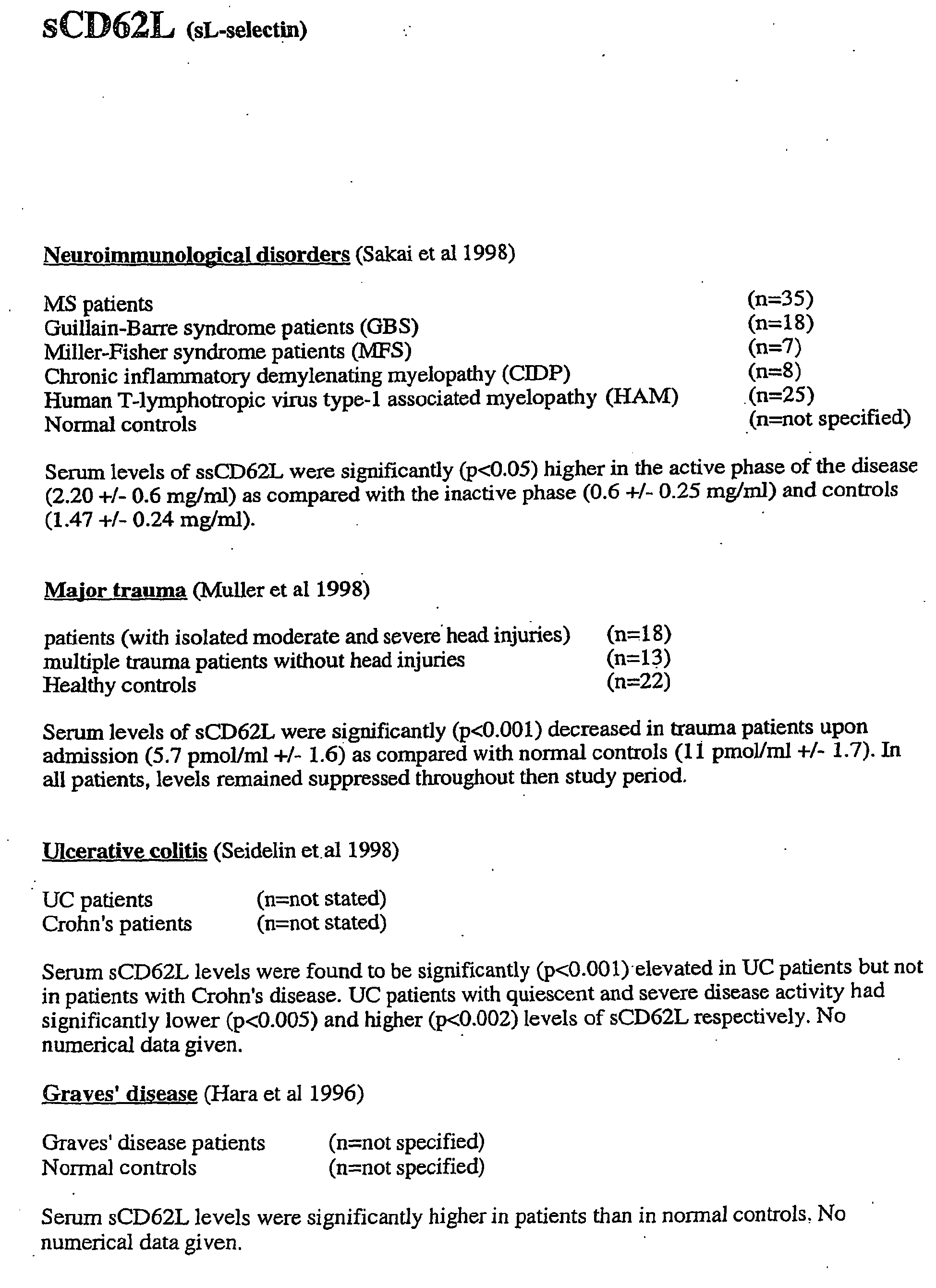

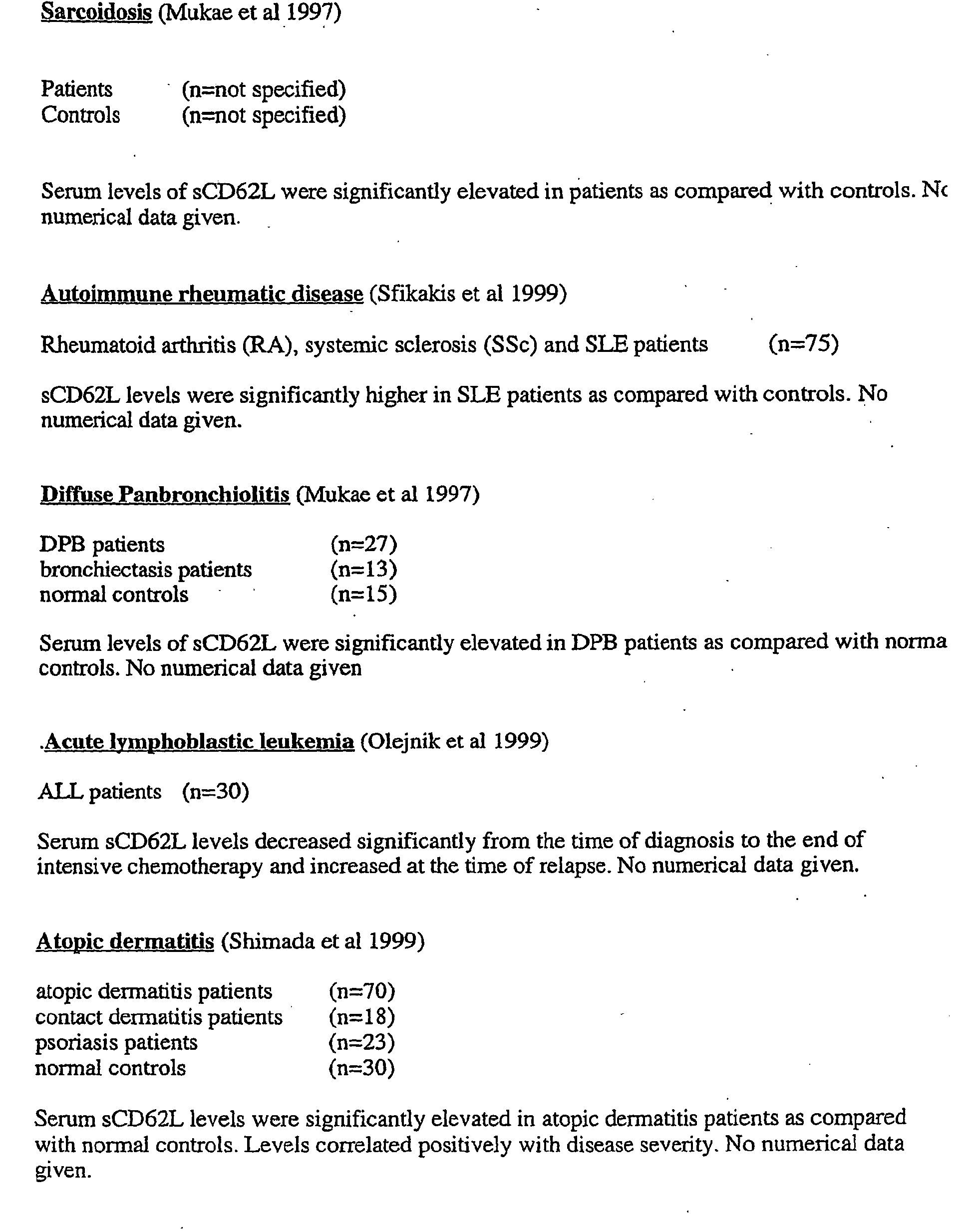

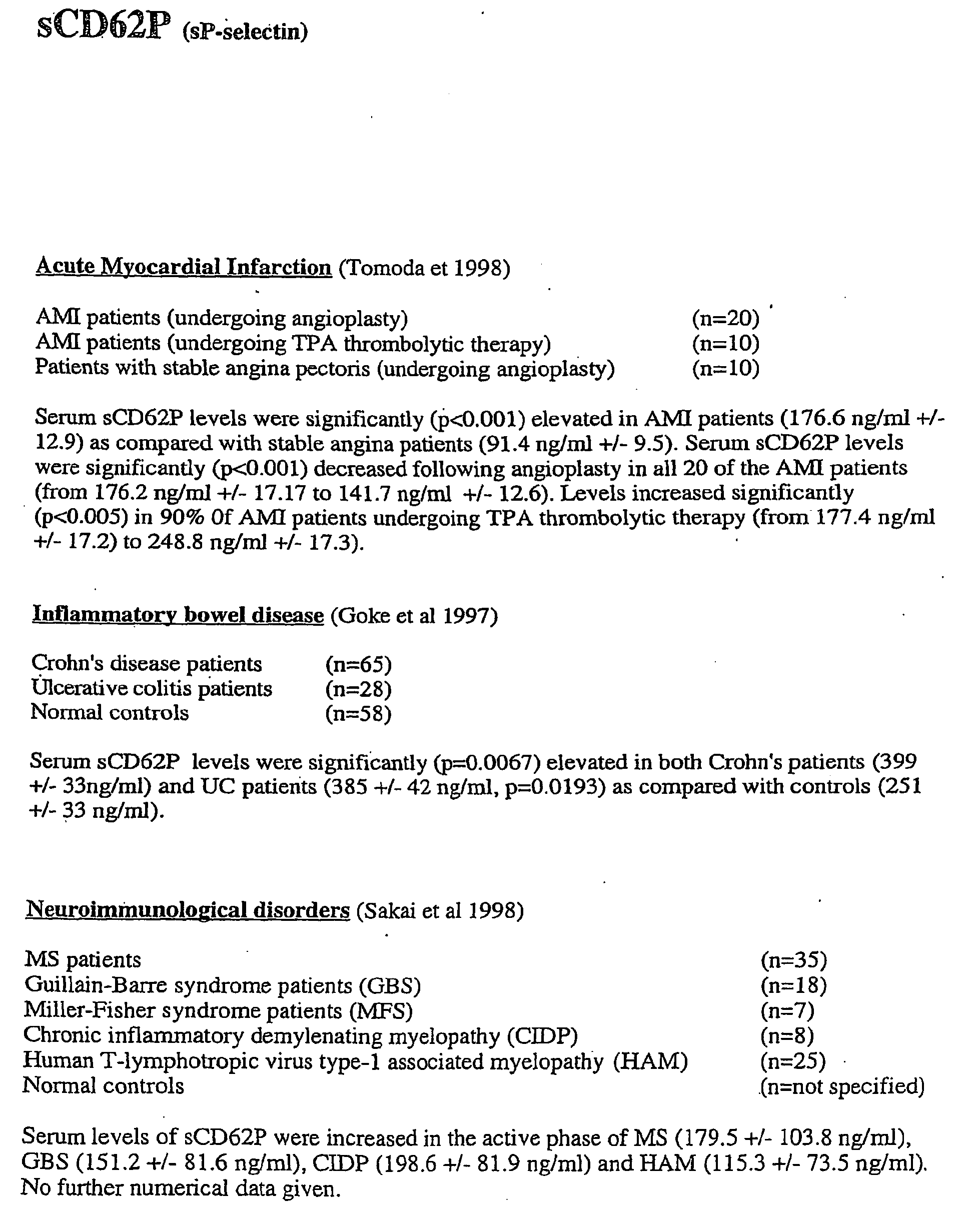

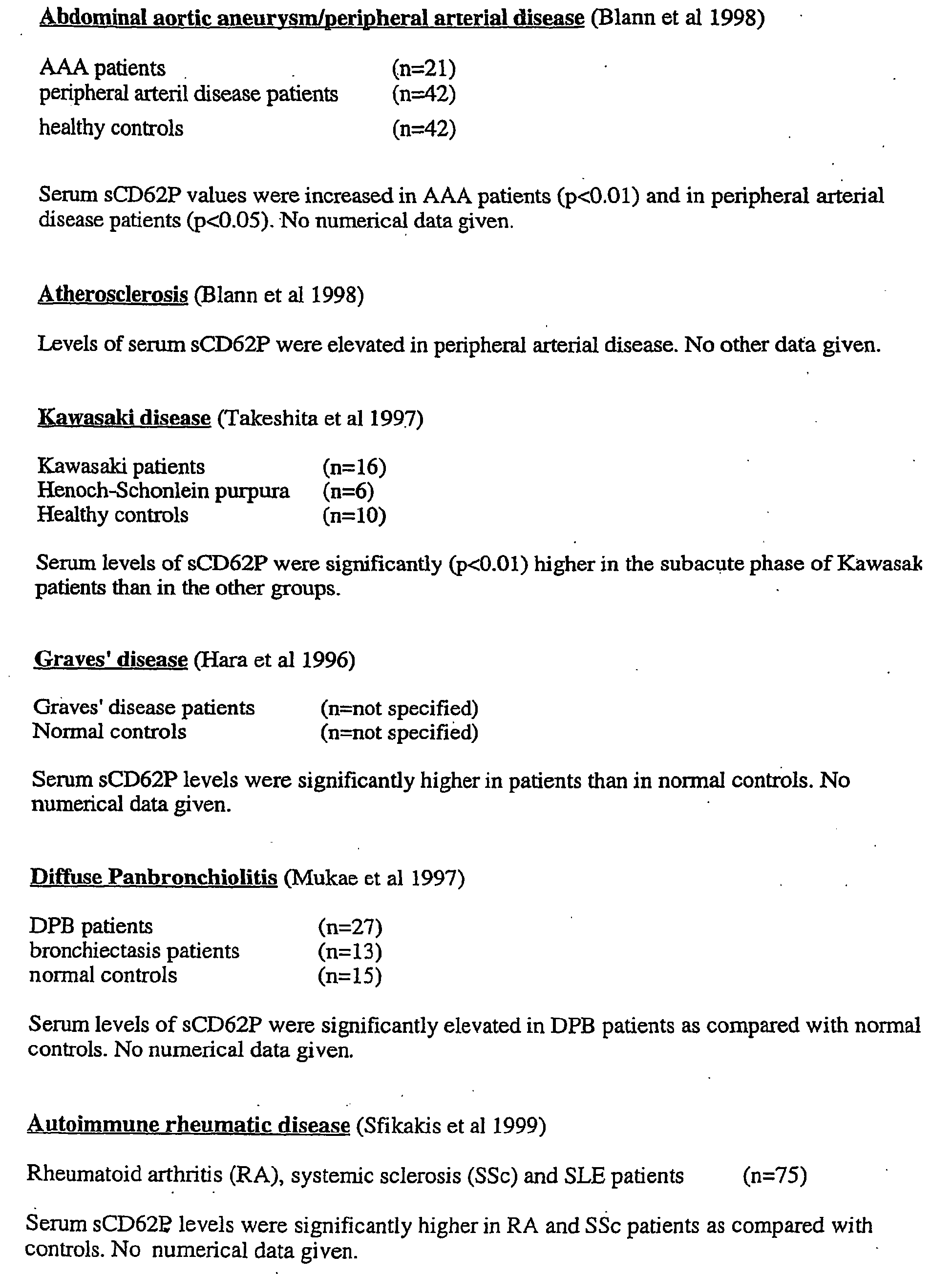

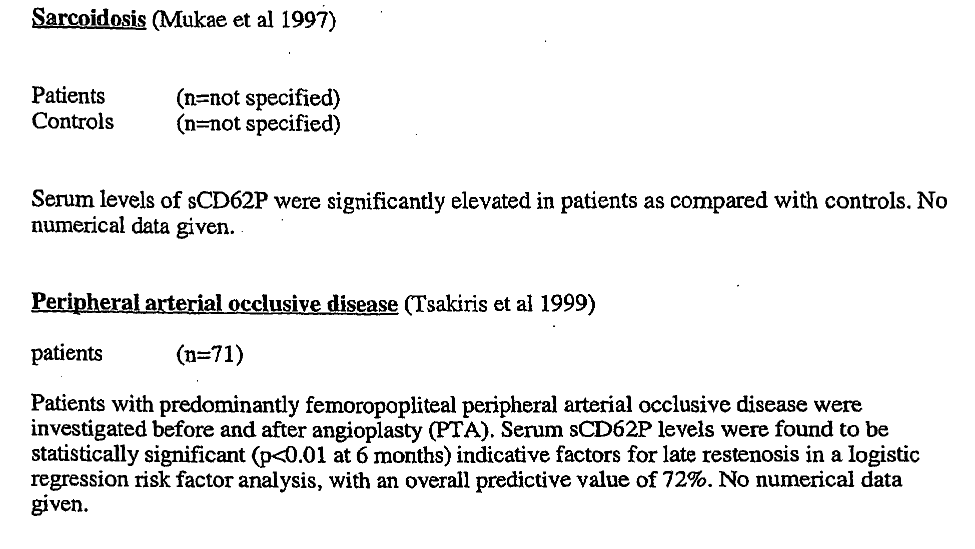

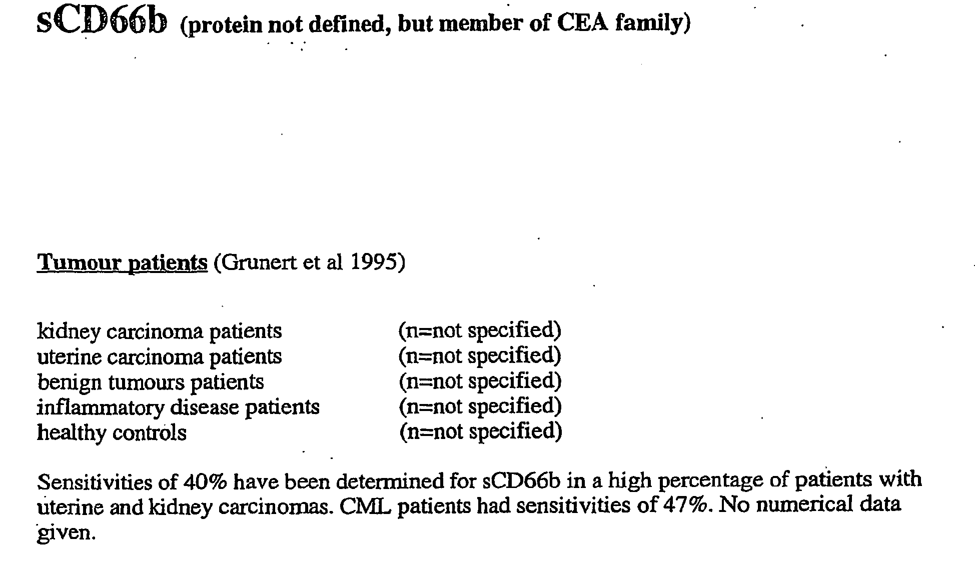

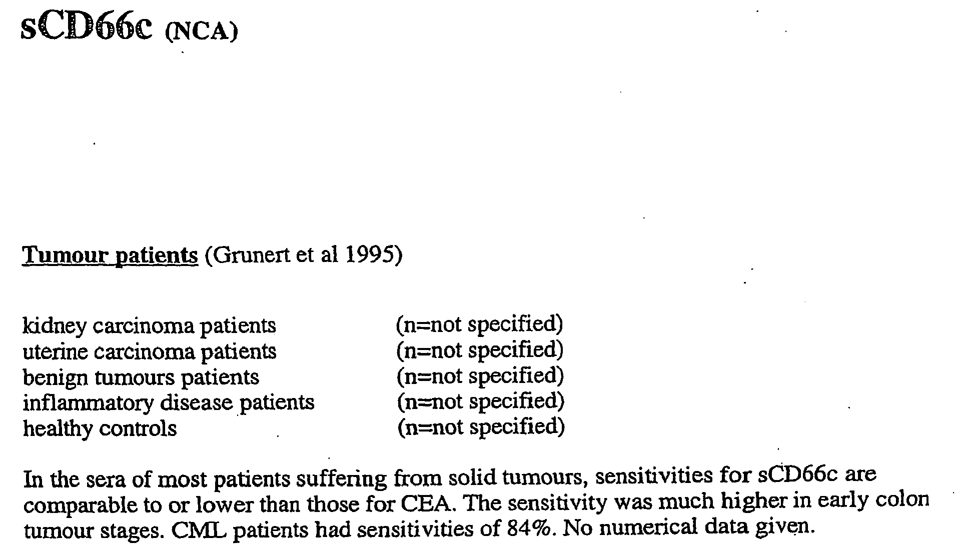

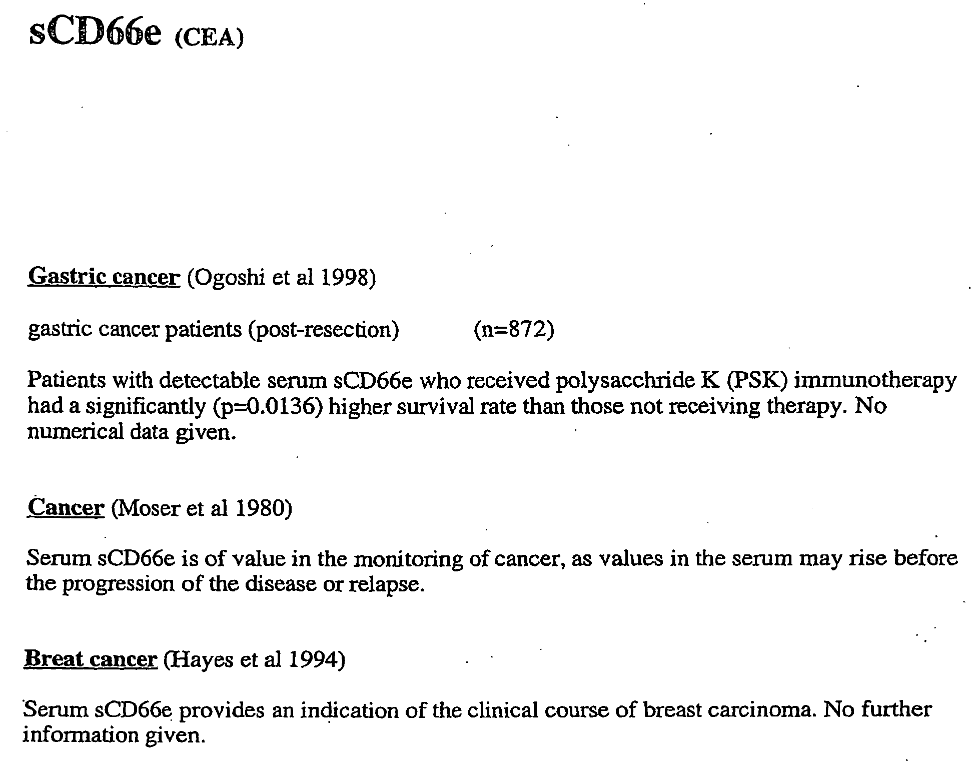

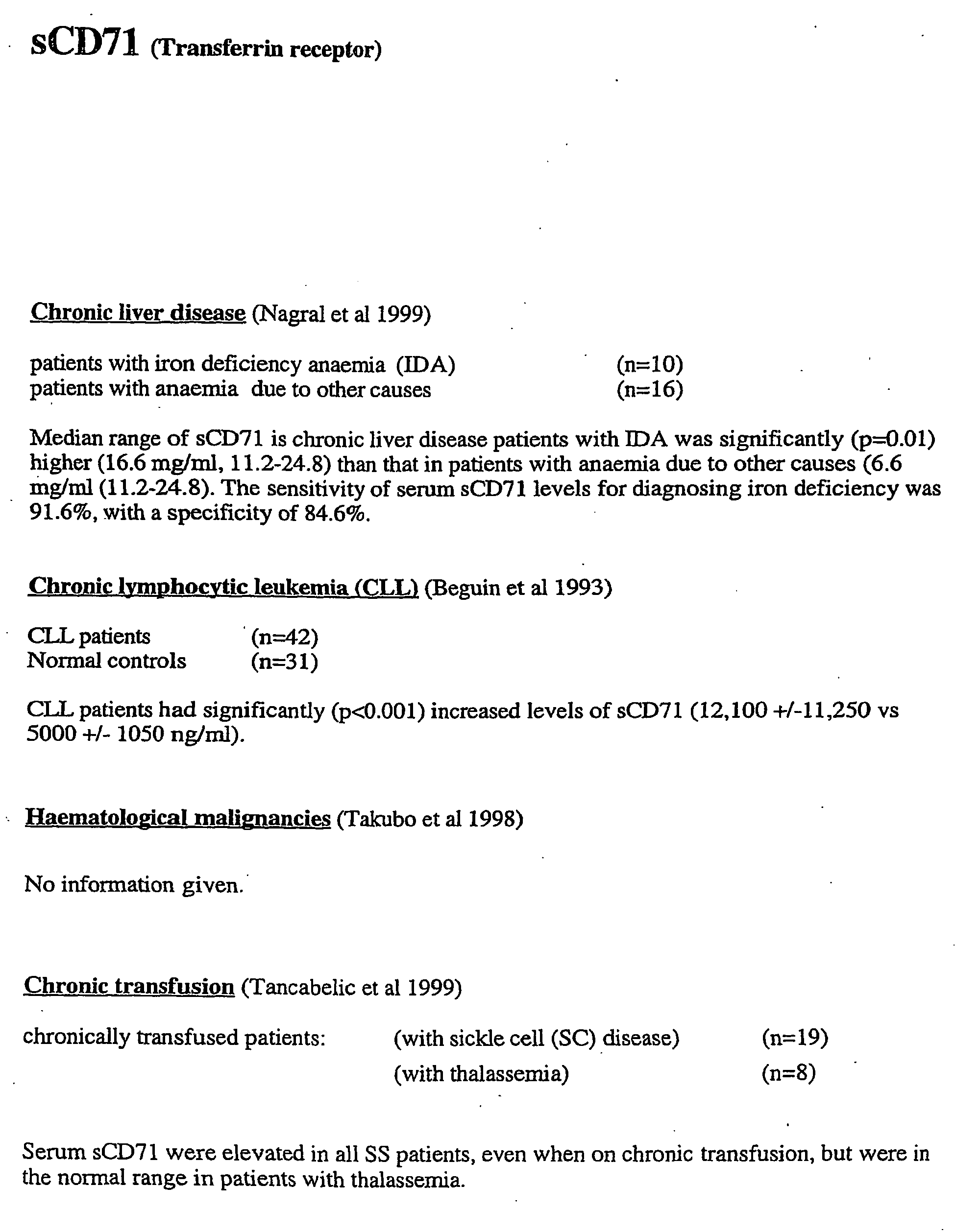

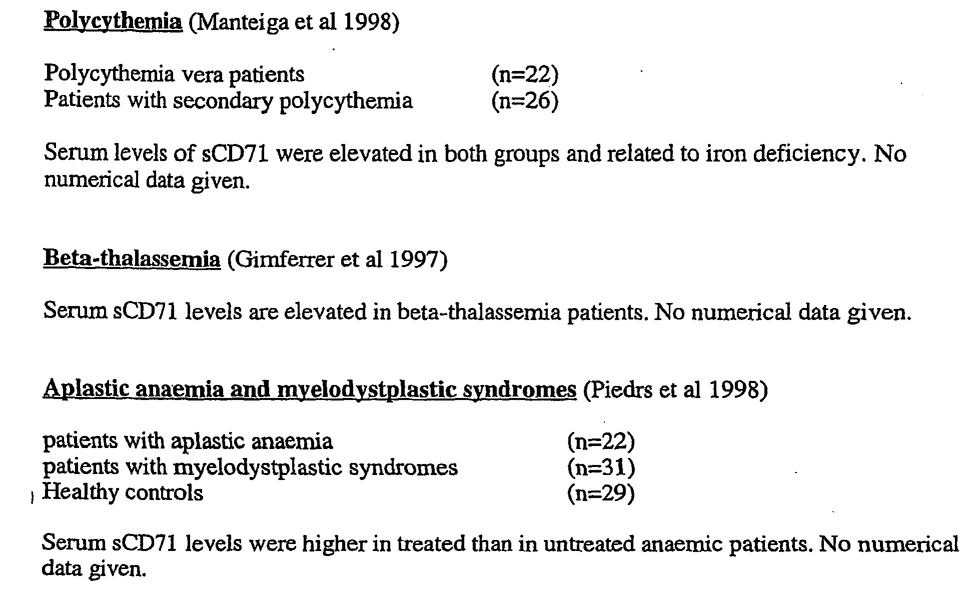

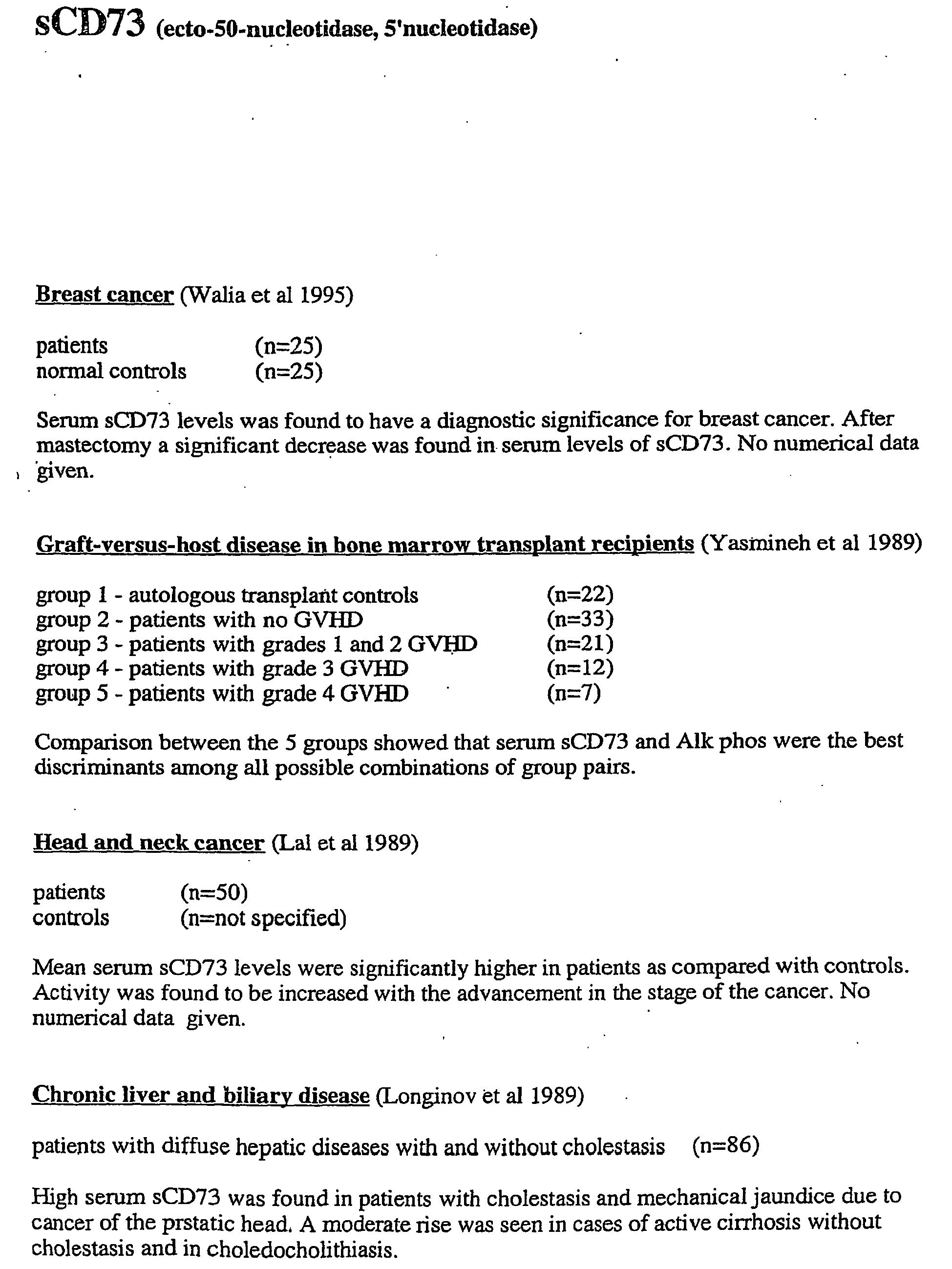

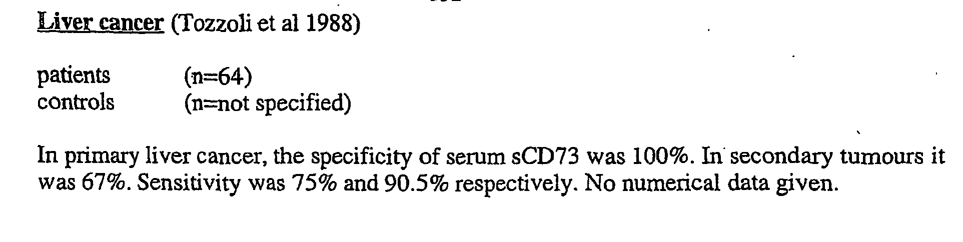

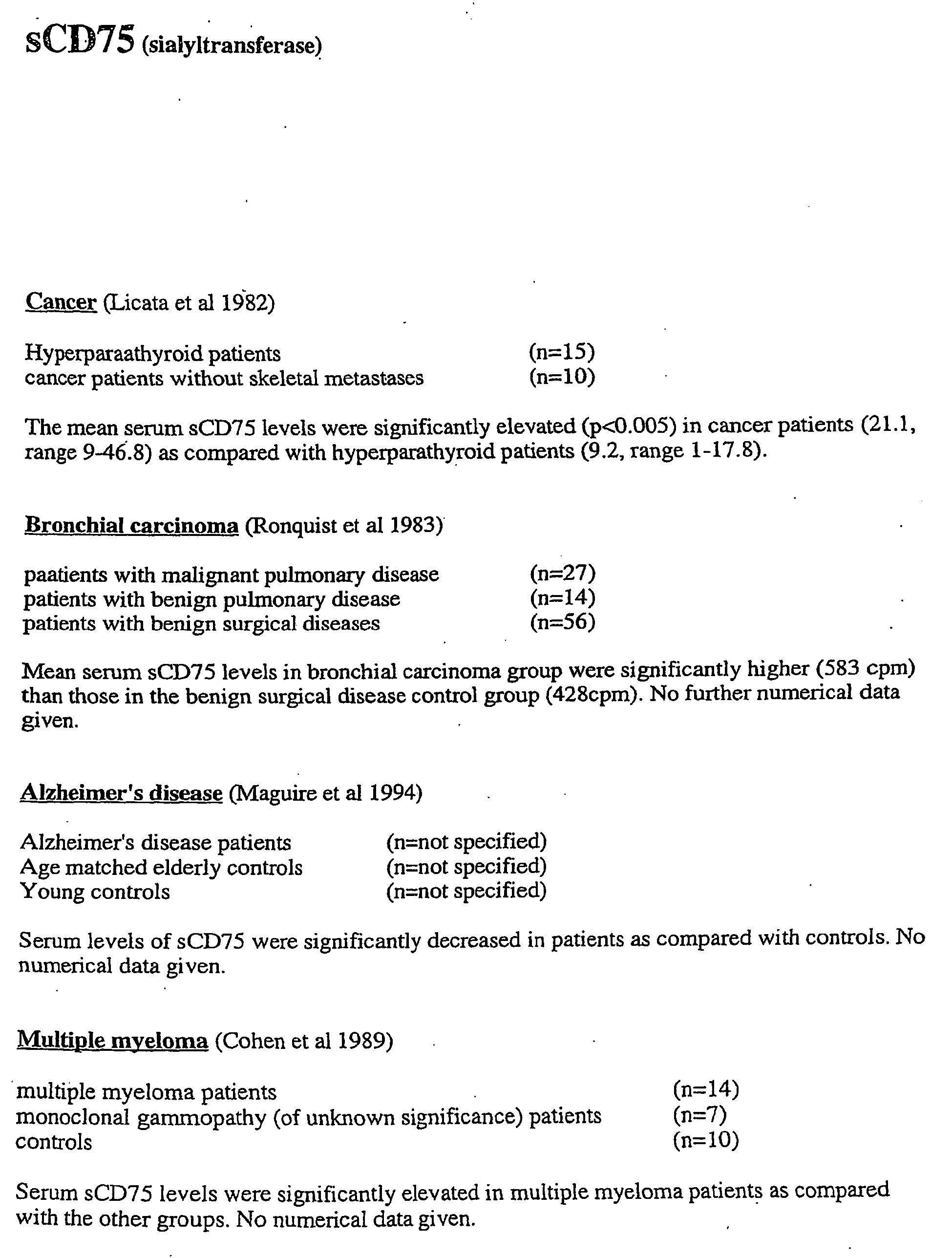

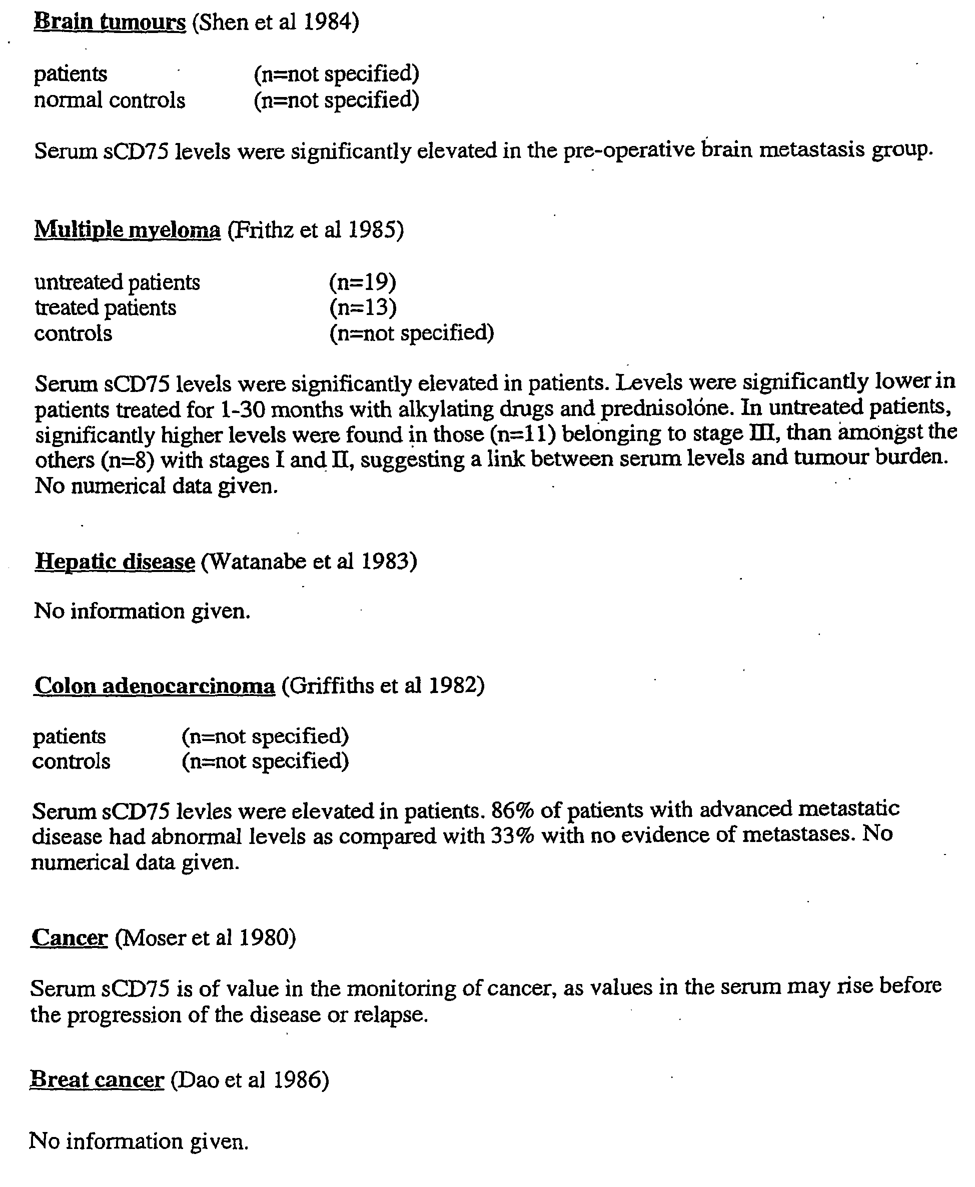

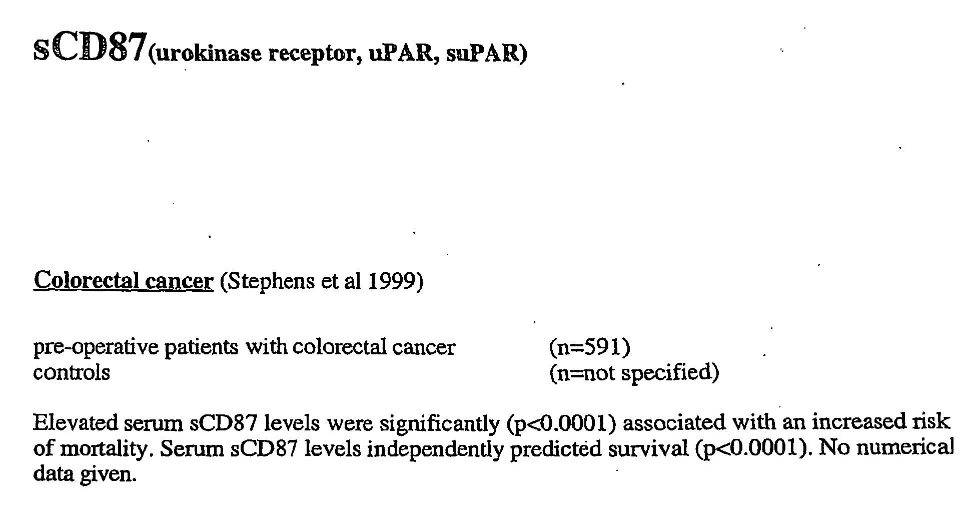

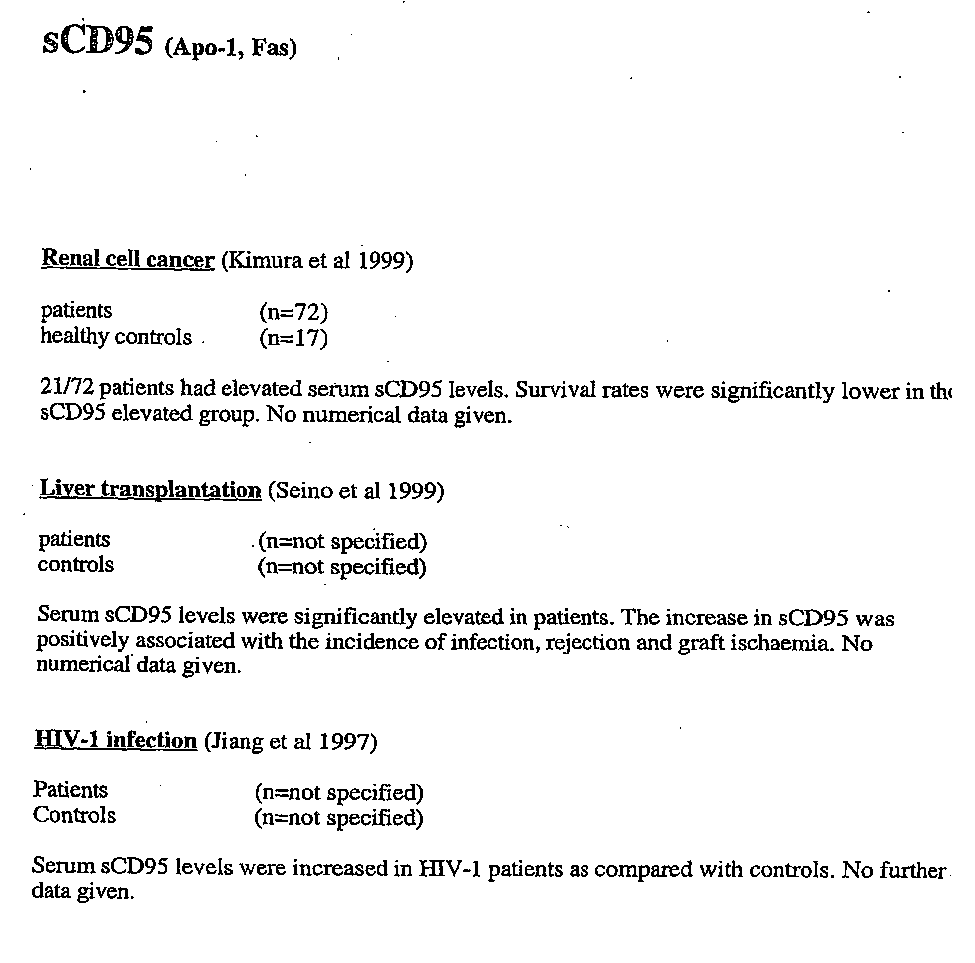

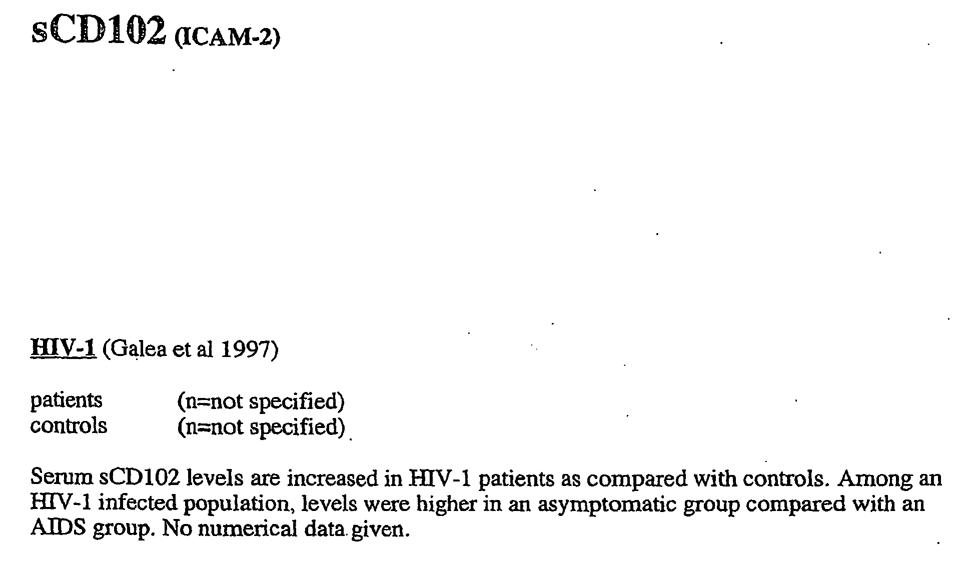

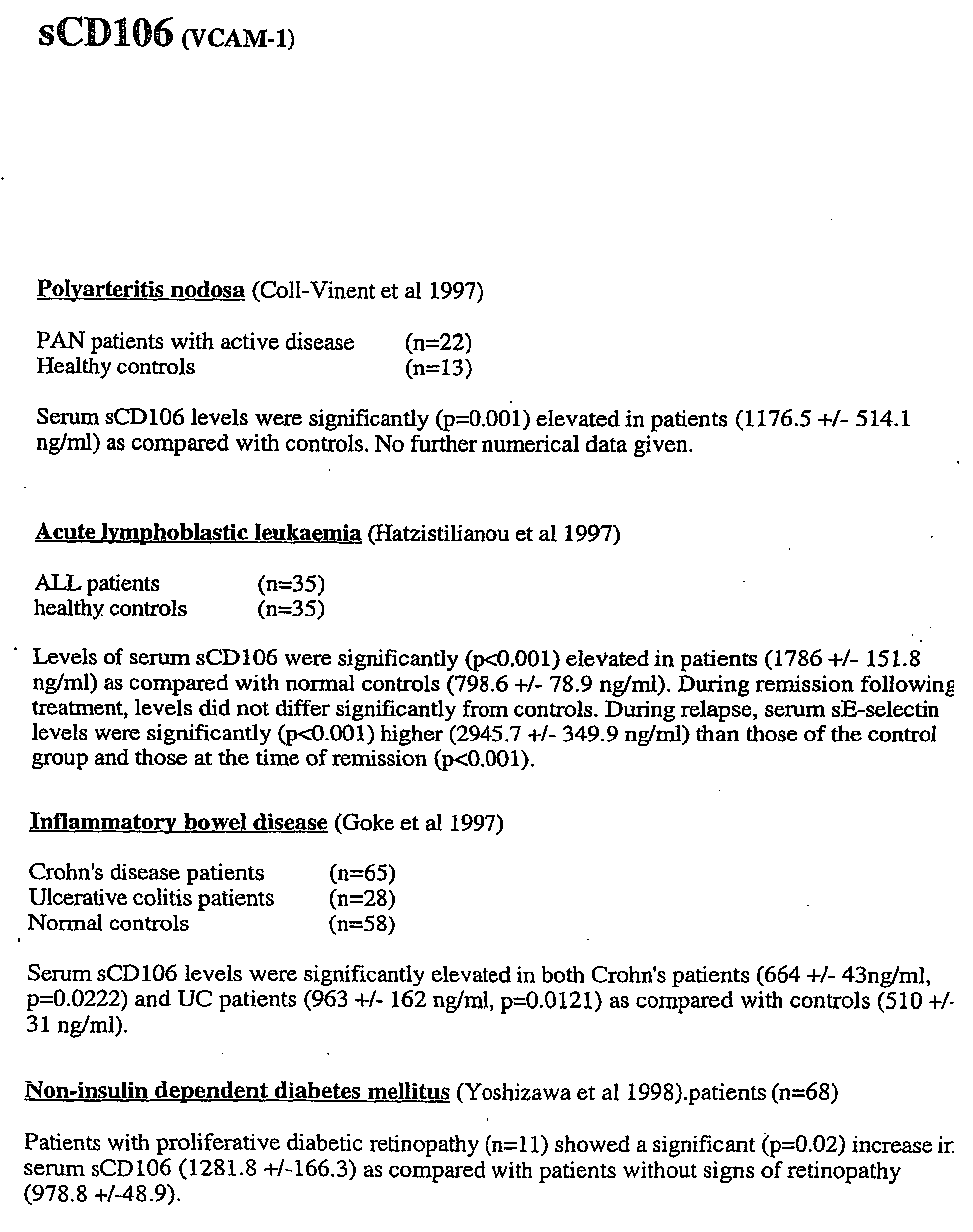

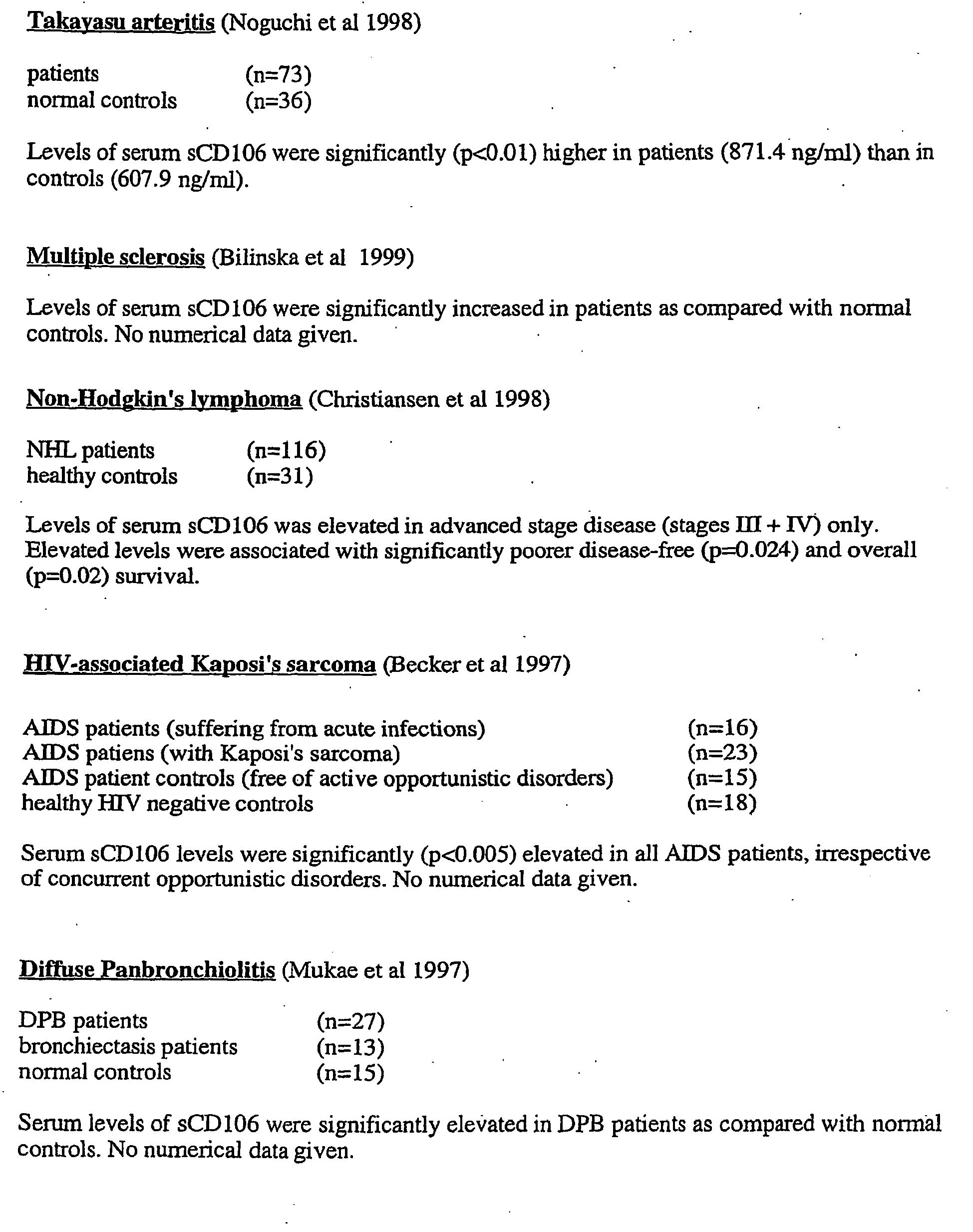

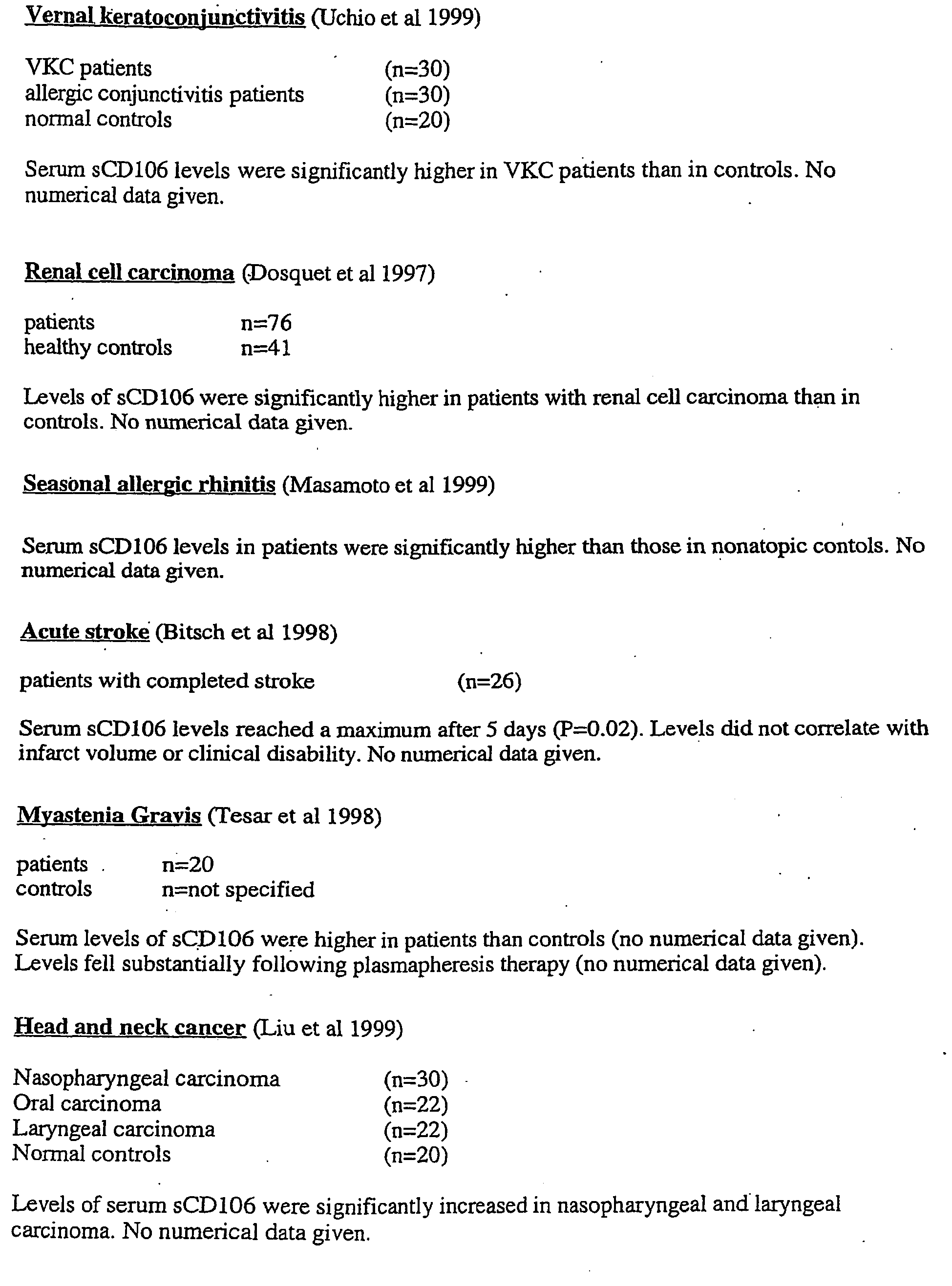

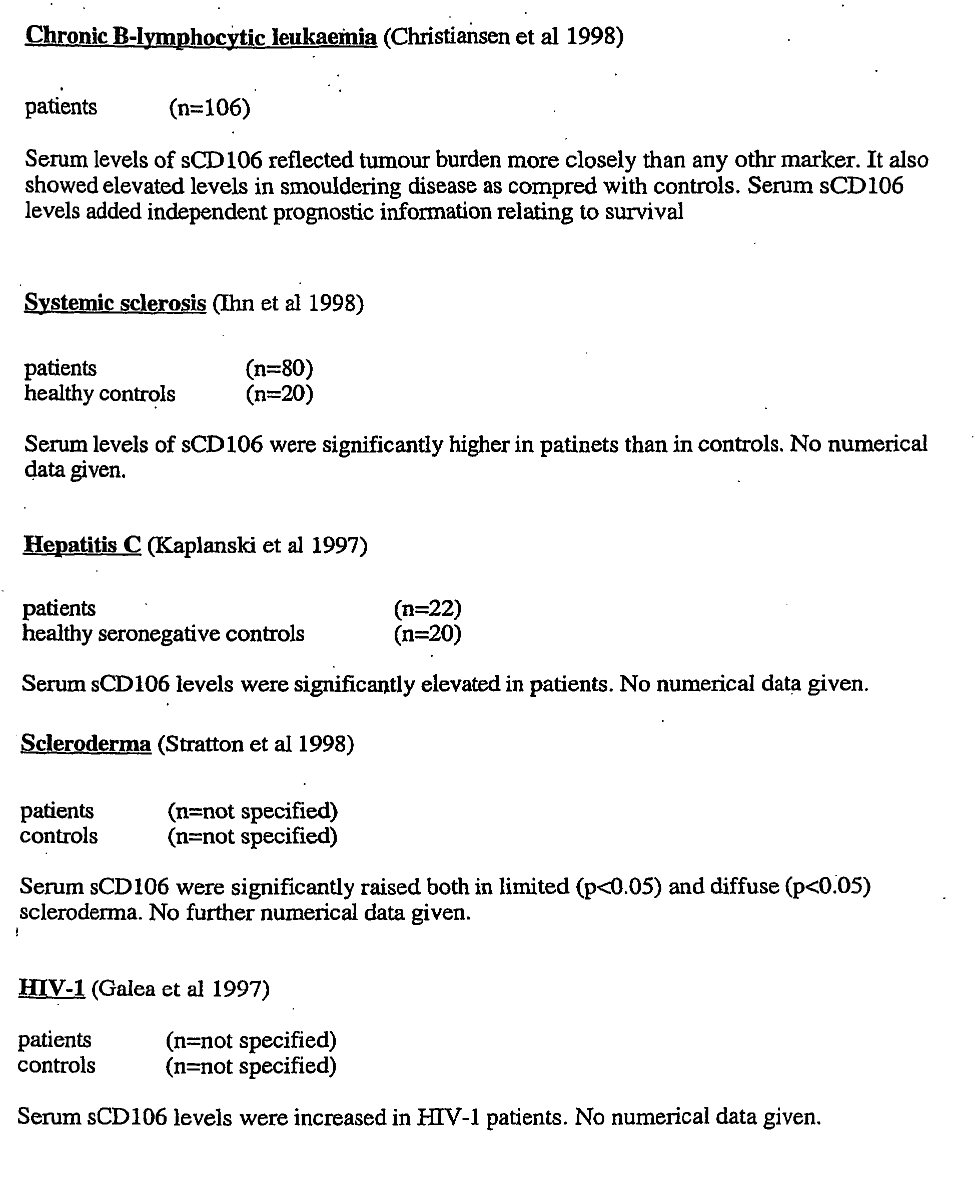

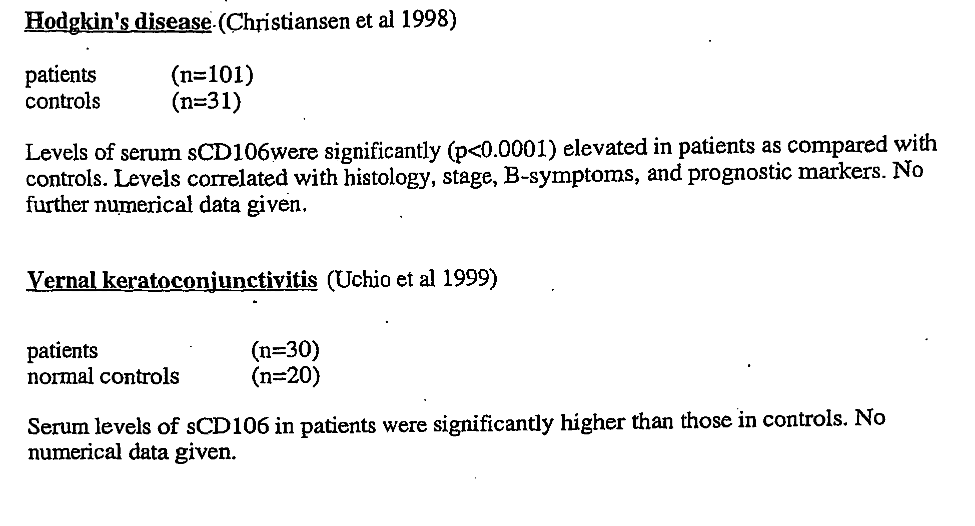

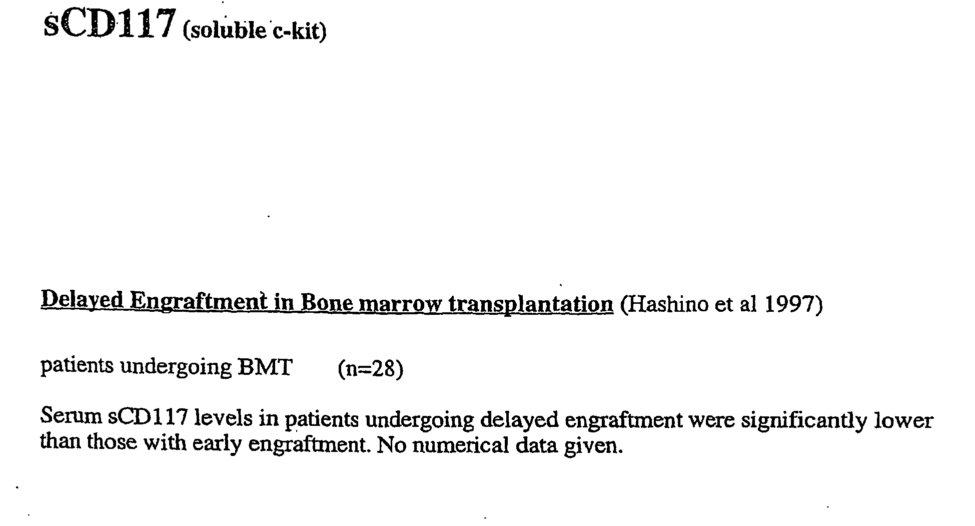

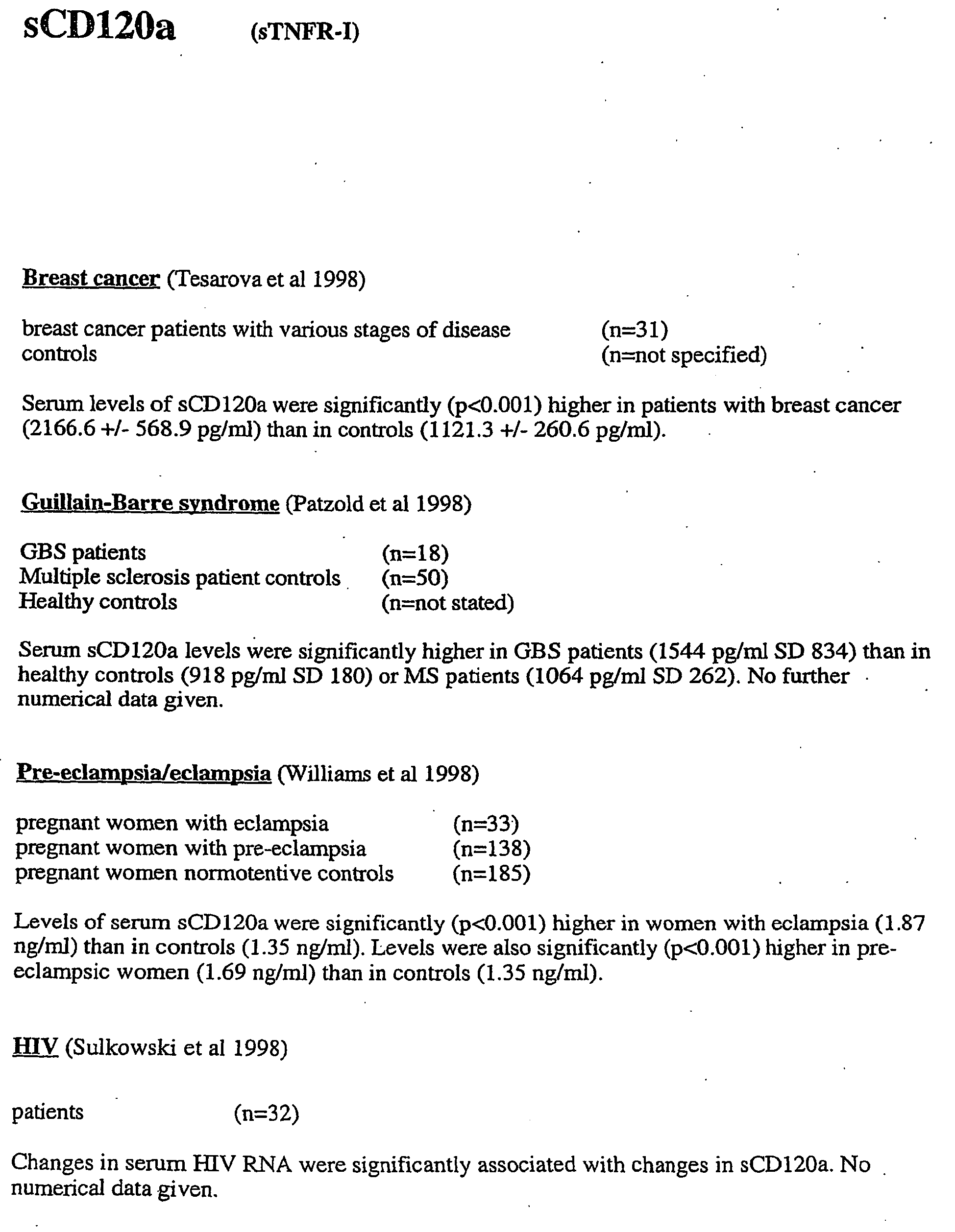

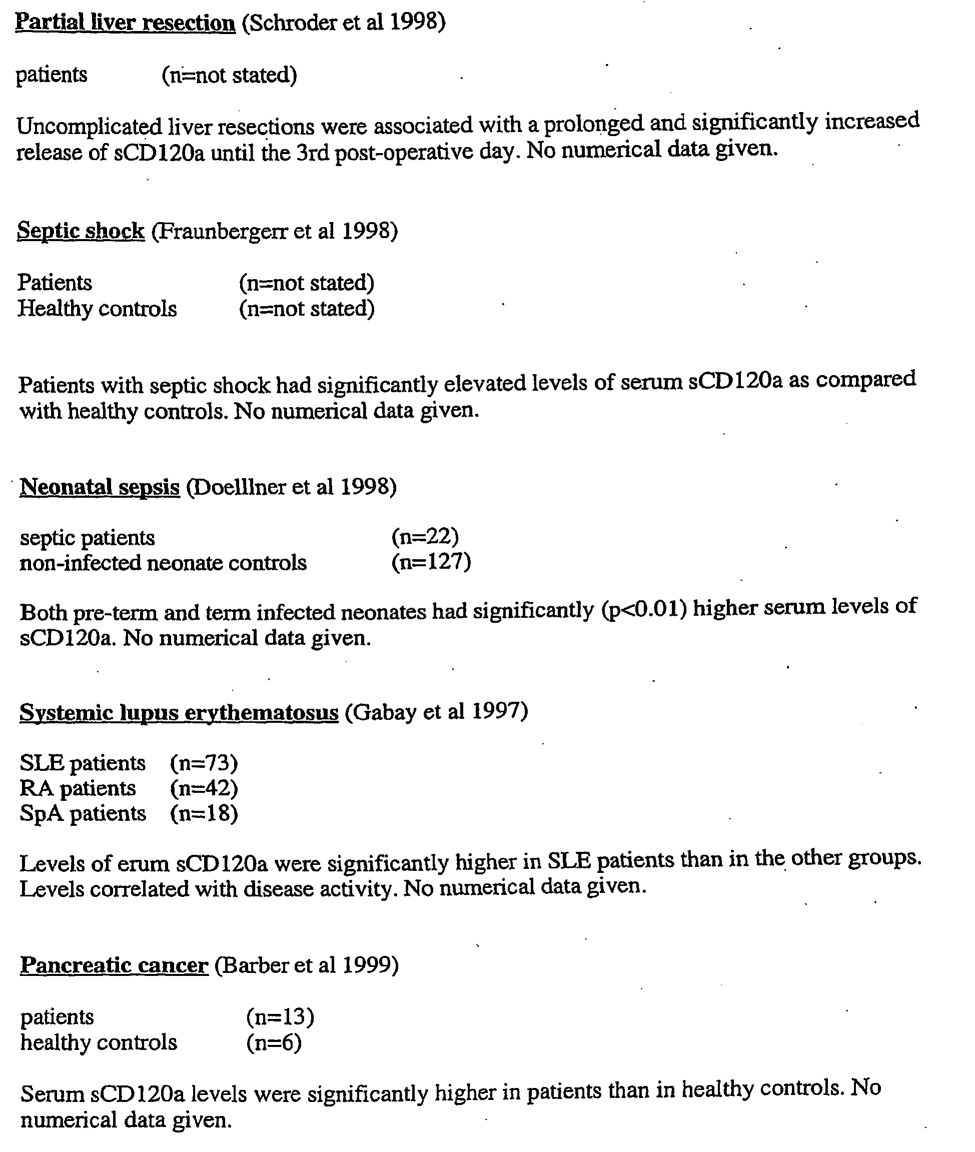

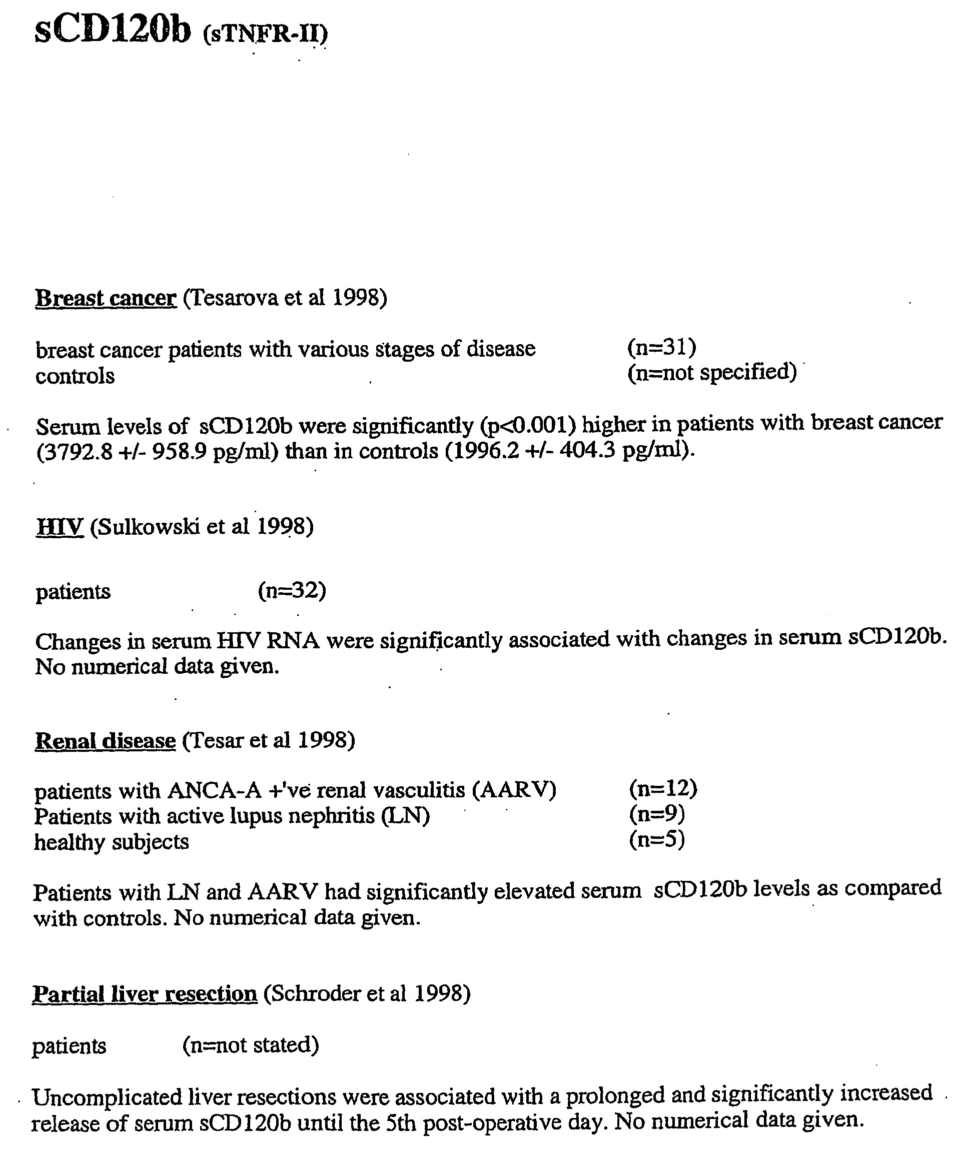

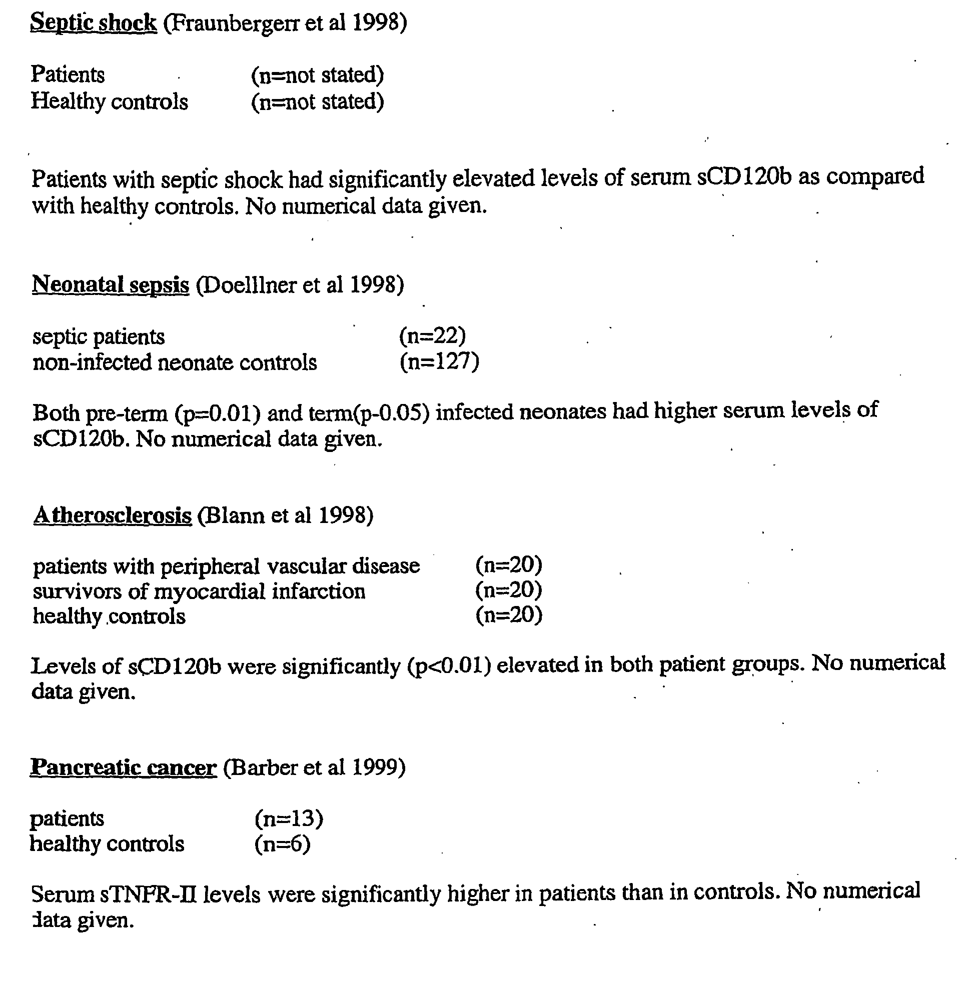

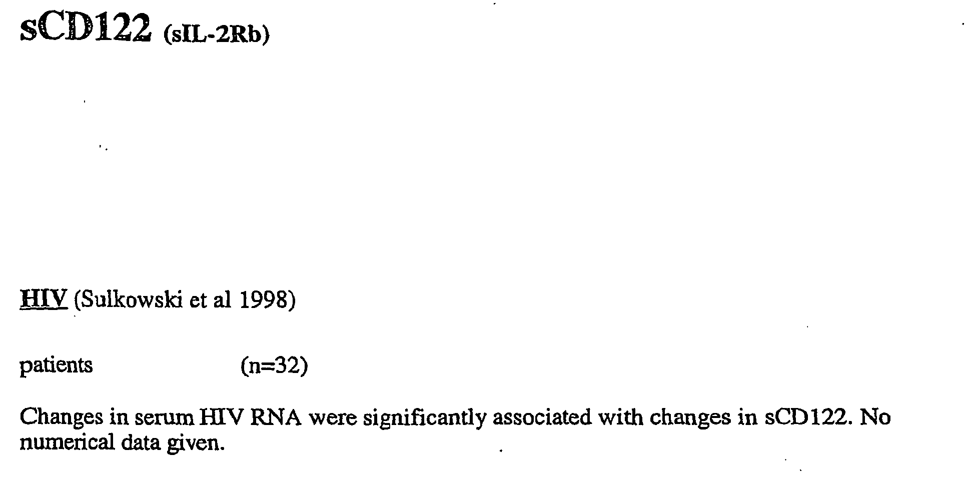

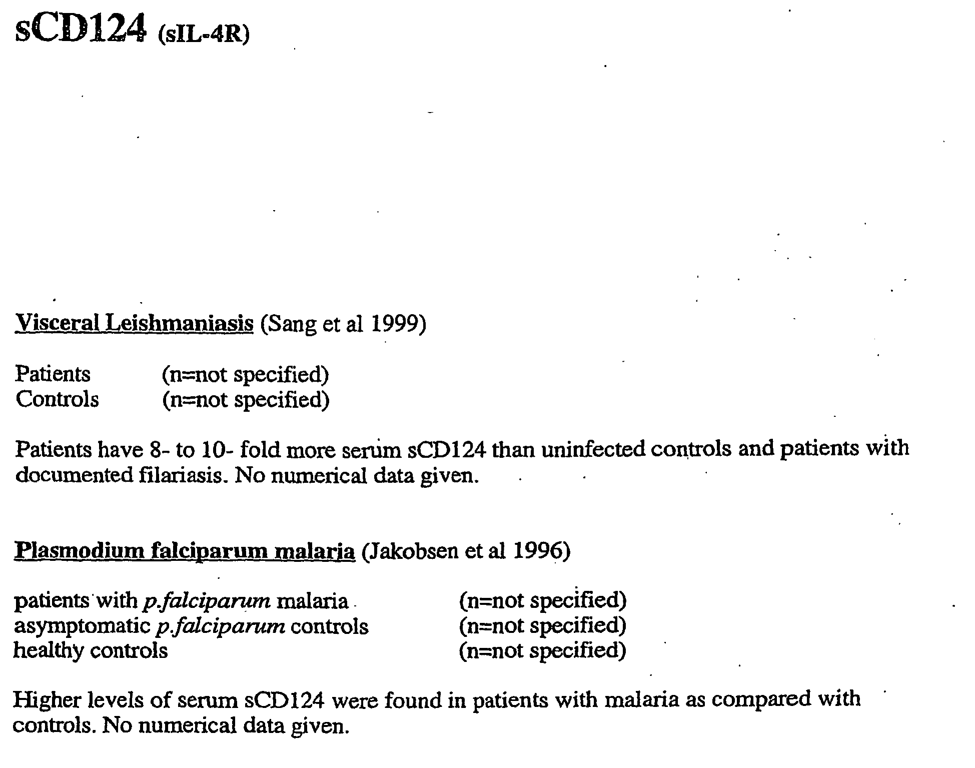

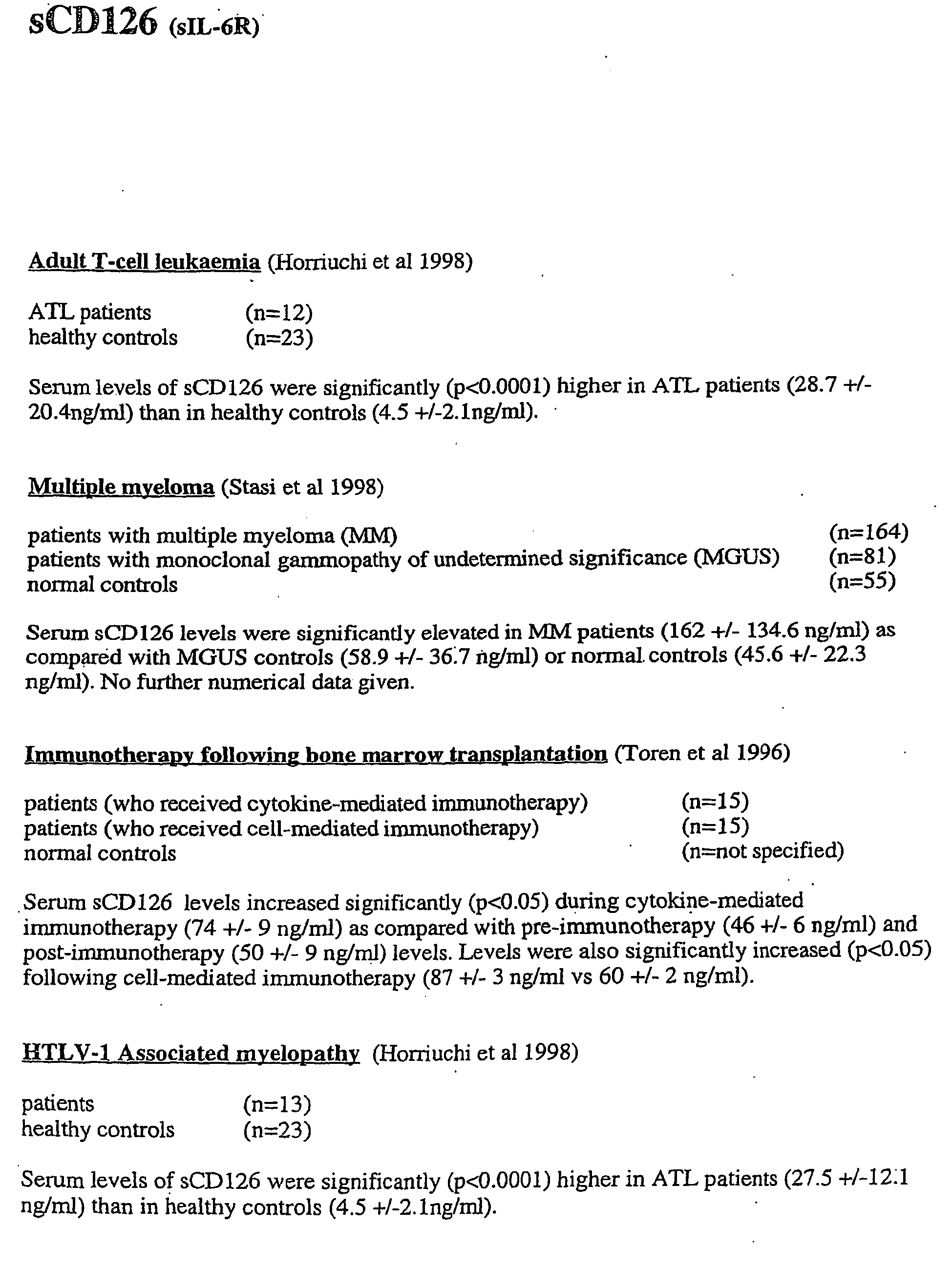

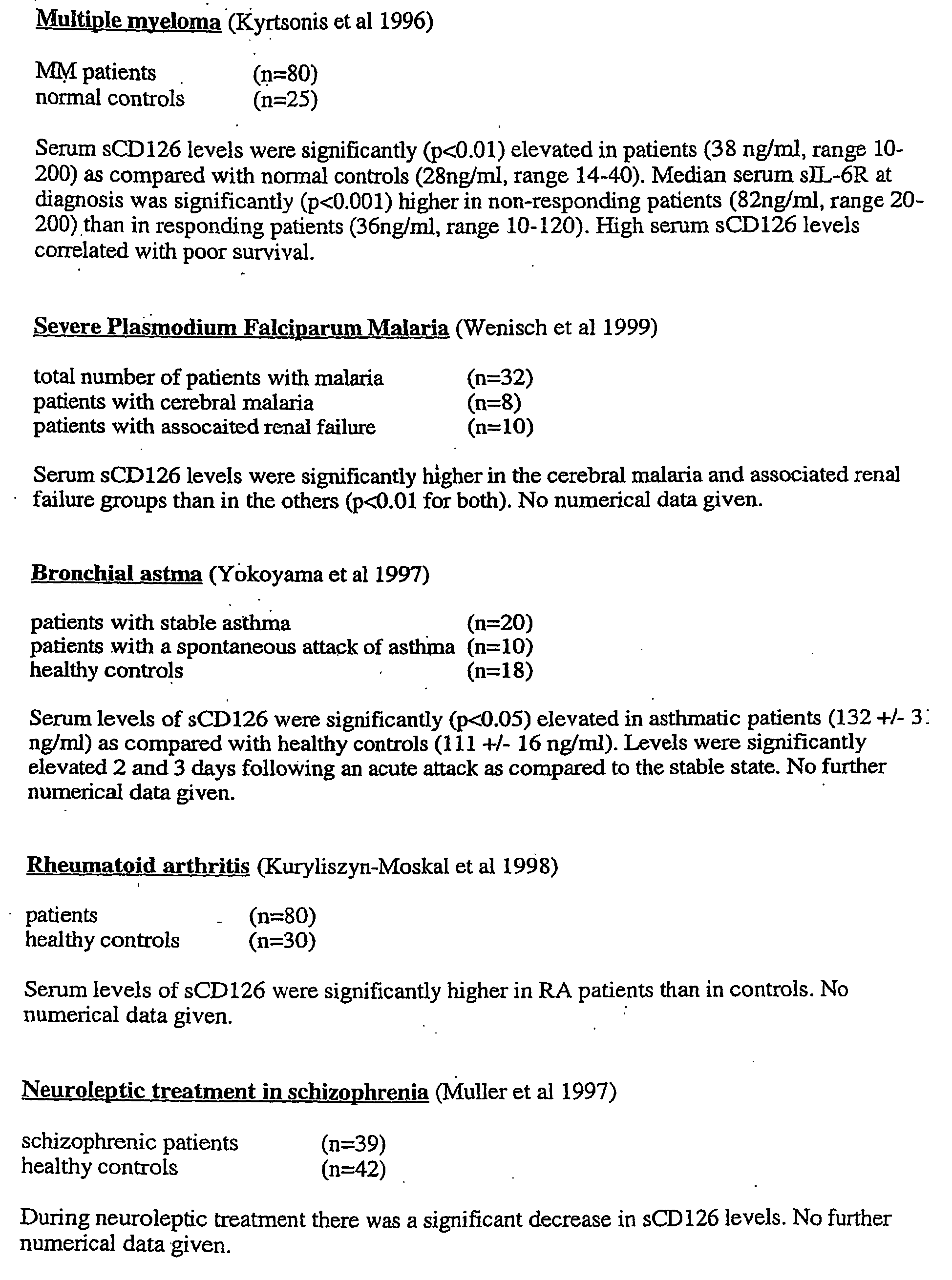

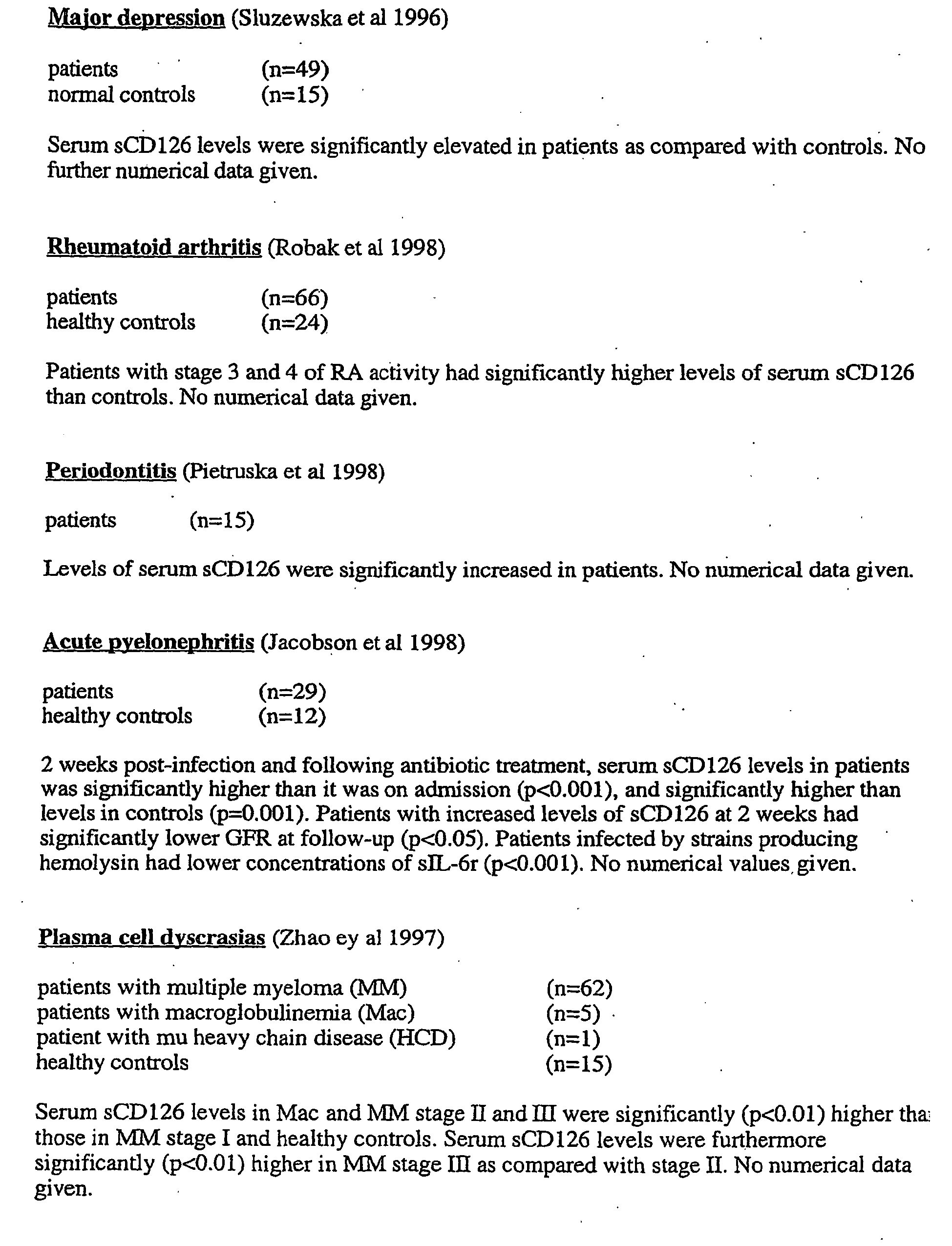

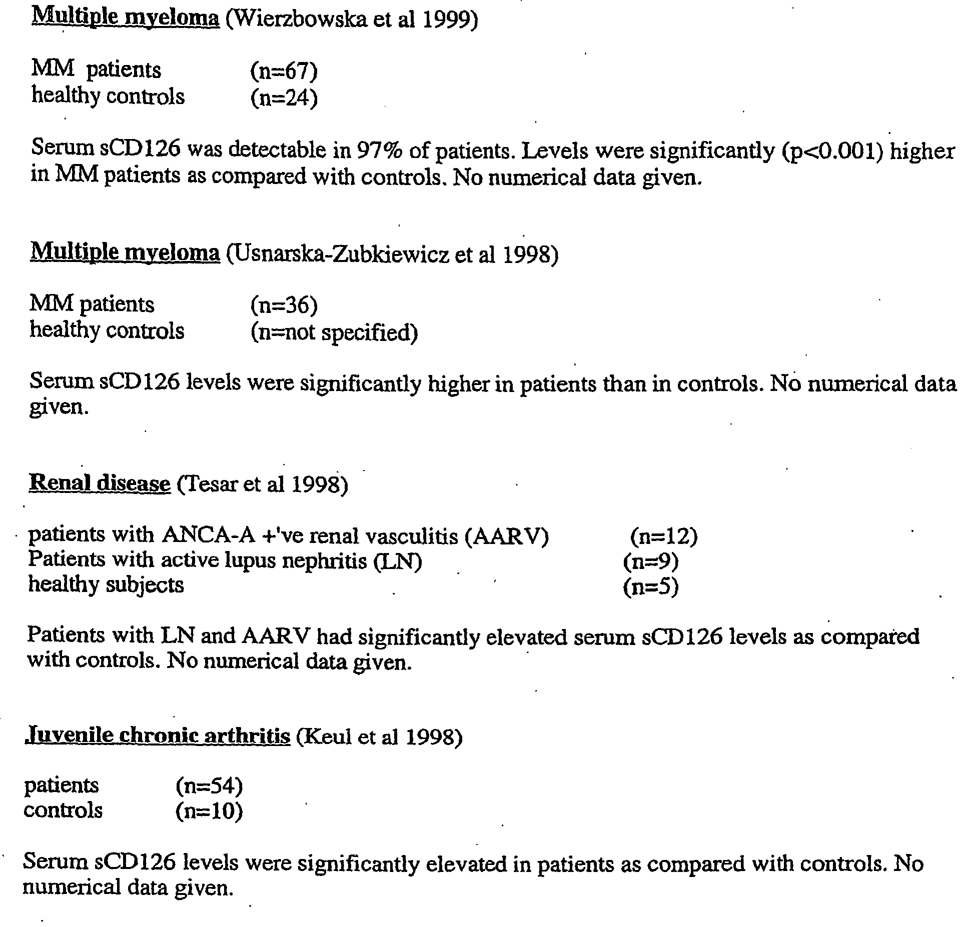

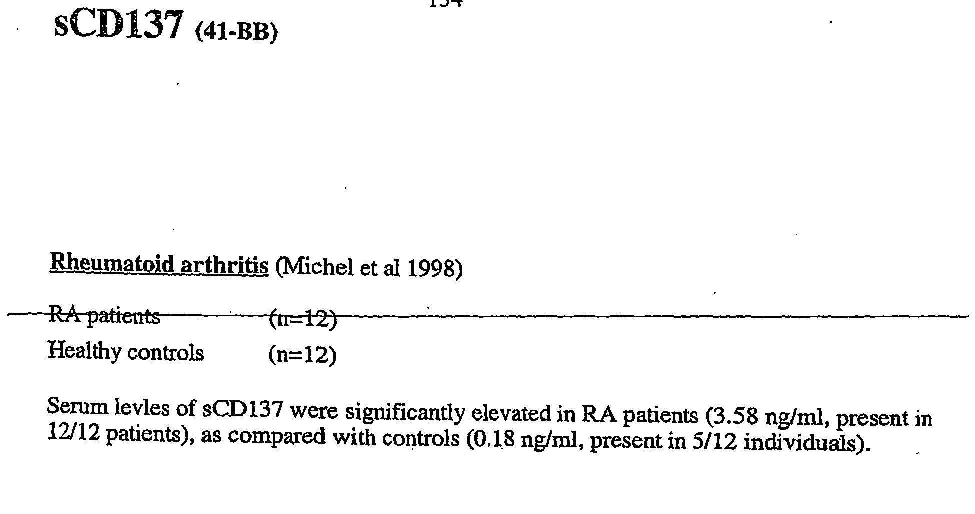

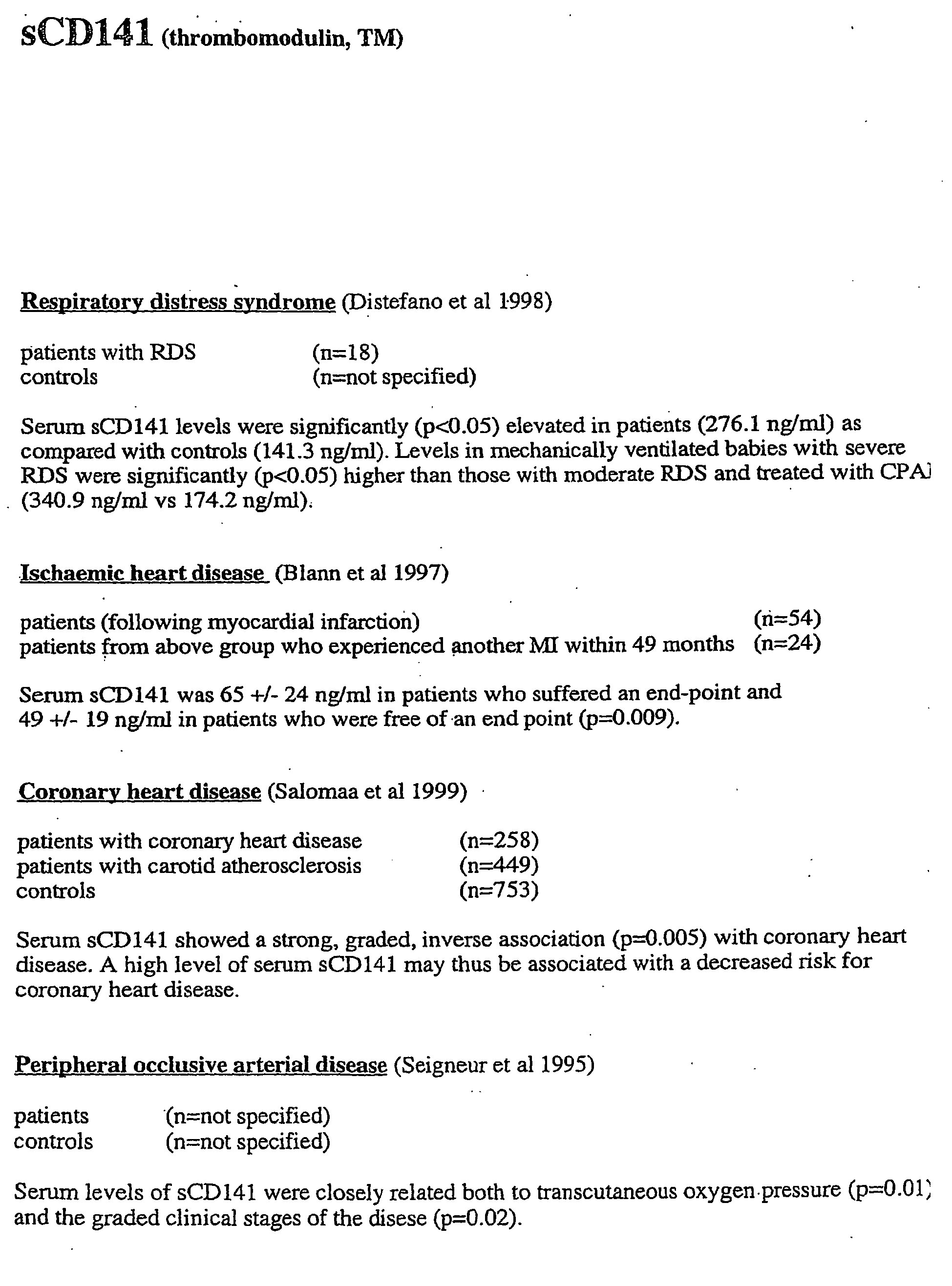

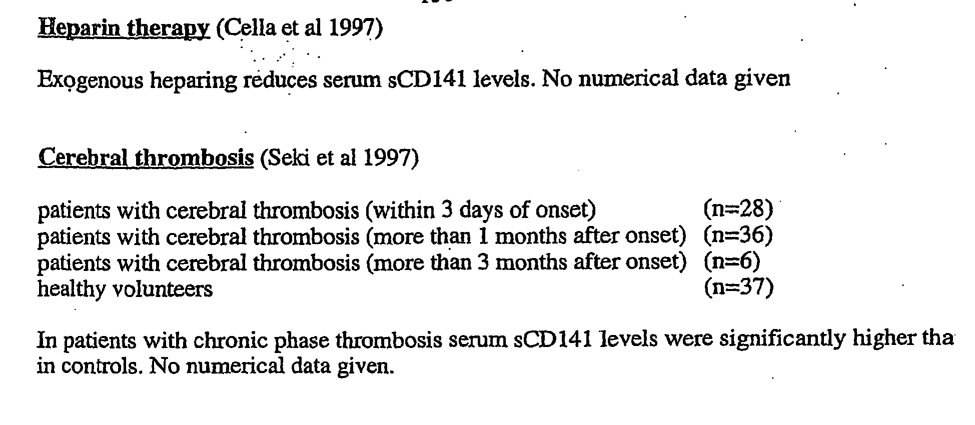

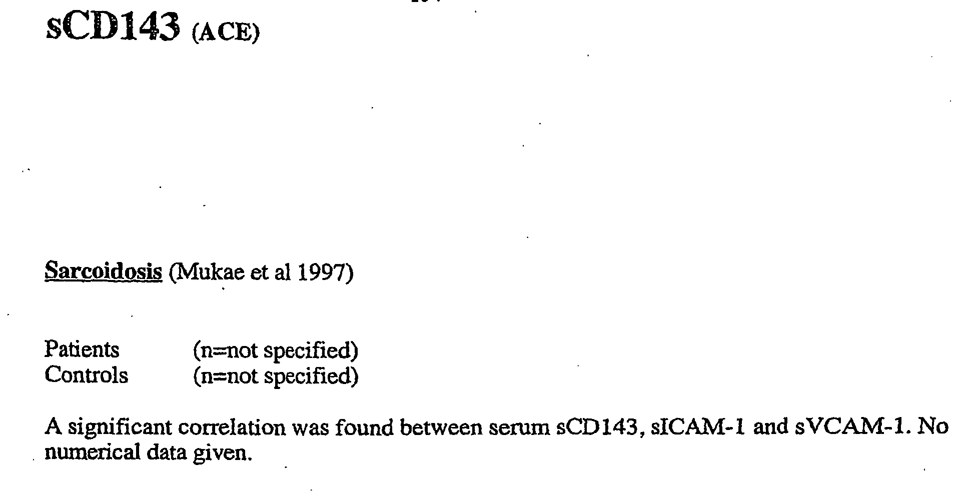

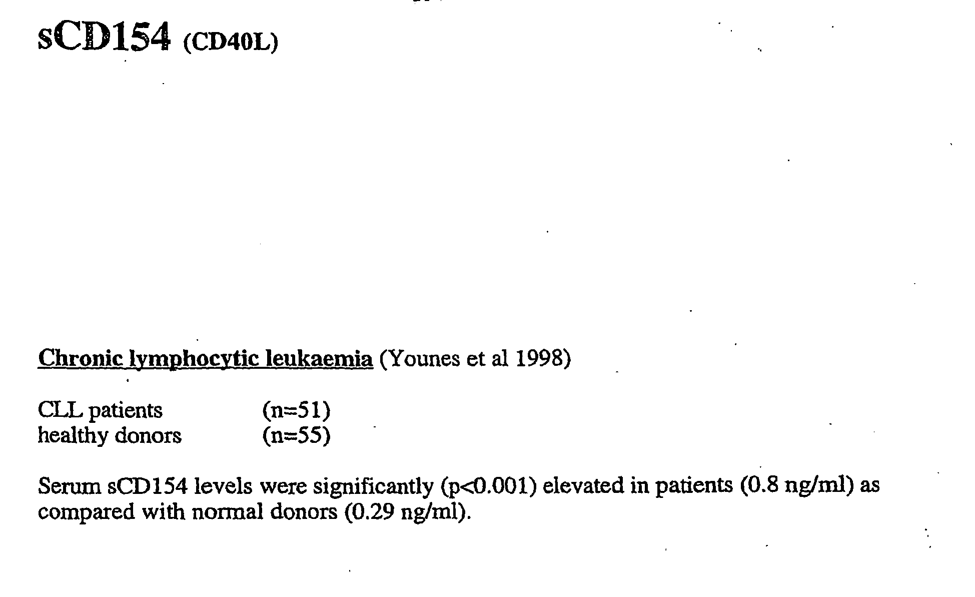

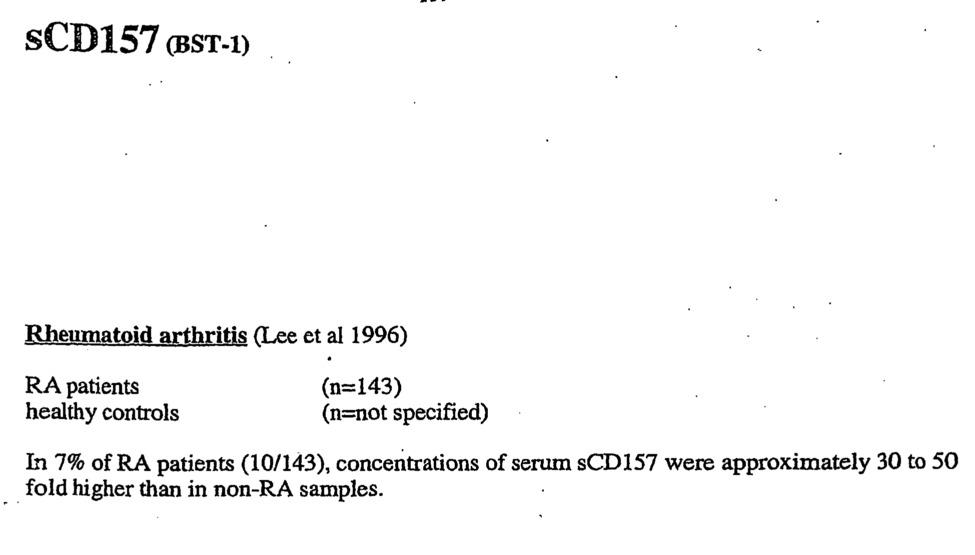

## Claims

1. A shed CD (sCD) fingerprint of one or more disease states.

2. A method of generating a shed CD (sCD) fingerprint of one or more disease state/s comprising the step of measuring the levels in parallel of more than one shed CDs from one or more individuals and collating the data.

3. A sCD fingerprint according to claim 1 or a method according to claim 2 wherein the disease state is any one or more selected form the group consisting of: infectious, neoplastic, autoimmune, metabolic, immunological, degenerative, psychological, psychiatric, iatrogenic, inflammatory, drug or toxin related, vascular, traumatic and endocrine diseases.

4. A sCD fingerprint or a method according any preceding claim wherein the disease state is any one or more selected from the group consisting of the following: infection, Bence Jones Proteinuria, Chronic Myeloid Leukemia, Colorectal cancer, chronic renal failure, Crohn's Disease, Diabetic Nephropathy, Cardiac pathology, Infection, Liver damage, Lymphoma, macrocytic anaemia, Prostate Cancer, Oligoclonal Banding and Pulmonary Embolism/Deep Vein Thrombosis and appendicitis.

5. A sCD fingerprint according to claim 1 or claim 3 or claim 4 or a method according to claim 2, claim 3 or claim 4 wherein the sCDs referred to comprise two or more selected from the group consisting: CD 14, CD25, CD31, CD44, CD50, CD54, CD62E, CD62L, CD86, CD95, CD106, CD116, CD124, CD138, CD141, CD40L, CD8, CD23, CD30, CD40 and their homologues present in other mammalian or non-mammalian species.

6. A method according to any of claims 2 to 5 wherein the sCD levels are measured in samples of one or more body fluids from an individual.

7. A method according to claim 6 wherein the body fluid is serum.

8. A method according to any of claims 2 to 7 wherein sCD levels are measured using one or more methods selected from the group consisting of: immunoassay and flow cytometry.

9. A method according to claim 8 wherein sCD levels are measured using any one or more method selected from the group consisting of the following: multiplexed particle flow cytometry, chip based monoclonal antibody technology, chips comprising engineered antibodies, non protein agents which bind to one or more sCDs.

10. A method for predicting the presence of one or more disease states in an individual comprising the step of comparing one or more sCD fingerprint/s generated from that individual with one or more reference sCD fingerprint/s.

11. A method for detecting the presence of one or more disease states in an individual comprising the step of comparing one or more sCD fingerprint/s generated from that individual with one or more reference sCD fingerprint/s.

12. A method for detecting the extent of one or more disease states in an individual comprising the step of comparing one or more sCD fingerprint/s generated from that individual with one or more reference sCD fingerprint/s.

13. A method for assessing the progression of a disease state in an individual comprising the step of comparing the sCD fingerprint of an individual at two or more periods during the life-span of the disease.

14. A method for assessing the effect of one or more agent/s on one or more disease states in an individual comprising the step of comparing a sCD fingerprint of an individual at two or more different time periods.

15. The use of a sCD fingerprint to assess the effect of one or more agent/s on an individual.

16. A method for sub-categorising a sCD fingerprint profile comprising the steps of identifying within one disease category one or more group/s of sCDs wherein each group of sCDs exhibits common characteristics distinguishing it from any other group within that disease category.

17. A sCD database comprising pathological and/or normal sCD fingerprint patterns.

18. A method for treating one or more diseases comprising the step of inhibiting the production of one or more sCDs within an individual.

19. A method according to claim 18 wherein the one or more sCDs are any one or more of those selected from the group consisting of the following: CD14, CD25, CD31, CD44, CD50, CD54, CD62E, CD62L, CD86, CD95, CD106, CD116, CD124, CD138, CD141, CD40L, CD8, CD23, CD30, CD40.

20. A method according to claim 19 wherein at least one sCD is sCD 1.

21. A method according to claim 18 or claim 19 wherein the production of one or more sCDs is inhibited by the use of one or more CD specific alternative splicing inhibitors.

22. A method according to any of claims 18 to 21 wherein the disease is any one or more of those selected from the group consisting of the following: tumourigenesis, infection, vascular disease, endocrine disease.

23. The use of an inhibitor of the production of one or more sCDs in the preparation of a medicament for the treatment of disease.

24. The use according to claim 23, wherein that use exhibits any one or more of the features of claims 18 to 22.
